# EUROPEAN PATENT SPECIFICATION

(11) **EP 4 288 435 B1**
(45) Date of publication and mention of the grant of the patent: **26.08.2026**
(21) Application number: 22705655.3
(22) Date of filing: 03.02.2022
(51) Int. Cl.: C07D 471/04, C07D 471/14, C07D 491/048, C07D 491/052, C07D 491/147, C07D 495/04, C07D 498/04, C07D 519/00, C07B 59/00, A61K 31/55, A61K 31/4375, A61K 31/519, A61K 31/4355, A61K 31/437, A61K 31/4365, A61P 35/00

(54) **TRICYCLIC-AMIDO-BICYCLIC PRMT5 INHIBITORS**
TRICYCLISCHE-AMIDO-BICYCLISCHE PRMT5-INHIBITOREN
INHIBITEURS DE PRMT5 TRICYCLIQUES-AMIDO-BICYCLIQUES

(30) Priority: 04.02.2021 US 202163145634 P
(43) Date of publication of application: 13.12.2023
(73) Proprietor: Amgen Inc., Thousand Oaks, CA 91320-1799 (US)
(72) Inventor: AMEGADZIE, Albert, Thousand Oaks, California 91320-1799 (US); BEYLKIN, Diane Jennifer, Thousand Oaks, California 91320-1799 (US); BOOKER, Shon, Thousand Oaks, California 91320-1799 (US); BOURBEAU, Matthew Paul, Thousand Oaks, California 91320-1799 (US); BUTLER, John R., Thousand Oaks, California 91320-1799 (US); GREENMAN, Kevin Lloyd, Thousand Oaks, California 91320-1799 (US); KOHN, Todd J., Thousand Oaks, California 91320-1799 (US); LI, Kexue, Thousand Oaks, California 91320-1799 (US); LIU, Qingyian, Thousand Oaks, California 91320-1799 (US); MINATTI, Ana Elena, Thousand Oaks, California 91320-1799 (US); NAVARATNE, Primali Vasundera, Thousand Oaks, California 91320-1799 (US); PETTUS, Liping H., Thousand Oaks, California 91320-1799 (US); RAHIMOFF, Rene, Thousand Oaks, California 91320-1799 (US); WANG, Hui-Ling, Thousand Oaks, California 91320-1799 (US); WEIRES, Nicholas Anthony, Thousand Oaks, California 91320-1799 (US)
(74) Representative: Grünecker Patent- und Rechtsanwälte PartG mbB
(86) International application number: PCT/US2022/015076
(87) International publication number: WO 2022/169948

(56) References cited:
- WO-A1-2019/002074
- WO-A1-2020/205660
- WO-A1-2020/206289

## Description

### BACKGROUND OF THE INVENTION

Epigenetic regulation of gene expression is an important biological determinant of protein production and cellular differentiation and plays a significant pathogenic role in a number of human diseases.

Epigenetic regulation involves heritable modification of genetic material without changing its nucleotide sequence. Typically, epigenetic regulation is mediated by selective and reversible modification (*e.g.,* methylation) of DNA and proteins (*e.g.,* histones) that control the conformational transition between transcriptionally active and inactive states of chromatin. These covalent modifications can be controlled by enzymes such as methyltransferases (*e.g*., PRMT5), many of which are associated with specific genetic alterations that can cause human disease. PRMT5 plays a role in diseases such as proliferative disorders, metabolic disorders, and blood disorders.

The homozygous deletion of tumor suppressor genes is a key driver of cancer, frequently resulting in the collateral loss of passenger genes located in close genomic proximity to the tumor suppressor. Deletion of these passenger genes can create therapeutically tractable vulnerabilities that are specific to tumor cells. Homozygous deletion of the chromosome 9p21 locus, which harbors the well-known tumor suppressor CDKN2A (cyclin dependent kinase inhibitor 2A), occurs in 15% of all tumors and frequently includes the passenger gene MTAP (methylthioadenosine phosphorylase), a key enzyme in the methionine and adenine salvage pathways. Deletion of MTAP results in accumulation of its substrate, methylthioadenosine (MTA). MTA shares close structural similarity to S-adenosylmethionine (SAM), the substrate methyl donor for the type II methyltransferase PRMT5. Elevated MTA levels, driven by loss of MTAP, selectively compete with SAM for binding to PRMT5, placing the methyltransferase in a hypomorphic state, vulnerable to further PRMT5 inhibition. Multiple genome scale shRNA drop out screens performed in large tumor cell line panels have identified a strong correlation between MTAP loss and cell line dependency on PRMT5, further highlighting the strength of this metabolic vulnerability. However, PRMT5 is a known cell essential gene and conditional PRMT5 knockout and siRNA knockdown studies suggest that significant liabilities could be associated with inhibiting PRMT5 in normal tissues (*e.g*., pan-cytopenia, infertility, skeletal muscle loss, cardiac hypertrophy). Therefore, novel strategies are required to exploit this metabolic vulnerability and preferentially target PRMT5 in MTAP null tumors while sparing PRMT5 in normal tissues (MTAP WT). Targeting PRMT5 with an MTA-cooperative small molecule inhibitor could preferentially target the MTA bound state of PRMT5, enriched in MTAP null tumor cells, while providing an improved therapeutic index over normal cells where MTAP is intact and MTA levels are low.

WO 2020/205660 A1, WO 2020/206289 A1 and WO 2019/002074 A1 refer to small molecules that are PRMT5 inhibitors.

### SUMMARY OF THE INVENTION

The present invention is as defined in the appended claims, all other subject matter (such as e.g. compounds 220 to 224 and 279 to 282) is present only for reference purposes.

In one aspect, the invention provides a compound of Formula **I** 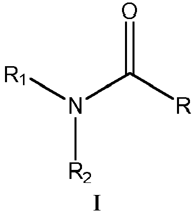
a tautomer thereof, a stereoisomer thereof, or a pharmaceutically acceptable salt of any of the foregoing; wherein R is a tricycle independently selected from the formula **IA:** 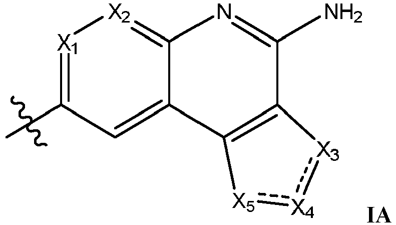 wherein 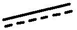 is a single or double bond;
X¹ and X² are in each instance independently selected from optionally substituted N and C, wherein substituents are independently selected from C₁₋₃ alkyl;
wherein both X¹ and X² cannot be N at the same time;
wherein if X¹ is C, it can be optionally substituted with halo, halo C₁₋₃ alkyl or -CN;

X³, X⁴ and X⁵ are at each instance independently selected from optionally substituted C, O and N, wherein the substituents are independently selected from C₁₋₃ alkyl, and C₁₋₃ alkyl(OH), wherein alkyl can be optionally substituted with halo;
wherein R¹ is a bicycle independently selected from the formulae **IB, IC** and **ID,** optionally substituted with R⁴: 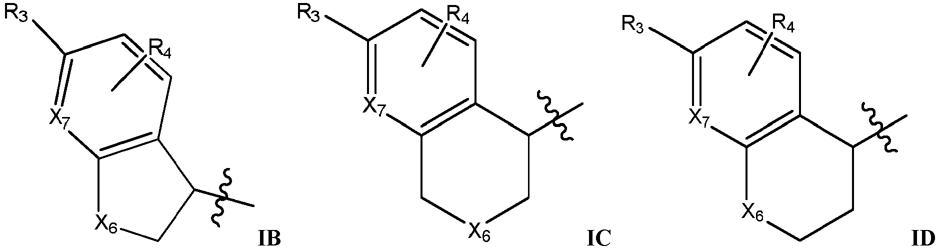
wherein X⁶ is in each instance independently selected from O and C;
wherein X⁷ is in each instance independently selected from N and C;
wherein R² is in each instance independently selected from an optionally substituted C₁₋₆ alkyl or optionally substituted C₁₋₆ cycloalkyl wherein the substituents are selected from -CN or C₁₋₆ cycloalkyl;
wherein R³ is in each instance independently selected from C₁₋₆ alkyl, C₁₋₆ cycloalkyl, halo, C₁₋₆ haloalkyl, -S(=O)₂C₁₋₆ alkyl, -S(O)(NH) C₁₋₆ alkyl, -S(O)(N-C₁₋₃ alkyl)C₁₋₆ alkyl, -CN, -OC₁₋₆ alkyl, -OC₁₋₆ haloalkyl, -N(=O)-OC₁₋₆ alkyl, -C(O)C₁₋₆ alkyl, -C(O)C₁₋₆ haloalkyl, 3,6-dihydro-2H-pyranyl and pentafluorosulfanyl;
wherein R⁴ is in each instance independently selected from C₁₋₆ alkyl, halo, and C₁₋₆ haloalkyl.

In one aspect, the invention provides the compounds, the tautomer thereof, the stereoisomer thereof, or the pharmaceutically acceptable salt of any of the foregoing, wherein R is

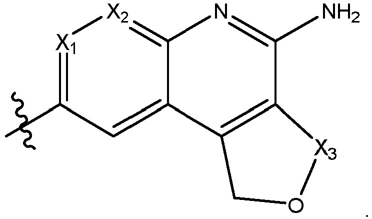

In a further aspect, X¹ can be C, optionally substituted with halo.

In another aspect, X¹ can be N.

In another aspect, X³ can be optionally substituted C.

The invention provides the compounds, the tautomer thereof, the stereoisomer thereof, or the pharmaceutically acceptable salt of any of the foregoing, wherein R can be

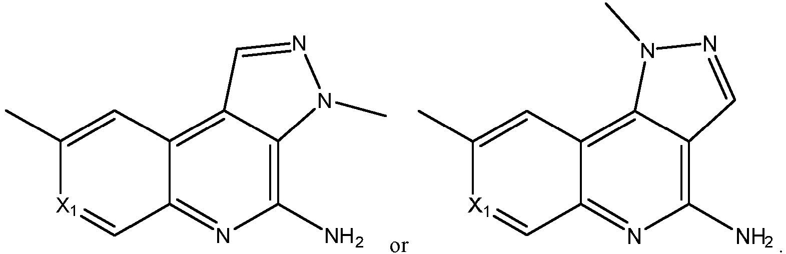

In one aspect, X¹ can be C substituted with halo.

The invention provides that R can be

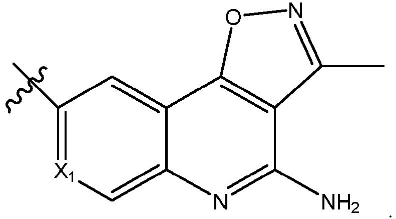

In one aspect, X1 can be C, optionally substituted with halo.

The invention provides compounds, the tautomer thereof, the stereoisomer thereof, or the pharmaceutically acceptable salt of any of the foregoing, wherein R¹ can be **IB.** In another aspect, R¹ can be **IC.** In another aspect, R¹ can be **ID.**

In one aspect, the invention discloses compounds, the tautomer thereof, the stereoisomer thereof, or the pharmaceutically acceptable salt of any of the foregoing, wherein R³ can be in each instance independently selected from C₁₋₆ alkyl, halo, and C₁₋₆ haloalkyl. In another aspect, R³ can be in each instance independently selected from -S(=O)₂C₁₋₆ alkyl and -CN.

The invention also discloses compounds therein the tautomer thereof, the stereoisomer thereof, or the pharmaceutically acceptable salt of any of the foregoing, wherein R¹ can be substituted with R⁴.

In one aspect, R⁴ can be halo.

In one aspect of the invention, R³ can be independently selected from methyl, ethyl and cyclopropyl.

The invention provides the compounds, the tautomer thereof, the stereoisomer thereof, or the pharmaceutically acceptable salt of any of the foregoing, wherein the compound is selected from:
4-amino-N-cyclopropyl-7-fluoro-1-methyl-N-((3S)-6-(trifluoromethyl)-2,3-dihydro-1-benzofuran-3-yl)-1H-pyrazolo[4,3-c]quinoline-8-carboxamide,
4-amino-N-((3R)-6-cyano-2,3-dihydro-1-benzofuran-3-yl)-N-methyl-1,3-dihydrofuro[3,4-c]quinoline-8-carboxamide,
4-amino-N-((3S)-6-cyano-2,3-dihydro-1-benzofuran-3-yl)-N-methyl-1,3-dihydrofuro[3,4-c]quinoline-8-carboxamide,
4-amino-N-methyl-N-((3S)-6-(1-methyl-1H-pyrazol-4-yl)-2,3-dihydro-1-benzofuran-3-yl)-1,3-dihydrofuro[3,4-c]quinoline-8-carboxamide,
(3R)-4-amino-N,3-dimethyl-N-((3S)-6-(1-methyl-1H-pyrazol-4-yl)-2,3-dihydro-1-benzofuran-3-yl)-1,3-dihydrofuro[3,4-c]quinoline-8-carboxamide,
(1R)-4-amino-N,1-dimethyl-N-((3S)-6-(trifluoromethyl)-2,3-dihydro-1-benzofuran-3-yl)-1,3-dihydrofuro[3,4-c]quinoline-8-carboxamide,
(1S)-4-amino-N,1-dimethyl-N-((3S)-6-(trifluoromethyl)-2,3-dihydro-1-benzofuran-3-yl)-1,3-dihydrofuro[3,4-c]quinoline-8-carboxamide,
4-amino-N-methyl-N-((3S)-6-(trifluoromethoxy)-2,3-dihydro-1-benzofuran-3-yl)-1,3-dihydrofuro[3,4-c][1,7]naphthyridine-8-carboxamide,
4-amino-N-methyl-N-((3R)-6-(trifluoromethoxy)-2,3-dihydro-1-benzofuran-3-yl)-1,3-dihydrofuro[3,4-c][1,7]naphthyridine-8-carboxamide,
4-amino-N-(cyanomethyl)-N-((3R)-6-(trifluoromethyl)-2,3-dihydro-1-benzofuran-3-yl)-1,3-dihydrofuro[3,4-c][1,7]naphthyridine-8-carboxamide,
4-amino-N-(cyanomethyl)-N-((3 S)-6-(trifluoromethyl)-2,3-dihydro-1-benzofuran-3-yl)-1,3-dihydrofuro[3,4-c][1,7]naphthyridine-8-carboxamide,
4-amino-N-((3S)-5,6-dichloro-2,3-dihydro-1-benzofuran-3-yl)-N-methyl-1,3-dihydrofuro[3,4-c][1,7]naphthyridine-8-carboxamide,
4-amino-N-((3R)-5,6-dichloro-2,3-dihydro-1-benzofuran-3-yl)-N-methyl-1,3-dihydrofuro[3,4-c][1,7]naphthyridine-8-carboxamide,
(3R)-4-amino-N,3-dimethyl-N-((3S)-6-(trifluoromethyl)-2,3-dihydro-1-benzofuran-3-yl)-1,3-dihydrofuro[3,4-c][1,7]naphthyridine-8-carboxamide,
4-amino-7-fluoro-N-methyl-N-((3R)-5-(trifluoromethyl)-2,3-dihydrofuro[2,3-b]pyridin-3-yl)-1,3-dihydrofuro[3,4-c]quinoline-8-carboxamide,
4-amino-7-fluoro-N-methyl-N-((3S)-5-(trifluoromethyl)-2,3-dihydrofuro[2,3-b]pyridin-3-yl)-1,3-dihydrofuro[3,4-c]quinoline-8-carboxamide,
4-amino-N-((5R)-6,6-dimethyl-2-(trifluoromethyl)-6,7-dihydro-5H-cyclopenta[b]pyridin-5-yl)-7-fluoro-N-methyl-1,3-dihydrofuro[3,4-c]quinoline-8-carboxamide,
4-amino-N-((5S)-6,6-dimethyl-2-(trifluoromethyl)-6,7-dihydro-5H-cyclopenta[b]pyridin-5-yl)-7-fluoro-N-methyl-1,3-dihydrofuro[3,4-c]quinoline-8-carboxamide,
4-amino-7-fluoro-N-methyl-N-((3 S)-6-(1-methyl-1H-pyrazol-4-yl)-2,3-dihydro-1-benzofuran-3-yl)-1,3-dihydrofuro[3,4-c]quinoline-8-carboxamide,
4-amino-N-((5R)-6,6-difluoro-2-(trifluoromethyl)-6,7-dihydro-5H-cyclopenta[b]pyridin-5-yl)-7-fluoro-N-methyl-1,3-dihydrofuro[3,4-c]quinoline-8-carboxamide,
4-amino-N-((5S)-6,6-difluoro-2-(trifluoromethyl)-6,7-dihydro-5H-cyclopenta[b]pyridin-5-yl)-7-fluoro-N-methyl-1,3-dihydrofuro[3,4-c]quinoline-8-carboxamide,
4-amino-N-(6-bromo-2,3-dihydrobenzofuran-3-yl)-7-fluoro-N-methyl-1,3-dihydrofuro[3,4-c]quinoline-8-carboxamide,
(3R)-4-amino-7-fluoro-N,3-dimethyl-N-((3S)-6-(1-methyl-1H-pyrazol-4-yl)-2,3-dihydro-1-benzofuran-3-yl)-1,3-dihydrofuro[3,4-c]quinoline-8-carboxamide,
4-amino-7-cyano-N-methyl-N-((3S)-6-(trifluoromethyl)-2,3-dihydro-1-benzofuran-3-yl)-1,3-dihydrofuro[3,4-c]quinoline-8-carboxamide,
4-amino-N-methyl-N-((3S)-6-(trifluoromethyl)-2,3-dihydro-1-benzofuran-3-yl)-1,3-dihydrothieno[3,4-c] quinoline-8-carboxamide,
5-amino-N-methyl-N-((3S)-6-(trifluoromethyl)-2,3-dihydro-1-benzofuran-3-yl)benzo[c][2,6]naphthyridine-9-carboxamide,
5-amino-N-methyl-N-((3S)-6-(trifluoromethyl)-2,3-dihydro-1-benzofuran-3-yl)pyrimido[4,5-c]quinoline-9-carboxamide,
5-amino-N,1-dimethyl-N-((3S)-6-(trifluoromethyl)-2,3-dihydro-1-benzofuran-3-yl)benzo[c][2,6]naphthyridine-9-carboxamide,
5-amino-N-methyl-N-((3S)-6-(trifluoromethyl)-2,3-dihydro-1-benzofuran-3-yl)pyrido[4,3-c][1,7]naphthyridine-9-carboxamide,
5-amino-N-methyl-N-((3S)-6-(trifluoromethyl)-2,3-dihydro-1-benzofuran-3-yl)pyrimido[4,5-c][1,7]naphthyridine-9-carboxamide,
4-amino-7-fluoro-N,3-dimethyl-N-((3S)-6-(trifluoromethyl)-2,3-dihydro-1-benzofuran-3-yl)[1,2]oxazolo[4,5-c]quinoline-8-carboxamide,
4-amino-N,1-dimethyl-N-((3R)-5-(trifluoromethyl)-2,3-dihydrofuro[2,3-b]pyridin-3-yl)-1H-pyrazolo[4,3-c] quinoline-8-carboxamide,
4-amino-N,1-dimethyl-N-((3S)-5-(trifluoromethyl)-2,3-dihydrofuro[2,3-b]pyridin-3-yl)-1H-pyrazolo[4,3-c] quinoline-8-carboxamide,
4-amino-N,1-dimethyl-N-((3S)-6-(trifluoromethyl)-2,3-dihydrofuro[3,2-c]pyridin-3-yl)-1H-pyrazolo[4,3-c] quinoline-8-carboxamide,
4-amino-N-ethyl-1-methyl-N-((3R)-6-(trifluoromethyl)-2,3-dihydro-1-benzofuran-3-yl)-1H-pyrazolo[4,3-c] quinoline-8-carboxamide,
4-amino-N,1-dimethyl-N-((3S)-6-(trifluoromethoxy)-2,3-dihydro-1-benzofuran-3-yl)-1H-pyrazolo[4,3-c] quinoline-8-carboxamide,
4-amino-N-((3S)-4-chloro-6-(trifluoromethyl)-2,3-dihydro-1-benzofuran-3-yl)-N,1-dimethyl-1H-pyrazolo[4,3-c]quinoline-8-carboxamide,
4-amino-N-((3R)-4-chloro-6-(trifluoromethyl)-2,3-dihydro-1-benzofuran-3-yl)-N,1-dimethyl-1H-pyrazolo[4,3-c]quinoline-8-carboxamide,
4-amino-N-((5R)-6,6-difluoro-2-(trifluoromethyl)-6,7-dihydro-5H-cyclopenta[b]pyridin-5-yl)-N,1-dimethyl-1H-pyrazolo[4,3-c]quinoline-8-carboxamide,
4-amino-N-((5S)-6,6-difluoro-2-(trifluoromethyl)-6,7-dihydro-5H-cyclopenta[b]pyridin-5-yl)-N,1-dimethyl-1H-pyrazolo[4,3-c]quinoline-8-carboxamide,
4-amino-N-((3S)-6-bromo-2,3-dihydro-1-benzofuran-3-yl)-N,1-dimethyl-1H-pyrazolo[4,3-c]quinoline-8-carboxamide,
4-amino-N-((3S)-6-bromo-2,3-dihydrofuro[3,2-b]pyridin-3-yl)-N,1-dimethyl-1H-pyrazolo[4,3-c] quinoline-8-carboxamide,
4-amino-N-((3R)-6-bromo-2,3-dihydrofuro[3,2-b]pyridin-3-yl)-N,1-dimethyl-1H-pyrazolo[4,3-c] quinoline-8-carboxamide,
4-amino-N,1-dimethyl-N-((3S)-6-(trifluoromethyl)-2,3-dihydro-1-benzofuran-3-yl)-1H-pyrazolo[4,3-c][1,7]naphthyridine-8-carboxamide,
4-amino-N-ethyl-1-methyl-N-((3S)-6-(trifluoromethyl)-2,3-dihydro-1-benzofuran-3-yl)-1H-pyrazolo[4,3-c][1,7]naphthyridine-8-carboxamide,
4-amino-N,1-dimethyl-N-((3S)-6-(trifluoromethoxy)-2,3-dihydro-1-benzofuran-3-yl)-1H-pyrazolo[4,3-c][1,7]naphthyridine-8-carboxamide,
4-amino-N,1-dimethyl-N-((3S)-6-(pentafluoro-lambda~6~-sulfanyl)-2,3-dihydro-1-benzofuran-3-yl)-1H-pyrazolo[4,3-c] [1,7]naphthyridine-8-carboxamide,
4-amino-N, 1 -dimethyl-N-((3S)-6-(1-(trifluoromethyl)- 1H-pyrazol-4-yl)-2,3-dihydro-1-benzofuran-3-yl)-1H-pyrazolo[4,3-c][1,7]naphthyridine-8-carboxamide,
4-amino-7-fluoro-N, 1-dimethyl-N-((3R)-6-(trifluoromethyl)-2,3-dihydrofuro[2,3-b]pyridin-3-yl)-1H-pyrazolo[4,3-c]quinoline-8-carboxamide,
4-amino-N-ethyl-7-fluoro-1-methyl-N-((3S)-6-(trifluoromethyl)-2,3-dihydro-1-benzofuran-3-yl)-1H-pyrazolo[4,3-c]quinoline-8-carboxamide,
4-amino-N-ethyl-7-fluoro-1-methyl-N-((3R)-6-(trifluoromethyl)-2,3-dihydro-1-benzofuran-3-yl)-1H-pyrazolo[4,3-c]quinoline-8-carboxamide,
4-amino-N-((5R)-6,6-difluoro-2-(trifluoromethyl)-6,7-dihydro-5H-cyclopenta[b]pyridin-5-yl)-7-fluoro-N,1-dimethyl-1H-pyrazolo[4,3-c]quinoline-8-carboxamide,
4-amino-N-((5S)-6,6-difluoro-2-(trifluoromethyl)-6,7-dihydro-5H-cyclopenta[b]pyridin-5-yl)-7-fluoro-N,1-dimethyl-1H-pyrazolo[4,3-c]quinoline-8-carboxamide,
4-amino-7-fluoro-N,1-dimethyl-N-((3S)-6-(1-(trifluoromethyl)-1H-pyrazol-4-yl)-2,3-dihydro-1-benzofuran-3-yl)-1H-pyrazolo[4,3-c]quinoline-8-carboxamide,
4-amino-7-chloro-N,1-dimethyl-N-((3S)-6-(1-(trifluoromethyl)-1H-pyrazol-4-yl)-2,3-dihydro-1-benzofuran-3-yl)-1H-pyrazolo[4,3-c]quinoline-8-carboxamide,
4-amino-N,1,7-trimethyl-N-((3S)-6-(trifluoromethyl)-2,3-dihydro-1-benzofuran-3-yl)-1H-pyrazolo[4,3-c] quinoline-8-carboxamide,
4-amino-N,1,7-trimethyl-N-((3S)-6-(trifluoromethyl)-2,3-dihydro-1-benzofuran-3-yl)-1H-pyrazolo[4,3-c] [1,8]naphthyridine-8-carboxamide,
4-amino-N-((3S)-5,6-dichloro-2,3-dihydro-1-benzofuran-3-yl)-N,1,7-trimethyl-1H-pyrazolo[4,3-c] [1,8]naphthyridine-8-carboxamide,
4-amino-N,1-dimethyl-7-(trifluoromethyl)-N-((3S)-6-(trifluoromethyl)-2,3-dihydro-1-benzofuran-3-yl)-1H-pyrazolo[4,3-c]quinoline-8-carboxamide,
4-amino-6-fluoro-N,1-dimethyl-N-((3S)-6-(trifluoromethyl)-2,3-dihydro-1-benzofuran-3-yl)-1H-pyrazolo[4,3-c]quinoline-8-carboxamide,
4-amino-N,3-dimethyl-N-((3S)-6-(pentafluoro-lambda~6~-sulfanyl)-2,3-dihydro-1-benzofuran-3-yl)-3H-pyrazolo[3,4-c]quinoline-8-carboxamide,
4-amino-N,3-dimethyl-N-((3S)-6-(trifluoromethyl)-2,3-dihydro-1-benzofuran-3-yl)-3H-pyrazolo[3,4-c][1,7]naphthyridine-8-carboxamide,
4-amino-N,3-dimethyl-N-((3S)-6-(pentafluoro-lambda~6~-sulfanyl)-2,3-dihydro-1-benzofuran-3-yl)-3H-pyrazolo[3,4-c] [1,7]naphthyridine-8-carboxamide,
4-amino-7-fluoro-N,3-dimethyl-N-((3S)-6-(trifluoromethyl)-2,3-dihydro-1-benzofuran-3-yl)-3H-pyrazolo[3,4-c]quinoline-8-carboxamide,
4-amino-7-fluoro-N,3-dimethyl-N-((3S)-6-(trifluoromethoxy)-2,3-dihydro-1-benzofuran-3-yl)-3H-pyrazolo[3,4-c]quinoline-8-carboxamide,
4-amino-7-fluoro-N,3-dimethyl-N-((3 S)-6-(1-(trifluoromethyl)-1H-pyrazol-4-yl)-2,3-dihydro-1-benzofuran-3-yl)-3H-pyrazolo[3,4-c]quinoline-8-carboxamide,
4-amino-N-methyl-N-((3S)-6-(trifluoromethyl)-2,3-dihydro-1-benzofuran-3-yl)-1H-pyrazolo[4,3-c] quinoline-8-carboxamide,
4-amino-N,3-dimethyl-N-((3S)-6-(trifluoromethyl)-2,3-dihydro-1-benzofuran-3-yl)-1H-pyrazolo[4,3-c] quinoline-8-carboxamide,
4-amino-N,1,3-trimethyl-N-((3S)-6-(trifluoromethyl)-2,3-dihydro-1-benzofuran-3-yl)-1H-pyrazolo[4,3-c] quinoline-8-carboxamide,
4-amino-N,3-dimethyl-N-((3S)-6-(trifluoromethyl)-2,3-dihydro-1-benzofuran-3-yl)-1H-pyrazolo[4,3-c][1,7]naphthyridine-8-carboxamide,
4-amino-N,1,3-trimethyl-N-((3S)-6-(trifluoromethyl)-2,3-dihydro-1-benzofuran-3-yl)-1H-pyrazolo[4,3-c][1,7]naphthyridine-8-carboxamide,
4-amino-N,1,3-trimethyl-N-((3S)-6-(pentafluoro-lambda~6~-sulfanyl)-2,3-dihydro-1-benzofuran-3-yl)-1H-pyrazolo[4,3-c][1,7]naphthyridine-8-carboxamide,
4-amino-7-fluoro-N, 1,3-trimethyl-N-((3S)-6-(trifluoromethyl)-2,3-dihydro-1-benzofuran-3-yl)-1H-pyrazolo[4,3-c]quinoline-8-carboxamide,
4-amino-7-fluoro-N,1,3-trimethyl-N-((3S)-6-(1-(trifluoromethyl)-1H-pyrazol-4-yl)-2,3-dihydro-1-benzofuran-3-yl)-1H-pyrazolo[4,3-c]quinoline-8-carboxamide,
(3R)-4-amino-N-((4S)-7-methoxy-3,4-dihydro-1H-2-benzopyran-4-yl)-N,3-dimethyl-1,3-dihydrofuro[3,4-c][1,7]naphthyridine-8-carboxamide,
(3R)-4-amino-N,3-dimethyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamide,
(3S)-4-amino-N,3-dimethyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamide,
4-amino-7-fluoro-N-methyl-N-((4S)-7-(trifluoromethyl)-3,4-dihydro-2H-chromen-4-yl)-1,3-dihydrofuro[3,4-c]quinoline-8-carboxamide,
4-amino-N-(7-bromoisochroman-4-yl)-7-fluoro-N-methyl-1,3-dihydrofuro[3,4-c]quinoline-8-carboxamide,
4-amino-7-chloro-N-methyl-N-((5S)-2-(trifluoromethyl)-5,8-dihydro-6H-pyrano[3,4-b]pyridin-5-yl)-1,3-dihydrofuro[3,4-c]quinoline-8-carboxamide,
4-amino-7-chloro-N-((4S)-7-cyano-3,4-dihydro-1H-2-benzopyran-4-yl)-N-methyl-1,3-dihydrofuro[3,4-c] [1,8]naphthyridine-8-carboxamide,
4-amino-7-chloro-N-methyl-N-((4S)-7-(trifluoromethyl)-3,4-dihydro-1H-2-benzopyran-4-yl)-1,3-dihydrofuro[3,4-c][1,8]naphthyridine-8-carboxamide,
4-amino-N,7-dimethyl-N-((5S)-2-(trifluoromethyl)-5,8-dihydro-6H-pyrano[3,4-b]pyridin-5-yl)-1,3-dihydrofuro[3,4-c]quinoline-8-carboxamide,
4-amino-N-methyl-7-(trifluoromethyl)-N-((5S)-2-(trifluoromethyl)-5,8-dihydro-6H-pyrano[3,4-b]pyridin-5-yl)-1,3-dihydrofuro[3,4-c]quinoline-8-carboxamide,
5-amino-N-methyl-N-((5S)-2-(trifluoromethyl)-5,8-dihydro-6H-pyrano[3,4-b]pyridin-5-yl)benzo[c][2,6]naphthyridine-9-carboxamide,
5-amino-N-methyl-N-((5S)-2-(trifluoromethyl)-5,8-dihydro-6H-pyrano[3,4-b]pyridin-5-yl)pyrimido[4,5-c] quinoline-9-carboxamide,
5-amino-N-methyl-N-((5S)-2-(trifluoromethyl)-5,8-dihydro-6H-pyrano[3,4-b]pyridin-5-yl)pyrido[4,3-c][1,7]naphthyridine-9-carboxamide,
5-amino-N-methyl-N-((5S)-2-(trifluoromethyl)-5,8-dihydro-6H-pyrano[3,4-b]pyridin-5-yl)pyrimido[4,5-c][1,7]naphthyridine-9-carboxamide,
4-amino-N-((5S)-2-methoxy-5,8-dihydro-6H-pyrano[3,4-b]pyridin-5-yl)-N,1-dimethyl-1H-pyrazolo[4,3-c] quinoline-8-carboxamide,
4-amino-N-((5R)-2-methoxy-5,8-dihydro-6H-pyrano[3,4-b]pyridin-5-yl)-N,1-dimethyl-1H-pyrazolo[4,3-c] quinoline-8-carboxamide,
4-amino-N-((5S)-2-ethoxy-5,8-dihydro-6H-pyrano[3,4-d]pyrimidin-5-yl)-N,1-dimethyl-1H-pyrazolo[4,3-c] quinoline-8-carboxamide,
4-amino-N,1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N-(7-bromoisochroman-4-yl)-N,1-dimethyl-1H-pyrazolo[4,3-c]quinoline-8-carboxamide,
4-amino-N,1-dimethyl-N-((4S)-7-(trifluoromethyl)-3,4-dihydro-1H-2-benzopyran-4-yl)-1H-pyrazolo[4,3-c][1,7]naphthyridine-8-carboxamide,
4-amino-N,1-dimethyl-N-((5S)-2-(trifluoromethyl)-5,8-dihydro-6H-pyrano[3,4-b]pyridin-5-yl)-1H-pyrazolo[4,3-c] [1,7]naphthyridine-8-carboxamide,
4-amino-7-fluoro-N,1-dimethyl-N-((4R)-7-(trifluoromethyl)-3,4-dihydro-2H-chromen-4-yl)-1H-pyrazolo[4,3-c]quinoline-8-carboxamide,
4-amino-7-fluoro-N,1-dimethyl-N-((4S)-7-(trifluoromethyl)-3,4-dihydro-2H-chromen-4-yl)-1H-pyrazolo[4,3-c]quinoline-8-carboxamide,
4-amino-7-fluoro-N,1-dimethyl-N-((4R)-7-(trifluoromethyl)-3,4-dihydro-2H-pyrano[2,3-b]pyridin-4-yl)-1H-pyrazolo[4,3-c]quinoline-8-carboxamide,
4-amino-7-fluoro-N,1-dimethyl-N-((4S)-7-(trifluoromethyl)-3,4-dihydro-2H-pyrano[2,3-b]pyridin-4-yl)-1H-pyrazolo[4,3-c]quinoline-8-carboxamide,
4-amino-7-fluoro-N,1-dimethyl-N-((4S)-7-(trifluoromethyl)-3,4-dihydro-1H-pyrano[4,3-c]pyridin-4-yl)-1H-pyrazolo[4,3-c]quinoline-8-carboxamide,
4-amino-7-fluoro-N,1-dimethyl-N-((4R)-7-(trifluoromethyl)-3,4-dihydro-1H-pyrano[4,3-c]pyridin-4-yl)-1H-pyrazolo[4,3-c]quinoline-8-carboxamide,
4-amino-7-chloro-N,1-dimethyl-N-((4S)-7-(trifluoromethyl)-3,4-dihydro-1H-2-benzopyran-4-yl)-1H-pyrazolo[4,3-c]quinoline-8-carboxamide,
4-amino-7-chloro-N,1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-7-chloro-N,1-dimethyl-N-((4S)-7-(trifluoromethyl)-3,4-dihydro-1H-2-benzopyran-4-yl)-1H-pyrazolo[4,3-c] [1,8]naphthyridine-8-carboxamide,
4-amino-N,1,7-trimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N-((5 S)-2-bromo-5,8-dihydro-6H-pyrano [3,4-b]pyridin-5-yl)-N,1,7-trimethyl-1H-pyrazolo [4,3-c] quinoline-8-carboxamide,
4-amino-N-((4S)-7-cyano-3,4-dihydro-1H-2-benzopyran-4-y1)-N,1,7-trimethyl-1H-pyrazolo[4,3-c] [1,8]naphthyridine-8-carboxamide,
4-amino-N,1,7-trimethyl-N-((4S)-7-(trifluoromethyl)-3,4-dihydro-1H-2-benzopyran-4-yl)-1H-pyrazolo[4,3-c] [1,8]naphthyridine-8-carboxamide,
4-amino-N,1,7-trimethyl-N-((5S)-2-(trifluoromethyl)-5,8-dihydro-6H-pyrano[3,4-b]pyridin-5-yl)-1H-pyrazolo[4,3-c] [1,8]naphthyridine-8-carboxamide,
4-amino-N,1-dimethyl-7-(trifluoromethyl)-N-((4S)-7-(trifluoromethyl)-3,4-dihydro-1H-2-benzopyran-4-yl)-1H-pyrazolo[4,3-c]quinoline-8-carboxamide,
4-amino-N,1-dimethyl-7-(trifluoromethyl)-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N,3-dimethyl-N-((5 S)-2-(trifluoromethyl)-5,8-dihydro-6H-pyrano[3,4-b]pyridin-5-yl)-3H-pyrazolo[3,4-c] [1, 7]naphthyridine-8-carboxamide,
4-amino-N-ethyl-7-fluoro-3-methyl-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-7-fluoro-N,3-dimethyl-N-((4R)-7-(trifluoromethoxy)-3,4-dihydro-1H-2-benzopyran-4-yl)-3H-pyrazolo[3,4-c]quinoline-8-carboxamide,
4-amino-7-fluoro-N,3-dimethyl-N-((4S)-7-(trifluoromethoxy)-3,4-dihydro-1H-2-benzopyran-4-yl)-3H-pyrazolo[3,4-c]quinoline-8-carboxamide,
4-amino-N-ethyl-3,7-dimethyl-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-N,3-dimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N,1,3-trimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N,3-dimethyl-N-((5S)-2-(trifluoromethyl)-5,8-dihydro-6H-pyrano[3,4-b]pyridin-5-yl)-1H-pyrazolo[4,3-c] [1,7]naphthyridine-8-carboxamide,
4-amino-N,1,3-trimethyl-N-((4S)-7-(trifluoromethyl)-3,4-dihydro-1H-2-benzopyran-4-yl)-1H-pyrazolo[4,3-c] [1,7]naphthyridine-8-carboxamide,
4-amino-N,1,3-trimethyl-N-((5S)-2-(trifluoromethyl)-5,8-dihydro-6H-pyrano[3,4-b]pyridin-5-yl)-1H-pyrazolo[4,3-c] [1,7]naphthyridine-8-carboxamide,
4-amino-7-fluoro-N,1,3-trimethyl-N-((4S)-7-(trifluoromethyl)-3,4-dihydro-1H-2-benzopyran-4-yl)-1H-pyrazolo[4,3-c]quinoline-8-carboxamide,
4-amino-7-fluoro-N,1,3-trimethyl-N-((5S)-2-(trifluoromethyl)-5,8-dihydro-6H-pyrano[3,4-b]pyridin-5-yl)-1H-pyrazolo[4,3-c]quinoline-8-carboxamide,
2-amino-3-iodo-N-methyl-N-((5S)-2-(trifluoromethyl)-5,8-dihydro-6H-pyrano[3,4-b]pyridin-5-yl)-6-quinolinecarboxamide,
4-amino-N,1,7-trimethyl-N-((5R)-2-(trifluoromethyl)-5,6,7,9-tetrahydrooxepino[3,4-b]pyridin-5-yl)-1H-pyrazolo[4,3-c]quinoline-8-carboxamide,
4-amino-N,1,7-trimethyl-N-((5S)-2-(trifluoromethyl)-5,6,7,9-tetrahydrooxepino[3,4-b]pyridin-5-yl)-1H-pyrazolo[4,3-c]quinoline-8-carboxamide,
4-amino-N-methyl-N-((3S)-6-(trifluoromethyl)-2,3-dihydro-1-benzofuran-3-yl)-1,3-dihydrofuro[3,4-c] quinoline-8-carboxamide,
4-amino-N-methyl-N-((3R)-6-(trifluoromethyl)-2,3-dihydro-1-benzofuran-3-yl)-1,3-dihydrofuro[3,4-c] quinoline-8-carboxamide,
4-amino-N-methyl-N-((3S)-6-nitro-2,3-dihydro-1-benzofuran-3-yl)-1,3-dihydrofuro[3,4-c]quinoline-8-carboxamide,
4-amino-N-methyl-N-((3R)-6-nitro-2,3-dihydro-1-benzofuran-3-yl)-1,3-dihydrofuro[3,4-c]quinoline-8-carboxamide,
4-amino-N-(~2~H_3_)methyl-N-((3R)-6-(trifluoromethyl)-2,3-dihydro-1-benzofuran-3-yl)-1,3-dihydrofuro[3,4-c]quinoline-8-carboxamide,
4-amino-N-(~2~H_3_)methyl-N-((3S)-6-(trifluoromethyl)-2,3-dihydro-1-benzofuran-3-yl)-1,3-dihydrofuro[3,4-c]quinoline-8-carboxamide,
4-amino-N-methyl-N-((3S)-6-(methylsulfonyl)-2,3-dihydro-1-benzofuran-3-yl)-1,3-dihydrofuro[3,4-c] quinoline-8-carboxamide,
4-amino-N-methyl-N-((3R)-6-(methylsulfonyl)-2,3-dihydro-1-benzofuran-3-yl)-1,3-dihydrofuro[3,4-c] quinoline-8-carboxamide,
4-amino-N-((3S)-4-fluoro-6-(trifluoromethyl)-2,3-dihydro-1-benzofuran-3-yl)-N-methyl-1,3-dihydrofuro[3,4-c]quinoline-8-carboxamide,
4-amino-N-((3R)-4-fluoro-6-(trifluoromethyl)-2,3-dihydro-1-benzofuran-3-yl)-N-methyl-1,3-dihydrofuro[3,4-c]quinoline-8-carboxamide,
4-amino-N-methyl-N-((3S)-6-(pentafluoro-lambda~6~-sulfanyl)-2,3-dihydro-1-benzofuran-3-yl)-1,3-dihydrofuro[3,4-c]quinoline-8-carboxamide,
4-amino-N-methyl-N-((3R)-6-(pentafluoro-lambda~6~-sulfanyl)-2,3-dihydro-1-benzofuran-3-yl)-1,3-dihydrofuro[3,4-c]quinoline-8-carboxamide,
4-amino-N-((3S)-6-bromo-2,3-dihydro-1-benzofuran-3-yl)-N-methyl-1,3-dihydrofuro[3,4-c]quinoline-8-carboxamide,
4-amino-N-((3R)-6-bromo-2,3-dihydro-1-benzofuran-3-yl)-N-methyl-1,3-dihydrofuro[3,4-c]quinoline-8-carboxamide,
(3R)-4-amino-N-((3S)-6-bromo-2,3-dihydro-1-benzofuran-3-yl)-N,3-dimethyl-1,3-dihydrofuro[3,4-c] quinoline-8-carboxamide,
(3R)-4-amino-N-((3R)-6-bromo-2,3-dihydro-1-benzofuran-3-yl)-N,3-dimethyl-1,3-dihydrofuro[3,4-c] quinoline-8-carboxamide,
4-amino-N-((3S)-6-chloro-2,3-dihydro-1-benzofuran-3-yl)-N-methyl-1,3-dihydrofuro[3,4-c][1,7]naphthyridine-8-carboxamide,
4-amino-N-((3R)-6-chloro-2,3-dihydro-1-benzofuran-3-yl)-N-methyl-1,3-dihydrofuro[3,4-c][1,7]naphthyridine-8-carboxamide,
4-amino-N-methyl-N-((3S)-6-(trifluoromethyl)-2,3-dihydrofuro[2,3-b]pyridin-3-yl)-1,3-dihydrofuro[3,4-c][1,7]naphthyridine-8-carboxamide,
4-amino-N-methyl-N-((3R)-6-(trifluoromethyl)-2,3-dihydrofuro[2,3-b]pyridin-3-yl)-1,3-dihydrofuro[3,4-c][1,7]naphthyridine-8-carboxamide,
4-amino-N-(~2~H_3_)methyl-N-((3S)-6-(trifluoromethyl)-2,3-dihydro-1-benzofuran-3-yl)-1,3-dihydrofuro[3,4-c][1,7]naphthyridine-8-carboxamide,
4-amino-N-(~2~H_3_)methyl-N-((3R)-6-(trifluoromethyl)-2,3-dihydro-1-benzofuran-3-yl)-1,3-dihydrofuro[3,4-c][1,7]naphthyridine-8-carboxamide,
4-amino-N-methyl-N-((3S)-6-(methylsulfonyl)-2,3-dihydro-1-benzofuran-3-yl)-1,3-dihydrofuro[3,4-c][ 1,7]naphthyridine-8-carboxamide,
4-amino-N-methyl-N-((3R)-6-(methylsulfonyl)-2,3-dihydro-1-benzofuran-3-yl)-1,3-dihydrofuro[3,4-c] [1, 7]naphthyridine-8-carboxamide,
4-amino-N-methyl-N-((1R)-5-(trifluoromethoxy)-2,3-dihydro-1H-inden-1-yl)-1,3-dihydrofuro[3,4-c][1,7]naphthyridine-8-carboxamide,
4-amino-N-methyl-N-((1S)-5-(trifluoromethoxy)-2,3-dihydro-1H-inden-1-yl)-1,3-dihydrofuro[3,4-c][1,7]naphthyridine-8-carboxamide,
4-amino-N-methyl-N-((3S)-6-(pentafluoro-lambda~6~-sulfanyl)-2,3-dihydro-1-benzofuran-3-yl)-1,3-dihydrofuro[3,4-c][1,7]naphthyridine-8-carboxamide,
4-amino-N-methyl-N-((3R)-6-(pentafluoro-lambda~6~-sulfanyl)-2,3-dihydro-1-benzofuran-3-yl)-1,3-dihydrofuro[3,4-c][1,7]naphthyridine-8-carboxamide,
4-amino-N-((3S)-6-bromo-2,3-dihydro-1-benzofuran-3-yl)-N-methyl-1,3-dihydrofuro[3,4-c][1,7]naphthyridine-8-carboxamide,
4-amino-N-((3R)-6-bromo-2,3-dihydro-1-benzofuran-3-yl)-N-methyl-1,3-dihydrofuro[3,4-c][1,7]naphthyridine-8-carboxamide,
4-amino-N-((3S)-6-chloro-2,3-dihydro-1-benzofuran-3-yl)-7-fluoro-N-methyl-1,3-dihydrofuro[3,4-c] quinoline-8-carboxamide,
4-amino-N-((3R)-6-chloro-2,3-dihydro-1-benzofuran-3-yl)-7-fluoro-N-methyl-1,3-dihydrofuro[3,4-c] quinoline-8-carboxamide,
4-amino-7-fluoro-N-methyl-N-((5R)-2-(trifluoromethyl)-6,7-dihydro-5H-cyclopenta[b]pyridin-5-yl)-1,3-dihydrofuro[3,4-c]quinoline-8-carboxamide,
4-amino-7-fluoro-N-methyl-N-((5S)-2-(trifluoromethyl)-6,7-dihydro-5H-cyclopenta[b]pyridin-5-yl)-1,3-dihydrofuro[3,4-c]quinoline-8-carboxamide,
4-amino-7-fluoro-N-methyl-N-((3S)-6-(trifluoromethyl)-2,3-dihydrofuro[2,3-b]pyridin-3-yl)-1,3-dihydrofuro[3,4-c]quinoline-8-carboxamide,
4-amino-7-fluoro-N-methyl-N-((3R)-6-(trifluoromethyl)-2,3-dihydrofuro[2,3-b]pyridin-3-yl)-1,3-dihydrofuro[3,4-c]quinoline-8-carboxamide,
4-amino-7-fluoro-N-methyl-N-((3S)-6-(methylsulfonyl)-2,3-dihydro-1-benzofuran-3-yl)-1,3-dihydrofuro[3,4-c]quinoline-8-carboxamide,
4-amino-7-fluoro-N-methyl-N-((3R)-6-(methylsulfonyl)-2,3-dihydro-1-benzofuran-3-yl)-1,3-dihydrofuro[3,4-c]quinoline-8-carboxamide,
4-amino-7-fluoro-N-((3S)-4-fluoro-6-(trifluoromethyl)-2,3-dihydro-1-benzofuran-3-yl)-N-methyl-1,3-dihydrofuro[3,4-c]quinoline-8-carboxamide,
4-amino-7-fluoro-N-((3R)-4-fluoro-6-(trifluoromethyl)-2,3-dihydro-1-benzofuran-3-yl)-N-methyl-1,3-dihydrofuro[3,4-c]quinoline-8-carboxamide,
4-amino-7-fluoro-N-methyl-N-((3S)-6-(pentafluoro-lambda~6~-sulfanyl)-2,3-dihydro-1-benzofuran-3-yl)-1,3-dihydrofuro[3,4-c]quinoline-8-carboxamide,
4-amino-7-fluoro-N-methyl-N-((3R)-6-(pentafluoro-lambda~6~-sulfanyl)-2,3-dihydro-1-benzofuran-3-yl)-1,3-dihydrofuro[3,4-c]quinoline-8-carboxamide,
(3R)-4-amino-7-fluoro-N,3-dimethyl-N-((5R)-2-(trifluoromethyl)-6,7-dihydro-5H-cyclopenta[b]pyridin-5-yl)-1,3-dihydrofuro[3,4-c]quinoline-8-carboxamide,
(3R)-4-amino-7-fluoro-N,3-dimethyl-N-((5S)-2-(trifluoromethyl)-6,7-dihydro-5H-cyclopenta[b]pyridin-5-yl)-1,3-dihydrofuro[3,4-c]quinoline-8-carboxamide,
4-amino-7-chloro-N-methyl-N-((3S)-6-(trifluoromethyl)-2,3-dihydrofuro[3,2-c]pyridin-3-yl)-1,3-dihydrofuro[3,4-c]quinoline-8-carboxamide,
4-amino-7-chloro-N-methyl-N-((3R)-6-(trifluoromethyl)-2,3-dihydrofuro[3,2-c]pyridin-3-yl)-1,3-dihydrofuro[3,4-c]quinoline-8-carboxamide,
4-amino-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-N,1-dimethyl-N-((5S)-2-(trifluoromethyl)-6,7-dihydro-5H-cyclopenta[b]pyridin-5-yl)-1H-pyrazolo[4,3-c]quinoline-8-carboxamide,
4-amino-N-ethyl-1-methyl-N-((3S)-6-(trifluoromethyl)-2,3-dihydro-1-benzofuran-3-yl)-1H-pyrazolo[4,3-c] quinoline-8-carboxamide,
4-amino-N,1-dimethyl-N-((3S)-6-(pentafluoro-lambda~6~-sulfanyl)-2,3-dihydro-1-benzofuran-3-yl)-1H-pyrazolo[4,3-c]quinoline-8-carboxamide,
4-amino-N,1-dimethyl-N-((3R)-6-(pentafluoro-lambda~6~-sulfanyl)-2,3-dihydro-1-benzofuran-3-yl)-1H-pyrazolo[4,3-c]quinoline-8-carboxamide,
4-amino-N-((1R)-5-bromo-2,3-dihydro-1H-inden-1-yl)-N,1-dimethyl-1H-pyrazolo[4,3-c]quinoline-8-carboxamide,
4-amino-N-((1S)-5-bromo-2,3-dihydro-1H-inden-1-yl)-N,1-dimethyl-1H-pyrazolo[4,3-c]quinoline-8-carboxamide,
4-amino-N-((5R)-2-bromo-6,7-dihydro-5H-cyclopenta[b]pyridin-5-yl)-N,1-dimethyl-1H-pyrazolo[4,3-c] quinoline-8-carboxamide,
4-amino-N,1-dimethyl-N-((5R)-2-(trifluoromethyl)-6,7-dihydro-5H-cyclopenta[b]pyridin-5-yl)-1H-pyrazolo[4,3-c] [1,7]naphthyridine-8-carboxamide,
4-amino-N-ethyl-1-methyl-N-((3R)-6-(trifluoromethyl)-2,3-dihydro-1-benzofuran-3-yl)-1H-pyrazolo[4,3-c][1,7]naphthyridine-8-carboxamide,
4-amino-N-((3S)-6-cyano-2,3-dihydro-1-benzofuran-3-yl)-7-fluoro-N,1-dimethyl-1H-pyrazolo[4,3-c] quinoline-8-carboxamide,
4-amino-N-((3R)-6-cyano-2,3-dihydro-1-benzofuran-3-yl)-7-fluoro-N,1-dimethyl-1H-pyrazolo[4,3-c] quinoline-8-carboxamide,
4-amino-7-fluoro-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-7-fluoro-N, 1-dimethyl-N-((5S)-2-(trifluoromethyl)-6,7-dihydro-5H-cyclopenta[b]pyridin-5-yl)-1H-pyrazolo[4,3-c]quinoline-8-carboxamide,
4-amino-7-fluoro-N, 1-dimethyl-N-((3S)-6-(trifluoromethyl)-2,3-dihydrofuro[2,3-b]pyridin-3-yl)-1H-pyrazolo[4,3-c]quinoline-8-carboxamide,
4-amino-7-fluoro-N,1-dimethyl-N-((3S)-6-(methylsulfonyl)-2,3-dihydro-1-benzofuran-3-yl)-1H-pyrazolo[4,3-c]quinoline-8-carboxamide,
4-amino-7-fluoro-N,1-dimethyl-N-((3R)-6-(methylsulfonyl)-2,3-dihydro-1-benzofuran-3-yl)-1H-pyrazolo[4,3-c]quinoline-8-carboxamide,
4-amino-7-fluoro-N-((3 S)-4-fluoro-6-(trifluoromethyl)-2,3-dihydro-1-benzofuran-3-yl)-N,1-dimethyl-1H-pyrazolo[4,3-c]quinoline-8-carboxamide,
4-amino-7-fluoro-N-((3R)-4-fluoro-6-(trifluoromethyl)-2,3-dihydro-1-benzofuran-3-yl)-N,1-dimethyl-1H-pyrazolo[4,3-c]quinoline-8-carboxamide,
4-amino-7-fluoro-N,1-dimethyl-N-((3S)-6-(pentafluoro-lambda~6~-sulfanyl)-2,3-dihydro-1-benzofuran-3-yl)-1H-pyrazolo[4,3-c]quinoline-8-carboxamide,
4-amino-7-fluoro-N,1-dimethyl-N-((3R)-6-(pentafluoro-lambda~6~-sulfanyl)-2,3-dihydro-1-benzofuran-3-yl)-1H-pyrazolo[4,3-c]quinoline-8-carboxamide,
4-amino-7-chloro-N,1-dimethyl-N-((5R)-5-methyl-2-(trifluoromethyl)-6,7-dihydro-5H-cyclopenta[b]pyridin-5-yl)-1H-pyrazolo[4,3-c]quinoline-8-carboxamide,
4-amino-7-chloro-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-7-chloro-N,1-dimethyl-N-((5S)-2-(trifluoromethyl)-6,7-dihydro-5H-cyclopenta[b]pyridin-5-yl)-1H-pyrazolo[4,3-c]quinoline-8-carboxamide,
4-amino-7-chloro-N,1-dimethyl-N-((3S)-6-(trifluoromethyl)-2,3-dihydrofuro[2,3-b]pyridin-3-yl)-1H-pyrazolo[4,3-c]quinoline-8-carboxamide,
4-amino-7-chloro-N,1-dimethyl-N-((3R)-6-(trifluoromethyl)-2,3-dihydrofuro[2,3-b]pyridin-3-yl)-1H-pyrazolo[4,3-c]quinoline-8-carboxamide,
4-amino-7-fluoro-N,3-dimethyl-N-((5R)-2-(trifluoromethyl)-6,7-dihydro-5H-cyclopenta[b]pyridin-5-yl)-3H-pyrazolo[3,4-c]quinoline-8-carboxamide,
4-amino-7-fluoro-N,3-dimethyl-N-((5S)-2-(trifluoromethyl)-6,7-dihydro-5H-cyclopenta[b]pyridin-5-yl)-3H-pyrazolo[3,4-c]quinoline-8-carboxamide,
4-amino-7-fluoro-N,1,3-trimethyl-N-((3S)-6-(pentafluoro-lambda~6~-sulfanyl)-2,3-dihydro-1-benzofuran-3-yl)-1H-pyrazolo[4,3-c]quinoline-8-carboxamide,
4-amino-7-fluoro-N,1,3-trimethyl-N-((3R)-6-(pentafluoro-lambda~6~-sulfanyl)-2,3-dihydro-1-benzofuran-3-yl)-1H-pyrazolo[4,3-c]quinoline-8-carboxamide,
4-amino-N-((4S)-7-methoxy-3,4-dihydro-1H-2-benzopyran-4-yl)-N-methyl-1,3-dihydrofuro[3,4-c] quinoline-8-carboxamide,
(3R)-4-amino-N-((4S)-7-bromo-3,4-dihydro-1H-2-benzopyran-4-yl)-N,3-dimethyl-1,3-dihydrofuro[3,4-c] quinoline-8-carboxamide,
(3R)-4-amino-N-((4R)-7-bromo-3,4-dihydro-1H-2-benzopyran-4-yl)-N,3-dimethyl-1,3-dihydrofuro[3,4-c] quinoline-8-carboxamide,
(3R)-4-amino-N-((4S)-7-bromo-3,4-dihydro-1H-2-benzopyran-4-yl)-N-ethyl-3-methyl-1,3-dihydrofuro[3,4-c]quinoline-8-carboxamide,
(3R)-4-amino-N-((4R)-7-bromo-3,4-dihydro-1H-2-benzopyran-4-yl)-N-ethyl-3-methyl-1,3-dihydrofuro[3,4-c]quinoline-8-carboxamide,
4-amino-N-((4S)-7-bromo-3,4-dihydro-1H-2-benzopyran-4-yl)-N-methyl-1,3-dihydrofuro[3,4-c][1,7]naphthyridine-8-carboxamide,
4-amino-N-((4R)-7-bromo-3,4-dihydro-1H-2-benzopyran-4-yl)-N-methyl-1,3-dihydrofuro[3,4-c][1,7]naphthyridine-8-carboxamide,
4-amino-7-fluoro-N-((4S)-7-methoxy-3,4-dihydro-1H-2-benzopyran-4-yl)-N-methyl-1,3-dihydrofuro[3,4-c] quinoline-8-carboxamide,
4-amino-7-fluoro-N-methyl-N-((4R)-7-(trifluoromethyl)-3,4-dihydro-2H-chromen-4-yl)-1,3-dihydrofuro[3,4-c]quinoline-8-carboxamide,
4-amino-7-chloro-N-((5 S)-2-cyano-5,8-dihydro-6H-pyrano [3,4-b]pyridin-5-yl)-N-methyl-1,3-dihydrofuro[3,4-c]quinoline-8-carboxamide,
4-amino-7-chloro-N-((5R)-2-cyano-5,8-dihydro-6H-pyrano[3,4-b]pyridin-5-yl)-N-methyl-1,3-dihydrofuro[3,4-c]quinoline-8-carboxamide,
4-amino-7-chloro-N-((4S)-8-fluoro-7-(trifluoromethyl)-3,4-dihydro-1H-2-benzopyran-4-yl)-N-methyl-1,3-dihydrofuro[3,4-c]quinoline-8-carboxamide,
4-amino-7-chloro-N-((4R)-8-fluoro-7-(trifluoromethyl)-3,4-dihydro-1H-2-benzopyran-4-yl)-N-methyl-1,3-dihydrofuro[3,4-c]quinoline-8-carboxamide,
4-amino-7-chloro-N-methyl-N-((1R,4S)-1-methyl-7-(trifluoromethyl)-3,4-dihydro-1H-2-benzopyran-4-yl)-1,3-dihydrofuro[3,4-c] [1,8]naphthyridine-8-carboxamide,
4-amino-N-((4S)-8-fluoro-3,4-dihydro-1H-pyrano[4,3-c]pyridin-4-yl)-N,1-dimethyl-1H-pyrazolo[4,3-c] quinoline-8-carboxamide,
4-amino-N-((4S)-7-methoxy-3,4-dihydro-1H-2-benzopyran-4-yl)-N,1-dimethyl-1H-pyrazolo[4,3-c] quinoline-8-carboxamide,
4-amino-N-((4S)-7,8-difluoro-3,4-dihydro-1H-2-benzopyran-4-yl)-N,1-dimethyl-1H-pyrazolo[4,3-c] quinoline-8-carboxamide,
4-amino-N-((5 S)-2-ethoxy-5,8-dihydro-6H-pyrano [3,4-b]pyridin-5-yl)-N,1-dimethyl-1H-pyrazolo [4,3-c] quinoline-8-carboxamide,
4-amino-N,1-dimethyl-N-((5R)-2-(trifluoromethyl)-5,6,7,8-tetrahydro-5-quinolinyl)-1H-pyrazolo[4,3-c] quinoline-8-carboxamide,
4-amino-N-((4S)-7-bromo-3,4-dihydro-1H-2-benzopyran-4-yl)-N,1-dimethyl-1H-pyrazolo[4,3-c] quinoline-8-carboxamide,
4-amino-N-((4R)-7-bromo-3,4-dihydro-1H-2-benzopyran-4-yl)-N,1-dimethyl-1H-pyrazolo[4,3-c] quinoline-8-carboxamide,
4-amino-N,1-dimethyl-N-((1R,4S)-1-methyl-7-(trifluoromethyl)-3,4-dihydro-1H-2-benzopyran-4-yl)-1H-pyrazolo[4,3-c]quinoline-8-carboxamide,
4-amino-N, 1-dimethyl-N-((1S,4R)-1-methyl-7-(trifluoromethyl)-3,4-dihydro-1H-2-benzopyran-4-yl)-1H-pyrazolo[4,3-c]quinoline-8-carboxamide,
4-amino-N-((4S)-8-fluoro-7-(trifluoromethyl)-3,4-dihydro-1H-2-benzopyran-4-yl)-N,1-dimethyl-1H-pyrazolo[4,3-c]quinoline-8-carboxamide,
4-amino-N-((4R)-8-fluoro-7-(trifluoromethyl)-3,4-dihydro-1H-2-benzopyran-4-yl)-N,1-dimethyl-1H-pyrazolo[4,3-c]quinoline-8-carboxamide,
4-amino-N-((5S)-2-bromo-5,8-dihydro-6H-pyrano[3,4-b]pyridin-5-yl)-N,1-dimethyl-1H-pyrazolo[4,3-c] quinoline-8-carboxamide,
4-amino-N-((5R)-2-bromo-5,8-dihydro-6H-pyrano[3,4-b]pyridin-5-yl)-N,1-dimethyl-1H-pyrazolo[4,3-c] quinoline-8-carboxamide,
4-amino-N-((4S)-7-methoxy-3,4-dihydro-1H-2-benzopyran-4-yl)-N,1-dimethyl-1H-pyrazolo[4,3-c][1,7]naphthyridine-8-carboxamide,
4-amino-N-((4R)-7-methoxy-3,4-dihydro-1H-2-benzopyran-4-yl)-N,1-dimethyl-1H-pyrazolo[4,3-c][1,7]naphthyridine-8-carboxamide,
4-amino-N-((4S)-8-fluoro-7-(trifluoromethyl)-3,4-dihydro-1H-2-benzopyran-4-yl)-N,1-dimethyl-1H-pyrazolo[4,3-c] [1,7]naphthyridine-8-carboxamide,
4-amino-N-((5S)-5,8-dihydro-6H-pyrano[3,4-b]pyridin-5-yl)-7-fluoro-N,1-dimethyl-1H-pyrazolo[4,3-c] quinoline-8-carboxamide,
4-amino-N-((5R)-5,8-dihydro-6H-pyrano[3,4-b]pyridin-5-yl)-7-fluoro-N,1-dimethyl-1H-pyrazolo[4,3-c] quinoline-8-carboxamide,
4-amino-7-fluoro-N-((4S)-8-fluoro-3,4-dihydro-1H-pyrano[4,3-c]pyridin-4-yl)-N,1-dimethyl-1H-pyrazolo[4,3-c]quinoline-8-carboxamide,
4-amino-7-fluoro-N-((4R)-8-fluoro-3,4-dihydro-1H-pyrano[4,3-c]pyridin-4-yl)-N,1-dimethyl-1H-pyrazolo[4,3-c]quinoline-8-carboxamide,
4-amino-7-fluoro-N-((4S)-7-methoxy-3,4-dihydro-1H-2-benzopyran-4-yl)-N,1-dimethyl-1H-pyrazolo[4,3-c]quinoline-8-carboxamide,
4-amino-7-fluoro-N-((4R)-7-methoxy-3,4-dihydro-1H-2-benzopyran-4-yl)-N,1-dimethyl-1H-pyrazolo[4,3-c]quinoline-8-carboxamide,
4-amino-7-fluoro-N,1-dimethyl-N-((5R)-2-(trifluoromethyl)-5,6,78-tetrahydro-5-quinolinyl)-1H-pyrazolo[4,3-c]quinoline-8-carboxamide,
4-amino-7-fluoro-N, 1-dimethyl-N-((4S)-7-(trifluoromethoxy)-3,4-dihydro-1H-2-benzopyran-4-yl)-1H-pyrazolo[4,3-c]quinoline-8-carboxamide,
4-amino-7-fluoro-N, 1-dimethyl-N-((4R)-7-(trifluoromethoxy)-3,4-dihydro-1H-2-benzopyran-4-yl)-1H-pyrazolo[4,3-c]quinoline-8-carboxamide,
4-amino-7-fluoro-N-((4S)-8-fluoro-7-(trifluoromethyl)-3,4-dihydro-1H-2-benzopyran-4-yl)-N,1-dimethyl-1H-pyrazolo[4,3-c]quinoline-8-carboxamide,
4-amino-7-fluoro-N-((4R)-8-fluoro-7-(trifluoromethyl)-3,4-dihydro-1H-2-benzopyran-4-yl)-N,1-dimethyl-1H-pyrazolo[4,3-c]quinoline-8-carboxamide,
4-amino-N-((5R)-7,7-dimethyl-2-(trifluoromethyl)-5,6,7,8-tetrahydro-5-quinolinyl)-7-fluoro-N,1-dimethyl-1H-pyrazolo[4,3-c]quinoline-8-carboxamide,
4-amino-N-((5S)-7,7-dimethyl-2-(trifluoromethyl)-5,6,7,8-tetrahydro-5-quinolinyl)-7-fluoro-N,1-dimethyl-1H-pyrazolo[4,3-c]quinoline-8-carboxamide,
4-amino-N-((4S)-8-fluoro-7-(trifluoromethyl)-3,4-dihydro-1H-2-benzopyran-4-yl)-N,1,7-trimethyl-1H-pyrazolo[4,3-c]quinoline-8-carboxamide,
4-amino-N-((4R)-8-fluoro-7-(trifluoromethyl)-3,4-dihydro-1H-2-benzopyran-4-yl)-N,1,7-trimethyl-1H-pyrazolo[4,3-c]quinoline-8-carboxamide,
4-amino-N-((5S)-2-ethoxy-5,8-dihydro-6H-pyrano[3,4-b]pyridin-5-yl)-N,3-dimethyl-3H-pyrazolo[3,4-c] quinoline-8-carboxamide,
4-amino-7-fluoro-N,3-dimethyl-N-((5R)-2-(trifluoromethyl)-5,6,7,8-tetrahydro-5-quinolinyl)-3H-pyrazolo[3,4-c]quinoline-8-carboxamide,
4-amino-7-fluoro-N-((4S)-8-fluoro-7-(trifluoromethyl)-3,4-dihydro-1H-2-benzopyran-4-yl)-N,3-dimethyl-3H-pyrazolo[3,4-c]quinoline-8-carboxamide,
4-amino-N,1-dimethyl-N-((5R)-2-(trifluoromethyl)-5,6,7,9-tetrahydrooxepino[3,4-b]pyridin-5-yl)-1H-pyrazolo[4,3-c]quinoline-8-carboxamide,
4-amino-7-chloro-N-cyclopropyl-N-((3S)-6-(trifluoromethyl)-2,3-dihydro-1-benzofuran-3-yl)-1,3-dihydrofuro[3,4-c]quinoline-8-carboxamide,
4-amino-7-chloro-N-cyclopropyl-N-((3R)-6-(trifluoromethyl)-2,3-dihydro-1-benzofuran-3-yl)-1,3-dihydrofuro[3,4-c]quinoline-8-carboxamide,
4-amino-N-methyl-N-((3R)-6-(trifluoromethyl)-2,3-dihydrofuro[3,2-c]pyridin-3-yl)-1,3-dihydrofuro[3,4-c] quinoline-8-carboxamide,
4-amino-N-methyl-N-((3S)-6-(trifluoromethyl)-2,3-dihydrofuro[3,2-c]pyridin-3-yl)-1,3-dihydrofuro[3,4-c] quinoline-8-carboxamide,
4-amino-N-methyl-N-((3S)-6-(S-methylsulfonimidoyl)-2,3-dihydro-1-benzofuran-3-yl)-1,3-dihydrofuro[3,4-c]quinoline-8-carboxamide,
4-amino-N-((3S)-6-((R)-N,S-dimethylsulfonimidoyl)-2,3-dihydro-1-benzofuran-3-yl)-N-methyl-1,3-dihydrofuro[3,4-c]quinoline-8-carboxamide,
4-amino-N-((3S)-6-((S)-N,S-dimethylsulfonimidoyl)-2,3-dihydro-1-benzofuran-3-yl)-N-methyl-1,3-dihydrofuro[3,4-c]quinoline-8-carboxamide,
4-amino-N-cyclopropyl-N-((3R)-6-(trifluoromethyl)-2,3-dihydro-1-benzofuran-3-yl)-1,3-dihydrofuro[3,4-c]quinoline-8-carboxamide,
4-amino-N-cyclopropyl-N-((3S)-6-(trifluoromethyl)-2,3-dihydro-l-benzofuran-3-yl)-1,3-dihydrofuro[3,4-c] quinoline-8-carboxamide,
4-amino-N-((3R)-6-bromo-2,3-dihydrofuro[3,2-b]pyridin-3-yl)-N-methyl-1,3-dihydrofuro[3,4-c] quinoline-8-carboxamide,
4-amino-N-((3S)-6-bromo-2,3-dihydrofuro[3,2-b]pyridin-3-yl)-N-methyl-1,3-dihydrofuro[3,4-c] quinoline-8-carboxamide,
4-amino-N-ethyl-N-(6-(trifluoromethyl)-1-benzofuran-3-yl)-1,3-dihydrofuro[3,4-c]quinoline-8-carboxamide,
4-amino-N-((3S)-5,6-difluoro-2,3-dihydro-1-benzofuran-3-yl)-N-methyl-1,3-dihydrofuro[3,4-c][1,7]naphthyridine-8-carboxamide,
4-amino-N-((3R)-6-(difluoromethoxy)-2,3-dihydro-1-benzofuran-3-yl)-N-methyl-1,3-dihydrofuro[3,4-c][1,7]naphthyridine-8-carboxamide,
4-amino-N-((3S)-6-(difluoromethoxy)-2,3-dihydro-1-benzofuran-3-yl)-N-methyl-1,3-dihydrofuro[3,4-c][1,7]naphthyridine-8-carboxamide,
4-amino-N-methyl-N-((3R)-6-(trifluoromethyl)-2,3-dihydrofuro[3,2-c]pyridin-3-yl)-1,3-dihydrofuro[3,4-c][1,7]naphthyridine-8-carboxamide,
4-amino-N-((3S)-6-((R)-N,S-dimethylsulfonimidoyl)-2,3-dihydro-1-benzofuran-3-yl)-N-methyl-1,3-dihydrofuro[3,4-c][1,7]naphthyridine-8-carboxamide,
4-amino-N-((3S)-6-((S)-N,S-dimethylsulfonimidoyl)-2,3-dihydro-1-benzofuran-3-yl)-N-methyl-1,3-dihydrofuro[3,4-c][1,7]naphthyridine-8-carboxamide,
4-amino-N-methyl-N-((3S)-6-(1-(trifluoromethyl)-1H-pyrazol-4-yl)-2,3-dihydro-1-benzofuran-3-yl)-1,3-dihydrofuro[3,4-c][1,7]naphthyridine-8-carboxamide,
4-amino-N-((3R)-6-bromo-2,3-dihydrofuro[3,2-b]pyridin-3-yl)-N-methyl-1,3-dihydrofuro[3,4-c][1,7]naphthyridine-8-carboxamide,
4-amino-N-((3S)-6-bromo-2,3-dihydrofuro[3,2-b]pyridin-3-yl)-N-methyl-1,3-dihydrofuro[3,4-c][1,7]naphthyridine-8-carboxamide,
4-amino-N-((3R)-6-(difluoromethoxy)-2,3-dihydro-1-benzofuran-3-yl)-7-fluoro-N-methyl-1,3-dihydrofuro[3,4-c]quinoline-8-carboxamide,
4-amino-N-((3S)-6-(difluoromethoxy)-2,3-dihydro-1-benzofuran-3-yl)-7-fluoro-N-methyl-1,3-dihydrofuro[3,4-c]quinoline-8-carboxamide,
4-amino-7-fluoro-N-methyl-N-((3R)-6-(trifluoromethyl)-2,3-dihydrofuro[3,2-c]pyridin-3-yl)-1,3-dihydrofuro[3,4-c]quinoline-8-carboxamide,
4-amino-7-fluoro-N-methyl-N-((3S)-6-(trifluoromethyl)-2,3-dihydrofuro[3,2-c]pyridin-3-yl)-1,3-dihydrofuro[3,4-c]quinoline-8-carboxamide,
4-amino-7-fluoro-N-methyl-N-((3R)-6-(trifluoromethyl)-2,3-dihydrofuro[3,2-b]pyridin-3-yl)-1,3-dihydrofuro[3,4-c]quinoline-8-carboxamide,
4-amino-7-fluoro-N-methyl-N-((3S)-6-(trifluoromethyl)-2,3-dihydrofuro[3,2-b]pyridin-3-yl)-1,3-dihydrofuro[3,4-c]quinoline-8-carboxamide,
4-amino-7-fluoro-N-methyl-N-((3S)-6-(S-methylsulfonimidoyl)-2,3-dihydro-1-benzofuran-3-yl)-1,3-dihydrofuro[3,4-c]quinoline-8-carboxamide,
4-amino-N-((3S)-6-((R)-N,S-dimethylsulfonimidoyl)-2,3-dihydro-1-benzofuran-3-yl)-7-fluoro-N-methyl-1,3-dihydrofuro[3,4-c]quinoline-8-carboxamide,
4-amino-N-((3S)-6-((S)-N,S-dimethylsulfonimidoyl)-2,3-dihydro-1-benzofuran-3-yl)-7-fluoro-N-methyl-1,3-dihydrofuro[3,4-c]quinoline-8-carboxamide,
4-amino-7-fluoro-N-methyl-N-((3R)-6-(2-propanylsulfonyl)-2,3-dihydro-1-benzofuran-3-yl)-1,3-dihydrofuro[3,4-c]quinoline-8-carboxamide,
4-amino-7-fluoro-N-methyl-N-((3S)-6-(2-propanylsulfonyl)-2,3-dihydro-1-benzofuran-3-yl)-1,3-dihydrofuro[3,4-c]quinoline-8-carboxamide,
4-amino-N-((3R)-6-bromo-2,3-dihydrofuro[3,2-b]pyridin-3-yl)-7-fluoro-N-methyl-1,3-dihydrofuro[3,4-c] quinoline-8-carboxamide,
4-amino-N-((3S)-6-bromo-2,3-dihydrofuro[3,2-b]pyridin-3-yl)-7-fluoro-N-methyl-1,3-dihydrofuro[3,4-c] quinoline-8-carboxamide,
(3R)-4-amino-7-fluoro-N,3-dimethyl-N-((3S)-6-(trifluoromethyl)-2,3-dihydro-1-benzofuran-3-yl)-1,3-dihydrofuro[3,4-c]quinoline-8-carboxamide,
(3R)-4-amino-N-cyclopropyl-7-fluoro-3-methyl-N-((3S)-6-(trifluoromethyl)-2,3-dihydro-1-benzofuran-3-yl)-1,3-dihydrofuro[3,4-c]quinoline-8-carboxamide,
(3R)-4-amino-7-fluoro-3-methyl-N-(2-propanyl)-N-((3S)-6-(trifluoromethyl)-2,3-dihydro-1-benzofuran-3-yl)-1,3-dihydrofuro[3,4-c]quinoline-8-carboxamide,
(3R)-4-amino-7-fluoro-3-methyl-N-(2-propanyl)-N-((3R)-6-(trifluoromethyl)-2,3-dihydro-1-benzofuran-3-yl)-1,3-dihydrofuro[3,4-c]quinoline-8-carboxamide,
(3R)-4-amino-N-cyclobutyl-7-fluoro-3-methyl-N-((3S)-6-(trifluoromethyl)-2,3-dihydro-1-benzofuran-3-yl)-1,3-dihydrofuro[3,4-c]quinoline-8-carboxamide,
(3R)-4-amino-N-cyclobutyl-7-fluoro-3-methyl-N-((3R)-6-(trifluoromethyl)-2,3-dihydro-1-benzofuran-3-yl)-1,3-dihydrofuro[3,4-c]quinoline-8-carboxamide,
4-amino-N,1-dimethyl-N-((3S)-6-(trifluoromethyl)-2,3-dihydro-1-benzofuran-3-yl)-1H-pyrazolo[4,3-c] quinoline-8-carboxamide,
4-amino-N-((3 S)-5,6-dichloro-2,3-dihydro-1-benzofuran-3-yl)-N,1-dimethyl-1H-pyrazolo [4,3-c][1,7]naphthyridine-8-carboxamide,
4-amino-N-((3 S)-5,6-difluoro-2,3-dihydro-1-benzofuran-3-yl)-7-fluoro-N,1-dimethyl-1H-pyrazolo [4,3-c] quinoline-8-carboxamide,
4-amino-N-((3S)-5,6-dichloro-2,3-dihydro-1-benzofuran-3-yl)-7-fluoro-N,1-dimethyl-1H-pyrazolo[4,3-c] quinoline-8-carboxamide,
4-amino-N-((3R)-5,6-dichloro-2,3-dihydro-1-benzofuran-3-yl)-7-fluoro-N,1-dimethyl-1H-pyrazolo[4,3-c] quinoline-8-carboxamide,
4-amino-7-fluoro-N,1-dimethyl-N-((3S)-6-(trifluoromethoxy)-2,3-dihydro-1-benzofuran-3-yl)-1H-pyrazolo[4,3-c]quinoline-8-carboxamide,
4-amino-N-((3R)-6-bromo-2,3-dihydro-1-benzothiophen-3-yl)-7-fluoro-N,1-dimethyl-1H-pyrazolo[4,3-c] quinoline-8-carboxamide,
4-amino-N-((3S)-6-bromo-2,3-dihydro-1-benzothiophen-3-yl)-7-fluoro-N, 1-dimethyl-1H-pyrazolo[4,3-c] quinoline-8-carboxamide,
4-amino-7-chloro-N,1-dimethyl-N-((3S)-6-(trifluoromethyl)-2,3-dihydro-1-benzofuran-3-yl)-1H-pyrazolo[4,3-c]quinoline-8-carboxamide,
4-amino-7-chloro-N,1-dimethyl-N-((3S)-6-(trifluoromethoxy)-2,3-dihydro-1-benzofuran-3-yl)-1H-pyrazolo[4,3-c]quinoline-8-carboxamide,
4-amino-N-((3R)-6-cyano-2,3-dihydro-1-benzofuran-3-yl)-7-fluoro-N,3-dimethyl-3H-pyrazolo[3,4-c] quinoline-8-carboxamide,
4-amino-N-((3S)-6-cyano-2,3-dihydro-1-benzofuran-3-yl)-7-fluoro-N,3-dimethyl-3H-pyrazolo[3,4-c] quinoline-8-carboxamide,
4-amino-N-((3S)-5,6-dichloro-2,3-dihydro-1-benzofuran-3-yl)-7-fluoro-N,3-dimethyl-3H-pyrazolo[3,4-c] quinoline-8-carboxamide,
4-amino-N-((3R)-6-bromo-2,3-dihydro-1-benzothiophen-3-yl)-7-fluoro-N,3-dimethyl-3H-pyrazolo[3,4-c] quinoline-8-carboxamide,
4-amino-N-((3S)-6-bromo-2,3-dihydro-1-benzothiophen-3-yl)-7-fluoro-N,3-dimethyl-3H-pyrazolo[3,4-c] quinoline-8-carboxamide,
4-amino-7-chloro-N,3-dimethyl-N-((5R)-2-(trifluoromethyl)-6,7-dihydro-5H-cyclopenta[b]pyridin-5-yl)-3H-pyrazolo[3,4-c]quinoline-8-carboxamide,
4-amino-7-chloro-N,3-dimethyl-N-((5S)-2-(trifluoromethyl)-6,7-dihydro-5H-cyclopenta[b]pyridin-5-yl)-3H-pyrazolo[3,4-c]quinoline-8-carboxamide,
4-amino-7-chloro-N,3-dimethyl-N-((3 S)-6-(trifluoromethyl)-2,3-dihydro-1-benzofuran-3-yl)-3H-pyrazolo[3,4-c]quinoline-8-carboxamide,
4-amino-7-chloro-N,3-dimethyl-N-((3S)-6-(trifluoromethoxy)-2,3-dihydro-1-benzofuran-3-yl)-3H-pyrazolo[3,4-c]quinoline-8-carboxamide,
(3R)-4-amino-N,3-dimethyl-N-((5S)-2-(trifluoromethyl)-5,8-dihydro-6H-pyrano[3,4-b]pyridin-5-yl)-1,3-dihydrofuro[3,4-c]quinoline-8-carboxamide,
4-amino-N-((4S)-7-cyano-3,4-dihydro-1H-2-benzopyran-4-yl)-N-methyl-1,3-dihydrofuro[3,4-c][1,7]naphthyridine-8-carboxamide,
4-amino-N-methyl-N-((4S)-7-(trifluoromethyl)-3,4-dihydro-1H-2-benzopyran-4-yl)-1,3-dihydrofuro[3,4-c][1,7]naphthyridine-8-carboxamide,
4-amino-N-methyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamide,
4-amino-N-methyl-N-((4S)-7-(trifluoromethyl)-3,4-dihydro-1H-pyrano[4,3-c]pyridin-4-yl)-1,3-dihydrofuro[3,4-c][1,7]naphthyridine-8-carboxamide,
4-amino-N-methyl-N-((4S)-7-(trifluoromethyl)-3,4-dihydro-1H-2-benzopyran-4-yl)-1,3-dihydrofuro[3,4-c] [1,8]naphthyridine-8-carboxamide,
4-amino-7-fluoro-N-methyl-N-((5S)-2-(trifluoromethyl)-5,8-dihydro-6H-pyrano[3,4-b]pyridin-5-yl)-1,3-dihydrofuro[3,4-c]quinoline-8-carboxamide,
(3R)-4-amino-7-fluoro-N,3-dimethyl-N-((5S)-2-(trifluoromethyl)-5,8-dihydro-6H-pyrano[3,4-b]pyridin-5-yl)-1,3-dihydrofuro[3,4-c]quinoline-8-carboxamide,
4-amino-7-chloro-N-((4S)-7-cyano-3,4-dihydro-1H-2-benzopyran-4-yl)-N-methyl-1,3-dihydrofuro[3,4-c] quinoline-8-carboxamide,
4-amino-N-((4S)-7-cyano-3,4-dihydro-1H-2-benzopyran-4-yl)-N,3-dimethyl[1,2]oxazolo[4,5-c] quinoline-8-carboxamide,
4-amino-N,3-dimethyl-N-((4S)-7-(trifluoromethyl)-3,4-dihydro-1H-2-benzopyran-4-yl)[1,2]oxazolo[4,5-c] quinoline-8-carboxamide,
4-amino-N,1-dimethyl-N-((4S)-7-(trifluoromethyl)-3,4-dihydro-1H-2-benzopyran-4-yl)-1H-pyrazolo[4,3-c] quinoline-8-carboxamide,
4-amino-N,1-dimethyl-N-((4S)-7-(trifluoromethyl)-3,4-dihydro-1H-pyrano[4,3-c]pyridin-4-yl)-1H-pyrazolo[4,3-c]quinoline-8-carboxamide,
4-amino-N-ethyl-1-methyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N-ethyl-1-methyl-N-((5R)-2-(trifluoromethyl)-5,8-dihydro-6H-pyrano|[ 3,4-b|pyridin-5-y1)-1H-pyrazolo[4,3-c]quinoline-8-carboxamide,
4-amino-N-cyclopropyl-1-methyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-1-methyl-N-(2-methylpropyl)-N-((5 S)-2-(trifluoromethyl)-5,8-dihydro-6H-pyrano[3,4-b]pyridin-5-yl)-1H-pyrazolo[4,3-c]quinoline-8-carboxamide,
4-amino-1-methyl-N-(2-methylpropyl)-N-((5R)-2-(trifluoromethyl)-5,8-dihydro-6H-pyrano[3,4-b]pyridin-5-yl)-1H-pyrazolo[4,3-c]quinoline-8-carboxamide,
4-amino-7-fluoro-N, 1-dimethyl-N-((4S)-7-(trifluoromethyl)-3,4-dihydro-1H-2-benzopyran-4-yl)-1H-pyrazolo[4,3-c]quinoline-8-carboxamide,
4-amino-7-fluoro-N,1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N-ethyl-7-fluoro-1-methyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N-((4S)-7-cyano-3,4-dihydro-1H-2-benzopyran-4-yl)-N,3-dimethyl-3H-pyrazolo[3,4-c] quinoline-8-carboxamide,
4-amino-N,3-dimethyl-N-((4S)-7-(trifluoromethyl)-3,4-dihydro-1H-2-benzopyran-4-yl)-3H-pyrazolo[3,4-c] quinoline-8-carboxamide,
4-amino-N,3-dimethyl-N-((4S)-7-(trifluoromethyl)-3,4-dihydro-1H-pyrano[4,3-c]pyridin-4-yl)-3H-pyrazolo[3,4-c]quinoline-8-carboxamide,
4-amino-N,3-dimethyl-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-7-fluoro-N-((5S)-2-methoxy-5,8-dihydro-6H-pyrano[3,4-b]pyridin-5-yl)-N,3-dimethyl-3H-pyrazolo[3,4-c]quinoline-8-carboxamide,
4-amino-7-fluoro-N,3-dimethyl-N-((4S)-7-(trifluoromethyl)-3,4-dihydro-1H-2-benzopyran-4-yl)-3H-pyrazolo[3,4-c]quinoline-8-carboxamide,
4-amino-7-fluoro-N,3-dimethyl-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-N-cyclopropyl-7-fluoro-3-methyl-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-7-fluoro-3-methyl-N-(2-methylpropyl)-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-7-fluoro-3-methyl-N-(2-methylpropyl)-N-((5R)-2-(trifluoromethyl)-5,8-dihydro-6H-pyrano[3,4-b]pyridin-5-yl)-3H-pyrazolo[3,4-c]quinoline-8-carboxamide,
4-amino-7-chloro-N,3-dimethyl-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-N,3,7-trimethyl-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-N-((4S)-7-cyano-3,4-dihydro-1H-2-benzopyran-4-yl)-N,1,3-trimethyl-1H-pyrazolo[4,3-c] [1,7]naphthyridine-8-carboxamide,
4-amino-N-methyl-N-((3 S)-6-(trifluoromethyl)-2,3-dihydro-1-benzofuran-3-yl)-1,3-dihydrofuro [3,4-c] [1,7]naphthyridine-8-carboxamide,
4-amino-N-methyl-N-((3R)-6-(trifluoromethyl)-2,3-dihydro-1-benzofuran-3-yl)-1,3-dihydrofuro[3,4-c] [1,7]naphthyridine-8-carboxamide,
4-amino-N-((3S)-6-methoxy-2,3-dihydrofuro[2,3-b]pyridin-3-yl)-N-methyl-1,3-dihydrofuro[3,4-c] quinoline-8-carboxamide,
4-amino-N-((3R)-6-methoxy-2,3-dihydrofuro[2,3-b]pyridin-3-yl)-N-methyl-1,3-dihydrofuro[3,4-c] quinoline-8-carboxamide,
4-amino-N-((3S)-6-(difluoromethoxy)-2,3-dihydro-1-benzofuran-3-yl)-N-methyl-1,3-dihydrofuro[3,4-c] quinoline-8-carboxamide,
4-amino-N-methyl-N-((5R)-2-(trifluoromethyl)-6,7-dihydro-5H-cyclopenta[b]pyridin-5-yl)-1,3-dihydrofuro[3,4-c]quinoline-8-carboxamide,
4-amino-N-methyl-N-((5S)-2-(trifluoromethyl)-6,7-dihydro-5H-cyclopenta[b]pyridin-5-yl)-1,3-dihydrofuro[3,4-c]quinoline-8-carboxamide,
4-amino-N-methyl-N-((3S)-6-(trifluoromethyl)-2,3-dihydrofuro[2,3-b]pyridin-3-yl)-1,3-dihydrofuro[3,4-c] quinoline-8-carboxamide,
4-amino-N-methyl-N-((3R)-6-(trifluoromethyl)-2,3-dihydrofuro[2,3-b]pyridin-3-yl)-1,3-dihydrofuro[3,4-c] quinoline-8-carboxamide,
4-amino-N-methyl-N-((1R)-5-(trifluoromethoxy)-2,3-dihydro-1H-inden-1-yl)-1,3-dihydrofuro[3,4-c] quinoline-8-carboxamide,
4-amino-N-methyl-N-((1S)-5-(trifluoromethoxy)-2,3-dihydro-1H-inden-1-yl)-1,3-dihydrofuro[3,4-c] quinoline-8-carboxamide,
4-amino-N-ethyl-N-((3S)-6-(trifluoromethyl)-2,3-dihydro-1-benzofuran-3-yl)-1,3-dihydrofuro[3,4-c] quinoline-8-carboxamide,
4-amino-N-ethyl-N-((3R)-6-(trifluoromethyl)-2,3-dihydro-1-benzofuran-3-yl)-1,3-dihydrofuro[3,4-c] quinoline-8-carboxamide,
(4S,6R)-4-(3-chloro-5-fluorophenyl)-1-(2-hydroxyethyl)-6-(3-methylphenyl)-2-piperidinone,
N-(6,8-dichloro-2-(3-chloro-4-(1,2,4-oxadiazol-5-yl)phenyl)imidazo[1,2-b]pyridazin-3-yl)-2,2,2-trifluoroacetamide,
(3R)-4-amino-N,3-dimethyl-N-((3S)-6-(trifluoromethyl)-2,3-dihydro-1-benzofuran-3-yl)-1,3-dihydrofuro[3,4-c]quinoline-8-carboxamide,
(3R)-4-amino-N,3-dimethyl-N-((3R)-6-(trifluoromethyl)-2,3-dihydro-1-benzofuran-3-yl)-1,3-dihydrofuro[3,4-c]quinoline-8-carboxamide,
(3R)-4-amino-N-ethyl-3-methyl-N-((3S)-6-(trifluoromethyl)-2,3-dihydro-1-benzofuran-3-yl)-1,3-dihydrofuro[3,4-c]quinoline-8-carboxamide,
(3R)-4-amino-N-ethyl-3-methyl-N-((3R)-6-(trifluoromethyl)-2,3-dihydro-1-benzofuran-3-yl)-1,3-dihydrofuro[3,4-c]quinoline-8-carboxamide,
4-amino-N-((3S)-6-cyano-2,3-dihydro-1-benzofuran-3-yl)-N-methyl-1,3-dihydrofuro[3,4-c] [1,7]naphthyridine-8-carboxamide,
4-amino-N-methyl-N-((3S)-6-(trifluoromethyl)-2,3-dihydrofuro[3,2-c]pyridin-3-yl)-1,3-dihydrofuro[3,4-c] [1,7]naphthyridine-8-carboxamide,
4-amino-N-ethyl-N-((3S)-6-(trifluoromethyl)-2,3-dihydro-1-benzofuran-3-yl)-1,3-dihydrofuro[3,4-c] [1,7]naphthyridine-8-carboxamide,
4-amino-N-ethyl-N-((3R)-6-(trifluoromethyl)-2,3-dihydro-1-benzofuran-3-yl)-1,3-dihydrofuro[3,4-c] [1,7]naphthyridine-8-carboxamide,
4-amino-N-((3S)-4-fluoro-6-(trifluoromethyl)-2,3-dihydro-1-benzofuran-3-yl)-N-methyl-1,3-dihydrofuro[3,4-c][1,7]naphthyridine-8-carboxamide,
4-amino-N-((3R)-4-fluoro-6-(trifluoromethyl)-2,3-dihydro-1-benzofuran-3-yl)-N-methyl-1,3-dihydrofuro[3,4-c][1,7]naphthyridine-8-carboxamide,
4-amino-N-cyclopropyl-N-((3S)-6-(trifluoromethyl)-2,3-dihydro-1-benzofuran-3-yl)-1,3-dihydrofuro[3,4-c] [1,7]naphthyridine-8-carboxamide,
4-amino-N-((3S)-6-chloro-5-(trifluoromethyl)-2,3-dihydro-1-benzofuran-3-yl)-N-methyl-1,3-dihydrofuro[3,4-c][1,7]naphthyridine-8-carboxamide,
4-amino-N-methyl-N-((3S)-6-(trifluoromethyl)-2,3-dihydro-1-benzofuran-3-yl)-1,3-dihydrofuro[3,4-c] [1,8]naphthyridine-8-carboxamide,
4-amino-N-methyl-N-((3R)-6-(trifluoromethyl)-2,3-dihydro-1-benzofuran-3-yl)-1,3-dihydrofuro[3,4-c] [1,8]naphthyridine-8-carboxamide,
4-amino-N-((3S)-6-cyano-2,3-dihydro-1-benzofuran-3-yl)-7-fluoro-N-methyl-1,3-dihydrofuro[3,4-c] quinoline-8-carboxamide,
4-amino-7-fluoro-N-methyl-N-((3 S)-6-(trifluoromethyl)-2,3-dihydro-1-benzofuran-3-yl)-1,3-dihydrofuro[3,4-c]quinoline-8-carboxamide,
4-amino-7-fluoro-N-methyl-N-((3R)-6-(trifluoromethyl)-2,3-dihydro-1-benzofuran-3-yl)-1,3-dihydrofuro[3,4-c]quinoline-8-carboxamide,
4-amino-N-cyclopropyl-7-fluoro-N-((3S)-6-(trifluoromethyl)-2,3-dihydro-1-benzofuran-3-yl)-1,3-dihydrofuro[3,4-c]quinoline-8-carboxamide,
4-amino-N-cyclopropyl-7-fluoro-N-((3R)-6-(trifluoromethyl)-2,3-dihydro-1-benzofuran-3-yl)-1,3-dihydrofuro[3,4-c]quinoline-8-carboxamide,
4-amino-N-(cyclopropylmethyl)-7-fluoro-N-((3S)-6-(trifluoromethyl)-2,3-dihydro-1-benzofuran-3-yl)-1,3-dihydrofuro[3,4-c]quinoline-8-carboxamide,
4-amino-N-(cyclopropylmethyl)-7-fluoro-N-((3R)-6-(trifluoromethyl)-2,3-dihydro-1-benzofuran-3-yl)-1,3-dihydrofuro[3,4-c]quinoline-8-carboxamide,
4-amino-7-chloro-N-methyl-N-((5R)-2-(trifluoromethyl)-6,7-dihydro-5H-cyclopenta[b]pyridin-5-yl)-1,3-dihydrofuro[3,4-c]quinoline-8-carboxamide,
4-amino-7-chloro-N-methyl-N-((5S)-2-(trifluoromethyl)-6,7-dihydro-5H-cyclopenta[b]pyridin-5-yl)-1,3-dihydrofuro[3,4-c]quinoline-8-carboxamide,
4-amino-7-chloro-N-methyl-N-((3 S)-6-(trifluoromethyl)-2,3-dihydro-1-benzofuran-3-yl)-1,3-dihydrofuro[3,4-c]quinoline-8-carboxamide,
4-amino-7-chloro-N-methyl-N-((3R)-6-(trifluoromethyl)-2,3-dihydro-1-benzofuran-3-yl)-1,3-dihydrofuro[3,4-c]quinoline-8-carboxamide,
4-amino-7-chloro-N-methyl-N-((3S)-6-(trifluoromethyl)-2,3-dihydrofuro[2,3-b]pyridin-3-yl)-1,3-dihydrofuro[3,4-c]quinoline-8-carboxamide,
4-amino-7-chloro-N-methyl-N-((3R)-6-(trifluoromethyl)-2,3-dihydrofuro[2,3-b]pyridin-3-yl)-1,3-dihydrofuro[3,4-c]quinoline-8-carboxamide,
4-amino-7-chloro-N-methyl-N-((3S)-6-(methylsulfonyl)-2,3-dihydro-1-benzofuran-3-yl)-1,3-dihydrofuro[3,4-c]quinoline-8-carboxamide,
4-amino-7-chloro-N-methyl-N-((3R)-6-(methylsulfonyl)-2,3-dihydro-1-benzofuran-3-yl)-1,3-dihydrofuro[3,4-c]quinoline-8-carboxamide,
4-amino-7-chloro-N-((3S)-4-fluoro-6-(trifluoromethyl)-2,3-dihydro-1-benzofuran-3-yl)-N-methyl-1,3-dihydrofuro[3,4-c]quinoline-8-carboxamide,
4-amino-7-chloro-N-((3R)-4-fluoro-6-(trifluoromethyl)-2,3-dihydro-1-benzofuran-3-yl)-N-methyl-1,3-dihydrofuro[3,4-c]quinoline-8-carboxamide,
4-amino-N,1-dimethyl-N-((3S)-6-(trifluoromethyl)-2,3-dihydrofuro[3,2-c]pyridin-3-yl)-1H-pyrazolo[4,3-c] [1,7]naphthyridine-8-carboxamide,
4-amino-N-((3S)-6-(difluoromethoxy)-2,3-dihydro-1-benzofuran-3-yl)-7-fluoro-N,1-dimethyl-1H-pyrazolo[4,3-c]quinoline-8-carboxamide,
4-amino-N-((3R)-6-(difluoromethoxy)-2,3-dihydro-1-benzofuran-3-yl)-7-fluoro-N,1-dimethyl-1H-pyrazolo[4,3-c]quinoline-8-carboxamide,
4-amino-7-fluoro-N,1-dimethyl-N-((3S)-6-(trifluoromethyl)-2,3-dihydro-1-benzofuran-3-yl)-1H-pyrazolo[4,3-c]quinoline-8-carboxamide,
4-amino-7-fluoro-N, 1-dimethyl-N-((3R)-6-(trifluoromethyl)-2,3-dihydro-1-benzofuran-3-yl)-1H-pyrazolo[4,3-c]quinoline-8-carboxamide,
4-amino-7-fluoro-N,1-dimethyl-N-((3S)-6-(trifluoromethyl)-2,3-dihydrofuro[3,2-c]pyridin-3-yl)-1H-pyrazolo[4,3-c]quinoline-8-carboxamide,
4-amino-7-fluoro-N,1-dimethyl-N-((3R)-6-(trifluoromethyl)-2,3-dihydrofuro[3,2-c]pyridin-3-yl)-1H-pyrazolo[4,3-c]quinoline-8-carboxamide,
4-amino-N,3-dimethyl-N-((3S)-6-(trifluoromethyl)-2,3-dihydro-1-benzofuran-3-yl)-3H-pyrazolo[3,4-c] quinoline-8-carboxamide,
4-amino-N,3-dimethyl-N-((3R)-6-(trifluoromethyl)-2,3-dihydro-1-benzofuran-3-yl)-3H-pyrazolo[3,4-c] quinoline-8-carboxamide,
4-amino-N-((3S)-6-(difluoromethoxy)-2,3-dihydro-1-benzofuran-3-yl)-7-fluoro-N,3-dimethyl-3H-pyrazolo[3,4-c]quinoline-8-carboxamide,
4-amino-N-methyl-N-((4R)-7-(trifluoromethyl)-3,4-dihydro-2H-chromen-4-yl)-1,3-dihydrofuro[3,4-c] quinoline-8-carboxamide,
4-amino-N-methyl-N-((4S)-7-(trifluoromethyl)-3,4-dihydro-2H-chromen-4-yl)-1,3-dihydrofuro[3,4-c] quinoline-8-carboxamide,
4-amino-N-((4S)-7-bromo-3,4-dihydro-1H-2-benzopyran-4-yl)-N-ethyl-1,3-dihydrofuro[3,4-c]quinoline-8-carboxamide,
4-amino-N-((4R)-7-bromo-3,4-dihydro-1H-2-benzopyran-4-yl)-N-ethyl-1,3-dihydrofuro[3,4-c]quinoline-8-carboxamide,
4-amino-N-((4S)-7-methoxy-3,4-dihydro-1H-2-benzopyran-4-yl)-N-methyl-1,3-dihydrofuro[3,4-c] [1,7]naphthyridine-8-carboxamide,
4-amino-N-methyl-N-((4S)-6-(trifluoromethyl)-3,4-dihydro-1H-2-benzopyran-4-yl)-1,3-dihydrofuro[3,4-c] [1,7]naphthyridine-8-carboxamide,
4-amino-N-methyl-N-((4R)-6-(trifluoromethyl)-3,4-dihydro-1H-2-benzopyran-4-yl)-1,3-dihydrofuro[3,4-c] [1,7]naphthyridine-8-carboxamide,
4-amino-N-methyl-N-((4R)-7-(trifluoromethyl)-3,4-dihydro-2H-chromen-4-yl)-1, 3 -dihydrofuro [3, 4-c] [1,7]naphthyridine-8-carboxamide,
4-amino-N-methyl-N-((4S)-7-(trifluoromethyl)-3,4-dihydro-2H-chromen-4-yl)-1,3-dihydrofuro[3,4-c] [1,7]naphthyridine-8-carboxamide,
4-amino-N-((4S)-7-bromo-3,4-dihydro-1H-2-benzopyran-4-yl)-N-ethyl-1,3-dihydrofuro|3,4-c] [1,7]naphthyridine-8-carboxamide,
4-amino-N-((4R)-7-bromo-3,4-dihydro-1H-2-benzopyran-4-yl)-N-ethyl-1,3-dihydrofuro[3,4-c] [1,7]naphthyridine-8-carboxamide,
4-amino-N-((4R)-7-bromo-3,4-dihydro-1H-2-benzopyran-4-yl)-N-methyl-1,3-dihydrofuro[3,4-c] [1,8]naphthyridine-8-carboxamide,
4-amino-N-((4S)-7-bromo-3,4-dihydro-1H-2-benzopyran-4-yl)-N-methyl-1,3-dihydrofuro[3,4-c] [1,8]naphthyridine-8-carboxamide,
4-amino-7-fluoro-N-methyl-N-((4S)-7-(methylsulfonyl)-3,4-dihydro-1H-2-benzopyran-4-yl)-1,3-dihydrofuro[3,4-c]quinoline-8-carboxamide,
(3R)-4-amino-7-fluoro-N,3-dimethyl-N-((4S)-7-(methylsulfonyl)-3,4-dihydro-1H-2-benzopyran-4-yl)-1,3-dihydrofuro[3,4-c]quinoline-8-carboxamide,
4-amino-N-(cyclopropylmethyl)-1-methyl-N-((5 S)-2-(trifluoromethyl)-5,8-dihydro-6H-pyrano [3,4-b]pyridin-5-yl)-1H-pyrazolo[4,3-c]quinoline-8-carboxamide,
4-amino-N-(cyclopropylmethyl)-1-methyl-N-((5R)-2-(trifluoromethyl)-5,8-dihydro-6H-pyrano[3,4-b]pyridin-5-yl)-1H-pyrazolo[4,3-c]quinoline-8-carboxamide,
4-amino-N-((4S)-7-cyano-3,4-dihydro-1H-2-benzopyran-4-yl)-7-fluoro-N,1-dimethyl-1H-pyrazolo[4,3-c] quinoline-8-carboxamide,
4-amino-N-((4R)-7-cyano-3,4-dihydro-1H-2-benzopyran-4-yl)-7-fluoro-N, 1 -dimethyl-1H-pyrazolo[4,3-c] quinoline-8-carboxamide,
4-amino-N-((4S)-7,8-difluoro-3,4-dihydro-1H-2-benzopyran-4-yl)-7-fluoro-N,1-dimethyl-1H-pyrazolo[4,3-c]quinoline-8-carboxamide,
4-amino-N-((4R)-7,8-difluoro-3,4-dihydro-1H-2-benzopyran-4-yl)-7-fluoro-N,1-dimethyl-1H-pyrazolo[4,3-c]quinoline-8-carboxamide,
4-amino-7-fluoro-N,1-dimethyl-N-((8S)-3-(trifluoromethyl)-7,8-dihydro-5H-pyrano[4,3-b]pyridin-8-yl)-1H-pyrazolo[4,3-c]quinoline-8-carboxamide,
4-amino-7-fluoro-N,1-dimethyl-N-((4S)-7-(methylsulfonyl)-3,4-dihydro-1H-2-benzopyran-4-yl)-1H-pyrazolo[4,3-c]quinoline-8-carboxamide,
4-amino-N,3-dimethyl-N-((1R,4S)-1-methyl-7-(trifluoromethyl)-3,4-dihydro-1H-2-benzopyran-4-yl)-3H-pyrazolo[3,4-c]quinoline-8-carboxamide,
4-amino-N,3-dimethyl-N-((1R,4R)-1-methyl-7-(trifluoromethyl)-3,4-dihydro-1H-2-benzopyran-4-yl)-3H-pyrazolo[3,4-c]quinoline-8-carboxamide,
4-amino-N,3-dimethyl-N-((1S,4S)-1-methyl-7-(trifluoromethyl)-3,4-dihydro-1H-2-benzopyran-4-yl)-3H-pyrazolo[3,4-c]quinoline-8-carboxamide,
4-amino-N,3-dimethyl-N-((1S,4R)-1-methyl-7-(trifluoromethyl)-3,4-dihydro-1H-2-benzopyran-4-yl)-3H-pyrazolo[3,4-c]quinoline-8-carboxamide,
4-amino-N-((4S)-7,8-difluoro-3,4-dihydro-1H-2-benzopyran-4-yl)-7-fluoro-N,3-dimethyl-3H-pyrazolo[3,4-c]quinoline-8-carboxamide,
4-amino-7-fluoro-N,3-dimethyl-N-((8S)-3-(trifluoromethyl)-7,8-dihydro-5H-pyrano[4,3-b]pyridin-8-yl)-3H-pyrazolo[3,4-c]quinoline-8-carboxamide,
4-amino-N-(cyclopropylmethyl)-7-fluoro-3-methyl-N-((5S)-2-(trifluoromethyl)-5,8-dihydro-6H-pyrano[3,4-b]pyridin-5-yl)-3H-pyrazolo[3,4-c]quinoline-8-carboxamide,
4-amino-N-((4S)-7-bromo-3,4-dihydro-1H-2-benzopyran-4-yl)-N-methyl-1,3-dihydrofuro[3,4-c] quinoline-8-carboxamide,
4-amino-N-((4R)-7-bromo-3,4-dihydro-1H-2-benzopyran-4-yl)-N-methyl-1,3-dihydrofuro[3,4-c] quinoline-8-carboxamide,
4-amino-N-((3 S)-6-cyclopropyl-2,3-dihydro-1-benzofuran-3-yl)-N-methyl-1,3-dihydrofuro [3,4-c] quinoline-8-carboxamide,
4-amino-N-((3R)-6-cyclopropyl-2,3-dihydro-1-benzofuran-3-yl)-N-methyl-1,3-dihydrofuro[3,4-c] quinoline-8-carboxamide,
4-amino-N-((3S)-6-(3,6-dihydro-2H-pyran-4-yl)-2,3-dihydro-1-benzofuran-3-yl)-N-methyl-1,3-dihydrofuro[3,4-c]quinoline-8-carboxamide,
4-amino-N-((3R)-6-(3,6-dihydro-2H-pyran-4-yl)-2,3-dihydro-1-benzofuran-3-yl)-N-methyl-1,3-dihydrofuro[3,4-c]quinoline-8-carboxamide,
2-methyl-2-propanyl 4-((3R)-3-(((4-amino-1,3-dihydrofuro[3,4-c]quinolin-8-yl)carbonyl)(methyl)amino)-2,3-dihydro-1-benzofuran-6-yl)-3,6-dihydro-1(2H)-pyridinecarboxylate,
2-methyl-2-propanyl 4-((3 S)-3-(((4-amino-1,3-dihydrofuro[3,4-c]quinolin-8-yl)carbonyl)(methyl)amino)-2,3-dihydro-1-benzofuran-6-yl)-3,6-dihydro-1(2H)-pyridinecarboxylate,
4-amino-N-((3S)-6-cyclopropyl-2,3-dihydro-1-benzofuran-3-yl)-N,1-dimethyl-1H-pyrazolo[4,3-c] quinoline-8-carboxamide,
4-amino-N,1-dimethyl-N-((3S)-6-(1-methyl-1H-pyrrol-3-yl)-2,3-dihydro-1-benzofuran-3-yl)-1H-pyrazolo[4,3-c]quinoline-8-carboxamide,
4-amino-N-((3 S)-6-(1-cyclohexen-1-yl)-2,3-dihydro-1-benzofuran-3-yl)-N,1-dimethyl-1H-pyrazolo [4,3-c] quinoline-8-carboxamide,
4-amino-N-((3S)-6-(3,6-dihydro-2H-pyran-4-yl)-2,3-dihydro-1-benzofuran-3-yl)-N, 1-dimethyl-1H-pyrazolo[4,3-c]quinoline-8-carboxamide,
4-amino-N-((3 S)-6-(4,4-difluoro-1-cyclohexen-1-yl)-2,3-dihydro-1-benzofuran-3-yl)-N,1-dimethyl-1H-pyrazolo[4,3-c]quinoline-8-carboxamide,
4-amino-N,1-dimethyl-N-((3S)-6-(1-(trifluoromethyl)-1H-pyrazol-4-yl)-2,3-dihydro-1-benzofuran-3-yl)-1H-pyrazolo[4,3-c]quinoline-8-carboxamide,
(3R)-4-amino-N-((4S)-7-cyclopropyl-3,4-dihydro-1H-2-benzopyran-4-yl)-N-ethyl-3-methyl-1,3-dihydrofuro[3,4-c]quinoline-8-carboxamide,
4-amino-N,1-dimethyl-N-((3S)-6-(1H-pyrazol-4-yl)-2,3-dihydro-1-benzofuran-3-yl)-1H-pyrazolo[4,3-c] quinoline-8-carboxamide,
4-amino-N,1-dimethyl-N-((1R)-5-(1-methyl-1H-pyrazol-4-yl)-2,3-dihydro-1H-inden-1-yl)-1H-pyrazolo[4,3-c]quinoline-8-carboxamide,
4-amino-N,1-dimethyl-N-((5S)-2-(1-methyl-1H-pyrazol-4-yl)-6,7-dihydro-5H-cyclopenta[b]pyridin-5-yl)-1H-pyrazolo[4,3-c]quinoline-8-carboxamide,
4-amino-N,1-dimethyl-N-((3S)-6-(3-oxetanyl)-2,3-dihydro-1-benzofuran-3-yl)-1H-pyrazolo[4,3-c] quinoline-8-carboxamide,
4-amino-N-((3S)-6-(3-furanyl)-2,3-dihydro-1-benzofuran-3-yl)-N,1-dimethyl-1H-pyrazolo[4,3-c] quinoline-8-carboxamide,
4-amino-N,1-dimethyl-N-((3S)-6-(4-pyridinyl)-2,3-dihydro-1-benzofuran-3-yl)-1H-pyrazolo[4,3-c] quinoline-8-carboxamide,
4-amino-N,1-dimethyl-N-((3S)-6-(3-pyridinyl)-2,3-dihydro-1-benzofuran-3-yl)-1H-pyrazolo[4,3-c] quinoline-8-carboxamide,
4-amino-N,1-dimethyl-N-((3S)-6-(1-methyl-1H-pyrazol-4-yl)-2,3-dihydro-1-benzofuran-3-yl)-1H-pyrazolo[4,3-c]quinoline-8-carboxamide,
4-amino-N,1-dimethyl-N-((3S)-6-(1-methyl-1H-pyrazol-5-yl)-2,3-dihydro-1-benzofuran-3-yl)-1H-pyrazolo[4,3-c]quinoline-8-carboxamide,
4-amino-N,1-dimethyl-N-((3S)-6-(1-methyl-1H-pyrazol-3-yl)-2,3-dihydro-1-benzofuran-3-yl)-1H-pyrazolo[4,3-c]quinoline-8-carboxamide,
4-amino-N,1-dimethyl-N-((3 S)-6-(5-methyl-3-furanyl)-2,3-dihydro-1-benzofuran-3-yl)-1H-pyrazolo[4,3-c] quinoline-8-carboxamide,
4-amino-N,1-dimethyl-N-((3 S)-6-(3-methyl-1,2-oxazol-5-yl)-2,3-dihydro-1-benzofuran-3-yl)-1H-pyrazolo[4,3-c]quinoline-8-carboxamide,
4-amino-N-((3S)-6-(5,6-dihydro-2H-pyran-3-yl)-2,3-dihydro-1-benzofuran-3-yl)-N,1-dimethyl-1H-pyrazolo[4,3-c]quinoline-8-carboxamide,
4-amino-N,1-dimethyl-N-((3S)-6-(1,3-thiazol-4-yl)-2,3-dihydro-1-benzofuran-3-yl)-1H-pyrazolo[4,3-c]quinoline-8-carboxamide,
4-amino-N,1-dimethyl-N-((3S)-6-(2-methyl-5-pyrimidinyl)-2,3-dihydro-1-benzofuran-3-yl)-1H-pyrazolo[4,3-c]quinoline-8-carboxamide,
4-amino-N,1-dimethyl-N-((3S)-6-(2-oxo-1,2-dihydro-5-pyrimidinyl)-2,3-dihydro-1-benzofuran-3-yl)-1H-pyrazolo[4,3-c]quinoline-8-carboxamide,
4-amino-N-((3S)-6-(6-fluoro-3-pyridinyl)-2,3-dihydro-1-benzofuran-3-yl)-N,1-dimethyl-1H-pyrazolo[4,3-c]quinoline-8-carboxamide,
4-amino-N-((3S)-6-(1-ethyl-1H-pyrazol-4-yl)-2,3-dihydro-1-benzofuran-3-yl)-N, 1-dimethyl-1H-pyrazolo[4,3-c]quinoline-8-carboxamide,
4-amino-N-((3S)-6-(1-cyclopropyl-1H-pyrazol-4-yl)-2,3-dihydro-1-benzofuran-3-yl)-N, 1-dimethyl-1H-pyrazolo[4,3-c]quinoline-8-carboxamide,
4-amino-N-((3S)-6-(3,5-difluorophenyl)-2,3-dihydro-1-benzofuran-3-yl)-N,1-dimethyl-1H-pyrazolo[4,3-c] quinoline-8-carboxamide,
4-amino-N-((3S)-6-(2,6-difluoro-3-pyridinyl)-2,3-dihydro-1-benzofuran-3-yl)-N,1-dimethyl-1H-pyrazolo[4,3-c]quinoline-8-carboxamide,
4-amino-N-((3S)-6-(2,3-difluoro-4-pyridinyl)-2,3-dihydro-1-benzofuran-3-yl)-N,1-dimethyl-1H-pyrazolo[4,3-c]quinoline-8-carboxamide,
4-amino-N,1-dimethyl-N-((3S)-6-(2-oxo-2,3-dihydro-1H-pyrrolo[2,3-b]pyridin-5-yl)-2,3-dihydro-1-benzofuran-3-yl)-1H-pyrazolo[4,3-c]quinoline-8-carboxamide,
4-amino-N,1-dimethyl-N-((3S)-6-(5-(trifluoromethyl)-1H-pyrazol-3-yl)-2,3-dihydro-1-benzofuran-3-yl)-1H-pyrazolo[4,3-c]quinoline-8-carboxamide,
4-amino-N,1-dimethyl-N-((3S)-6-(6-(methylcarbamoyl)-3-pyridinyl)-2,3-dihydro-1-benzofuran-3-yl)-1H-pyrazolo[4,3-c]quinoline-8-carboxamide,
N-((3S)-6-(6-acetamido-3-pyridinyl)-2,3-dihydro-1-benzofuran-3-yl)-4-amino-N,1-dimethyl-1H-pyrazolo[4,3-c]quinoline-8-carboxamide,
4-amino-N,1-dimethyl-N-((3S)-6-(6-(trifluoromethyl)-2-pyridinyl)-2,3-dihydro-1-benzofuran-3-yl)-1H-pyrazolo[4,3-c]quinoline-8-carboxamide,
4-amino-N,1-dimethyl-N-((3S)-6-(2-(trifluoromethyl)-3-pyridinyl)-2,3-dihydro-1-benzofuran-3-yl)-1H-pyrazolo[4,3-c]quinoline-8-carboxamide,
4-amino-N,1-dimethyl-N-((3S)-6-(4-(trifluoromethyl)-3-pyridinyl)-2,3-dihydro-1-benzofuran-3-yl)-1H-pyrazolo[4,3-c]quinoline-8-carboxamide,
4-amino-N,1-dimethyl-N-((3S)-6-(5-(trifluoromethyl)-3-pyridinyl)-2,3-dihydro-1-benzofuran-3-yl)-1H-pyrazolo[4,3-c]quinoline-8-carboxamide,
4-amino-N,1-dimethyl-N-((3S)-6-(5-(trifluoromethyl)-2-pyridinyl)-2,3-dihydro-1-benzofuran-3-yl)-1H-pyrazolo[4,3-c]quinoline-8-carboxamide,
4-amino-N,1-dimethyl-N-((3S)-6-(1-methyl-4-(trifluoromethyl)-1H-pyrazol-5-yl)-2,3-dihydro-1-benzofuran-3-yl)-1H-pyrazolo[4,3-c]quinoline-8-carboxamide,
4-amino-N,1-dimethyl-N-((3S)-6-(1-methyl-3-(trifluoromethyl)-1H-pyrazol-5-yl)-2,3-dihydro-1-benzofuran-3-yl)-1H-pyrazolo[4,3-c]quinoline-8-carboxamide,
4-amino-N,1-dimethyl-N-((3S)-6-(2-(trifluoromethyl)-1,3-thiazol-4-yl)-2,3-dihydro-1-benzofuran-3-yl)-1H-pyrazolo[4,3-c]quinoline-8-carboxamide,
4-amino-N,1-dimethyl-N-((3S)-6-(2-(trifluoromethyl)-1,3-thiazol-5-yl)-2,3-dihydro-1-benzofuran-3-yl)-1H-pyrazolo[4,3-c]quinoline-8-carboxamide,
4-amino-N-((3S)-6-(2,2-difluoro-1,3-benzodioxol-5-yl)-2,3-dihydro-1-benzofuran-3-yl)-N,1-dimethyl-1H-pyrazolo[4,3-c]quinoline-8-carboxamide,
4-amino-N,1-dimethyl-N-((3S)-6-(4-(trifluoromethoxy)phenyl)-2,3-dihydro-1-benzofuran-3-yl)-1H-pyrazolo[4,3-c]quinoline-8-carboxamide,
4-amino-N-((3S)-6-(3-(difluoromethoxy)-5-fluorophenyl)-2,3-dihydro-1-benzofuran-3-yl)-N,1-dimethyl-1H-pyrazolo[4,3-c]quinoline-8-carboxamide,
4-amino-N-((3S)-6-(2-fluoro-5-(trifluoromethyl)phenyl)-2,3-dihydro-1-benzofuran-3-yl)-N,1-dimethyl-1H-pyrazolo[4,3-c]quinoline-8-carboxamide,
4-amino-N-((3S)-6-(2-fluoro-4-(trifluoromethyl)phenyl)-2,3-dihydro-1-benzofuran-3-yl)-N,1-dimethyl-1H-pyrazolo[4,3-c]quinoline-8-carboxamide,
4-amino-N,1-dimethyl-N-((3S)-6-(2-(2,2,2-trifluoroethoxy)-4-pyridinyl)-2,3-dihydro-1-benzofuran-3-yl)-1H-pyrazolo[4,3-c]quinoline-8-carboxamide,
4-amino-N,1-dimethyl-N-((4S)-7-(1-(trifluoromethyl)-1H-pyrazol-4-yl)-3,4-dihydro-1H-2-benzopyran-4-yl)-1H-pyrazolo[4,3-c]quinoline-8-carboxamide,
4-amino-N,1-dimethyl-N-((4R)-7-(1-(trifluoromethyl)-1H-pyrazol-4-yl)-3,4-dihydro-1H-2-benzopyran-4-yl)-1H-pyrazolo[4,3-c]quinoline-8-carboxamide,
4-amino-7-fluoro-N-methyl-N-((4R)-7-(4-(trifluoromethyl)phenyl)-3,4-dihydro-1H-2-benzopyran-4-yl)-1,3-dihydrofuro[3,4-c]quinoline-8-carboxamide,
4-amino-7-fluoro-N-methyl-N-((4S)-7-(4-(trifluoromethyl)phenyl)-3,4-dihydro-1H-2-benzopyran-4-yl)-1,3-dihydrofuro[3,4-c]quinoline-8-carboxamide,
4-amino-7-fluoro-N-methyl-N-((3S)-6-(4-(pentafluoro-lambda~6~-sulfanyl)phenyl)-2,3-dihydro-1-benzofuran-3-yl)-1,3-dihydrofuro[3,4-c]quinoline-8-carboxamide,
4-amino-N,1-dimethyl-N-((3S)-6-(4-(pentafluoro-lambda~6~-sulfanyl)phenyl)-2,3-dihydro-1-benzofuran-3-yl)-1H-pyrazolo[4,3-c]quinoline-8-carboxamide,
4-amino-N,1-dimethyl-N-((4S)-7-(4-(trifluoromethyl)phenyl)-3,4-dihydro-1H-2-benzopyran-4-yl)-1H-pyrazolo[4,3-c]quinoline-8-carboxamide,
4-amino-N,1-dimethyl-N-((4R)-7-(4-(trifluoromethyl)phenyl)-3,4-dihydro-1H-2-benzopyran-4-yl)-1H-pyrazolo[4,3-c]quinoline-8-carboxamide,
4-amino-N-((3S)-6-methoxy-2,3-dihydro-1-benzofuran-3-yl)-N,1-dimethyl-1H-pyrazolo[4,3-c]quinoline-8-carboxamide,
4-amino-N-((3S)-6-(cyclopropyloxy)-2,3-dihydro-1-benzofuran-3-yl)-N,1-dimethyl-1H-pyrazolo[4,3-c] quinoline-8-carboxamide,
4-amino-N-((3S)-2,3-dihydro-1-benzofuran-3-yl)-N,1-dimethyl-1H-pyrazolo[4,3-c]quinoline-8-carboxamide,
4-amino-N-((3S)-6-ethoxy-2,3-dihydro-1-benzofuran-3-yl)-N-methyl-1,3-dihydrofuro[3,4-c] [1,7]naphthyridine-8-carboxamide,
4-amino-N-((3S)-6-ethoxy-2,3-dihydro-1-benzofuran-3-yl)-N,1-dimethyl-1H-pyrazolo[4,3-c]quinoline-8-carboxamide,
4-amino-N-((3S)-6-(cyclobutyloxy)-2,3-dihydro-1-benzofuran-3-yl)-N,1-dimethyl-1H-pyrazolo[4,3-c] quinoline-8-carboxamide,
4-amino-N,1-dimethyl-N-((3S)-6-(3-oxetanyloxy)-2,3-dihydro-1-benzofuran-3-yl)-1H-pyrazolo[4,3-c] quinoline-8-carboxamide,
4-amino-N-((3S)-6-(2-methoxyethoxy)-2,3-dihydro-1-benzofuran-3-yl)-N,1-dimethyl-1H-pyrazolo[4,3-c] quinoline-8-carboxamide,
4-amino-N,1-dimethyl-N-((5S)-2-methyl-5,8-dihydro-6H-pyrano[3,4-b]pyridin-5-yl)-1H-pyrazolo[4,3-c] quinoline-8-carboxamide,
4-amino-N,1-dimethyl-N-((1R)-5-methyl-2,3-dihydro-1H-inden-1-yl)-1H-pyrazolo[4,3-c]quinoline-8-carboxamide,
4-amino-N-((1R)-2,3-dihydro-1H-inden-1-yl)-N,1-dimethyl-1H-pyrazolo[4,3-c]quinoline-8-carboxamide,
4-amino-N-((1S)-2,3-dihydro-1H-inden-1-yl)-N,1-dimethyl-1H-pyrazolo[4,3-c]quinoline-8-carboxamide,
4-amino-N,1-dimethyl-N-((5R)-2-methyl-6,7-dihydro-5H-cyclopenta[b]pyridin-5-yl)-1H-pyrazolo[4,3-c] quinoline-8-carboxamide,
4-amino-N,1-dimethyl-N-((5S)-2-methyl-6,7-dihydro-5H-cyclopenta[b]pyridin-5-yl)-1H-pyrazolo[4,3-c] quinoline-8-carboxamide,
4-amino-N,1-dimethyl-N-((3S)-6-methyl-2,3-dihydro-1-benzofuran-3-yl)-1H-pyrazolo[4,3-c]quinoline-8-carboxamide,
4-amino-N-((5 S)-2-cyclopropyl-5,8-dihydro-6H-pyrano [3,4-b]pyridin-5-yl)-N,1-dimethyl-1H-pyrazolo[4,3-c]quinoline-8-carboxamide,
methyl (3S)-3-(((4-amino-1-methyl-1H-pyrazolo[4,3-c]quinolin-8-yl)carbonyl)(methyl)amino)-2,3-dihydro-1-benzofuran-6-carboxylate,
4-amino-N-((3S)-6-(hydroxymethyl)-2,3-dihydro-1-benzofuran-3-yl)-N,1-dimethyl-1H-pyrazolo[4,3-c] quinoline-8-carboxamide,
4-amino-N-((3S)-6-chloro-2,3-dihydro-1-benzofuran-3-yl)-N,1-dimethyl-1H-pyrazolo[4,3-c]quinoline-8-carboxamide,
4-amino-N,1-dimethyl-N-((3S)-6-(tetrahydro-2H-pyran-4-yl)-2,3-dihydro-1-benzofuran-3-yl)-1H-pyrazolo[4,3-c]quinoline-8-carboxamide,
N-((3R)-6-(1-acetyl-1,2,3,6-tetrahydro-4-pyridinyl)-2,3-dihydro-1-benzofuran-3-yl)-4-amino-N-methyl-1,3-dihydrofuro[3,4-c]quinoline-8-carboxamide and
N-((3 S)-6-(1-acetyl-1,2,3,6-tetrahydro-4-pyridinyl)-2,3-dihydro-1-benzofuran-3-yl)-4-amino-N-methyl-1,3-dihydrofuro[3,4-c]quinoline-8-carboxamide.

The invention provides the compounds, the tautomer thereof, the stereoisomer thereof, or the pharmaceutically acceptable salt of any of the foregoing, wherein the compound is selected from: 4-amino-7-chloro-N,1-dimethyl-N-((5R)-2-(trifluoromethyl)-6,7-dihydro-5H-cyclopenta[b]pyridin-5-yl)-1H-pyrazolo[4,3-c]quinoline-8 -carboxamide,
4-amino-N-((4S)-8-fluoro-7-(trifluoromethyl)-3,4-dihydro-1H-2-benzopyran-4-yl)-N,1-dimethyl-1H-pyrazolo[4,3-c]quinoline-8-carboxamide,
4-amino-N-((5S)-2-bromo-5,8-dihydro-6H-pyrano[3,4-b]pyridin-5-yl)-N,1-dimethyl-1H-pyrazolo[4,3-c] quinoline-8-carboxamide,
4-amino-7-chloro-N,1-dimethyl-N-((5S)-2-(trifluoromethyl)-5,8-dihydro-6H-pyrano[3,4-b]pyridin-5-yl)-1H-pyrazolo[4,3-c]quinoline-8 -carboxamide,
4-amino-7-fluoro-N-methyl-N-((5R)-2-(trifluoromethyl)-6,7-dihydro-5H-cyclopenta[b]pyridin-5-yl)-1,3-dihydrofuro[3,4-c]quinoline-8-carboxamide,
(3R)-4-amino-7-fluoro-N,3-dimethyl-N-((5R)-2-(trifluoromethyl)-6,7-dihydro-5H-cyclopenta[b]pyridin-5-yl)-1,3-dihydrofuro[3,4-c]quinoline-8-carboxamide,
4-amino-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-N,1-dimethyl-N-((3S)-6-(pentafluoro-lambda~6~-sulfanyl)-2,3-dihydro-1-benzofuran-3-yl)-1H-pyrazolo[4,3-c]quinoline-8-carboxamide,
4-amino-N,1-dimethyl-N-((5R)-2-(trifluoromethyl)-6,7-dihydro-5H-cyclopenta[b]pyridin-5-yl)-1H-pyrazolo[4,3-c] [1,7]naphthyridine-8-carboxamide,
4-amino-7-fluoro-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-7-fluoro-N,1-dimethyl-N-((3S)-6-(trifluoromethyl)-2,3-dihydrofuro[2,3-b]pyridin-3-yl)-1H-pyrazolo[4,3-c]quinoline-8-carboxamide,
4-amino-7-chloro-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-N-((4S)-8-fluoro-7-(trifluoromethyl)-3,4-dihydro-1H-2-benzopyran-4-yl)-N,1-dimethyl-1H-pyrazolo[4,3-c]quinoline-8-carboxamide,
4-amino-N-((5S)-2-bromo-5,8-dihydro-6H-pyrano[3,4-b]pyridin-5-yl)-N,1-dimethyl-1H-pyrazolo[4,3-c] quinoline-8-carboxamide,
4-amino-N,1-dimethyl-N-((3S)-6-(trifluoromethyl)-2,3-dihydro-1-benzofuran-3-yl)-1H-pyrazolo[4,3-c]quinoline-8-carboxamide,
(3R)-4-amino-N,3-dimethyl-N-((5S)-2-(trifluoromethyl)-5,8-dihydro-6H-pyrano[3,4-b]pyridin-5-yl)-1,3-dihydrofuro[3,4-c]quinoline-8-carboxamide,
4-amino-N-methyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamide,
4-amino-7-fluoro-N,1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N-methyl-N-((3S)-6-(trifluoromethyl)-2,3-dihydro-1-benzofuran-3-yl)-1,3-dihydrofuro[3,4-c][1,7]naphthyridine-8-carboxamide,
4-amino-7-fluoro-N,1-dimethyl-N-((3S)-6-(trifluoromethyl)-2,3-dihydro-1-benzofuran-3-yl)-1H-pyrazolo[4,3-c]quinoline-8-carboxamide, and
4-amino-7-fluoro-N,1-dimethyl-N-((3 S)-6-(trifluoromethyl)-2,3-dihydrofuro [3,2-c]pyridin-3-yl)-1H-pyrazolo[4,3-c]quinoline-8-carboxamide.

Any method of treatment mentioned herein as referring to the present invention is not to be understood as belonging to the claimed invention as such. However, the invention as defined in the appended claims may relate to compounds or compositions for use in such methods, as can be taken from the claims.

The invention further provides methods of treating cancer comprising administering to a subject an effective amount of the compound of the invention, the tautomer thereof, the stereoisomer thereof, or the pharmaceutically acceptable salt of any of the foregoing. In one aspect, the cancer is selected from lung, Head and Neck Squamous Cell Carcinoma (HNSCC), esophagus, lymphoid, glioblastoma, colon, melanoma, gastric, pancreatic, bile or bladder cancer. In one aspect, lung cancer could be Non-Small Cell Lung Carcinoma (NSCLC).

The invention further provides pharmaceutical compositions, comprising the compounds of the invention, the tautomer thereof, the stereoisomer thereof, or the pharmaceutically acceptable salt of any of the foregoing or a pharmaceutically acceptable salt thereof, and at least one pharmaceutically acceptable excipient.

The invention also provides the compound of the invention, the tautomer thereof, the stereoisomer thereof, or the pharmaceutically acceptable salt of any of the foregoing for use in a method of treating a cancer, the method comprising administering to a subject an effective amount of such compound. In one aspect, the cancer can lung, Head and Neck Squamous Cell Carcinoma (HNSCC), esophagus, lymphoid, glioblastoma, colon, melanoma, gastric, pancreatic bile or bladder cancer. In one aspect, lung cancer could be Non-Small Cell Lung Carcinoma (NSCLC).

Other objects, features and advantages of the invention will become apparent to those skilled in the art from the following description and claims.

### DETAILED DESCRIPTION OF THE INVENTION

### Definitions

As used herein, if any variable occurs more than one time in a chemical formula, its definition on each occurrence is independent of its definition at every other occurrence. If the chemical structure and chemical name conflict, the chemical structure is determinative of the identity of the compound. The compounds of the present disclosure may contain one or more chiral centers and/or double bonds and therefore, may exist as stereoisomers, such as double-bond isomers *(i.e.,* geometric isomers), enantiomers, or diastereomers. Accordingly, any chemical structures within the scope of the specification depicted, in whole or in part, with a relative configuration encompass all possible enantiomers and stereoisomers of the illustrated compounds including the stereoisomerically pure form *(e.g.,* geometrically pure, enantiomerically pure or diastereomerically pure) and enantiomeric and stereoisomeric mixtures. Enantiomeric and stereoisomeric mixtures can be resolved into the component enantiomers or stereoisomers using separation techniques or chiral synthesis techniques well known to the skilled artisan.

Certain compounds of the invention may possess asymmetric carbon atoms (optical centers) or double bonds; the racemates, enantiomers, diastereomers, geometric isomers and individual isomers are all intended to be encompassed within the scope of the invention. Furthermore, atropisomers and mixtures thereof such as those resulting from restricted rotation about two aromatic or heteroaromatic rings bonded to one another are intended to be encompassed within the scope of the invention. For example, when substituent is a phenyl group and is substituted with two groups bonded to the C atoms adjacent to the point of attachment to the N atom of the triazole, then rotation of the phenyl may be restricted. In some instances, the barrier of rotation is high enough that the different atropisomers may be separated and isolated.

As used herein and unless otherwise indicated, the term "stereoisomer" or "stereomerically pure" means one stereoisomer of a compound that is substantially free of other stereoisomers of that compound. For example, a stereomerically pure compound having one chiral center will be substantially free of the mirror image enantiomer of the compound. A stereomerically pure compound having two chiral centers will be substantially free of other diastereomers of the compound. A typical stereomerically pure compound comprises greater than about 80% by weight of one stereoisomer of the compound and less than about 20% by weight of other stereoisomers of the compound, more preferably greater than about 90% by weight of one stereoisomer of the compound and less than about 10% by weight of the other stereoisomers of the compound, even more preferably greater than about 95% by weight of one stereoisomer of the compound and less than about 5% by weight of the other stereoisomers of the compound, and most preferably greater than about 97% by weight of one stereoisomer of the compound and less than about 3% by weight of the other stereoisomers of the compound. If the stereochemistry of a structure or a portion of a structure is not indicated with, for example, bold or dashed lines, the structure or portion of the structure is to be interpreted as encompassing all stereoisomers of it. A bond drawn with a wavy line indicates that both stereoisomers are encompassed. This is not to be confused with a wavy line drawn perpendicular to a bond which indicates the point of attachment of a group to the rest of the molecule.

As known by those skilled in the art, certain compounds of the invention may exist in one or more tautomeric forms. Because one chemical structure may only be used to represent one tautomeric form, it will be understood that for convenience, referral to a compound of a given structural formula includes tautomers of the structure represented by the structural formula. Depending on the compound, some compounds may exist primarily in one form more than another. Also, depending on the compound and the energy required to convert one tautomer to the other, some compounds may exist as mixtures at room temperature whereas others may be isolated in one tautomeric form or the other. Examples of other tautomers associated with compounds of the invention are those with a pyridone group (a pyridinyl) for which hydroxypyridine is a tautomer and compounds with a ketone group with the enol tautomer. Examples of these are shown below.

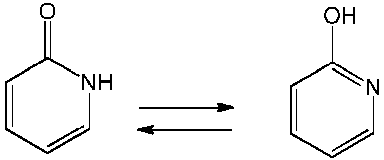

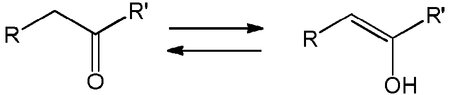

Compounds of the present disclosure include, but are not limited to, compounds of Formula I and all pharmaceutically acceptable forms thereof. Pharmaceutically acceptable forms of the compounds recited herein include pharmaceutically acceptable salts, solvates, crystal forms (including polymorphs and clathrates), chelates, non-covalent complexes, and mixtures thereof. In certain embodiments, the compounds described herein are in the form of pharmaceutically acceptable salts. As used herein, the term "compound" encompasses not only the compound itself, but also a pharmaceutically acceptable salt thereof, a solvate thereof, a chelate thereof, a non-covalent complex thereof, and mixtures of any of the foregoing. In some embodiments, the term "compound" encompasses the compound itself, pharmaceutically acceptable salts thereof, tautomers of the compound, and pharmaceutically acceptable salts of the tautomers. In other embodiments, the term "compound" encompasses the compound itself, pharmaceutically acceptable salts thereof, tautomers of the compound, pharmaceutically acceptable salts of the tautomers.

Pharmaceutically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and p-toluenesulfonic, acids; base addition salts formed with alkali metals and alkaline earth metals and organic bases such as N,N-dibenzylethylenediamine, chloroprocaine, choline, diethanolamine, ethylenediamine, meglumaine (N-methylglucamine), lysine and procaine; and internally formed salts. Suitable salts include those described in P. Heinrich Stahl, Camille G. Wermuth (Eds.), Handbook of Pharmaceutical Salts Properties, Selection and Use; 2002. Salts having a non-pharmaceutically acceptable anion or cation are within the scope of the invention as useful intermediates for the preparation of pharmaceutically acceptable salts and/or for use in non-therapeutic, for example, in vitro, situations.

The term "solvate" refers to the compound formed by the interaction of a solvent and a compound. Solvates of a compound includes solvates of all forms of the compound. In certain embodiments, solvents are volatile, non-toxic, and/or acceptable for administration to humans in trace amounts. Suitable solvates are pharmaceutically acceptable solvates, such as hydrates, including monohydrates and hemi-hydrates.

The invention discloses compounds which may also contain naturally occurring or unnatural proportions of atomic isotopes at one or more of the atoms that constitute such compounds. For example, the compounds may be radiolabeled with radioactive isotopes, such as for example tritium (3H), iodine-125 (125I) or carbon-14 (14C). Radiolabeled compounds are useful as therapeutic or prophylactic agents, research reagents, e.g., assay reagents, and diagnostic agents, e.g., in vivo imaging agents. All isotopic variations of the compounds of the invention, whether radioactive or not, are intended to be encompassed within the scope of the invention. For example, the invention also includes deuterium (D) or tritium (T) containing compounds.

"Alkyl" refers to a saturated branched or straight-chain monovalent hydrocarbon group derived by the removal of one hydrogen atom from a single carbon atom of a parent alkane. Typical alkyl groups include, but are not limited to, methyl, ethyl, propyls such as propan-1-yl and propan-2-yl, butyls such as butan-1-yl, butan-2-yl, 2-methyl-propan-1-yl, 2-methyl-propan-2-yl, tert-butyl, and the like. In certain embodiments, an alkyl group comprises 1 to 20 carbon atoms. In some embodiments, alkyl groups include 1 to 10 carbon atoms or 1 to 6 carbon atoms whereas in other embodiments, alkyl groups include 1 to 4 carbon atoms. In still other embodiments, an alkyl group includes 1 or 2 carbon atoms. Branched chain alkyl groups include at least 3 carbon atoms and typically include 3 to 7, or in some embodiments, 3 to 6 carbon atoms. An alkyl group having 1 to 6 carbon atoms may be referred to as a (C₁-C₆)alkyl group and an alkyl group having 1 to 4 carbon atoms may be referred to as a (C₁-C₄)alkyl. This nomenclature may also be used for alkyl groups with differing numbers of carbon atoms.

"Alkenyl" refers to an unsaturated branched or straight-chain hydrocarbon group having at least one carbon-carbon double bond derived by the removal of one hydrogen atom from a single carbon atom of a parent alkene. The group may be in either the Z- or E- form (*cis* or *trans*) about the double bond(s). Typical alkenyl groups include, but are not limited to, ethenyl; propenyls such as prop-1-en-1-yl, prop-1-en-2-yl, prop-2-en-1-yl (allyl), and prop-2-en-2-yl; butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, and buta-1,3-dien-2-yl; and the like. In certain embodiments, an alkenyl group has 2 to 20 carbon atoms and in other embodiments, has 2 to 6 carbon atoms. An alkenyl group having 2 to 6 carbon atoms may be referred to as a (C₂-C₆)alkenyl group.

"Alkynyl" refers to an unsaturated branched or straight-chain hydrocarbon having at least one carbon-carbon triple bond derived by the removal of one hydrogen atom from a single carbon atom of a parent alkyne. Typical alkynyl groups include, but are not limited to, ethynyl; propynyl; butynyl, 2-pentynyl, 3-pentynyl, 2-hexynyl, 3-hexynyl and the like. In certain embodiments, an alkynyl group has 2 to 20 carbon atoms and in other embodiments, has 2 to 6 carbon atoms. An alkynyl group having 2 to 6 carbon atoms may be referred to as a -(C₂-C₆)alkynyl group.

"Alkoxy" refers to a radical -OR where R represents an alkyl group as defined herein. Representative examples include, but are not limited to, methoxy, ethoxy, propoxy, butoxy, and the like. Typical alkoxy groups include 1 to 10 carbon atoms, 1 to 6 carbon atoms or 1 to 4 carbon atoms in the R group. Alkoxy groups that include 1 to 6 carbon atoms may be designated as -O-(C₁-C₆) alkyl or as -O-(C₁-C₆ alkyl) groups. In some embodiments, an alkoxy group may include 1 to 4 carbon atoms and may be designated as -O-(C₁-C₄) alkyl or as -O-(C₁-C₄ alkyl) groups group.

"Aryl" refers to a monovalent aromatic hydrocarbon group derived by the removal of one hydrogen atom from a single carbon atom of a parent aromatic ring system. Aryl encompasses monocyclic carbocyclic aromatic rings, for example, benzene. Aryl also encompasses bicyclic carbocyclic aromatic ring systems where each of the rings is aromatic, for example, naphthalene. Aryl groups may thus include fused ring systems where each ring is a carbocyclic aromatic ring. In certain embodiments, an aryl group includes 6 to 10 carbon atoms. Such groups may be referred to as C₆-C₁₀ aryl groups. Aryl, however, does not encompass or overlap in any way with heteroaryl as separately defined below. Hence, if one or more carbocyclic aromatic rings is fused with an aromatic ring that includes at least one heteroatom, the resulting ring system is a heteroaryl group, not an aryl group, as defined herein.

"Carbonyl" refers to the radical -C(O) which may also be referred to as -C(=O) group.

"Carboxy" refers to the radical -C(O)OH which may also be referred to as -C(=O)OH.

"Cyano" refers to the radical -CN.

"Cycloalkyl" refers to a saturated cyclic alkyl group derived by the removal of one hydrogen atom from a single carbon atom of a parent cycloalkane. Typical cycloalkyl groups include, but are not limited to, groups derived from cyclopropane, cyclobutane, cyclopentane, cyclohexane, cycloheptane, cyclooctane, and the like. Cycloalkyl groups may be described by the number of carbon atoms in the ring. For example, a cycloalkyl group having 3 to 8 ring members may be referred to as a (C₃-C₈)cycloalkyl, a cycloalkyl group having 3 to 7 ring members may be referred to as a (C₃-C₇)cycloalkyl and a cycloalkyl group having 4 to 7 ring members may be referred to as a (C₄-C₇)cycloalkyl. In certain embodiments, the cycloalkyl group can be a (C₃-C₁₀)cycloalkyl, a (C₃-C₈)cycloalkyl, a (C₃-C₇)cycloalkyl, a (C₃-C₆)cycloalkyl, or a (C₄-C₇)cycloalkyl group and these may be referred to as C₃-C₁₀ cycloalkyl, C₃-C₈ cycloalkyl, C₃-C₇ cycloalkyl, C₃-C₆ cycloalkyl, or C₄-C₇ cycloalkyl groups using alternative language.

"Heterocyclyl" refers to a cyclic group that includes at least one saturated, partially unsaturated, cyclic ring. Heterocyclyl groups include at least one heteroatom as a ring member. Typical heteroatoms include, O, S and N and are independently chosen. Heterocyclyl groups include monocyclic ring systems and bicyclic ring systems. Bicyclic heterocyclyl groups include at least one non-aromatic ring with at least one heteroatom ring member that may be fused to a cycloalkyl ring or may be fused to an aromatic ring where the aromatic ring may be carbocyclic or may include one or more heteroatoms. The point of attachment of a bicyclic heterocyclyl group may be at the non-aromatic cyclic ring that includes at least one heteroatom or at another ring of the heterocyclyl group. For example, a heterocyclyl group derived by removal of a hydrogen atom from one of the 9 membered heterocyclic compounds shown below may be attached to the rest of the molecule at the 5-membered ring or at the 6-membered ring.

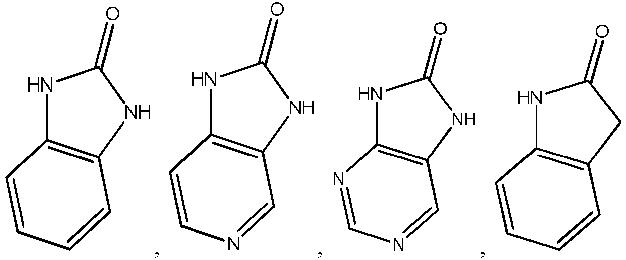

In some embodiments, a heterocyclyl group includes 5 to 10 ring members of which 1, 2, 3 or 4 or 1, 2, or 3 are heteroatoms independently selected from O, S, or N. In other embodiments, a heterocyclyl group includes 3 to 7 ring members of which 1, 2, or 3 heteroatom are independently selected from O, S, or N. In such 3-7 membered heterocyclyl groups, only 1 of the ring atoms is a heteroatom when the ring includes only 3 members and includes 1 or 2 heteroatoms when the ring includes 4 members. In some embodiments, a heterocyclyl group includes 3 or 4 ring members of which 1 is a heteroatom selected from O, S, or N. In other embodiments, a heterocyclyl group includes 5 to 7 ring members of which 1, 2, or 3 are heteroatoms independently selected from O, S, or N. Typical heterocyclyl groups include, but are not limited to, groups derived from epoxides, aziridine, azetidine, imidazolidine, morpholine, piperazine, piperidine, hexahydropyrimidine, 1,4,5,6-tetrahydropyrimidine, pyrazolidine, pyrrolidine, quinuclidine, tetrahydrofuran, tetrahydropyran, benzimidazolone, pyridinone, and the like. Heterocyclyl groups may be fully saturated but may also include one or more double bonds. Examples of such heterocyclyl groups include, but are not limited to, 1,2,3,6-tetrahydropyridinyl, 3,6-dihydro-2H-pyranyl, 3,4-dihydro-2H-pyranyl, 2,5-dihydro-1H-pyrolyl, 2,3-dihydro-1H-pyrolyl, 1H-azirinyl, 1,2-dihydroazetenyl, and the like. Substituted heterocyclyl also includes ring systems substituted with one or more oxo (=O) or oxide (-O⁻) substituents, such as piperidinyl N-oxide, morpholinyl-N-oxide, 1-oxo-1-thiomorpholinyl, pyridinonyl, benzimidazolonyl, benzo[d]oxazol-2(3H)-onyl, 3,4-dihydroisoquinolin-1(2H)-onyl, indolin-onyl, 1H-imidazo[4,5-c]pyridin-2(3H)-onyl, 7H-purin-8(9H)-onyl, imidazolidin-2-onyl, 1H-imidazol-2(3H)-onyl, 1,1-dioxo-1-thiomorpholinyl, and the like.

The term "comprising" is meant to be open ended, i.e., all-encompassing and non-limiting. It may be used herein synonymously with "having" or "including". Comprising is intended to include each and every indicated or recited component or element(s) while not excluding any other components or elements.

"Disease" refers to any disease, disorder, condition, symptom, or indication.

"Halo" or "halogen" refers to a fluoro, chloro, bromo, or iodo group.

"Haloalkyl" refers to an alkyl group in which at least one hydrogen is replaced with a halogen. Thus, the term "haloalkyl" includes monohaloalkyl (alkyl substituted with one halogen atom) and polyhaloalkyl (alkyl substituted with two or more halogen atoms). Representative "haloalkyl" groups include difluoromethyl, 2,2,2-trifluoroethyl, 2,2,2-trichloroethyl, and the like. The term "perhaloalkyl" means, unless otherwise stated, an alkyl group in which each of the hydrogen atoms is replaced with a halogen atom. For example, the term "perhaloalkyl", includes, but is not limited to, trifluoromethyl, pentachloroethyl, 1,1,1-trifluoro-2-bromo-2-chloroethyl, and the like.

"Heteroaryl" refers to a monovalent heteroaromatic group derived by the removal of one hydrogen atom from a single atom of a parent heteroaromatic ring system. Heteroaryl groups typically include 5- to 14-membered, but more typically include 5- to 10-membered aromatic, monocyclic, bicyclic, and tricyclic rings containing one or more, for example, 1, 2, 3, or 4, or in certain embodiments, 1, 2, or 3, heteroatoms chosen from O, S, or N, with the remaining ring atoms being carbon. In monocyclic heteroaryl groups, the single ring is aromatic and includes at least one heteroatom. In some embodiments, a monocyclic heteroaryl group may include 5 or 6 ring members and may include 1, 2, 3, or 4 heteroatoms, 1, 2, or 3 heteroatoms, 1 or 2 heteroatoms, or 1 heteroatom where the heteroatom(s) are independently selected from O, S, or N. In bicyclic aromatic rings, both rings are aromatic. In bicyclic heteroaryl groups, at least one of the rings must include a heteroatom, but it is not necessary that both rings include a heteroatom although it is permitted for them to do so. For example, the term "heteroaryl" includes a 5- to 7-membered heteroaromatic ring fused to a carbocyclic aromatic ring or fused to another heteroaromatic ring. In tricyclic aromatic rings, all three of the rings are aromatic and at least one of the rings includes at least one heteroatom. For fused, bicyclic and tricyclic heteroaryl ring systems where only one of the rings contains one or more heteroatoms, the point of attachment may be at the ring including at least one heteroatom or at a carbocyclic ring. When the total number of S and O atoms in the heteroaryl group exceeds 1, those heteroatoms are not adjacent to one another. In certain embodiments, the total number of S and O atoms in the heteroaryl group is not more than 2. In certain embodiments, the total number of S and O atoms in the aromatic heterocycle is not more than 1. Heteroaryl does not encompass or overlap with aryl as defined above. Examples of heteroaryl groups include, but are not limited to, groups derived from acridine, carbazole, cinnoline, furan, imidazole, indazole, indole, indolizine, isobenzofuran, isochromene, isoindole, isoquinoline, isothiazole, 2H-benzo[d][1,2,3]triazole, isoxazole, naphthyridine, oxadiazole, oxazole, perimidine, phenanthridine, phenanthroline, phenazine, phthalazine, pteridine, purine, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, and the like. In certain embodiments, the heteroaryl group can be between 5 to 20 membered heteroaryl, such as, for example, a 5 to 14 membered or 5 to 10 membered heteroaryl. In certain embodiments, heteroaryl groups can be those derived from thiophene, pyrrole, benzothiophene, 2H-benzo[d][1,2,3]triazole benzofuran, indole, pyridine, quinoline, imidazole, benzimidazole, oxazole, tetrazole, and pyrazine.

"MTAP" refers to a mammalian methylthioadenosine phosphorylase enzyme.

"Pharmaceutically acceptable" refers to generally recognized for use in animals, and more particularly in humans.

"Pharmaceutically acceptable salt" refers to a salt of a compound that is pharmaceutically acceptable and that possesses the desired pharmacological activity of the parent compound.

"Pharmaceutically acceptable excipient" refers to a broad range of ingredients that may be combined with a compound or salt of the present invention to prepare a pharmaceutical composition or formulation. Typically, excipients include, but are not limited to, diluents, colorants, vehicles, anti-adherants, glidants, disintegrants, flavoring agents, coatings, binders, sweeteners, lubricants, sorbents, preservatives, and the like.

"PRMT5" refers to a mammalian Protein Arginine N-Methyl Transferase 5 (PRMT5) enzyme.

"PRMT5 inhibitor"" refers to compounds that inhibit or negatively modulate all or a portion of the PRMT5 enzymatic activity.

"MTA-cooperative PRMT5 inhibitor" refers to compounds that inhibit or negatively modulate all or a portion of the PRMT5 enzymatic activity in the presence of bound MTA, *in vitro* or *in vivo,* in the cells with elevated levels of MTA.

"Stereoisomer" refers to an isomer that differs in the arrangement of the constituent atoms in space. Stereoisomers that are mirror images of each other and optically active are termed "enantiomers," and stereoisomers that are not mirror images of one another and are optically active are termed "diastereomers."

"Subject" includes mammals and humans. The terms "human" and "subject" are used interchangeably herein.

"Therapeutically effective amount" refers to the amount of a compound that, when administered to a subject for treating a disease, or at least one of the clinical symptoms of a disease or disorder, is sufficient to affect such treatment for the disease, disorder, or symptom. As those skilled in the art will recognize. this amount is typically not limited to a single dose but may comprise multiple dosages over a significant period of time as required to bring about a therapeutic or prophylactic response in the subject. Thus, a "therapeutically effective amount" is not limited to the amount in a single capsule or tablet, but may include more than one capsule or tablet, which is the dose prescribed by a qualified physician or medical care provider. The "therapeutically effective amount" can vary depending on the compound, the disease, disorder, and/or symptoms of the disease or disorder, severity of the disease, disorder, and/or symptoms of the disease or disorder, the age of the subject to be treated, and/or the weight of the subject to be treated. An appropriate amount in any given instance can be readily apparent to those skilled in the art or capable of determination by routine experimentation.

"Treating" or "treatment" of any disease or disorder refers to arresting or ameliorating a disease, disorder, or at least one of the clinical symptoms of a disease or disorder, reducing the risk of acquiring a disease, disorder, or at least one of the clinical symptoms of a disease or disorder, reducing the development of a disease, disorder or at least one of the clinical symptoms of the disease or disorder, or reducing the risk of developing a disease or disorder or at least one of the clinical symptoms of a disease or disorder. "Treating" or "treatment" also refers to inhibiting the disease or disorder, either physically, (e.g., stabilization of a discernible symptom), physiologically, (e.g., stabilization of a physical parameter), or both, or inhibiting at least one physical parameter which may not be discernible to the subject. Further, "treating" or "treatment" refers to delaying the onset of the disease or disorder or at least symptoms thereof in a subject which may be exposed to or predisposed to a disease or disorder even though that subject does not yet experience or display symptoms of the disease or disorder.

Also provided are pharmaceutical compositions that include the compound or the pharmaceutically acceptable salt thereof, the tautomer thereof, the pharmaceutically acceptable salt of the tautomer, the stereoisomer of any of the foregoing, or the mixture thereof according to any one of the examples and at least one pharmaceutically acceptable excipient, carrier or diluent. In some examples, the compound or the pharmaceutically acceptable salt thereof, the tautomer thereof, the pharmaceutically acceptable salt of the tautomer, the stereoisomer of any of the foregoing, or the mixture thereof according to any one of the aspects is present in an amount effective for the treatment of PRMT5-dependent cancers. In some aspects, the pharmaceutical composition is formulated for oral delivery whereas in other embodiments, the pharmaceutical composition is formulated for intravenous delivery. In some embodiments, the pharmaceutical composition is formulated for oral administration once a day or QD, and in some such formulations is a tablet where the effective amount of the active ingredient ranges from 1 mg to 1000 mg.

In some aspects, the subject is a mammal. In some such aspects, the mammal is a rodent. In other aspects, the mammal is a canine. In still other embodiments, the subject is a primate and, in some such embodiments, is a human.

The pharmaceutical compositions or formulations for the administration of the compounds of this invention may conveniently be presented in unit dosage form and may be prepared by any of the methods well known in the art. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition, the active object compound is included in an amount sufficient to produce the desired effect upon the process or condition of diseases.

The compounds of the invention may be administered via oral, mucosal (including sublingual, buccal, rectal, nasal, or vaginal), parenteral (including subcutaneous, intramuscular, bolus injection, intra-arterial, or intravenous), transdermal, or topical administration. In some aspects, the compounds of the invention are administered via mucosal (including sublingual, buccal, rectal, nasal, or vaginal), parenteral (including subcutaneous, intramuscular, bolus injection, intra-arterial, or intravenous), transdermal, or topical administration. In other aspects, the compounds of the invention are administered via oral administration. In still other embodiments, the compounds of the invention are not administered via oral administration.

The compounds of the invention, the pharmaceutically acceptable salt thereof, the tautomer thereof, the pharmaceutically acceptable salt of the tautomer, the stereoisomer of any of the foregoing, or the mixture thereof may find use in treating a number of conditions.

Compounds and compositions described herein are generally useful for the inhibition of PRMT5. In some aspects, methods of treating PRMT5-mediated disorder in a subject are disclosed which comprise
administering an effective amount of a compound described herein (*e.g.,* a compound of Formula I or a pharmaceutically acceptable salt thereof), to a subject in need of treatment. In certain aspects, the effective amount is a therapeutically effective amount. In certain aspects, the effective amount is a prophylactically effective amount. In certain aspects, the subject is suffering from a PRMT5-mediated disorder (*e.g.,* a cancer, for example a lymphoma, breast cancer, or pancreatic cancer). In other aspects, the subject is susceptible to a PRMT5-mediated disorder (*e.g.,* a cancer, for example a lymphoma, breast cancer, or pancreatic cancer).

As used herein, the term "PRMT5-mediated disorder" means any disease, disorder, or other pathological condition in which PRMT5 is known to play a role. Accordingly, in some aspects, the present disclosure relates to treating or lessening the severity of one or more diseases in which PRMT5 is known to play a role.

In some aspects, herein disclosed is a method of inhibiting PRMT5 activity in a subject in need thereof comprising administering to the subject an effective amount of a compound described herein (*e.g*., a compound of Formula I, or a pharmaceutically acceptable salt thereof, or a pharmaceutical composition thereof.

The invention discloses methods of treating cancers and other disorders arising from homozygous deletion of the chromosome 9p21 locus, which harbors the well-known tumor suppressor CDKN2A (cyclin dependent kinase inhibitor 2A). In one aspect, the invention encompasses methods of treating cancers and tumors which are MTAP (methylthioadenosine phosphorylase) - null. In some embodiments, these types of cancer display accumulation of MTAP substrate, methylthioadenosine (MTA).

The methods of treating PRMT5 disorders preferentially target PRMT5 in MTAP null tumors while sparing PRMT5 in normal tissues (MTAP WT). The compounds of the present invention thus include MTA-cooperative small molecule inhibitors which could preferentially target the MTA bound state of PRMT5, enriched in MTAP null tumor cells, while providing an improved therapeutic index over normal cells where MTAP is intact and MTA levels are low.

In further aspects, a PRMT5 inhibitor MTA coopertative compound contemplated by the present invention is useful in treating a proliferative disorder, such as cancer. In some embodiments, the compounds described herein are useful for treating pancreatic cancer. In some aspects, the compounds described herein are useful for treating multiple myeloma (MM). In further embodiments, the compounds described herein are useful for treating breast cancer. The breast cancer can be estrogen receptor negative (ER-) or the breast cancer can be progesterone receptor negative (PR-). In further embodiments, the breast cancer can be HER2 negative. In some embodiments, the breast cancer is estrogen receptor negative, progesterone receptor negative and HER2 negative, also referred to herein as "triple negative breast cancer".

In further aspects, a breast cancer can be a lobular carcinoma in situ (LCIS), a ductal carcinoma in situ (DCIS), an invasive ductal carcinoma (IDC), inflammatory breast cancer, Paget disease of the nipple, Phyllodes tumor, Angiosarcoma, adenoid cystic carcinoma, low-grade adenosquamous carcinoma, medullary carcinoma, mucinous carcinoma, papillary carcinoma, tubular carcinoma, metaplastic carcinoma, micropapary carcinoma, mixed carcinoma, or another breast cancer, including but not limited to triple negative, HER positive, estrogen receptor positive, progesterone receptor positive, HER and estrogen receptor positive, HER and progesterone receptor positive, estrogen and progesterone receptor positive, and HER and estrogen and progesterone receptor positive.

In one embodiment, compounds of the invention are useful for treating pancreatic cancer.

In another embodiment, compounds of the invention are useful for treating NSCLC (non-small cell lung carcinoma. In one embodiment, the NSCLC can be squamous NSCLC. In another embodiment, it can be adenocarcinoma.

In a further aspect, cancer can be glioblastoma (GBM). In a further aspect, cancer can be mesothelioma. In one aspect, cancer can be bladder cancer. In another aspect, cancer can be esophageal cancer. In a further aspect, cancer can be melanoma. In one aspect, cancer can be DLBCL, HNSCC or cholangiocarcinoma.

In some aspects, one or more compounds described herein are useful for treating any PRMT5-mediated or PRMT5-responsive proliferative cell disorder, for example a cancer that is PRMT5 responsive.

In one aspect, a cancer that lacks p53 (*e.g.,* a p53 null cancer) is less sensitive to PRMT5 inhibition than a cancer that is p53 positive. Accordingly, a cancer that is PRMT5 responsive can be a p53 positive cancer. The term "p53 positive" refers to a cancer that does not lack p53 expression and/or activity. In some embodiments, one or more compounds described herein are useful for treating a p53 positive cancer. In some aspects, a greater amount of one or more compounds described herein may be required to treat a p53 negative cancer (*e.g.* , a p53 null cancer) than a p53 positive cancer.

In some aspects, the disclosure provides a method for identifying subjects having a cancer that is sensitive to treatment with a PRMT5 inhibitor. In some embodiments, the method comprises obtaining a sample from the subject; detecting the presence or absence of p53; and, identifying the subject as having a cancer that is sensitive to treatment with a PRMT5 inhibitor if p53 is present in the sample. Accordingly, in some embodiments, a subject having a p53 positive cancer is identified as a subject for treatment with a PRMT5 inhibitor. In some embodiments, the method further comprises administering to the subject a composition comprising a PRMT5 inhibitor.

In some embodiments, aspects of the disclosure relate to a method for identifying subjects having a cancer that is insensitive (or that has low sensitivity) to treatment with a PRMT5 inhibitor. In some embodiments, the method comprises obtaining a sample from the subject; detecting the presence or absence of p53; and, identifying the subject as having a cancer that is not sensitive (for example, a cancer that is less sensitive than a p53 positive cancer) to treatment with a PRMT5 inhibitor if p53 is absent from the sample (*e.g.,* if the cancer is a p53 null cancer). In some embodiments, a p53 negative cancer (*e.g.,* a p53 null cancer) is to be treated with a PRMT5 inhibitor, but a greater amount of PRMT5 inhibitor may be required to treat the p53 negative cancer than a p53 positive cancer. However, in some embodiments, a subject having a p53 negative cancer (*e.g.* , a p53 null cancer) is to be treated with a therapeutic agent that is not a PRMT5 inhibitor.

By "sample" is meant any biological sample derived from the subject, includes but is not limited to, cells, tissues samples, body fluids (including, but not limited to, mucus, blood, plasma, serum, urine, saliva, and semen), cancer cells, and cancer tissues. Detection of the presence or absence of p53 in the sample may be achieved by any suitable method for detecting p53 nucleic acid or protein, for example, nucleic acid sequencing (*e.g*., DNA or RNA sequencing), quantitative PCR, Western blotting, etc., or any combination of thereof.

It should be appreciated that in some aspects, one or more of the compounds described herein may be useful for treating other types of cancer, including, but not limited to, acoustic neuroma, adenocarcinoma, adrenal gland cancer, anal cancer, angiosarcoma (*e.g*., lymphangiosarcoma, lymphangioendotheliosarcoma, hemangio sarcoma), appendix cancer, benign monoclonal gammopathy, biliary cancer (e.g. , cholangiocarcinoma), bladder cancer, brain cancer (e.g., meningioma; glioma, e.g. , astrocytoma, oligodendroglioma; medulloblastoma), bronchus cancer, carcinoid tumor, cervical cancer (e.g. , cervical adenocarcinoma), choriocarcinoma, chordoma, craniopharyngioma, colorectal cancer (e.g., colon cancer, rectal cancer, colorectal adenocarcinoma), epithelial carcinoma, ependymoma, endothelio sarcoma (e.g., Kaposi's sarcoma, multiple idiopathic hemorrhagic sarcoma), endometrial cancer (e.g., uterine cancer, uterine sarcoma), esophageal cancer (e.g. , adenocarcinoma of the esophagus, Barrett' s adenocarinoma), Ewing sarcoma, eye cancer (e.g., intraocular melanoma, retinoblastoma), familiar hypereosinophilia, gall bladder cancer, gastric cancer (e.g. , stomach adenocarcinoma), gastrointestinal stromal tumor (GIST), head and neck cancer (e.g., head and neck squamous cell carcinoma, oral cancer (e.g., oral squamous cell carcinoma (OSCC), throat cancer (e.g., laryngeal cancer, pharyngeal cancer, nasopharyngeal cancer, oropharyngeal cancer)), hematopoietic cancers (e.g., leukemia such as acute lymphocytic leukemia (ALL) (e.g., B-cell ALL, T-cell ALL), acute myelocytic leukemia (AML) (e.g. , B-cell AML, T-cell AML), chronic myelocytic leukemia (CML) (e.g. , B-cell CML, T-cell CML), and chronic lymphocytic leukemia (CLL) (e.g. , B-cell CLL, T- cell CLL), follicular lymphoma, chronic lymphocytic leukemia/small lymphocytic lymphoma (CLL/SLL), marginal zone B-cell lymphomas (e.g. , mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (e.g., "Waldenstrom's macro globulinemia"), hairy cell leukemia (HCL), immunoblastic large cell lymphoma, precursor B -lymphoblastic lymphoma and primary central nervous system (CNS) lymphoma; and T-cell NHL such as precursor T-lymphoblastic lymphoma/leukemia, peripheral T-cell lymphoma (PTCL) (e.g., cutaneous T-cell lymphoma (CTCL) (e.g. , mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma); a mixture of one or more leukemia/lymphoma as described above; and multiple myeloma (MM)), heavy chain disease (e.g., alpha chain disease, gamma chain disease, mu chain disease), hemangioblastoma, inflammatory myofibroblastic tumors, immunocytic amyloidosis, kidney cancer (e.g., nephroblastoma a.k.a. Wilms' tumor, renal cell carcinoma), liver cancer (e.g. , hepatocellular cancer (HCC), malignant hepatoma), lung cancer (e.g., bronchogenic carcinoma, small cell lung cancer (SCLC), non-small cell lung cancer (NSCLC), adenocarcinoma of the lung), leiomyosarcoma (LMS), mastocytosis (e.g. , systemic mastocytosis), myelodysplasia syndrome (MDS), mesothelioma, myeloproliferative disorder (MPD) (e.g., polycythemia Vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES)), neuroblastoma, neurofibroma (e.g. , neurofibromatosis (NF) type 1 or type 2, schwannomatosis), neuroendocrine cancer (e.g., gastroenteropancreatic neuroendoctrine tumor (GEP-NET), carcinoid tumor), osteosarcoma, ovarian cancer (e.g. , cystadenocarcinoma, ovarian embryonal carcinoma, ovarian adenocarcinoma), papillary adenocarcinoma, penile cancer (e.g., Paget' s disease of the penis and scrotum), pinealoma, primitive neuroectodermal tumor (PNT), prostate cancer (e.g., prostate adenocarcinoma), rectal cancer, rhabdomyosarcoma, salivary gland cancer, skin cancer (*e.g.* , squamous cell carcinoma (SCC), keratoacanthoma (KA), melanoma, basal cell carcinoma (BCC)), small bowel cancer (e.g. , appendix cancer), soft tissue sarcoma (*e.g*., malignant fibrous histiocytoma (MFH), liposarcoma, malignant peripheral nerve sheath tumor (MPNST), chondrosarcoma, fibrosarcoma, myxosarcoma), sebaceous gland carcinoma, sweat gland carcinoma, synovioma, testicular cancer (e.g., seminoma, testicular embryonal carcinoma), thyroid cancer (*e.g.,* papillary carcinoma of the thyroid, papillary thyroid carcinoma (PTC), medullary thyroid cancer), urethral cancer, vaginal cancer and vulvar cancer (*e.g*., Paget's disease of the vulva).

In some aspects, the method of treating cancer in a subject comprises administering a composition comprising a PRMT5 inhibitor to the subject, wherein treatment with the PRMT5 inhibitor inhibits tumor growth of the cancer by more than about 25%, more than about 50%, more than about 75%, more than about 90% (e.g., 25%-50%, 50%-75%, 75%- 90%, or 90%-100% for example). In some embodiments, the method of treating cancer in a subject comprises administering a composition comprising a PRMT5 inhibitor to the subject, wherein methyl mark of the cancer is reduced more than about 50%, more than about 75%, more than about 80% (e.g., 50%-75%, 50%-80%, 80%-90%, 80%-100%, or 90%-100% for example). A methyl mark refers to protein methylation, for example a histone methylation (e.g., methylation of one or more lysines and/or arginines of a histone protein), or DNA methylation (e.g., epigenetic DNA methylation, for example methylated CpG sites). In some embodiments, the methyl mark level of a cell is a measure of the extent to which histones are methylated in the cell (*e*.*g.,* at one or more particular lysine and/or arginine positions).

The invention is further described by reference to the following examples, which are intended to exemplify the claimed invention but not to limit it in any way.

### EXAMPLES

Unless otherwise noted, all materials were obtained from commercial suppliers and were used without further purification.

The following abbreviations are used to refer to various reagents, solvents, or instruments:

| | |
|---|---|
| AcOH | acetic acid |
| aq or aq. | aqueous |
| Boc | *tert*-butyloxycarbonyl |
| CLND | chemiluminescent nitrogen detection |
| CMPI | 2-Chloro-1-methylpyridinium iodide |
| DAD | diode array detector |
| DCE | 1,2-dichloroethane |
| DCM | dichloromethane |
| DEA | diethylamine |
| DIAD | diisopropyl azodicarboxylate |
| DMA or DMAc | *N*,*N*-dimethylacetamide |
| DMF | *N,N-*dimethylformamide |
| DMSO | dimethyl sulfoxide |
| dppf | 1,1'-bis(diphenylphosphino)ferrocene |
| EDC·HCl or EDCI | 3-((ethylimino)methyleneamino)-*N*,*N*-dimethylpropan-1-amonium chloride |
| ESI or ES | electrospray ionization |
| Et | ethyl |
| Et₂O | diethyl ether |
| EtOH | ethyl alcohol |
| EtOAc | ethyl acetate |
| g | grams |
| h | hour |
| HPLC | high pressure liquid chromatography |
| HATU | 1-[bis(dimethylamino)methylene]-1*H*-1,2,3-triazolo[4,5-*b*]pyridinium 3-oxid hexafluorophosphate |
| HBTU | N,N,N',N'-Tetramethyl-O-(1H-benzotriazol-1-yl)uronium hexafluorophosphate, O-(Benzotriazol-1-yl)-N,N,N',N'-tetramethyluronium hexafluorophosphate |
| HOAt | 1-hydroxy-7-azabenzotriazole |
| iPr | isopropyl |
| *i*Pr₂NEt or DIPEA | *N*-ethyl diisopropylamine (Hünig's base) |
| LC MS, LCMS, LC-MS or LC/MS | liquid chromatography mass spectroscopy |
| LG | leaving group (e.g., halogen, mesylate, triflate) |
| LiHMDS | lithium bis(trimethylsilyl)amide |
| m/z | mass divided by charge |
| Me | methyl |
| MeCN/ACN | acetonitrile |
| MeOH | methanol |
| Met | metal species for cross-coupling (e.g., MgX, ZnX, SnR₃, SiR₃, B(OR)₂) |
| mg | milligrams |
| min | minutes |
| mL | milliliters |
| MS | mass spectra |
| MsCl | methanesulfonyl chloride |
| MTBE | *tert-*butyl methyl ether |
| NMP | 1-methyl-2-pyrrolidine |
| *n*-BuLi | n-butyllithium |
| NMR | nuclear magnetic resonance |
| Pd₂(dba)₃ | tris(dibenzylideneacetone)dipalladium(0) |
| Pd(dppf)Cl₂·DCM | [1,1'-bis(diphenylphosphino)ferrocene]dichloropalladium(II), complex with DCM |
| Pd(PPh₃)₄ | tetrakis(triphenylphosphine)palladium(0) |
| Ph | phenyl |
| PG or Prot. group | protecting group |
| Prep | preparative |
| PyBrOP | bromotripyrrolidinophosphonium hexafluorophosphate |
| rbf | round-bottom flask |
| RP-HPLC | reverse phase high pressure liquid chromatography |
| RT or rt | room temperature |
| R.T. | retention time |
| RuPhos | 2-dicyclohexylphosphino-2',6'-diisopropoxybiphenyl |
| sat. or sat'd | saturated |
| SFC | supercritical fluid chromatography |
| *t*-BuOH | *tert-*butanol |
| TEA or Et₃N | triethylamine |
| TEOS | tetraethyl orthosilicate |
| TFA | trifluoroacetic acid |
| THF | tetrahydrofuran |
| TBTU | N,N,N',N'-Tetramethyl-O-(benzotriazol-1-yl)uronium tetrafluoroborate |
| TOF | time of flight |
| UHPLC | ultra-high-performance liquid chromatography |
| Xantphos | 4,5-bis(diphenylphosphino)-9,9-dimethylxanthene |

### General Synthetic Schemes:

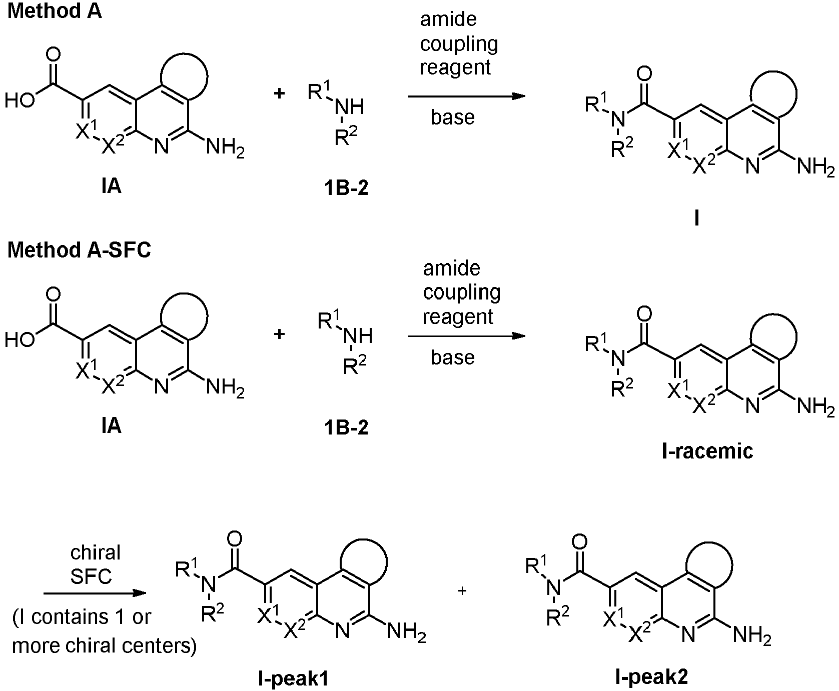

### Method B

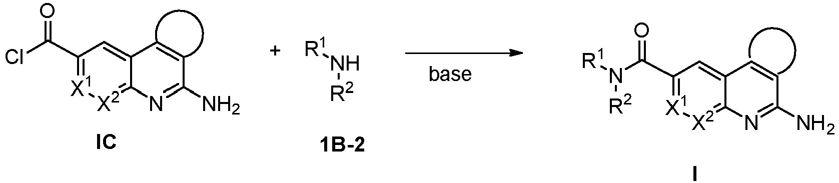

### Method B-SFC

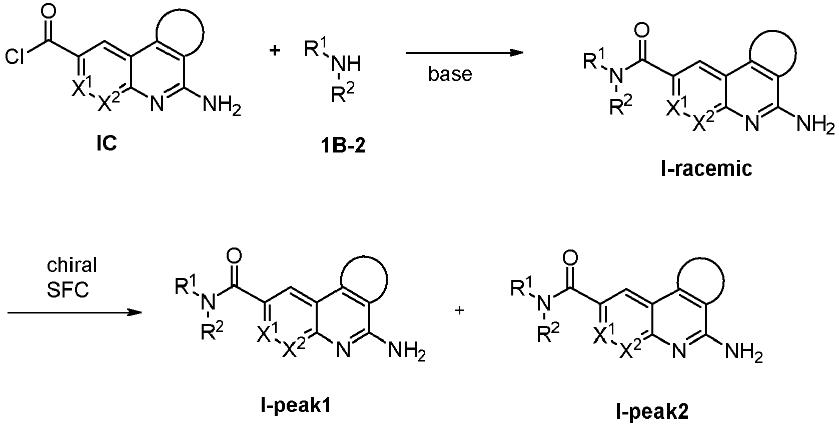

Method A: Compound **I** can be prepared from the reaction of acid **IA** and secondary amine **IB-1** in the presence of a base such as Et₃N or DIPEA, an activating reagent such as HATU or PyBrOP, in a solvent such as DMF or DMAc. If racemic amine or acid is employed in Method A, chiral SFC can be used to separate the stereoisomers, in which case stereochemistry was arbitrarily assigned to each isomer. Method B: Compound **I** can be prepared from the reaction of acid chloride **IC** and secondary amine **IB** in the presence of a base such as Et₃N or DIPEA or pyridine, in a solvent such as THF or dioxane or DCM or DCE. Alternatively, compound **I** can be prepared from the reaction of acid chloride **IC** and secondary amine **IB** in the presence of DMAP in pyridine. If racemic amine or acid is employed in Method B, chiral SFC can be used to separate the stereoisomers, in which case stereochemistry was arbitrarily assigned to each isomer.

### Analytical U/HPLC

The following equipment was used for analytical UHPLC: Waters Acquity system equipped with an Acquity BEH C18 (1.7µm, 2.1 x 50 mm) with a linear gradient of a binary solvent system using a flow rate of 0.5 mL/min and DAD at ambient temperature, combined with MS detection SQD I. Linear gradients used (H₂O/CH₃CN/HCO₂H (95/5/0.1% to 0/100/0.1%)). Agilent Infinity I/II -TOF6230B /CLND Antek 8060 equipped with Acquity BEH C18 (1.7µm, 2.1 x 50 mm) with a linear gradient of a binary solvent system using a flow rate of 0.75 mL/min combined with DAD. Linear gradients used (H₂O/MeOH/HCO₂H (95/5/0.1% to 0/100/0.1%)).

### Preparative HPLC

The following equipment was used for Prep-HPLC: Shimadzu Nexera X2 equipped with a Merck Chromolith SpeedROD RP-18E (5µm, 10 x 100 mm) with a linear gradient of a binary solvent system using a flow rate between 4 and 7 mL/min and UV detection at 254 nm, combined with MS detecting on a Shimadzu LCMS-2020. Linear gradients used (H₂O/MeOH/HCO₂H (95/5/0.1% to 0/100/0.1%)).

### Intermediates

### Intermediate 1: N-methyl-2-(trifluoromethyl)-6,7-dihydro-5H-cyclopenta[b]pyridin-5-amine

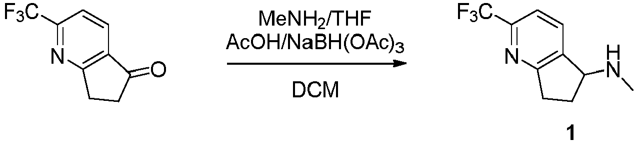

To a stirred mixture of 2-(trifluoromethyl)-6,7-dihydro-5H-cyclopenta[b]pyridin-5-one (1.50 g, 7.46 mmol, Angel Pharmatech) and aminomethane (2 M solution in THF) (9.32 mL, 18.64 mmol, Sigma-Aldrich Corporation) in DCM (7 mL) was added acetic acid (1.120 g, 1.076 mL, 18.64 mmol, Sigma-Aldrich Corporation). The resulting mixture was stirred at rt for 10 min before sodium triacetoxyborohydride (2.055 g, 9.69 mmol, Sigma-Aldrich Corporation) was added in one portion as a solid. The resulting mixture was stirred at rt for 42 h. The reaction was quenched with methanol. The volatiles were removed in vacuo and the residue was basified at 0 °C with ammonium hydroxide, directly loaded onto a silica gel precolumn (25 g), and subjected to combi-flash column chromatography on a 24-g ISCO gold column eluting with MeOH (with 0.5% ammonium hydroxide)/DCM (0 to 20%) (2 x) to give N-methyl-2-(trifluoromethyl)-6,7-dihydro-5H-cyclopenta[b]pyridin-5-amine (1) (1.40 g, 6.48 mmol, 87 % yield) as a dark-colored solid. *m*/*z* (ESI): 217.20 (M+H)⁺. ¹H NMR (CHLOROFORM-d, 400 MHz) δ 7.78 (d, 1H, J=7.7 Hz), 7.52 (d, 1H, J=7.7 Hz), 4.26 (t, 1H, J=6.9 Hz), 3.1-3.2 (m, 1H), 3.0-3.1 (m, 1H), 2.5-2.6 (m, 4H), 1.9-2.0 (m, 1H), 1.31 (br s, 1H). ¹⁹F NMR (CHLOROFORM-d, 376 MHz) δ -67.39 (s, 3F).

The following amines in Table 1 were prepared in a manner similar to that described for Intermediate **1.**

**Table 1**

| **Int. #** | **Chemical Structure** | **Name** | ***m*/*z* (ESI): (M+H)⁺** |
|---|---|---|---|
| **2** | 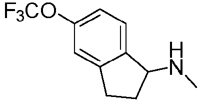 | N-methyl-5-(trifluoromethoxy)-2,3-dihydro-1H-inden-1-amine | 232.15 |
| **3** | 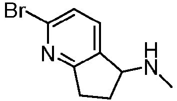 | 2-bromo-N-methyl-6,7-dihydro-5H-cyclopenta[b]pyridin-5-amine | 227, 229 |
| **4** | 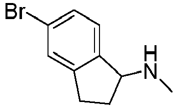 | 5-bromo-N-methyl-2,3-dihydro-1H-inden-1-amine | 226, 228 |
| **5** | 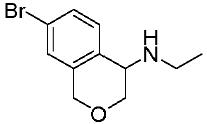 | 7-bromo-N-ethylisochroman-4-amine | 256.15, 258.10 |
| **6** | 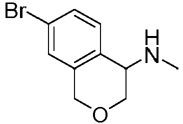 | 7-bromo-N-methylisochroman-4-amine | 242.05, 244.00 |
| **7** | 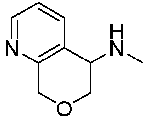 | N-methyl-5,8-dihydro-6H-pyrano[3,4-b]pyridin-5-amine | 165.1 |
| **8** | 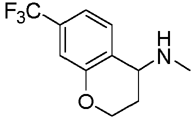 | N-methyl-7-(trifluoromethyl)chroman-4-amine | 232.00 |
| **9** | 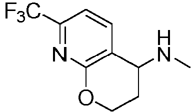 | N-methyl-7-(trifluoromethyl)-3,4-dihydro-2H-pyrano[2,3-b]pyridin-4-amine | 233.2 |

### Intermediate 11: N-methyl-6-(trifluoromethyl)-2,3-dihydrofuro[2,3-b]pyridin-3-amine

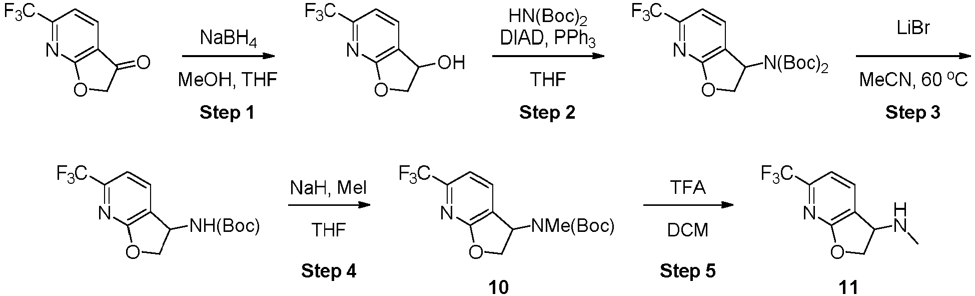

Step 1. To a stirred ice-cooled solution of 6-(trifluoromethyl)furo[2,3-b]pyridin-3(2H)-one (3.000 g, 14.77 mmol, eNovation) in tetrahydrofuran (25 mL) and MeOH (20 mL) was added under nitrogen sodium borohydride (0.950 g, 25.1 mmol, Sigma-Aldrich Corporation) in one portion as a solid. The resulting mixture was stirred at 0 °C for 20 min and at ambient temperature for 15 min. The volatiles were removed in vacuo and the residue was directly loaded onto a silica gel precolumn (25 g) and subjected to combi-flash column chromatography on a 40-g ISCO gold column eluting with MeOH/DCM (0 to 16%) to give 6-(trifluoromethyl)-2,3-dihydrofuro[2,3-b]pyridin-3-ol (2.65 g, 12.92 mmol, 87 % yield) as a colorless oil. *m*/*z* (ESI): 206.20 (M+H)⁺. ¹H NMR (CHLOROFORM-d, 400 MHz) δ 7.89 (d, 1H, J=7.3 Hz), 7.32 (d, 1H, J=7.5 Hz), 5.51 (br d, 1H, J=2.7 Hz), 4.75 (dd, 1H, J=7.1, 10.9 Hz), 4.57 (dd, 1H, *J=3.1,* 10.9 Hz), 2.41 (br d, 1H, J=7.1 Hz). ¹⁹F NMR (CHLOROFORM-d, 376 MHz) δ -67.96 (s, 3F).

Step 2. To a stirred ice-cooled solution of 6-(trifluoromethyl)-2,3-dihydrofuro[2,3-b]pyridin-3-ol (1.300 g, 6.34 mmol), bis(Boc)amine (1.446 g, 6.65 mmol, Oakwood Products) and triphenyl phosphine (1.745 g, 6.65 mmol, Sigma-Aldrich) in THF (20 mL) was slowly added under nitrogen via a syringe a solution of diisopropyl azodiformate (1.346 g, 1.310 mL, 6.65 mmol, Oakwood Products) in THF (6 mL) over a period of 15 min. The resulting mixture was allowed to warm to rt and stirred at rt overnight. The volatiles were removed, and the crude residue was directly loaded onto a silica gel precolumn (25 g) and subjected to combi-flash column chromatography on a 40-g ISCO gold column eluting with MeOH/DCM (0 to 1%) to give an impure product. This was dissolved in DCM and loaded onto a silica gel precolumn (25 g) and subjected to combi-flash column chromatography on a 40-g ISCO gold column eluting with EtOAc/heptane (0 to 40%) to give impure N,N-bis(Boc)-6-(trifluoromethyl)-2,3-dihydrofuro[2,3-b]pyridin-3-amine (1.75 g, 4.33 mmol, 68 % yield) as a white solid. *m*/*z* (ESI): 426.80 (M+Na)⁺. ¹H NMR (CHLOROFORM-d, 400 MHz) δ 7.67 (d, 1H, J=7.7 Hz), 7.25 (d, 1H, J=7.5 Hz), 6.17 (dd, 1H, J=5.5, 10.3 Hz), 4.8-4.9 (m, 1H), 4.70 (dd, 1H, *J=*5.4, 10.0 Hz), 1.40 (s, 18H). ¹⁹F NMR (CHLOROFORM-d, 376 MHz) δ -67.95 (s, 3F).

Step 3. A mixture of N,N-bis(Boc)-6-(trifluoromethyl)-2,3-dihydrofuro[2,3-b]pyridin-3-amine (3.65 g, 9.03 mmol) and lithium bromide (2.352 g, 27.1 mmol, Sigma-Aldrich Corporation) in acetonitrile (55 mL) in a 250-mL RBF was stirred at 58 °C for 18 h. The volatiles were removed and the crude residue was directly loaded onto a silica gel precolumn (25 g) and subjected to combi-flash column chromatography on a 40-g ISCO gold column eluting with MeOH/DCM (0 to 8%) (3 x) to give an impure tert-butyl (6-(trifluoromethyl)-2,3-dihydrofuro[2,3-b]pyridin-3-yl)carbamate (1.46 g, 4.80 mmol, 53% yield) as a white solid, which was taken onto the next step without further purification. *m*/*z* (ESI): 326.95 (M+Na)⁺. ¹H NMR (CHLOROFORM-d, 400 MHz) δ 7.84 (br d, 1H, J=7.3 Hz), 7.29 (d, 1H, J=7.5 Hz), 5.50 (br d, 1H, J=2.1 Hz), 4.7-5.1 (m, 2H), 4.44 (dd, 1H, J=4.7, 10.3 Hz), 1.48 (s, 9H). ¹⁹F NMR (CHLOROFORM-d, 376 MHz) δ -67.98 (s, 3F).

Step 4. To a stirred solution of tert-butyl (6-(trifluoromethyl)-2,3-dihydrofuro[2,3-b]pyridin-3-yl)carbamate (935 mg, 3.07 mmol) in THF (20 mL) was added under nitrogen, sodium hydride, 60% in mineral oil (307 mg, 7.68 mmol, Sigma-Aldrich Corporation) in two portions over 5 min. The resulting mixture was stirred at 0 °C for 15 min before iodomethane (872 mg, 0.383 mL, 6.15 mmol, Sigma-Aldrich Corporation) was added slowly dropwise via a syringe. The resulting mixture was stirred at 0 °C for 15 min and at rt for 1 h. The reaction was cooled in an ice-water bath before quenched with MeOH (3 mL). The volatiles were removed in vacuo and the residue was dissolve in DCM/MeOH, loaded onto a silica gel precolumn (25 g), and subjected to combi-flash column chromatography on a 24-g ISCO gold column eluting with MeOH/DCM (0 to 2%) to give tert-butyl methyl(6-(trifluoromethyl)-2,3-dihydrofuro[2,3-b]pyridin-3-yl)carbamate (10) (950 mg, 2.98 mmol, 97 % yield) as a nearly colorless oil, which solidified at rt upon standing. *m*/*z* (ESI): 340.90 (M+Na)⁺. ¹H NMR (CHLOROFORM-d, 400 MHz) δ 7.71 (br d, 1H, J=7.3 Hz), 7.30 (d, 1H, J=7.3 Hz), 5.5-6.4 (m, 1H), 4.79 (dd, 1H, J=9.5, 10.3 Hz), 4.3-4.6 (m, 1H), 2.5-2.8 (m, 3H), 1.49 (br s, 9H). ¹⁹F NMR (CHLOROFORM-d, 376 MHz) δ -67.95 (s, 3F).

Step 5. To a stirred ice-cooled solution of tert-butyl methyl(6-(trifluoromethyl)-2,3-dihydrofuro[2,3-b]pyridin-3-yl)carbamate (10) (1.160 g, 3.64 mmol) in DCM (20 mL) was added 2,2,2-trifluoroacetic acid (1.039 g, 3.0 mL, 9.11 mmol, Sigma-Aldrich Corporation) dropwise via a syringe. The resulting mixture was stirred at rt for 2 h. The volatiles were removed in vacuo and the residue was dissolved in DCM/MeOH and ammonium hydroxide (0.4 mL) and loaded onto a silica gel precolumn (25 g) and subjected to combi-flash column chromatography on a 24-g ISCO gold column eluting with MeOH (with 0.5% ammonium hydroxide)/DCM (1 to 20%) (2 x) to give N-methyl-6-(trifluoromethyl)-2,3-dihydrofuro[2,3-b]pyridin-3-amine (11) (625 mg, 2.86 mmol, 79 % yield) as a lightly brownish film with a total yield of about 20% over 5 steps. *m*/*z* (ESI): 219.10 (M+H)⁺. ¹H NMR (CHLOROFORM-d, 400 MHz) δ 7.78 (d, 1H, J=7.3 Hz), 7.0-7.4 (m, 1H), 4.7-4.8 (m, 1H), 4.4-4.6 (m, 2H), 2.48 (s, 3H), 1.3-1.6 (m, 1H). ¹⁹F NMR (CHLOROFORM-d, 376 MHz) δ -67.92 (s, 3F).

### Intermediate 12: N-methyl-6-(trifluoromethyl)-2,3-dihydrobenzofuran-3-amine

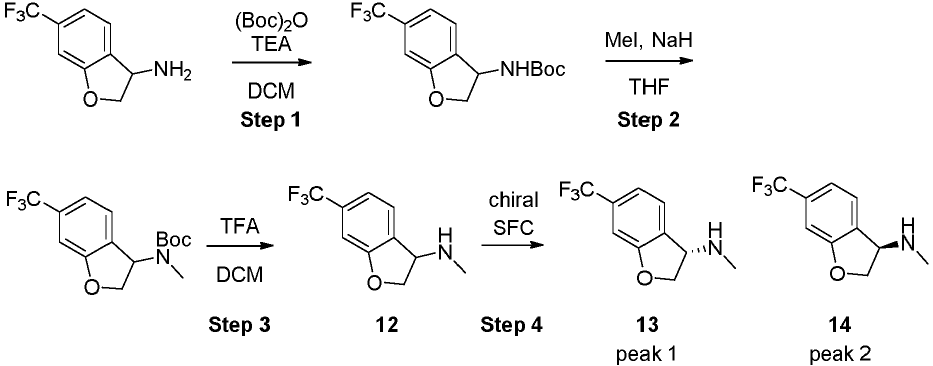

Step 1. To a stirred ice-cooled solution of 6-(trifluoromethyl)-2,3-dihydrobenzofuran-3-amine (0.440 g, 2.166 mmol, eNovation) and triethylamine (0.219 g, 0.304 mL, 2.166 mmol, Sigma-Aldrich Corporation) in DCM (8 mL) was added di-tert-butyl dicarbonate (0.473 g, 2.166 mmol, TCI America). The resulting mixture was stirred at 0 °C for 15 min and at rt for 2 days. The crude mixture was directly loaded onto a silica gel precolumn (25 g) and subjected to combi-flash column chromatography on a 24-g ISCO gold column eluting with EtOAc/heptane (0 to 60%) to give tert-butyl (6-(trifluoromethyl)-2,3-dihydrobenzofuran-3-yl)carbamate (630 mg, 2.077 mmol, 96% yield) as an off-white solid. *m*/*z* (ESI): 303.10 (M+H)⁺. ¹H NMR (CHLOROFORM-d, 400 MHz) δ 7.45 (d, 1H, J=7.9 Hz), 7.20 (d, 1H, J=7.7 Hz), 7.08 (s, 1H), 5.42 (br s, 1H), 4.86 (br d, 1H, *J=3.1* Hz), 4.75 (dd, 1H, J=8.3, 9.9 Hz), 4.40 (dd, 1H, *J=*4.5, 10.1 Hz), 1.48 (s, 9H). ¹⁹F NMR (CHLOROFORM-d, 376 MHz) δ -62.52 (s, 3F).

Step 2. To a stirred ice-cooled solution of tert-butyl (6-(trifluoromethyl)-2,3-dihydrobenzofuran-3-yl)carbamate (330 mg, 1.088 mmol) in THF (5 mL) was added under nitrogen, sodium hydride 60% in mineral oil (65.3 mg, 1.632 mmol, Sigma-Aldrich Corporation). The resulting mixture was stirred at 0 °C for 15 min before iodomethane (154 mg, 0.154 mL, 1.088 mmol, Sigma-Aldrich Corporation) was added via a syringe. The resulting mixture was stirred at 0 °C for 15 min and at ambient temperature for 16 h. The reaction mixture was again cooled in an ice bath before it was quenched with MeOH. The volatiles were removed in vacuo and the residue was directly loaded onto a silica gel precolumn (25 g) and subjected to combi-flash column chromatography on a 12-g ISCO gold column eluting with EtOAc/heptane (0 to 40%) to give tert-butyl methyl(6-(trifluoromethyl)-2,3-dihydrobenzofuran-3-yl)carbamate (340 mg, 1.072 mmol, 98% yield) as a colorless oil, which was taken onto the next step without further purification. *m*/*z* (ESI): 340.15 (M+Na)⁺.

Step 3. To a stirred solution of tert-butyl methyl(6-(trifluoromethyl)-2,3-dihydrobenzofuran-3-yl)carbamate (340 mg, 1.072 mmol) in DCM (2 mL) was added 2,2,2-trifluoroacetic acid (122 mg, 2.0 mL, 1.072 mmol, Sigma-Aldrich Corporation). The resulting mixture was stirred at rt for 2 h. The volatiles were removed in vacuo. The residue was carefully basified with ammonium hydroxide (0.5 mL). The crude residue was directly loaded onto a silica gel precolumn (25 g) previously covered with a layer of Na₂CO₃, and subjected to combi-flash column chromatography on a 12-g ISCO gold column, eluting with MeOH (with 0.5% ammonium hydroxide)/DCM (1 to 20%) to give N-methyl-6-(trifluoromethyl)-2,3-dihydrobenzofuran-3-amine **(12)** (140 mg, 0.645 mmol, 60.2% yield) as a colorless oil. Note that the low yield was due to loss of material during rotary evaporation due to its high volatility. No higher than 32 °C was later found optimal for handling the compound without loss. ¹H NMR (CHLOROFORM-d, 400 MHz) δ 7.43 (d, 1H, J=7.7 Hz), 7.18 (d, 1H, J=7.7 Hz), 7.07 (s, 1H), 4.6-4.7 (m, 1H), 4.4-4.5 (m, 2H), 2.46 (s, 3H). *m*/*z* (ESI): 218.20 (M+H)⁺.

Step 4. The racemate was separated via preparative SFC using a Chiral Technologies IG column (250 x 21 mm, 5 mm) x 2 with a mobile phase of 80% Liquid CO₂ and 20% MeOH with 0.2% TEA using a flow rate of 60 mL/min to generate (S)-N-methyl-6-(trifluoromethyl)-2,3-dihydrobenzofuran-3-amine **(13)** as peak 1 with an ee of > 99% and (R)-N-methyl-6-(trifluoromethyl)-2,3-dihydrobenzofuran-3-amine **(14)** as peak 2 with an ee of > 99%.

The following amine intermediates in Table 2 were prepared in a manner similar to that described for Intermediate **12.** Chiral amines in Table 2 were synthesized from the corresponding chiral primary amines (Intermediate **25** from (3S)-6-Bromo-2,3-dihydro-3-benzofuranamine, CAS#1228568-69-1).

**Table 2**

| **Int. #** | **Chemical Structure** | **Name** | ***m*/*z* (ESI): (M+H)⁺** |
|---|---|---|---|
| **15** | 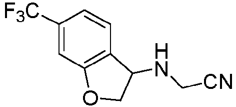 | 2-((6-(trifluoromethyl)-2,3-dihydrobenzofuran-3-yl)amino)acetonitrile | 243.0 |
| **16** | 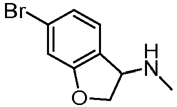 | 6-bromo-N-methyl-2,3-dihydrobenzofuran-3-amine | 197.0, 199.0 |
| **17** | 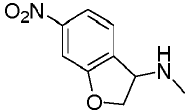 | N-methyl-6-nitro-2,3-dihydrobenzofuran-3-amine | 317.20 |
| **18** | 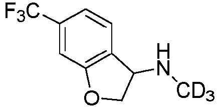 | N-(methyl-d3)-6-(trifluoromethyl)-2,3-dihydrobenzofuran-3-amine | 221.10 |
| **19** | 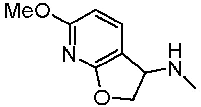 | 6-methoxy-N-methyl-2,3-dihydrofuro[2,3-b]pyridin-3-amine | 181.15 |
| **20** | 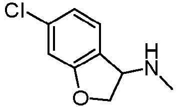 | 6-chloro-N-methyl-2,3-dihydrobenzofuran-3-amine | 184.2 |
| **21** | 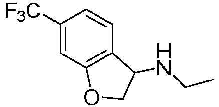 | N-ethyl-6-(trifluoromethyl)-2,3-dihydrobenzofuran-3-amine | 232 |
| **22** | 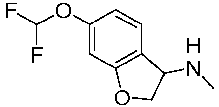 | 6-(difluoromethoxy)-N-methyl-2,3-dihydrobenzofuran-3-amine | 216.1 |
| **23** | 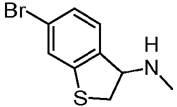 | 6-bromo-N-methyl-2,3-dihydrobenzo[b]thiophen-3-amine | 242.97 |
| **24** | 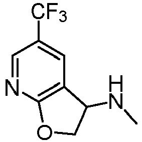 | N-methyl-5-(trifluomethyl)-2,3-dihydrofuro[2,3-b]pyridin-3-amine | 241.0 (M+Na)+ |
| **25** | 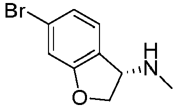 | (S)-6-bromo-N-methyl-2,3-dihydrobenzofuran-3-amine | 250, 252 (M+Na)+ |

### Intermediate 27: 3-(methylamino)-2,3-dihydrobenzofuran-6-carbonitrile

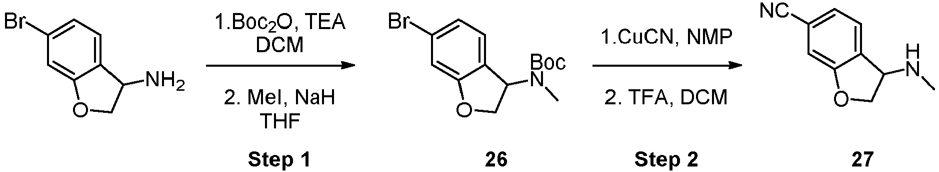

Step 1. To a stirred ice-cooled solution of 6-bromo-2,3-dihydrobenzofuran-3-amine (2.000 g, 9.34 mmol, Aurum Pharmatech) and triethylamine (1.040 g, 1.444 mL, 10.28 mmol, Sigma-Aldrich Corporation) in DCM (14 mL) was added di-tert-butyl dicarbonate (2.039 g, 9.34 mmol, TCI America). The resulting mixture was stirred at 0 °C for 15 min and then at rt for 48 h. The crude mixture was directly loaded on a silica gel precolumn (25 g) and subjected to combi-flash column chromatography on a 40-g ISCO gold column eluting with MeOH/DCM (0 to 4%) to give tert-butyl (6-bromo-2,3-dihydrobenzofuran-3-yl)carbamate (2.79 g, 8.88 mmol, 95% yield) as an off-white solid. *m*/*z* (ESI): 335.95 and 338.05 (M+Na)⁺. ¹H NMR (CHLOROFORM-d, 400 MHz) δ 7.20 (d, 1H, J=7.9 Hz), 7.06 (dd, 1H, J=1.7, 7.9 Hz), 7.01 (d, 1H, J=1.7 Hz), 5.32 (br s, 1H), 4.82 (br s, 1H), 4.70 (dd, 1H, J=8.2, 10.0 Hz), 4.36 (dd, 1H, J=4.2, 10.0 Hz), 1.47 (s, 9H).

To a stirred ice-cooled solution of tert-butyl (6-bromo-2,3-dihydrobenzofuran-3-yl)carbamate (1.46 g, 4.65 mmol) in THF (18 mL) was added under nitrogen sodium hydride, 60% in mineral oil (0.279 g, 6.97 mmol, Aldrich) in two aliquots. The resulting mixture was stirred at 0 °C for 15 min before iodomethane (0.660 g, 0.289 mL, 4.65 mmol, Sigma-Aldrich Corporation) was added via a syringe. The resulting mixture was stirred at 0 °C for 15 min and at ambient temperature for 2 days. The reaction mixture was cooled in an ice bath before it was quenched with MeOH. The volatiles were removed in vacuo and the residue was directly loaded onto a silica gel precolumn (25 g) and subjected to combi-flash column chromatography on a 24-g ISCO gold column eluting with MeOH/DCM (0 to 2%) to give tert-butyl (6-bromo-2,3-dihydrobenzofuran-3-yl)(methyl)carbamate (26) (1.55 g, 4.72 mmol, 102 % yield) as a coloress oil. *m*/*z* (ESI): 350.05 and 352.00 (M+Na)⁺. ¹H NMR (CHLOROFORM-d, 400 MHz) δ 7.0-7.2 (m, 2H), 7.01 (d, 1H, *J=1.5* Hz), 5.6-6.2 (m, 1H), 4.63 (br t, 1H, J=9.6 Hz), 4.39 (br dd, 1H, J=3.4, *10.1* Hz), 2.55 (br s, 3H), 1.50 (s, 9H).

Step 2. To a stirred solution of tert-butyl (6-bromo-2,3-dihydrobenzofuran-3-yl)(methyl)carbamate (200 mg, 0.609 mmol) in NMP (3.5 mL) in a 10-mL microwave vessel was added cyanocopper (218 mg, 2.437 mmol, Sigma-Aldrich Corporation). The vessel was sealed and subjected to microwave condition (4 h at 135 °C). The crude was directly loaded onto a silica gel precolumn (25 g) and subjected to combi-flash column chromatography on a 24-g ISCO gold column eluting with MeOH/DCM (25 min from 0 to 1%) to give 240 mg of an impure mixture of tert-butyl (6-cyano-2,3-dihydrobenzofuran-3-yl)(methyl)carbamate and the unreacted starting material as a nearly colorless oil, which was taken onto the next step without further purification. m/z (ESI): 365.00 (M+Na)⁺.

To a stirred solution of a mixture of tert-butyl (6-cyano-2,3-dihydrobenzofuran-3-yl)(methyl)carbamate (220 mg, 0.802 mmol) and tert-butyl (6-bromo-2,3-dihydrobenzofuran-3-yl)(methyl)carbamate (263 mg, 0.802 mmol) (240 mg as the total weight of the impure mixture) in DCM (6 mL) was added at rt 2,2,2-trifluoroacetic acid (366 mg, 3 mL, 3.21 mmol, Sigma-Aldrich Corporation) via a syringe. The resulting mixture was stirred at rt for 1 h. The volatiles were removed to give a crude mixture of 3-(methylamino)-2,3-dihydrobenzofuran-6-carbonitrile (27) and its bromo counterpart resulting from tert-butyl (6-bromo-2,3-dihydrobenzofuran-3-yl)(methyl)carbamate as an oil. This was diluted in MeOH/DCM and filtered through a layer of solid sodium carbonate to remove the residual acid and the filtrate was concentrated in vacuo. The crude 3-(methylamino)-2,3-dihydrobenzofuran-6-carbonitrile was taken onto the next step. *m*/*z* (ESI): 197.00 (M+Na)⁺.

### Intermediate 29: 3-(methylamino)-2,3-dihydrobenzofuran-6-carbonitrile

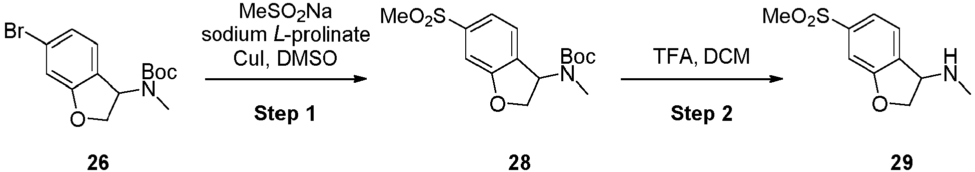

Step 1. To a mixture of tert-butyl (6-bromo-2,3-dihydrobenzofuran-3-yl)(methyl)carbamate (26) (330 mg, 1.005 mmol), methanesulfinic acid, sodium salt (205 mg, 2.011 mmol, TCI America), (S)-pyrrolidine-2-carboxylic acid, sodium salt (55.1 mg, 0.402 mmol, Combi-Blocks), and copper (i) iodide (38.3 mg, 0.201 mmol, Sigma-Aldrich Corporation) in a 5-mL microwave vessel was added dimethyl sulfoxide (2.5 mL). The resulting solution was purged with nitrogen for 10 min before it was sealed and subjected to microwave irradiation (16 h at 90 °C). The crude was directly loaded onto a silica gel precolumn (25 g) and subjected to combi-flash column chromatography on a 24-g ISCO gold column eluting with MeOH (with 0.5% ammonium hydroxide)/DCM (0 to 4%) to give tert-butyl methyl(6-(methylsulfonyl)-2,3-dihydrobenzofuran-3-yl)carbamate **(28)** (300 mg, 0.916 mmol, 91% yield) as a colorless film. *m*/*z* (ESI): 350.05 (M+Na)⁺. ¹H NMR (CHLOROFORM-d, 400 MHz) δ 7.51 (dd, 1H, J=1.6, 7.8 Hz), 7.40 (br d, 1H, J=7.7 Hz), 7.36 (d, 1H, *J=1.5* Hz), 5.7-6.2 (m, 1H), 4.70 (t, 1H, J=9.8 Hz), 4.46 (br dd, 1H, J=3.8, 9.8 Hz), 3.04 (s, 3H), 2.55 (br s, 3H), 1.48 (s, 9H).

Step 2. To a stirred solution of tert-butyl methyl(6-(methylsulfonyl)-2,3-dihydrobenzofuran-3-yl)carbamate **(28)** (300 mg, 0.916 mmol) in DCM (8 mL) was added 2,2,2-trifluoroacetic acid (120 mg, 2.0 mL, 1.054 mmol, Sigma-Aldrich Corporation) at rt. The resulting mixture was stirred at rt for 1 h. The volatiles were removed and the residue was dissolved in MeOH/DCM and ammonium hydroxide (0.4 mL). The crude residue was directly loaded onto a silica gel precolumn (25 g) previously covered by a layer of sodium carbonate, and subjected to combi-flash column chromatography on a 24-g ISCO gold column eluting with MeOH (with 0.5% ammonium hydroxide)/DCM (2 to 20%) to give N-methyl-6-(methylsulfonyl)-2,3-dihydrobenzofuran-3-amine **(29)** (190 mg, 0.836 mmol, 91% yield) as a colorless oil. *m*/*z* (ESI): 228.00 (M+H)⁺. ¹H NMR (CHLOROFORM-d, 400 MHz) δ 7.4-7.6 (m, 2H), 7.34 (s, 1H), 4.6-4.7 (m, 1H), 4.4-4.5 (m, 2H), 3.02 (s, 3H), 2.44 (s, 3H), 1.40 (br s, 1H).

### Intermediate 30: 6-(isopropylsulfonyl)-N-methyl-2,3-dihydrobenzofuran-3-amine

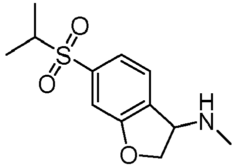

Intermediate **30** was prepared in a similar fashion to Intermediate 29 above, *m*/*z* (ESI): 256.1 (M+H)⁺.

### Intermediate 31: N-cyclopropyl-6-(trifluoromethyl)-2,3-dihydrobenzofuran-3-amine

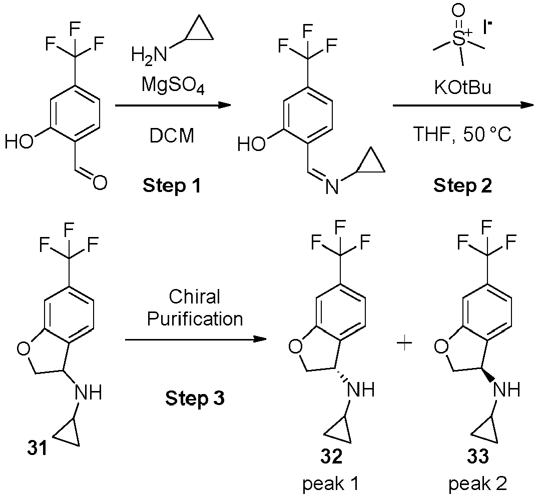

Step 1. A mixture of 2-hydroxy-4-(trifluoromethyl)benzaldehyde (2.00 g, 10.52 mmol, PharmaBlock), cyclopropylamine (1.201 g, 21.04 mmol, Acros), and anhydrous magnesium sulfate (5.06 g, 42.1 mmol, Sigma-Aldrich Corporation) in DCM (20 mL) was stirred at rt for 18 hours. The reaction mixture was filtered under gravity and the filtrate concentrated to afford (Z)-2-((cyclopropylimino)methyl)-5-(trifluoromethyl)phenol (2.1 g, 9.16 mmol, 87% yield) as yellow solid. The product was used in next step without further purification. *m*/*z* (ESI): 230.0 (M+H)⁺. ¹H NMR (400 MHz, DMSO-*d*₆) δ ppm 12.98 (br s, 1 H) 8.83 (s, 1 H) 7.67 (d, J=7.94 Hz, 1 H) 7.24 (dd, J=7.94, 1.05 Hz, 1 H) 7.17 (s, 1 H) 3.20 (tt, J=6.85, 3.40 Hz, 1 H) 0.98 - 1.09 (m, 2 H) 0.89 - 0.98 (m, 2 H). ¹⁹F NMR (376 MHz, DMSO-*d*₆) δ ppm -61.60 (s, 3 F).

Step 2. To a suspension of trimethylsulfoxonium iodide (2.54 g, 11.56 mmol, Sigma-Aldrich Corporation) in tetrahydrofuran (20 mL) was added potassium t-butoxide (1.297 g, 11.56 mmol, Sigma-Aldrich Corporation) portion wise. The suspension was stirred at rt for 30 minutes and then treated with a solution of (Z)-2-((cyclopropylimino)methyl)-5-(trifluoromethyl)phenol (1.06 g, 4.62 mmol) in THF (4 mL) dropwise. The resulting suspension was stirred at rt for 1 hour and then at 50 °C for 3 hours. The reaction was cooled to rt, an additional 1 eq of potassium t-butoxide (0.519 g, 4.62 mmol, Sigma-Aldrich Corporation) was added, and the resulting suspension was stirred at rt for 12 hours. The reaction mixture was filtered, and the filtrate diluted with water and extracted with EtOAc. The organic layer was concentrated, and the residue purified with by column chromatography using EtOAc/EtOH (3:1) in heptane (0-60%) to afford N-cyclopropyl-6-(trifluoromethyl)-2,3-dihydrobenzofuran-3-amine (31, 0.750 g, 3.08 mmol, 66.7 % yield) as an oil. ¹H NMR (400 MHz, CHLOROFORM-d) δ ppm 7.46 (d, J=7.73 Hz, 1 H) 7.19 (d, J=7.59 Hz, 1 H) 7.08 (s, 1 H) 4.56 - 4.68 (m, 2 H) 4.49 (dd, J=9.41, 3.76 Hz, 1 H) 2.26 (tt, J=6.71, 3.42 Hz, 1 H) 1.75 - 2.06 (m, 1 H) 0.50 - 0.55 (m, 2 H) 0.41 - 0.46 (m, 2 H). ¹⁹F NMR (376 MHz, CHLOROFORM-*d*) δ ppm -62.36 (s, 3 F).

Step 3. N-cyclopropyl-6-(trifluoromethyl)-2,3-dihydrobenzofuran-3-amine (0.750 g) was purified via preparative SFC using a Chiral Technologies AD column (150 x 30 mm, 5 mm) with a mobile phase of 90% Liquid CO₂ and 10% MeOH with 0.2% TEA using a flow rate of 175 mL/min to generate (S)-N-cyclopropyl-6-(trifluoromethyl)-2,3-dihydrobenzofuran-3-amine (32) (0.413 g) as peak 1 with an ee of >99%. ¹H NMR (400 MHz, CHLOROFORM-d) δ ppm 7.46 (d, J=7.67 Hz, 1 H) 7.19 (d, J=7.67 Hz, 1 H) 7.08 (s, 1 H) 4.61 - 4.68 (m, 1 H) 4.56 - 4.61 (m, 1 H) 4.47 - 4.51 (m, 1 H) 2.23 - 2.29 (m, 1 H) 1.59 (br s, 1 H) 0.42 - 0.56 (m, 4 H). ¹⁹F NMR (376 MHz, CHLOROFORM-d) δ ppm -62.36 (s, 3 F). (R)-N-cyclopropyl-6-(trifluoromethyl)-2,3-dihydrobenzofuran-3-amine (33) (0.314 g) was isolated as peak 2 with an ee of 98.56%. ¹H NMR (400 MHz, CHLOROFORM-d) δ ppm 7.46 (d, J=7.88 Hz, 1 H) 7.19 (d, J=7.67 Hz, 1 H) 7.08 (s, 1 H) 4.56 - 4.68 (m, 2 H) 4.45 - 4.52 (m, 1 H) 2.26 (tt, J=6.66, 3.50 Hz, 1 H) 1.87 (br s, 1 H) 0.40 - 0.56 (m, 4 H). ¹⁹F NMR (376 MHz, CHLOROFORM-d) δ ppm -62.36 (s, 3 F).

The following amines in table 3 were prepared in a manner similar to that described for Intermediates **31-33.**

**Table 3**

| **Int. #** | **Chemical Structure** | **Name** | **SFC** | ***m*/*z* (ESI): (M+H)⁺** |
|---|---|---|---|---|
| **34** | 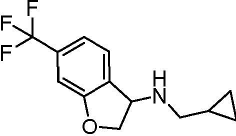 | N-(cyclopropylmethyl)-6-(trifluoromethyl)-2,3-dihydrobenzofuran-3-amine | | 258.1 |
| **35** | 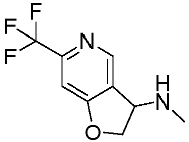 | N-methyl-6-(trifluoromethyl)-2,3-dihydrofuro[3,2-c]pyridin-3-amine | | 219.10 |
| **36** | 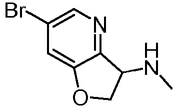 | 6-bromo-N-methyl-2,3-dihydrofuro[3,2-b]pyridin-3-amine | | 227.1, 229.0 |
| **37** | 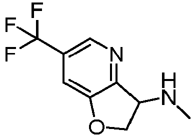 | N-methyl-6-(trifluoromethyl)-2,3-dihydrofuro[3,2-b]pyridin-3-amine | | 219.3 |
| **38** | 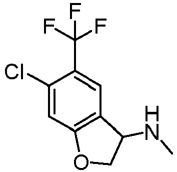 | 6-chloro-N-methyl-5-(trifluoromethyl)-2,3-dihydrobenzofuran-3-amine | | 252.1 |
| **39** | 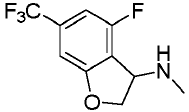 | 4-fluoro-N-methyl-6-(trifluoromethyl)-2,3-dihydrobenzofuran-3-amine | | 236.2 |
| **40** | 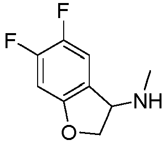 | 5,6-difluoro-N-methyl-2,3-dihydrobenzofuran-3-amine | Fmoc-protected before chiral purification; see Intermeidate **89** below | 184.1 (oxidized) |
| **41** | 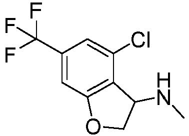 | 4-chloro-N-methyl-6-(trifluoromethyl)-2,3-dihydrobenzofuran-3-amine | Fmoc-protected before chiral purification; see Intermediates **90** and **91** below | 232.1 (oxidized) |
| **42** | 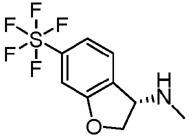 | Peak 1: (*S*)-N-methyl-6-(pentafluoro-l6-sulfaneyl)-2,3-dihydrobenzofuran-3-amine | Chiralpak AD column (21 x 300 mm) with a mobile phase of 85% Liquid CO₂ and 15% methanol with 0.2% TEA using a flow rate of 80 mL/min | 276.2 |
| **43** | 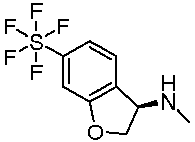 | Peak 2: (*R*)-N-methyl-6-(pentafluoro-l6-sulfaneyl)-2,3-dihydrobenzofuran-3-amine | | |
| **44** | 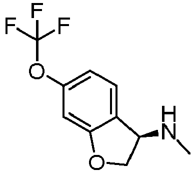 | Peak 1: (R)-N-methyl-6-(trifluoromethoxy)-2,3-dihydrobenzofuran-3-amine | Chiralpak IG column (250 x 4.6 mm) with a mobile phase of 90% Liquid CO₂ and 10% MeOH with 0.2% isopropylamine, using a CO₂ flow rate of 2.7 mL/min and a cosolvent flow rate of 0.3 mL/min | No ion |
| **45** | 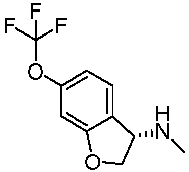 | Peak 2: (S)-N-methyl-6-(trifluoromethoxy)-2,3-dihydrobenzofuran-3-amine | | |
| **46** | 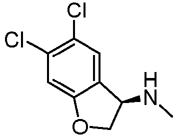 | Peak 1: (R)-5,6-dichloro-N-methyl-2,3-dihydrobenzofuran-3-amine | Chiralpak IG column (250 x 4.6 mm) with a mobile phase of 80% Liquid CO₂ and 20% MeOH with 0.2% isopropylamine, using a CO₂ flow rate of 2.4 mL/min and a cosolvent flow rate of 0.6 mL/min | No ion |
| **47** | 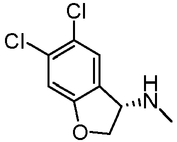 | Peak 2: (S)-5,6-dichloro-N-methyl-2,3-dihydrobenzofuran-3-amine | | |

### Intermediate 48: N,5-dimethyl-2-(trifluoromethyl)-6,7-dihydro-5H-cyclopenta[b]pyridin-5-amine

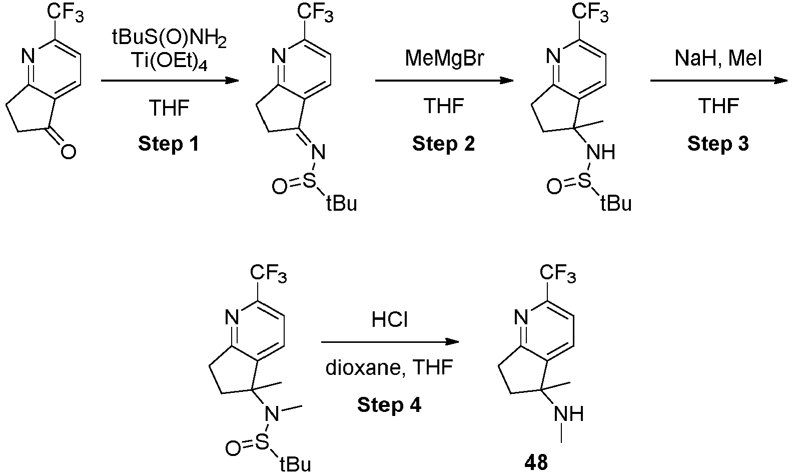

Step 1. To a stirred solution of 2-(trifluoromethyl)-6,7-dihydro-5H-cyclopenta[b]pyridin-5-one (1.000 g, 4.97 mmol, Angel Pharmatech) in THF (15 mL) in a 20-mL microwave reaction vessel was added ethyl titanate (2.495 g, 2.495 mL, 10.94 mmol, Sigma-Aldrich Corporation) via syringe followed by 2-methylpropane-2-sulfinamide (0.603 g, 4.97 mmol, Sigma-Aldrich Corporation) in one portion as a solid. The vessel was sealed and subjected to microwave irradiation (4 h, 70°C). After being cooled to rt, the crude reaction mixture was poured into brine (20 mL). The mixture was vigorously stirred for 15 min before it was vacuum filtered through a layer of celite. The filter cake was washed with EtOAc and the organic layer of the filtrate was dried over anhydrous sodium sulfate and concentrated in vacuo. The residue was dissolved in DCM, loaded onto a silica gel precolumn (25 g), and subjected to combi-flash column chromatography on a 24-g ISCO gold column eluting with EtOAc/heptane (0 to 100%) to give (E)-2-methyl-N-(2-(trifluoromethyl)-6,7-dihydro-5H-cyclopenta[b]pyridin-5-ylidene)propane-2-sulfinamide (710 mg, 2.333 mmol, 46.9 % yield) as a dark film. *m*/*z* (ESI): 327.0 (M+Na)⁺. ¹H NMR (CHLOROFORM-d, 400 MHz) δ 8.19 (d, 1H, J=7.9 Hz), 7.66 (d, 1H, J=8.2 Hz), 3.5-3.7 (m, 1H), 3.35 (dd, 2H, *J*=5.0, 7.3 Hz), 3.2-3.3 (m, 1H), 1.35 (s, 9H).

Step 2. To a stirred ice-cooled solution of (E)-2-methyl-N-(2-(trifluoromethyl)-6,7-dihydro-5H-cyclopenta[b]pyridin-5-ylidene)propane-2-sulfinamide (710 mg, 2.333 mmol) in DCM (15 mL) in a 250-mL single-necked round-bottomed flask was added, under nitrogen atmosphere, methylmagnesium bromide, 3.0 M in diethyl ether (4.67 mL, 14.00 mmol, Sigma-Aldrich Corporation) via syringe. The resulting mixture was stirred at 0 °C for 2 h and allowed to warm up to rt and stirred at rt overnight. The reaction was cooled in an ice-water bath, carefully quenched with ice-cold saturated ammonium chloride, and extracted with DCM (3 x). The combined organics were dried over anhydrous sodium sulfate and concentrated in vacuo. The residue was dissolved in DCM, loaded onto a silica gel precolumn (25 g), and subjected to combi-flash column chromatography on a 40-g ISCO gold column eluting with MeOH/DCM (0 to 5%) to give a single diastereomer, 2-methyl-N-(5-methyl-2-(trifluoromethyl)-6,7-dihydro-5H-cyclopenta[b]pyridin-5-yl)propane-2-sulfinamide (100 mg, 0.312 mmol, 13.38% yield) as a dark film. *m*/*z* (ESI): 321.0 (M+H)⁺. ¹H NMR (CHLOROFORM-d, 400 MHz) δ 7.90 (d, 1H, J=7.9 Hz), 7.54 (d, 1H, J=7.7 Hz), 3.50 (s, 1H), 3.2-3.3 (m, 1H), 3.0-3.1 (m, 1H), 2.52 (ddd, 1H, J=7.0, 8.8, 13.5 Hz), 2.30 (ddd, 1H, J=5.1, 8.4, 13.4 Hz), 1.65 (s, 3H), 1.2-1.3 (m, 8H). ¹⁹F NMR (CHLOROFORM-d, 376 MHz) δ -67.45 (s, 3F).

Step 3. To a stirred solution of 2-methyl-N-(5-methyl-2-(trifluoromethyl)-6,7-dihydro-5H-cyclopenta[b]pyridin-5-yl)propane-2-sulfinamide (100 mg, 0.312 mmol) in THF (5 mL) under nitrogen was added sodium hydride, 60% in mineral oil (14.98 mg, 0.375 mmol, Sigma-Aldrich Corporation). The resulting mixture was stirred at 0 °C for 20 min before iodomethane (89 mg, 0.039 mL, 0.624 mmol, Sigma-Aldrich Corporation) was added slowly dropwise via a syringe. The resulting mixture was stirred at 0 °C for 2 h and at ambient temperature for 3.5 h. The reaction was cooled in an ice-water bath before it was quenched with MeOH (5 mL). The volatiles were removed in vacuo and the residue was dissolved in DCM/MeOH and loaded onto a silica gel precolumn (25 g) and subjected to combi-flash column chromatography on a 24-g ISCO gold column eluting with MeOH/DCM (0 to 4%) to give N,2-dimethyl-N-(5-methyl-2-(trifluoromethyl)-6,7-dihydro-5H-cyclopenta[b]pyridin-5-yl)propane-2-sulfinamide (64 mg, 0.191 mmol, 61.3 % yield) as a nearly colorless film. *m*/*z* (ESI): 335.0 (M+H)⁺. ¹H NMR (CHLOROFORM-d, 400 MHz) δ 7.82 (d, 1H, *J*=7.9 Hz), 7.53 (d, 1H, *J*=7.9 Hz), 3.2-3.3 (m, 1H), 3.0-3.1 (m, 1H), 2.62 (ddd, 1H, *J*=6.7, 9.1, 13.7 Hz), 2.46 (s, 3H), 2.10 (ddd, 1H, *J*=5.3, 8.7, 13.7 Hz), 1.65 (s, 3H), 1.22 (s, 9H). ¹⁹F NMR (CHLOROFORM-d, 376 MHz) δ -67.44 (s, 3F).

Step 4. To a stirred solution of N,2-dimethyl-N-(5-methyl-2-(trifluoromethyl)-6,7-dihydro-5H-cyclopenta[b]pyridin-5-yl)propane-2-sulfinamide (64 mg, 0.191 mmol) in MeOH (1.0 mL) was added at rt hydrogen chloride in dioxane, 4 M solution (3.0 mL, 12.00 mmol, Sigma-Aldrich Corporation). The resulting mixture was stirred at rt for 0.5 h. The volatiles were removed to give crude N,5-dimethyl-2-(trifluoromethyl)-6,7-dihydro-5H-cyclopenta[b]pyridin-5-amine **(48),** which was directly taken onto the next step. *m*/*z* (ESI): 231.20 (M+H)⁺.

### Intermediate 49: 6,6-dimethyl-2-(trifluoromethyl)-6,7-dihydro-5H-cyclopenta[b]pyridin-5-ol

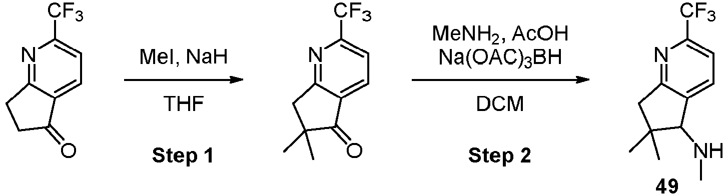

Step 1. To a stirred ice-cooled solution of 2-(trifluoromethyl)-6,7-dihydro-5H-cyclopenta[b]pyridin-5-one (600 mg, 2.98 mmol, Angel Pharma) in THF (8 mL) under nitrogen was added sodium hydride, 60% in mineral oil (298 mg, 7.46 mmol, Sigma-Aldrich Corporation) in one portion. The resulting mixture was stirred at 0 °C for 25 min before iodomethane (889 mg, 0.390 mL, 6.26 mmol, Sigma-Aldrich Corporation) was added via a syringe. The resulting mixture was stirred 0 °C for 1.5 h and at rt for 2 h. The mixture was poured onto ice and saturated aqueous ammonium chloride solution and extracted with DCM (3x). The combined organics were dried over anhydrous sodium sulfate and concentrated in vacuo. The crude residue was dissolved in DCM, loaded onto a silica gel precolumn (25 g), and subjected to combi-flash column chromatography on a 24-g ISCO gold column eluting with MeOH/DCM (0 to 6%) to give 6,6-dimethyl-2-(trifluoromethyl)-6,7-dihydro-5H-cyclopenta[b]pyridin-5-one (65 mg, 0.284 mmol, 9.51 % yield) as a nearly colorless film/solid. *m*/*z* (ESI): 230.20 (M+H)⁺. ¹H NMR (CHLOROFORM-d, 400 MHz) δ 8.21 (d, 1H, J=7.9 Hz), 7.73 (d, 1H, J=7.9 Hz), 3.22 (s, 2H), 1.33 (s, 6H). ¹⁹F NMR (CHLOROFORM-d, 376 MHz) δ -68.00 (s, 3F).

Step 2. To a stirred mixture of 6,6-dimethyl-2-(trifluoromethyl)-6,7-dihydro-5H-cyclopenta[b]pyridin-5-one (65 mg, 0.284 mmol) and aminomethane, 2.0 M solution in THF (0.922 mL, 1.843 mmol, Sigma-Aldrich Corporation) in DCM (7 mL) was added acetic acid (102 mg, 0.098 mL, 1.702 mmol, Sigma-Aldrich Corporation). The resulting mixture was stirred at rt for 25 min before sodium triacetoxyborohydride (78 mg, 0.369 mmol, Sigma-Aldrich Corporation) was added in one portion as a solid. The resulting mixture was stirred at rt overnight. MeOH (0.5 mL) was added to the mixture and stirring continued for 2 h before it was directly loaded onto a silica gel precolumn (25 g) and subjected to combi-flash column chromatography eluting on a 24-g ISCO gold column eluting with MeOH/DCM (0 to 4%) and (MeOH with 0.5% ammonium hydroxide)/DCM (2 to 20%) to give impure N,6,6-trimethyl-2-(trifluoromethyl)-6,7-dihydro-5H-cyclopenta[b]pyridin-5-amine **(49)** (10 mg, 0.041 mmol, 14.44 % yield) as a colorless film. *m*/*z* (ESI): 245.20 (M+H)⁺. ¹H NMR (CHLOROFORM-d, 400 MHz) δ 7.75 (d, 1H, *J*=7.7 Hz), 7.49 (d, 1H, *J*=7.7 Hz), 5.50 (br d, 1H, *J*=2.3 Hz), 3.73 (s, 1H), 2.64 (s, 3H), 2.08 (s, 2H), 1.28 (s, 3H), 1.06 (s, 3H). ¹⁹F NMR (CHLOROFORM-d, 376 MHz) δ -67.35 (s, 3F).

### Intermediate 50: 6,6-difluoro-N-methyl-2-(trifluoromethyl)-6,7-dihydro-5H-cyclopenta[b]pyridin-5-amine

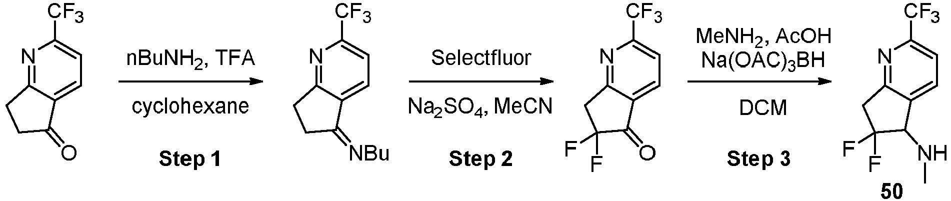

Step 1. To a stirred mixture of 2-trifluoromethyl-6,7-dihydro-[1]pyrindin-5-one (1.000 g, 4.97 mmol, Synnovator) and butan-1-amine (0.436 g, 0.590 mL, 5.97 mmol, Sigma-Aldrich Corporation) in cyclohexane (50 mL) in a 100-mL single-necked RBF was added a few drops of 2,2,2-trifluoroacetic acid (0.028 g, 0.249 mmol, Sigma-Aldrich Corporation) via a syringe under nitrogen. The flask was then equipped with a Dean-Stark condenser and the mixture was refluxed overnight. After cooled to rt, the crude mixture was poured into ice and a sat. aq solution of NaHCO₃ and extracted with EtOAc (3 x). The combined organics were washed with brine and dried over anhydrous sodium sulfate. Removal of the volatiles in vacuo gave (Z)-5-(butyl-14-azaneylidene)-2-(trifluoromethyl)-6,7-dihydro-5H-cyclopenta[b]pyridine (1.00 g, 3.89 mmol, 78 % yield) as a colorless film, which was used in the next step without purification. *m*/*z* (ESI): 257.20 (M+H)⁺. ¹H NMR (CHLOROFORM-d, 400 MHz) δ 8.21 (d, 1H, *J*=7.9 Hz), 7.61 (d, 1H, *J*=7.9 Hz), 3.51 (t, 2H, *J*=7.1 Hz), 3.2-3.3 (m, 2H), 2.8-2.9 (m, 2H), 1.7-1.8 (m, 2H), 1.4-1.6 (m, 3H), 0.99 (t, 3H, J=7.4 Hz). ¹⁹F NMR (CHLOROFORM-d, 376 MHz) δ -67.57 (s, 3F).

Step 2. To a stirred mixture of (Z)-5-(butyl-14-azaneylidene)-2-(trifluoromethyl)-6,7-dihydro-5H-cyclopenta[b]pyridine (1.000 g, 3.50 mmol) and sodium sulfate (0.497 g, 3.50 mmol, Sigma-Aldrich Corporation) in MeCN (30 mL) in a 250-mL single-necked RBF under nitrogen was added 1-(chloromethyl)-4-fluoro-1,4-diazabicyclo[2.22]octane-1,4-diium tetrafluoroborate (2.478 g, 7.00 mmol, Sigma-Aldrich Corporation) in one portion as a solid. The resulting mixture was heated to reflux overnight. After it was cooled to rt, aqueous HCl solution (37%) (3.0 mL) was added and the mixture was stirred at rt for 35 min. The volatiles were removed in vacuo. The residue was mixed with DCM and washed with ice-cold saturated sodium bicarbonate aqueous solution. The aqueous layer was extracted with DCM (2 x). The combined organics were dried over anhydrous sodium sulfate and concentrated in vacuo. The crude residue was directly loaded onto a silica gel precolumn (25 g) and subjected to combi-flash column chromatography on a 24-g ISCO gold column eluting with MeOH/DCM (15 min from 0 to 4%) to give impure 6,6-difluoro-2-(trifluoromethyl)-6,7-dihydro-5H-cyclopenta[b]pyridin-5-one (410 mg, 1.729 mmol, 49.4 % yield) as an brownish solid. ¹H NMR (CHLOROFORM-d, 400 MHz) δ 8.34 (d, 1H, J=8.2 Hz), 7.96 (d, 1H, J=7.9 Hz), 7.86 (d, 1H, J=7.9 Hz), 7.67 (d, 1H, J=7.9 Hz), 3.80 (t, 2H, J=12.4 Hz). ¹⁹F NMR (CHLOROFORM-d, 376 MHz) δ -68.42 (s, 3F), -110.96 (s, 2F).

Step 3. To a stirred mixture of 6,6-difluoro-2-(trifluoromethyl)-6,7-dihydro-5H-cyclopenta[b]pyridin-5-one (260 mg, 1.096 mmol) and aminomethane, 2.0 M solution in THF (2.193 mL, 4.39 mmol, Sigma-Aldrich Corporation) in DCM (4 mL) was added acetic acid (263 mg, 0.253 mL, 4.39 mmol, Sigma-Aldrich Corporation). The resulting mixture was stirred at rt for 30 min before sodium triacetoxyborohydride (302 mg, 1.425 mmol, Sigma-Aldrich Corporation) was added in one portion as a solid. The resulting mixture was stirred at rt for 2 days. MeOH (0.5 mL) was added to the mixture and stirring continued for 20 min before it was directly loaded onto a silica gel precolumn (25 g) and subjected to combi-flash column chromatography eluting on a 24-g ISCO gold column eluting with (MeOH with 0.5% ammonium hydroxide)/DCM (20 min from 0 to 6%) (2 x) to give impure 6,6-difluoro-N-methyl-2-(trifluoromethyl)-6,7-dihydro-5H-cyclopenta[b]pyridin-5-amine (50) (104 mg, 0.412 mmol, 37.6 % yield) as a colorless film. *m*/*z* (ESI): 253.00 (M+H)⁺. ¹H NMR (CHLOROFORM-d, 400 MHz) δ 7.88 (d, 1H, J=7.7 Hz), 7.62 (d, 1H, J=7.9 Hz), 4.3-4.4 (m, 1H), 3.5-3.7 (m, 2H), 2.71 (d, 3H, *J*=1.0 Hz), 1.67 (br s, 1H). ¹⁹F NMR (CHLOROFORM-d, 376 MHz) δ -67.70 (s, 3F), -99.8--97.2 (m, 1F), -112.08 (d, 1F, J=233.2 Hz).

### Intermediate 51 and Intermediate 52: methyl((S)-3-(methylamino)-2,3-dihydrobenzofuran-6-yl)(methylimino)-λ6-sulfanone and imino(methyl)((S)-3-(methylamino)-2,3-dihydrobenzofuran-6-yl)-λ6-sulfanone

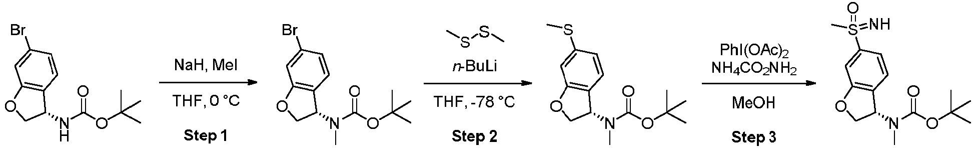

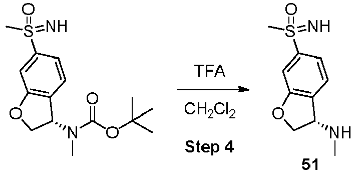

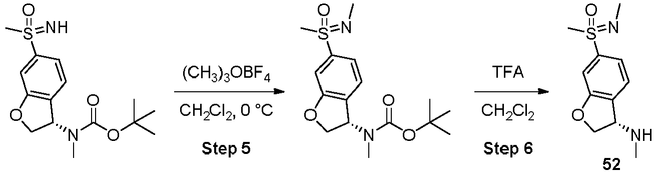

Step 1. An oven-dried round-bottom flask was charged with tert-butyl (S)-(6-bromo-2,3-dihydrobenzofuran-3-yl)carbamate (500 mg, 1.591 mmol) and tetrahydrofuran (15.9 mL). The resulting solution was cooled to 0 °C and sodium hydride (60% dispersion in mineral oil, 115 mg, 2.86 mmol) was added as a solid in one portion. The resulting mixture was allowed to stir at 0°C for 15 min, after which iodomethane (407 mg, 178 µL, 2.86 mmol) was added, and the resulting mixture was allowed to warm to 23 °C. After 1 h, the reaction mixture was cooled to 0°C and quenched by slow addition of MeOH (5 mL). The solution was then warmed to room temperature and concentrated in vacuo, and the resulting crude residue was purified by flash chromatography (0 to 50% 3:1 EtOAc:EtOH in heptane) to afford tert-butyl (S)-(6-bromo-2,3-dihydrobenzofuran-3-yl)(methyl)carbamate (439.6 mg, 1.339 mmol, 84% yield) as a clear oil. *m*/*z* (ESI): 352.0 (M+Na)⁺. ¹H NMR (400 MHz, CHLOROFORM-d) δ ppm 6.99 - 7.16 (m, 3 H), 6.03 (br s, 1 H), 4.65 (br t, J=9.7 Hz, 1 H), 4.32 - 4.47 (m, 1 H), 2.56 (br s, 3 H), 1.45 - 1.53 (m, 9 H).

Step 2. A round-bottom flask was charged with tert-butyl (S)-(6-bromo-2,3-dihydrobenzofuran-3-yl)(methyl)carbamate (439.6 mg, 1.339 mmol) and tetrahydrofuran (13.4 mL). The resulting solution was flushed with nitrogen, cooled to -78 °C, and n-butyllithium (729 µL, 1.473 mmol, 2.02 M in hexanes) was added dropwise. The resulting mixture was allowed to stir at -78°C for 30 min under a nitrogen atmosphere, after which dimethyl disulfide (252 mg, 241 µL, 2.68 mmol) was added dropwise. The reaction mixture was allowed to stir at -78°C. After 1 h, the reaction mixture was allowed to warm to 0 °C and was quenched by slow addition of H₂O (20 mL). The mixture was then transferred to a separatory funnel with EtOAc (20 mL) and H₂O, and the aqueous layer was extracted with EtOAc (2 x 20 mL). The combined organics were dried over Na₂SO₄, filtered, and concentrated in vacuo. The resulting crude residue was purified by flash chromatography (0 to 100% EtOAc in heptane) to afford tert-butyl (S)-methyl(6-(methylthio)-2,3-dihydrobenzofuran-3-yl)carbamate (237.9 mg, 0.805 mmol, 60.1% yield) as a clear oil. *m*/*z* (ESI): 318.2 (M+Na)⁺. ¹H NMR (400 MHz, CHLOROFORM-d) δ ppm 7.09 - 7.21 (m, 1 H), 6.80 - 6.88 (m, 1 H), 6.76 (s, 1 H), 5.67 - 6.22 (m, 1 H), 4.56 - 4.69 (m, 1 H), 4.39 (br dd, J=9.7, 3.1 Hz, 1 H), 2.56 (br s, 3 H), 2.45 - 2.52 (m, 3 H), 1.52 (s, 9 H).

Step 3. A vial was charged with tert-butyl (S)-methyl(6-(methylthio)-2,3-dihydrobenzofuran-3-yl)carbamate (223.5 mg, 0.757 mmol) and methanol (1.51 mL). To the resulting solution were added ammonium carbamate (118 mg, 1.513 mmol) and iodobenzene diacetate (609 mg, 1.892 mmol). The resulting mixture was allowed to stir at 23 °C. After 30 min, the reaction mixture was concentrated in vacuo and the resulting crude residue was purified by flash chromatography (0 to 100% 3:1 EtOAc:EtOH in heptane) to afford tert-butyl methyl((3S)-6-(S-methylsulfonimidoyl)-2,3-dihydrobenzofuran-3-yl)carbamate (177.8 mg, 0.545 mmol, 72.0% yield) as a yellow oil. *m*/*z* (ESI): 327.1 (M+H)⁺.

Step 4. A vial was charged with tert-butyl methyl((3S)-6-(S-methylsulfonimidoyl)-2,3-dihydrobenzofuran-3-yl)carbamate (178 mg, 0.545 mmol) and dichloromethane (5.45 mL). To the resulting solution was added 2,2,2-trifluoroacetic acid (1.55 g, 1.04 mL, 13.6 mmol) and the reaction mixture was allowed to stir at 23 °C. After 30 min, the reaction mixture was concentrated to dryness and the resulting crude imino(methyl)((S)-3-(methylamino)-2,3-dihydrobenzofuran-6-yl)-λ⁶-sulfanone (51) was used in the subsequent step without further purification. *m*/*z* (ESI): 227.2 (M+H)⁺.

Step 5. A round-bottom flask was charged with tert-butyl methyl((3S)-6-(S-methylsulfonimidoyl)-2,3-dihydrobenzofuran-3-yl)carbamate (271.7 mg, 0.832 mmol) and dichloromethane (16.6 mL). The resulting solution was cooled to 0°C, trimethyloxonium tetrafluoroborate (197 mg, 1.332 mmol) was added as a solid in one portion, and the reaction vessel was flushed with nitrogen. The resulting mixture was then allowed to warm to 23°C. After 1 h, the reaction mixture was cooled to 0 °C and quenched by slow addition of H₂O (10 mL). The resulting biphasic mixture was then transferred to a separatory funnel with DCM (20 mL), sat. aq. Na₂CO₃ (20 mL), and brine (20 mL), and the aqueous layer was extracted with DCM (2 x 20 mL). The combined organic layers were dried with Na₂SO₄, filtered, and concentrated to dryness. The resulting crude residue was purified by flash chromatography (0 to 100% 3:1 EtOAc:EtOH in heptane) to afford tert-butyl ((3S)-6-(N,S-dimethylsulfonimidoyl)-2,3-dihydrobenzofuran-3-yl)(methyl)carbamate (212 mg, 0.622 mmol, 74.7 % yield) as a clear oil. *m*/*z* (ESI): 341.2 (M+H)⁺.

Step 6. A vial was charged with tert-butyl ((3S)-6-(N,S-dimethylsulfonimidoyl)-2,3-dihydrobenzofuran-3-yl)(methyl)carbamate (212 mg, 0.622 mmol) and dichloromethane (6.22 µL). To the resulting solution was added 2,2,2-trifluoroacetic acid (1.77 g, 1.19 mL, 15.6 mmol) and the reaction mixture was allowed to stir at 23 °C. After 2 h, the reaction mixture was concentrated to dryness and the resulting crude methyl((S)-3-(methylamino)-2,3-dihydrobenzofuran-6-yl)(methylimino)-16-sulfanone (52) was used in the subsequent step without further purification. *m*/*z* (ESI): 241.2 (M+H)⁺.

### Intermediate 53: (S)-N-methyl-6-(1-(trifluoromethyl)-1H-pyrazol-4-yl)-2,3-dihydrobenzofuran-3-amine

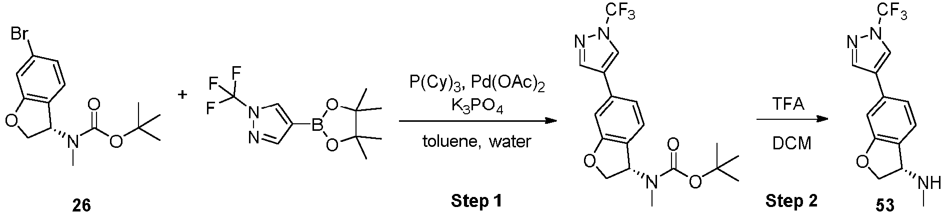

Step 1. A mixture of tert-butyl (S)-(6-bromo-2,3-dihydrobenzofuran-3-yl)(methyl)carbamate (0.3434 g, 1.046 mmol), 4-(4,4,5,5-tetramethyl-1,3,2-dioxaborolan-2-yl)-1-(trifluoromethyl)-1H-pyrazole (0.411 g, 1.569 mmol, Enamine) and toluene (10 mL) was purged with Ar, then potassium phosphate tribasic monohydrate (0.723 g, 3.14 mmol, Sigma-Aldrich Corporation) and water (1.111 mL) were added. The mixture was stirred for 10 min at rt, then tricyclohexylphosphine (0.059 g, 0.209 mmol, Strem Chemicals) and palladium (II) acetate (0.023 g, 0.105 mmol, Sigma-Aldrich Corporation) were added. The mixture was stirred in a sealed vial at 90°C overnight. The crude product was diluted with ethyl acetate, filtered through celite, and concentrated in vacuo. The residue was purified by silica gel flash column chromatography using 0-60% EtOAc in heptane. tert-butyl (S)-methyl(6-(1-(trifluoromethyl)-1H-pyrazol-4-yl)-2,3-dihydrobenzofuran-3-yl)carbamate (0.300 g, 0.783 mmol, 74.8% yield) was obtained as off-white solid. *m*/*z* (ESI): 384.1 (M+H)⁺.

Step 2. To a mixture of tert-butyl (S)-methyl(6-(1-(trifluoromethyl)-1H-pyrazol-4-yl)-2,3-dihydrobenzofuran-3-yl)carbamate (0.0375 g, 0.098 mmol) and DCM (1 mL) was added TFA (0.044 g, 0.030 mL, 0.392 mmol, Sigma-Aldrich Corporation). The mixture was stirred at rt overnight, then concentrated in vacuo. The crude was dissoved in 4M HCl in dioxane to convert the TFA salt to HCl salt and was concentrated to dryness. (S)-N-methyl-6-(1-(trifluoromethyl)-1H-pyrazol-4-yl)-2,3-dihydrobenzofuran-3-amine (53) (0.028 g, 0.099 mmol, 101% yield) was obtained as white solid. *m*/*z* (ESI): 306.1 (M+Na)⁺.

### Intermediate 54: (S)-N-methyl-6-(1-methyl-1H-pyrazol-4-yl)-2,3-dihydrobenzofuran-3-amine

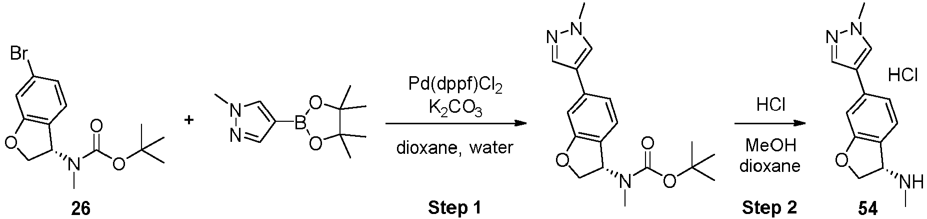

Step 1. A mixture of tert-butyl (S)-(6-bromo-2,3-dihydrobenzofuran-3-yl)(methyl)carbamate (0.3287 g, 1.002 mmol), 1-methyl-4-(4,4,5,5-tetramethyl-1,3,2-dioxaborolan-2-yl)-1H-pyrazole (0.417 g, 2.003 mmol, Apollo), potassium carbonate (0.415 g, 3.00 mmol, Sigma-Aldrich Corporation), dichloro[1,1'-bis(diphenylphosphino)ferrocene]palladium (II) dichloromethane adduct (0.095 g, 0.130 mmol, Strem Chemicals), 1,4-dioxane (5 mL), and water (0.556 mL) was purged with Ar, then was stirred in a sealed vial at 85 °C overnight. The crude product was diluted with ethyl acetate, filtered through celite and concentrated in vacuo. The crude product was purified by silica gel flash column chromatography using 0-60% EtOAc in heptane. tert-butyl (S)-methyl(6-(1-methyl-1H-pyrazol-4-yl)-2,3-dihydrobenzofuran-3-yl)carbamate (0.289 g, 0.877 mmol, 88% yield) was obtained as white solid. *m*/*z* (ESI): 330, 352 (M+H)⁺. ¹H NMR (400 MHz, CHLOROFORM-d) δ ppm 7.73 (s, 1 H), 7.58 (s, 1 H), 7.21 (br d, J=7.7 Hz, 1 H), 7.04 (dd, J=7.7, 1.5 Hz, 1 H), 6.93 (d, *J=1.5* Hz, 1 H), 5.70 - 6.11 (m, 1 H), 4.62 (br t, J=9.4 Hz, 1 H), 4.38 (br dd, *J*=10.2, 3.8 Hz, 1 H), 3.94 (s, 3 H), 2.57 (br s, 3 H), 1.54 (s, 6 H), 1.24 (s, 3 H).

Step 2. To a mixture of tert-butyl (S)-methyl(6-(1-methyl-1H-pyrazol-4-yl)-2,3-dihydrobenzofuran-3-yl)carbamate (0.2876 g, 0.873 mmol) in 1,4-dioxane (8 mL) was added hydrogen chloride, 4M in dioxane (1.091 mL, 4.37 mmol, Sigma-Aldrich Corporation). MeOH (0.699 g, 0.883 mL, 21.83 mmol, Sigma-Aldrich Corporation) was added to dissolve the salt. Then, 0.3 mL HCl was added and the reaction was continued overnight before it was concentrated in vacuo. (S)-N-methyl-6-(1-methyl-1H-pyrazol-4-yl)-2,3-dihydrobenzofuran-3-amine hydrochloride (54) (0.227 g, 0.854 mmol, 98% yield) was obtained as off-white solid. *m*/*z* (ESI): 230, 252 (M+Na)⁺.

### Intermediates 56 and 57: (R)-N-methyl-7-(trifluoromethyl)isochroman-4-amine and (S)-N-methyl-7-(trifluoromethyl)isochroman-4-amine

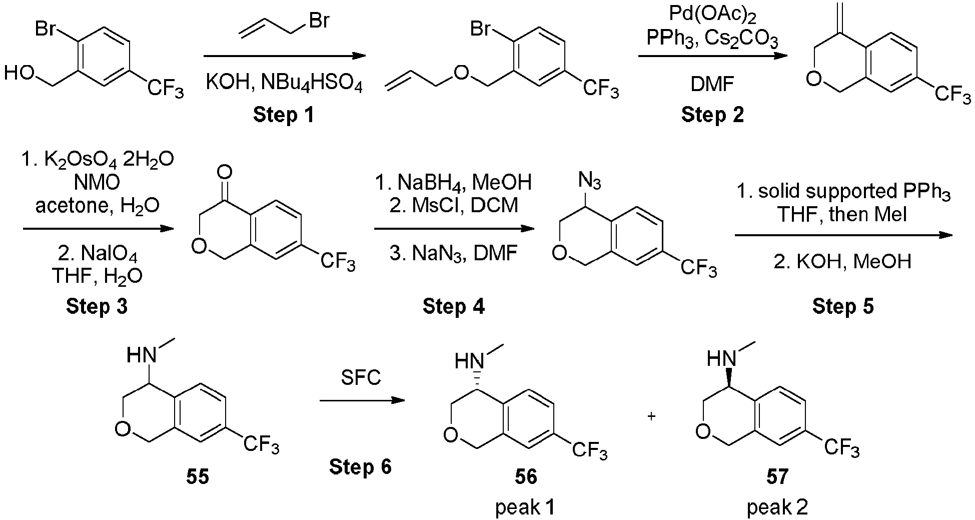

Step 1. To a solution of (2-bromo-5-(trifluoromethyl)phenyl)methanol (3.8552 g, 15.12 mmol, AA Blocks) in allyl bromide (1.829 g, 1.316 mL, 15.12 mmol, Sigma-Aldrich Corporation) was added potassium hydroxide (1.611 g, 28.7 mmol, Sigma-Aldrich Corporation), and tetrabutylammonium hydrogen sulfate (0.770 g, 2.267 mmol, Sigma-Aldrich Corporation). The mixture was stirred at room temperature overnight. Water (20 mL) was then added, and the aqueous layer was extracted with ethyl acetate (3-5 times). The combined organic layers were washed once with water, and once with brine before being dried over magnesium sulfate, filtered, and concentrated in-vacuo to afford the crude product. The crude product was isolated as a yellow oil and purified via column chromatography (0-17% EA/Heptanes) to yield ((allyloxy)methyl)-1-bromo-4-(trifluoromethyl)benzene (4.3595 g, 14.77 mmol, 98 % yield) as a clear oil. ¹H NMR (400 MHz, CHLOROFORM-d) δ ppm 7.80 (d, *J=1.5* Hz, 1 H), 7.66 (d, J=8.4 Hz, 1 H), 7.37 - 7.47 (m, 1 H), 6.00 (ddt, *J*=17.2, 10.4, 5.6, 5.6 Hz, 1 H), 5.23 - 5.44 (m, 2 H), 4.60 (s, 2 H), 4.16 (dt, J=5.6, 1.4 Hz, 2 H).

Step 2. To a solution of 2-((allyloxy)methyl)-1-bromo-4-(trifluoromethyl)benzene (4.3595 g, 14.77 mmol) in N, N-dimethylformamide (87 mL) was added cesium carbonate (5.78 g, 17.73 mmol, Sigma-Aldrich Corporation), triphenylphosphine (1.744 g, 6.65 mmol, Sigma-Aldrich Corporation) and palladium (ii) acetate (0.497 g, 2.216 mmol, Strem Chemicals). The reaction mixture was heated to 90 °C overnight, then it was filtered and concentrated to remove the majority of the DMF. Water and EtOAc were added and the resulting biphasic mixture was transferred to a separatory funnel. The layers were separated, and the aqueous phase was extracted with EtOAc. The organic phase was washed with brine. The combined organics were dried over anhydrous MgSO₄, filtered, and concentrated under reduced pressure. The resulting oil was purified by flash column chromatography (0-25% EtOAc/heptanes) to afford 4-methylene-7-(trifluoromethyl)isochromane (2.4102 g, 11.25 mmol, 76 % yield) as a yellow oil. ¹H NMR (400 MHz, CHLOROFORM-d) δ ppm 7.79 (d, J=8.4 Hz, 1 H), 7.45 - 7.52 (m, 1 H), 7.31 (s, 1 H), 5.73 (s, 1 H), 5.16 (s, 1 H), 4.85 (s, 2 H), 4.48 (t, *J*=1.3 Hz, 2 H).

Step 3. To a 100-mL round-bottomed flask was added 4-methylene-7-(trifluoromethyl)isochromane (2.4102 g, 11.25 mmol) in acetone (26.8 mL) and water (5.36 mL). Then, potassium osmate (vi) dihydrate (0.415 g, 1.125 mmol, Sigma-Aldrich Corporation) followed by 4-methylmorpholine 4-oxide (4.61 g, 39.4 mmol, Sigma-Aldrich Corporation) was added to the reaction mixture. The overall reaction mixture was allowed to stir under an inert (N₂) atmosphere, while at rt overnight. The reaction mixture was quenched with the addition of solid sodium sulfite and the mixture was stirred 10 min. The reaction mixture was partially concentrated in vacuo then diluted with EtOAc and brine. The layers were separated, and the aqueous layer was extracted with EtOAc. The organics were combined, dried over MgSO₄, filtered and concentrated in vacuo. The crude residue was purified by column chromatography, eluting with a gradient of 0-100% EA in Heptanes to obtain 4-(hydroxymethyl)-7-(trifluoromethyl)isochroman-4-ol (2.4765 g, 9.98 mmol, 89% yield). ¹H NMR (400 MHz, CHLOROFORM-d) δ ppm 7.74 (d, J=8.1 Hz, 1 H), 7.56 (br d, J=7.5 Hz, 1 H), 7.29 (s, 1 H), 4.85 (s, 2 H), 4.18 (d, *J*=11.4 Hz, 1 H), 3.92 (dd, *J*=11.1, 6.7 Hz, 1 H), 3.72 (ddd, *J*=11.2, 5.0, 1.0 Hz, 1 H), 3.64 (dd, *J*=11.3, 1.1 Hz, 1 H), 2.76 (s, 1 H), 2.02 - 2.10 (m, 1 H).

The diol was diluted with THF (36 mL) then sodium (meta)periodate (7.22 g, 33.8 mmol, Sigma-Aldrich Corporation), followed by water (1.2 mL) was added into the mixture. The resulting reaction mixture was allowed to stir under an inert (N₂) atmosphere. After stirring overnight, the mixture was diluted with a mixture of EtOAc/Heptane (1: 1). The mixture was filtered through a pad of Celite and the filtrate was collected and concentrated. The filtrate was treated with sat. aq. NaHCO₃ . The layers were separated, and the aqueous layer was extracted with EtOAc. The combined organic extracts were washed with brine solution, then dried over MgSO₄, filtered through a pad of Celite and concentrated in vacuo. The crude product was purified by column chromatography, eluting with a gradient of 0-20% EA/Heptanes to give 7-(trifluoromethyl)isochroman-4-one (1.7116 g, 7.92 mmol, 70.4% yield) as a white solid. *m*/*z* (ESI): 216.8 (M+H)⁺, ¹H NMR (400 MHz, CHLOROFORM-d) δ ppm 8.19 (d, J=8.1 Hz, 1 H), 7.71 (d, J=8.1 Hz, 1 H), 7.54 (s, 1 H), 4.97 (s, 2 H), 4.44 (s, 2 H).

Step 4. To a stirred solution of 7-(trifluoromethyl)isochroman-4-one (1.300 g, 6.01 mmol) in methanol (20.05 mL) was added sodium borohydride (0.296 g, 7.82 mmol, Sigma-Aldrich Corporation) by portion at 0°C. After being stirred for 15 min at that temperature, the reaction mixture was brought to room temperature and stirred. After 20 minutes, MeOH was evaporated from the reaction mixture by rotary evaporator. The reaction mixture was extracted with DCM and brine solution three times. The organics were combined, dried over MgSO₄, filtered, and the solvent was evaporated. The crude alcohol, 7-(trifluoromethyl)isochroman-4-ol (1.312 g, 6.01 mmol, 100% yield) was used for the next step without further purification. ¹H NMR (400 MHz, CHLOROFORM-d) δ ppm 7.53 - 7.64 (m, 2 H), 7.31 (s, 1 H), 4.69 - 4.93 (m, 2 H), 4.62 (dt, J=9.7, 2.8 Hz, 1 H), 4.11 (dd, *J*=11.9, 3.1 Hz, 1 H), 3.93 (dd, *J*=11.9, 2.9 Hz, 1 H), 2.28 - 2.39 (m, 1 H).

The crude alcohol was dissolved in DCM (20 mL) and stirred at 0 °C. Mesyl chloride (0.827 g, 0.559 mL, 7.22 mmol, Sigma-Aldrich Corporation) was added dropwise via syringe under a N₂ atmosphere at the same temperature followed by the dropwise addition of triethylamine (0.791 g, 1.099 mL, 7.82 mmol, Sigma-Aldrich Corporation). The reaction mixture was stirred for 30 min at the same temperature and then stirred at room temperature for another 30 min until the alcohol was consumed. The reaction mixture was extracted with dichloromethane and water. The organics were combined and dried over MgSO₄. The solvent was evaporated with a rotary evaporator, and the crude 7-(trifluoromethyl)isochroman-4-yl methanesulfonate (1.782 g, 6.01 mmol, 100% yield) was isolated as a white solid and used for the next step without further purification. ¹H NMR (400 MHz, CHLOROFORM-d) δ ppm 7.55 - 7.76 (m, 2 H), 7.37 (s, 1 H), 5.73 (t, J=2.7 Hz, 1 H), 4.88 - 5.01 (m, 1 H), 4.68 - 4.82 (m, 1 H), 4.39 (dd, *J=13.2,* 2.9 Hz, 1 H), 4.04 (dd, *J=13.1,* 2.8 Hz, 1 H), 3.12 (s, 3 H).

To a solution of the crude 7-(trifluoromethyl)isochroman-4-yl methanesulfonate (1.782 g, 6.01 mmol) in N, N-dimethylformamide (20.05 mL) was added sodium azide (0.782 g, 0.425 mL, 12.03 mmol, Sigma-Aldrich Corporation), and the mixture was stirred at rt for 2 h. After the reaction had reached completion, it was partially concentrated to remove the DMF, and extracted with EtOAc and brine. The organics were combined and dried over MgSO₄, and the solvent was evaporated using a rotary evaporator. The crude was purified by column chromatography (0-40% EtOAc in n-heptanes) on silica gel to yield 4-azido-7-(trifluoromethyl)isochromane (1.0389 g, 4.27 mmol, 71.0 % yield) as a clear oil. ¹H NMR (400 MHz, CHLOROFORM-d) δ ppm 7.61 (d, J=8.6 Hz, 1 H), 7.55 (dd, J=8.2, 1.0 Hz, 1 H), 7.38 (s, 1 H), 4.92 (d, *J=*15.3 Hz, 1 H), 4.79 (d, *J=*15.5 Hz, 1 H), 4.19 - 4.33 (m, 2 H), 4.02 (dd, *J*=11.9*,* 2.9 Hz, 1 H)

Step 5. Anhydrous tetrahydrofuran (9698 µL) was added to solid supported PPh₃ (1.9 g, 4.11 mmol, Sigma-Aldrich Corporation) (2.15 mmol/g). The mixture was left to stand for 5 min, then a solution of 4-azido-7-(trifluoromethyl)isochromane (500 mg, 2.056 mmol) in THF was added. The suspension was agitated at rt overnight then iodomethane (1751 mg, 768 µL, 12.34 mmol, Sigma-Aldrich Corporation) was added. The mixture was stirred at room temperature overnight, filtered and the resin was washed with anhydrous THF and DCM. The resin was suspended in MeOH (2 mL) in a rbf and potassium hydroxide (254 mg, 4.52 mmol, Sigma-Aldrich Corporation) was added. The suspension was agitated at 65°C for 4 hours, cooled to rt, filtered and the resin washed with DCM and MeOH. The filtrate and washings were combined and concentrated to dryness. The crude product was partitioned between DCM and aqueous NaHCO₃, and the aqueous layer extracted with DCM. The combined organic extracts were dried over MgSO₄, filtered, and concentrated to give the amine. The crude material was absorbed onto a plug of silica gel and purified by chromatography through a Biotage pre-packed silica gel column, eluting with a gradient of 0% to 100% EtOAc in Heptanes, to yield N-methyl-7-(trifluoromethyl)isochroman-4-amine **(55,** 435.8 mg, 1.885 mmol, 92 % yield). *m*/*z* (ESI): 232.2 (M+H)⁺. ¹H NMR (400 MHz, CHLOROFORM-d) δ ppm 7.45 - 7.52 (m, 2 H), 7.28 (s, 1 H), 4.69 - 4.91 (m, 2 H), 4.19 (dd, *J=11.8,* 2.7 Hz, 1 H), 3.81 (dd, *J=11.8,* 2.9 Hz, 1 H), 3.55 (br s, 1 H), 2.54 (s, 3 H).

Step 6. N-methyl-7-(trifluoromethyl)isochroman-4-amine (435.8 mg) was purified via preparative SFC using a Chiral Technologies IG column x 2 (250 x 21 mm, 5 mm) with a mobile phase of 90% Liquid CO₂ and 10% MeOH with 0.2% TEA using a flow rate of 70 mL/min to generate 138.2 mg of peak 1 with an ee of >99% and 157.2 mg of peak 2 with an ee of 97.22%. Peak assignment was determined by SFC with an IG column with 10% MeOH and 0.2% TEA. Peak 1: (R)-N-methyl-7-(trifluoromethyl)isochroman-4-amine (56, 138.2 mg, 0.598 mmol, 29.1 % yield), *m*/*z* (ESI): 232.2 (M+H)⁺. ¹H NMR (400 MHz, CHLOROFORM-d) δ ppm 7.45 - 7.52 (m, 2 H), 7.28 (s, 1 H), 4.69 - 4.91 (m, 2 H), 4.19 (dd, *J=11.8,* 2.7 Hz, 1 H), 3.81 (dd, *J=11.8,* 2.9 Hz, 1 H), 3.55 (br s, 1 H), 2.54 (s, 3 H) Peak 2: (S)-N-methyl-7-(trifluoromethyl)isochroman-4-amine (57, 157.2 mg, 0.680 mmol, 33.1 % yield), *m*/*z* (ESI): 232.2 (M+H)⁺, ¹H NMR (400 MHz, CHLOROFORM-d) δ ppm 7.49 (s, 2 H), 7.28 (s, 1 H), 4.68 - 4.92 (m, 2 H), 4.19 (dd, *J*=11.6, 2.7 Hz, 1 H), 3.81 (dd, *J*=11.8, 2.9 Hz, 1 H), 3.55 (br s, 1 H), 2.53 (s, 3 H)

The following amines in Table 4 were prepared in a manner similar to that described for Intermediate **55-57.**

**Table 4**

| **Int.** | **Structure** | **Name** | **SFC Conditions** | ***m*/*z* (ESI): (M+H)⁺** |
|---|---|---|---|---|
| **58** | 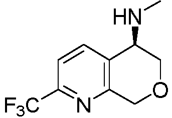 | Peak 1: (R)-N-methyl-2-(trifluoromethyl)-5,8-dihydro-6H-pyrano[3,4-b]pyridin-5-amine | Chiralpak IG column (21 x 500 mm, 5 µm) with a mobile phase of 90% Liquid CO₂ and 10% 1:1 ACN:MeOH with 0.2% DEA | 233.0 |
| **59** | 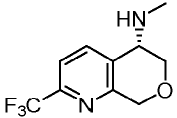 | Peak 2: (S)-N-methyl-2-(trifluoromethyl)-5,8-dihydro-6H-pyrano[3,4-b]pyridin-5-amine | | |
| **60** | 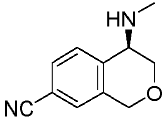 | Peak 1: (R)-4-(methylamino)isochromane-7-carbonitrile | Chiral Technologies AD column (250 x 21 mm, 5 mm) with a mobile phase of 90% Liquid CO₂ and 10% MeOH with 0.2% TEA | 189.2 |
| **61** | 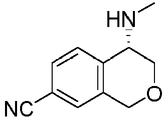 | Peak 2: (S)-4-(methylamino)isochromane-7-carbonitrile | | |
| **62** | 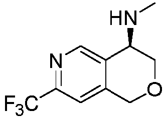 | Peak 1: (R)-N-methyl-7-(trifluoromethyl)-3,4-dihydro-1H-pyrano[4,3-c]pyridin-4-amine | Chiral Technologies AD-H column (250 x 21 mm, 5 µm) with a mobile phase of 80% Liquid CO₂ and 20% MeOH with 0.2% TEA | 233.0 |
| **63** | 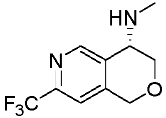 | Peak 2: (S)-N-methyl-7-(trifluoromethyl)-3,4-dihydro-1H-pyrano[4,3-c]pyridin-4-amine | | |
| **64** | 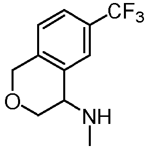 | N-methyl-6-(trifluoromethyl)isochroman-4-amine | | 232.0 |
| **65** | 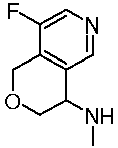 | 8-fluoro-N-methyl-3,4-dihydro-1H-pyrano[4,3-c]pyridin-4-amine | | 183.2 |
| **66** | 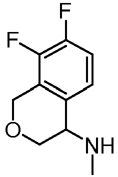 | 7,8-difluoro-N-methylisochroman-4-amine | | 200.2 |
| **67** | 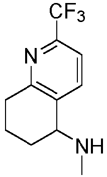 | N-methyl-2-(trifluoromethyl)-5,6,7,8-tetrahydroquinolin-5-amine | | 231.0 |
| **68** | 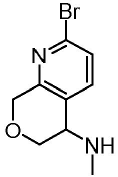 | 2-bromo-N-methyl-5,8-dihydro-6H-pyrano[3,4-b]pyridin-5-amine | | 243.0, 245.0 |
| **69** | 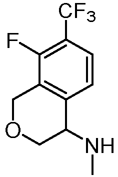 | 8-fluoro-N-methyl-7-(trifluoromethyl)isochroman-4-amine | | 250.0 |
| **70** | 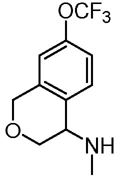 | N-methyl-7-(trifluoromethoxy)isochroman-4-amine | | 248.0 |
| **71** | 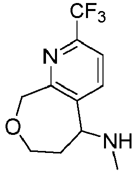 | N-methyl-2-(trifluoromethyl)-5,6,7,9-tetrahydrooxepino[3,4-b]pyridin-5-amine | | 247.2 |

### Intermediates 72 and 73: trans N,1-dimethyl-7-(trifluoromethyl)isochroman-4-amine and cis-N,1-dimethyl-7-(trifluoromethyl)isochroman-4-amine

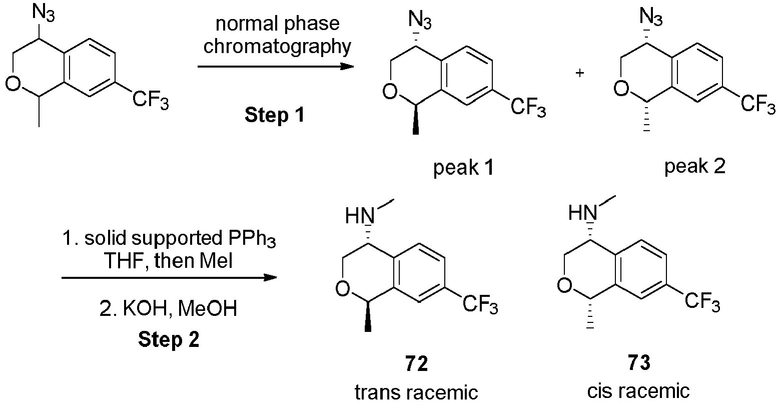

Step 1. 4-azido-1-methyl-7-(trifluoromethyl)isochromane was prepared using the same methods up to Step 4 for Intermediate 55. Crude 4-azido-1-methyl-7-(trifluoromethyl)isochromane was purified by column chromatography on silica gel with 0-40% EtOAc in n-heptanes. Peak 1 was determined to be the trans isomer and peak 2 was determined to be the cis isomer by looking at the crystal strucutre of the corresponding final analogues. Peak 1: trans-4-azido-1-methyl-7-(trifluoromethyl)isochromane (141.4 mg, 0.550 mmol, 24.4% yield) ¹H NMR (400 MHz, CHLOROFORM-d) δ ppm 7.56 - 7.60 (m, 2 H), 7.39 (s, 1 H), 5.01 (q, J=6.6 Hz, 1 H), 4.45 (t, J=5.3 Hz, 1 H), 4.30 (dd, J=11.7, 4.5 Hz, 1 H), 3.86 (dd, J=11.6, 6.4 Hz, 1 H), 1.58 (d, J=6.6 Hz, 3 H) Peak 2: cis-4-azido-1-methyl-7-(trifluoromethyl)isochromane (296.6 mg, 1.153 mmol, 51.5% yield) ¹H NMR (400 MHz, CHLOROFORM-d) δ ppm 7.60 (d, J=8.1 Hz, 1 H), 7.44 - 7.52 (m, 2 H), 4.85 (q, J=6.6 Hz, 1 H), 4.35 (dd, J=12.1, 1.6 Hz, 1 H), 4.13 (s, 1 H), 3.96 (dd, J=12.2, 2.3 Hz, 1 H), 1.65 (d, J=6.6 Hz, 3 H)

Step 2. Separately, both isomers were subjected to the same procedure; the following procedure is for the cis isomer (peak 2). Anhydrous tetrahydrofuran (5439 µL) was added to solid supported PPh₃ (1.07 g, 2.306 mmol, Sigma-Aldrich Corporation) (2.15 mmol/g). The mixture was left to stand for 5 min, then a solution of the cis-4-azido-1-methyl-7-(trifluoromethyl)isochromane (296.6 mg, 1.153 mmol) in THF was added. The suspension was agitated at rt overnight, then iodomethane (982 mg, 431 µL, 6.92 mmol, Sigma-Aldrich Corporation) was added. The mixture was stirred at rt overnight, filtered and the resin washed with THF and DCM. The resin was suspended in MeOH (2 mL) and transferred to a rbf, and a solution of potassium hydroxide (142 mg, 2.54 mmol, Sigma-Aldrich Corporation) (2% in MeOH) was added. The suspension was agitated at 65°C overnight, cooled to rt, filtered and the resin washed with DCM and MeOH. The filtrate and washings were combined and concentrated to dryness. The crude product was partitioned between DCM and aqueous NaHCO₃, and the aqueous layer extracted with EtOAc. The combined organic extracts were dried over anhyd MgSO₄, filtered, and concentrated to give the amine. The crude material was absorbed onto a plug of silica gel and purified by chromatography through a Biotage pre-packed silica gel column, eluting with a gradient of 0% to 100% EtOAc in Heptanes, to provide the product, cis-N,1-dimethyl-7-(trifluoromethyl)isochroman-4-amine (73) (213.1 mg, 0.869 mmol, 75% yield). *m*/*z* (ESI): 246.2 (M+H)⁺. ¹H NMR (400 MHz, CHLOROFORM-d) δ ppm 7.41 - 7.53 (m, 2 H), 7.33 - 7.39 (m, 1 H), 4.79 (q, J=6.4 Hz, 1 H), 4.30 (dd, J=11.8, 1.2 Hz, 1 H), 3.68 - 3.79 (m, 2 H), 3.46 (s, 1 H), 2.54 (s, 3 H), 1.60 (d, J=6.4 Hz, 3 H). The identical procedure was followed for peak 1, which yielded trans-N,1-dimethyl-7-(trifluoromethyl)isochroman-4-amine (72) (81.7 mg, 0.333 mmol, 60.6% yield). *m*/*z* (ESI): 246.2 (M+H)⁺. ¹H NMR (400 MHz, CHLOROFORM-d) δ ppm 7.55 - 7.60 (m, 1 H), 7.48 - 7.51 (m, 1 H), 7.31 (s, 1 H), 4.96 (q, J=6.5 Hz, 1 H), 4.16 (dd, J=11.5, 3.8 Hz, 1 H), 3.75 - 3.84 (m, 1 H), 3.67 - 3.74 (m, 1 H), 2.52 (s, 3 H), 1.54 (d, J=6.6 Hz, 3 H).

### Intermediate 74: N-methyl-3-(trifluoromethyl)-7,8-dihydro-5H-pyrano[4,3-b]pyridin-8-amine

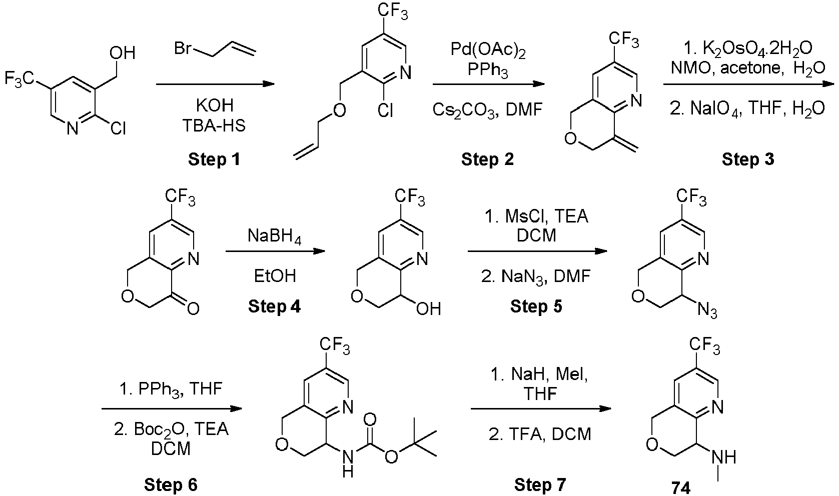

Step 1. To a solution of [2-chloro-5-(trifluoromethyl)-3-pyridyl]methanol (1.00 g, 1 mL, 4.73 mmol, Aurum Pharmatech LLC.) in dichloromethane (5 mL), was added allyl bromide (0.572 g, 0.411 mL, 4.73 mmol, Sigma-Aldrich Corporation), potassium hydroxide (0.504 g, 8.98 mmol, Sigma-Aldrich Corporation) and tetrabutylammonium hydrogen sulfate (0.241 g, 0.709 mmol, Sigma-Aldrich Corporation). The overall reaction mixture was stirred at rt overnight. The reaction mixture was diluted with DCM and water. The layers were separated and the aqueous layer was extracted with DCM (3x). The combined organic extracts were dried over MgSO₄, filtered and concentrated in vacuo. The crude material was absorbed onto a plug of silica gel and purified by chromatography through a silica gel column, eluting with a gradient of 0-25% EtOAc in heptane, to provide 3-((allyloxy)methyl)-2-chloro-5-(trifluoromethyl)pyridine (1.116 g, 4.44 mmol, 94% yield) as light-yellow oil. *m*/*z* (ESI): 252.0 (M+H)⁺. ¹H NMR (400 MHz, DMSO-*d*₆) δ ppm 8.81 (br s, 1 H), 8.21 (br s, 1 H), 5.88 - 6.03 (m, 1 H), 5.15 - 5.37 (m, 2 H), 4.53 - 4.63 (m, 2 H), 4.09 - 4.17 (m, 2 H).

Step 2. To an oven-dried 2-neck 100-mL round-bottomed flask was added 3-((allyloxy)methyl)-2-chloro-5-(trifluoromethyl)pyridine (1.116 g, 4.44 mmol), triphenylphosphine (0.523 g, 1.996 mmol, Sigma-Aldrich Corporation) and cesium carbonate (1.734 g, 5.32 mmol, Sigma-Aldrich Corporation) in N, N-dimethylformamide (15 mL). The reaction mixture was sparged with Argon (gas) for 5 minutes, then palladium (ii) acetate (0.149 g, 0.665 mmol, Sigma-Aldrich Corporation) was added to the reaction mixture. The resulting reaction mixture was stirred and heated at 90°C for 16 h. The reaction mixture was cooled to rt, then filtered through a pad of Celite. The filtrate was collected, then partially concentrated in vacuo (to remove most DMF). The residue was diluted with EtOAc and water. The aqueous layer was extracted with EtOAc (3x). The combined organic extracts were dried over MgSO₄, filtered and concentrated in vacuo. The crude material was absorbed onto a plug of silica gel and purified by chromatography through a silica gel column, eluting with a gradient of 0-20% EtOAc in heptane, to provide 8-methylene-3-(trifluoromethyl)-7,8-dihydro-5H-pyrano[4,3-b]|pyridine (0.256 g, 1.190 mmol, 26.8 % yield) as light-yellow solid. ¹H NMR (400 MHz, DMSO-*d*₆) δ ppm 8.86 (s, 1 H), 8.06 (s, 1 H), 6.32 (d, *J=*1.3 Hz, 1 H), 5.36 (d, *J=1.5* Hz, 1 H), 4.88 (s, 2 H), 4.58 (s, 2 H). *m*/*z* (ESI): 216.0 (M+H)⁺.

Step 3. To a stirred solution of 8-methylene-3-(trifluoromethyl)-7,8-dihydro-5H-pyrano[4,3-b]pyridine (0.240 g, 1.115 mmol) in acetone (5 mL)/ water (1 mL) was added potassium osmate (vi) dihydrate (0.041 g, 0.112 mmol, Acros Organics) and 4-methylmorpholine 4-oxide (0.457 g, 3.90 mmol, Sigma-Aldrich Corporation). The resulting reaction mixture was stirred at rt for 1.5 h. The reaction mixture was quenched with the addition of solid sodium sulfite (240 mg) and stirred 10 min. The reaction mixture was partially concentrated (to remove acetone) in vacuo. The residue was diluted with EtOAc and brine solution. The layers were separated and the aqueous layer was extracted with EtOAc (3x). The combined organic extracts were dried over MgSO₄, filtered and concentrated in vacuo. The crude material was absorbed onto a plug of silica gel and purified by chromatography through a silica gel column, eluting with a gradient of 0-10% MeOH in DCM, to provide 8-(hydroxymethyl)-3-(trifluoromethyl)-7,8-dihydro-5H-pyrano[4,3-b]pyridin-8-ol (0.270 g, 1.084 mmol, 97% yield). *m*/*z* (ESI): 250.0 (M+H)⁺.

The previous residue was diluted with THF (6 mL), then sodium (meta)periodate (0.716 g, 3.35 mmol, Sigma-Aldrich Corporation) and water (0.2 mL) were added to the reaction mixture. The resulting mixture was stirred at rt for 16 h. The reaction mixture was diluted with a mixture of EtOAc: Heptane (1: 1). The heterogeneous mixture was filtered through a pad of Celite and the filtrate was collected. The filtrate was treated with sat. aq. NaHCO₃, then the layers were separated and the aqueous layer was extractred with EtOAc (3x). The combined organic extracts were dried over MgSO₄, filtered and concentrated in vacuo. The crude material was absorbed onto a plug of silica gel and purified by chromatography through a silica gel column, eluting with a gradient of 0-30% EtOAc:EtOH (3: 1) in heptane, to provide 3-(trifluoromethyl)-5H-pyrano[4,3-b]pyridin-8(7H)-one (0.190 g, 0.875 mmol, 78 % yield) as off-white solid. *m*/*z* (ESI): 218.2 (M+H)⁺.

Step 4. To a solution of 3-(trifluoromethyl)-5H-pyrano[4,3-b]pyridin-8(7H)-one (0.171 g, 0.787 mmol) in ethanol (4 mL) was added sodium borohydride (0.030 g, 0.787 mmol, Sigma-Aldrich Corporation). The reaction mixture was stirred at rt for 1 h. The reaction mixture was diluted with EtOAc and sat. aq. NH₄Cl, then the aqueous layer was extracted with EtOAc (3x). The combined organic extracts were dried over MgSO₄, filtered and concentrated in vacuo, to afford crude 3-(trifluoromethyl)-7,8-dihydro-5H-pyrano[4,3-b]pyridin-8-ol. This material was used without further purification. *m*/*z* (ESI): 220.1 (M+H)⁺.

Step 5. To a 50-mL round-bottomed flask was added 3-(trifluoromethyl)-7,8-dihydro-5H-pyrano[4,3-b]pyridin-8-ol (0.170 g, 0.776 mmol) in dichloromethane (2 mL). The mixture was cooled to 0 °C, then methanesulfonyl chloride (0.107 g, 0.07 mL, 0.931 mmol, Sigma-Aldrich Corporation), followed by triethylamine (0.102 g, 0.14 mL, 1.008 mmol, Sigma-Aldrich Corporation) were added to the reaction mixture. The reaction mixutre was stirred at 0°C for 15 min, then at rt for 30 min. The reaction mixture was diluted with DCM and brine solution, then the biphasic solution was transferred to a separatory funnel. The aqueous layer was extracted with DCM (3x). The combined organic extracts were dried over MgSO₄, filtered and concentrated in vacuo, to afford 3-(trifluoromethyl)-7,8-dihydro-5H-pyrano[4,3-b]pyridin-8-yl methanesulfonate. This material was used without further purification. *m*/*z* (ESI): 298.0 (M+H)⁺.

The previous residue was diluted with N, N-dimethylformamide (2 mL), then sodium azide (0.101 g, 1.551 mmol, Sigma-Aldrich Corporation) was added slowly to the reaction mixture. The resulting reaction mixture was stirred at rt for 1.5 h. The reaction mixture was diluted with EtOAc and brine. The biphasic solution was transferred to a separatory funnel and the aqueous layer was extracted with EtOAc (3x). The combined organic extracts were dried over MgSO₄, filtered and concentrated in vacuo. The crude material was absorbed onto a plug of silica gel and purified by chromatography through a silica gel column, eluting with a gradient of 0-25% EtOAc in heptane, to provide 8-azido-3-(trifluoromethyl)-7,8-dihydro-5H-pyrano[4,3-b]pyridine (0.080 g, 0.328 mmol, 42.2% yield) as colorless oil. *m*/*z* (ESI): 245.0 (M+H)⁺.

Step 6. To a 50-mL round-bottomed flask was added 8-azido-3-(trifluoromethyl)-7,8-dihydro-5H-pyrano[4,3-b]pyridine (0.075 g, 0.307 mmol) in tetrahydrofuran (1 mL). Then triphenylphosphine (0.161 g, 0.614 mmol, Sigma-Aldrich Corporation) was added to the reaction mixture and stirred at rt for 16 h. The reaction mixture was diluted with EtOAc and brine. The layers were separated and the aqueous layer was extracted with EtOAc (3x). The combined organic extracts were dried over MgSO₄, filtered and concentrated in vacuo. *m*/*z* (ESI): 219.0 (M+H)⁺.

The previous residue was diluted with dichloromethane (1 mL), then di-tert-butyl dicarbonate (0.101 g, 0.107 mL, 0.461 mmol, Oakwood Products) and triethylamine (0.093 g, 0.130 mL, 0.921 mmol, Sigma-Aldrich Corporation) were added. The overall reaction mixture was stirred at rt for 16 h. The reaction mixture was treated with sat. aq. NaHCO₃ and diluted with DCM. The layers were separated and the aqueous layer was extracted with DCM (3x). The combined organic extracts were dried over MgSO₄, filtered and concentrated in vacuo. The crude material was absorbed onto a plug of silica gel and purified by chromatography through a silica gel column, eluting with a gradient of 0-30% EtOAc in heptane, to provide tert-butyl (3-(trifluoromethyl)-7,8-dihydro-5H-pyrano[4,3-b]pyridin-8-yl)carbamate (0.076 g, 0.239 mmol, 78 % yield) as off-white solid. *m*/*z* (ESI): 319.1 (M+H)⁺.

Step 7. To a 50-mL round-bottomed flask was added tert-butyl (3-(trifluoromethyl)-7,8-dihydro-5H-pyrano[4,3-b]pyridin-8-yl)carbamate (0.070 g, 0.220 mmol) in tetrahydrofuran (1 mL). The mixture was cooled to 0 °C, then sodium hydride (60% dispersion in mineral oil) (10.56 mg, 0.264 mmol, Oakwood Products) was added to the reaction mixture. The resulting mixture was stirred at 0 °C for 2 h, then iodomethane (0.037 g, 0.04 mL, 0.264 mmol, Sigma-Aldrich Corporation) was added dropwise. The reaction mixture was stirred an additional 20 min, while the temperature was maintained at 0°C, then it was stirred at rt overnight. The reaction mixture was quenched with MeOH and concentrated *in vacuo.* The crude material was absorbed onto a plug of silica gel and purified by chromatography through a silica-gel column, eluting with a gradient of 0-25% EtOAc in heptane, to provide tert-butyl methyl(3-(trifluoromethyl)-7,8-dihydro-5H-pyrano[4,3-b]pyridin-8-yl)carbamate as colorless oil. *m*/*z* (ESI): 333.0 (M-BOC+H)⁺.

The previous residue was dissolved in dichloromethane (1 mL), then treated with trifluoroacetic acid (0.251 g, 0.2 mL, 2.199 mmol, Sigma-Aldrich Corporation). The reaction mixture was stirred at rt for 1 h. The reaction mixture was concentrated in vacuo. The residue was diluted with DCM, then treated with sat. aq. NaHCO₃. The layers were separated and the aqueous layer was extracted with DCM (3x). The combined organic extracts were dried over MgSO₄, filtered and concentrated *in vacuo.* The crude N-methyl-3-(trifluoromethyl)-7,8-dihydro-5H-pyrano[4,3-b]pyridin-8-amine (74) was used, without further purification. *m*/*z* (ESI): 233.0 (M+H)⁺.

### Intermediate 75: 2-methoxy-N-methyl-5,8-dihydro-6H-pyrano[3,4-b]pyridin-5-amine

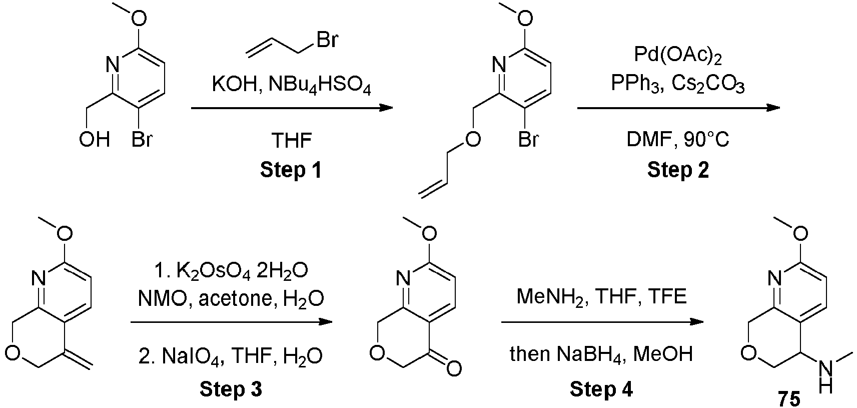

Step 1. To a stirred solution of (3-bromo-6-methoxypyridin-2-yl)methanol (1.0 g, 4.59 mmol) in tetrahydrofuran (20 mL) was added allyl bromide (0.476 mL, 5.50 mmol) at rt. Then KOH (0.515 g, 9.17 mmol) was added followed by tetrabutylammonium hydrogen sulfate (0.234 g, 0.688 mmol) at rt. The reaction mixture was stirred for 16 h at rt. The reaction mixture was diluted with ethyl acetate and washed with water and the organic layer was dried over anhydrous Na₂SO₄ and concentrated under vacuum to get the crude material. The crude was purified by column chromatography, eluting with 10% EtOAc in petroleum ether, to get 2-((allyloxy)methyl)-3-bromo-6-methoxypyridine (900 mg, 3.49 mmol, 76% yield) as a colourless oil. *m*/*z* (ESI): 258.1, 260.1 (M+H)⁺.

Step 2. To a stirred solution of 2-((allyloxy)methyl)-3-bromo-6-methoxypyridine (250 mg, 0.969 mmol) in N, N-dimethylformamide (2.5 mL) were added cesium carbonate (379 mg, 1.162 mmol), palladium (II) acetate (43.5 mg, 0.194 mmol) and triphenylphosphine (127 mg, 0.484 mmol) at rt and stirred at 90 °C for 30 min. The reaction mixture was filtered through celite and washed with ethyl acetate. Then the filterate was washed with water and the layers were separated out. The organic layer was washed with brine and dried over anhy. Na₂SO₄, filtered and concentrated under reduced pressure. The crude residue was purified by column chromatography over silica gel (230-400mesh) using 5% ethyl acetate in hexanes as an eluent to give 2-methoxy-5-methylene-5,8-dihydro-6H-pyrano[3,4-b]pyridine (90 mg, 0.508 mmol, 52.4% yield) as a yellow solid. *m*/*z* (ESI): 178.2 (M+H)⁺. ¹H NMR (400 MHz, Chloroform-d) δ 7.85 (d, J = 8.7 Hz, 1H), 6.65 (dq, J = 8.5, 0.9 Hz, 1H), 5.44 (d, J = 1.2 Hz, 1H), 5.00 - 4.97 (m, 1H), 4.76 (s, 2H), 4.43 (t, J = 1.2 Hz, 2H), 3.93 (s, 3H).

Step 3. To a stirred solution of 2-methoxy-5-methylene-5,8-dihydro-6H-pyrano[3,4-b]pyridine (3.8 g, 21.44 mmol) in mixture of acetone (50 mL) and water (10 mL) was added potassium osmate (vi) dihydrate (0.790 g, 2.144 mmol) followed by addition of 4-methylmorpholine 4-oxide (8.79 g, 75 mmol) at rt and the reaction mixture was stirred at rt for 16 h. The reaction mixture was quenched with the addition of solid sodium sulfite and the mixture was stirred for 10 min. The reaction mixture was partially concentrated (to remove acetone) in vacuo, then it was diluted with ethyl acetate and extracted with ethyl acetate (3 x 100 mL), washed with water and the organic layer was dried over anhydrous Na₂SO₄ and concentrated under vacuum to get crude 5-(hydroxymethyl)-2-methoxy-5,8-dihydro-6H-pyrano[3,4-b]pyridin-5-ol as brown colour oil. *m*/*z* (ESI): 212.3 (M+H)⁺.

To a stirred solution of 5-(hydroxymethyl)-2-methoxy-5,8-dihydro-6H-pyrano[3,4-b]pyridin-5-ol (4.8 g, 22.73 mmol) in tetrahydrofuran (100 mL) and water (20 mL) was added sodium periodate (12.15 g, 56.8 mmol) at rt and the reaction mixture was stirred at rt for 1h. The reaction mixture was diluted with ethyl acetate and washed with sat. NaHCO₃ solution and the organic layer was dried over anhydrous Na₂SO₄ and concentrated under vacuum to get crude compound, which was purified by column chromatography by eluting with 20% EtOAc in petroleum ether, to get 2-methoxy-6H-pyrano[3,4-b]pyridin-5(8H)-one (2.6 g, 14.51 mmol, 63.9 % yield) as tan solid. *m*/*z* (ESI): 180.1 (M+H)⁺. ¹H NMR (400 MHz, Chloroform-d) δ 8.17 (d, J = 8.6 Hz, 1H), 6.76 (d, J = 8.6 Hz, 1H), 4.86 (s, 2H), 4.36 (s, 2H), 4.02 (d, J = 1.2 Hz, 3H).

Step 4. To a stirred solution of 2-methoxy-6H-pyrano[3,4-b]pyridin-5(8H)-one (2.0 g, 11.16 mmol) in trifluoroethanol (14.46 mL, 201 mmol) was added methylamine in THF (27.9 mL, 55.8 mmol) and the reaction mixture was stirred at rt for 16 h. After 16 h, methanol (4.00 mL) was added followed by sodium borohydride (2.111 g, 55.8 mmol) at 0°C. The reaction mixture was allowed to come to rt and stirred for 1 h. The reaction mixture was concentrated under reduced pressure, then quenched with 10% sodium bicarbonate and extracted with 10% MeOH in DCM. The combined organic layer was dried over sodium sulphate, and concentrated. The crude material was purified by column chromatography eluting with 5% MeOH in DCM, to get 2-methoxy-N-methyl-5,8-dihydro-6H-pyrano[3,4-b]pyridin-5-amine **(75)** (1.5 g, 7.72 mmol, 69.2 % yield) as pale yellow liquid. *m*/*z* (ESI): 195.1 (M+H)⁺.

The following amines in Table 5 were prepared in a manner similar to that described for Intermediate **75.**

**Table 5**

| **Int. #** | **Chemical Structure** | **Name** | **SFC Conditions** | ***m*/*z* (ESI: (M+H)⁺** |
|---|---|---|---|---|
| 76 | 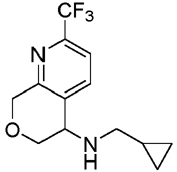 | N-(cyclopropylmethyl)-2-(trifluoromethyl)-5,8-dihydro-6H-pyrano[3,4-b]pyridin-5-amine | | 273.1 |
| 77 | 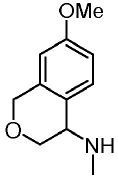 | 7-methoxy-N-methylisochroman-4-amine | | 194.2 |
| 78 | 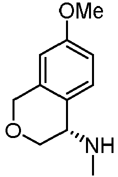 | (S)-7-methoxy-N-methylisochroman-4-amine | 2nd peak, Chiralpak AZ column (21x250 mm) with a mobile phase of 85% Liquid CO₂ and 15% methanol with 0.2% diethylamine using a flow rate of 80 ml/min | 194.2 |
| 79 | 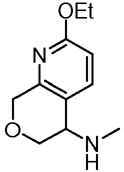 | 2-ethoxy-N-methyl-5,8-dihydro-6H-pyrano[3,4-b]pyridin-5-amine | | 209.2 |
| **80** | 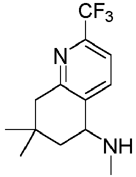 | N,7,7-trimethyl-2-(trifluoromethyl)-5,6,7,8-tetrahydroquinolin-5-amine | | 259.2 |

### Intermediate 81: (R)-2-methoxy-N-methyl-5,8-dihydro-6H-pyrano[3,4-b]pyridin-5-amine

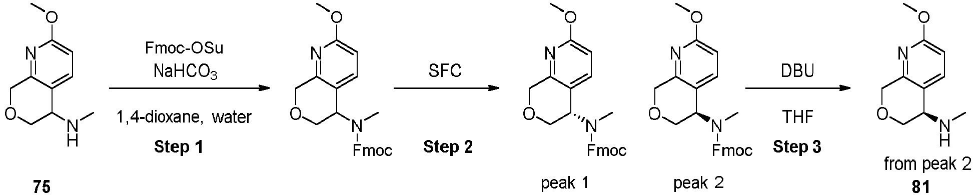

Step 1. To a stirred solution of 2-methoxy-N-methyl-5,8-dihydro-6H-pyrano[3,4-b]pyridin-5-amine **(75)** (1.25g, 6.44 mmol) in 1,4-dioxane (12.50 mL) and water (12.50 mL) were added sodium bicarbonate (0.811 g, 9.65 mmol) and Fmoc-OSu (3.26 g, 9.65 mmol) at 0°C, the reaction was allowed to come to rt and stirred for 1 h. After completion of the reaction, the reaction mixture quenched with water, extracted with ethyl acetate, dried over sodium sulphate and concentrated under reduced pressure. The obtained crude was purified by column chromatography; the compound eluted in 15% ethyl acetate in pet ether to yield 9H-fluoren-9-yl)methyl (2-methoxy-5,8-dihydro-6H-pyrano[3,4-b]pyridin-5-yl)(methyl)carbamate as the racemic compound.

Step 2. Racemic 9H-fluoren-9-yl)methyl (2-methoxy-5,8-dihydro-6H-pyrano[3,4-b]pyridin-5-yl)(methyl)carbamate was separated by chiral SFC using a Chiralcel OD-H column (250 x 21 mm, 5µm), with a mobile phase of 79% Liquid CO₂ and 21% MeOH with 0.2% TEA using a flow rate of 70 mL/min to get 840 mg of each isomer. Peak 1 is the more potent isomer.

Step 3. To a 100-mL round-bottomed flask were added (9H-fluoren-9-yl)methyl (R)-(2-methoxy-5,8-dihydro-6H-pyrano[3,4-b]pyridin-5-yl)(methyl)carbamate (840mg, 2.017 mmol) and DBU, 20% in THF (1520 µL, 2.017 mmol) at 0°C. The reaction was stirred at 0°C for 30 min. After completion of reaction, the reaction mixture was diluted with water and extracted with 20% MeOH in DCM. The combined organic layer dried over sodium sulphate, and concentrated under reduced pressure. The obtained crude was purified by column chromatography and the compound eluted in 5% MeOH in DCM to give (R)-2-methoxy-N-methyl-5,8-dihydro-6H-pyrano[3,4-b]pyridin-5-amine **(81)** (289 mg, 1.488 mmol, 73.8 % yield) as brown liquid. *m*/*z* (GCMS): 194.1. ¹H NMR (401 MHz, DMSO-*d*₆) δ 7.69 (d, *J* = 8.4 Hz, 1H), 6.69 (d, *J* = 8.4 Hz, 1H), 4.53 (q, *J =* 16.0 Hz, 2H), 3.89 (dd, *J* = 11.5, 4.1 Hz, 1H), 3.80 (s, 3H), 3.77 (d, *J = 3.7* Hz, 1H), 3.52 (t, *J=* 3.9 Hz, 1H), 2.30 (s, 3H).

The following amines in Table 6 were prepared in a manner similar to that described for Intermediate **81** with the shown SFC conditions being used during Step 2.

**Table 6**

| **Int.** | **Structure** | **Name** | **SFC Conditions** | ***m*/*z* (ESI: (M+H)⁺** |
|---|---|---|---|---|
| **82** | 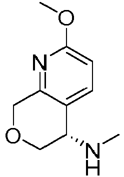 | (S)-2-methoxy-N-methyl-5,8-dihydro-6H-pyrano[3,4-b]pyridin-5-amine | 1st peak, Chiralcel OD-H column (250 x 21 mm, 5µm) with a mobile phase of 79% Liquid CO₂ and 21% MeOH with 0.2% TEA using a flow rate of 70 mL/min | 194.1 (GCMS) |
| **83** | 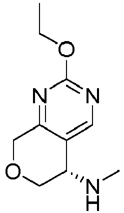 | (S)-2-ethoxy-N-methyl-5,8-dihydro-6H-pyrano[3,4-d]pyrimidin-5-amine | 1st peak, Chiralpak OJ-H column (250 x 21 mm, 5 micron) with a mobile phase of 85% Liquid CO₂ and 15% methanol using a flow rate of 120 mL/min | 210.3 |
| **84** | 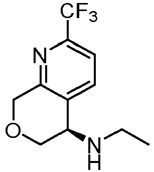 | (R)-N-ethyl-2-(trifluoromethyl)-5,8-dihydro-6H-pyrano[3,4-b]pyridin-5-amine | 1st peak, Chiralpak IG column (250 x 30 mm, 5 micron) with a mobile phase of 60% Liquid CO₂ and 40% MeOH with 0.2% TEA using a flow rate of 90 mL/min | 246.0 (GCMS) |
| **85** | 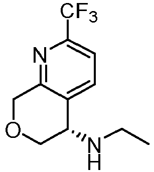 | (S)-N-ethyl-2-(trifluoromethyl)-5,8-dihydro-6H-pyrano[3,4-b]pyridin-5-amine | 2nd peak, Chiralpak IG column (250 x 30 mm, 5 micron) with a mobile phase of 60% Liquid CO₂ and 40% MeOH with 0.2% TEA using a flow rate of 90 mL/min | 245.9 (GCMS) |
| **86** | 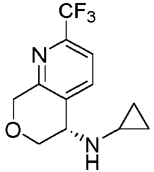 | (S)-N-cyclopropyl-2-(trifluoromethyl)-5,8-dihydro-6H-pyrano[3,4-b]pyridin-5-amine | 2nd peak, Chiralcel OD-H column (250 x 30 mm, 5 micron) with a mobile phase of 60% Liquid CO₂ and 40% methanol with 0.2% TEA using a flow rate of 90 mL/min | 259.1 |
| **87** | 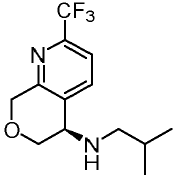 | (R)-N-isobutyl-2-(trifluoromethyl)-5,8-dihydro-6H-pyrano[3,4-b]pyridin-5-amine | 1st peak, Chiralpak OD-H column (250 x 21 mm, 5 micron) with a mobile phase of 85% Liquid CO₂ and 15% methanol using a flow rate of 70 mL/min | 274.1 (GCMS) |
| **88** | 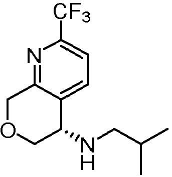 | (S)-N-isobutyl-2-(trifluoromethyl)-5,8-dihydro-6H-pyrano[3,4-b]pyridin-5-amine | 2nd peak, Chiralpak OD-H column (250 x 21 mm, 5 micron) with a mobile phase of 85% Liquid CO₂ and 15% methanol using a flow rate of 70 mL/min | 274.1 (GCMS) |
| **89** | 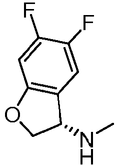 | (S)-5,6-difluoro-N-methyl-2,3-dihydrobenzofuran-3-amine | 1st peak, Chiralpak IG column (250 x 30 mm, 5 micron) with a mobile phase of 90% Liquid CO₂ and 10% MeOH with 0.2% TEA using a flow rate of 90 mL/min | 184.1 (oxidized) |
| **90** | 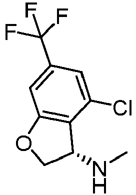 | (S)-4-chloro-N-methyl-6-(trifluoromethyl)-2,3-dihydrobenzofuran-3-amine | 1st peak, Chiralcel OD-H column (150 x 4.6 mm) with a mobile phase of 80% Liquid CO₂ and 20% MeOH using a flow rate of 3 mL/min | 232.1 (oxidized) |
| **91** | 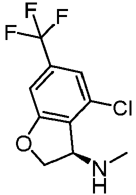 | (R)-4-chloro-N-methyl-6-(trifluoromethyl)-2,3-dihydrobenzofuran-3-amine | 2nd peak, Chiralcel OD-H column (150 x 4.6 mm) with a mobile phase of 80% Liquid CO₂ and 20% MeOH using a flow rate of 3 mL/min | 232.1 (oxidized) |

### Intermediate 92: 5-(methylamino)-5,8-dihydro-6H-pyrano[3,4-b]pyridine-2-carbonitrile

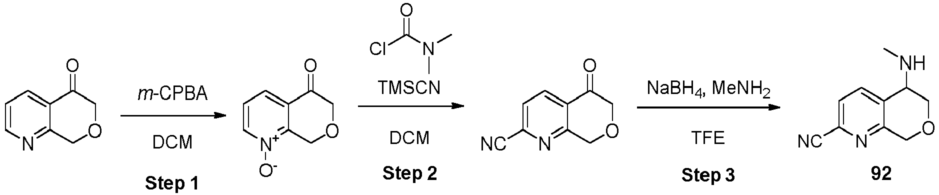

Step 1. 6H-pyrano[3,4-b]pyridin-5(8H)-one (80.0 mg, 0.536 mmol, 1.0 equiv, Enamine) was dissolved in DCM (5.36 mL) and m-CPBA (93.0 mg, 0.536 mmol, 1.0 equiv, Sigma-Aldrich Corporation) was added. The reaction was stirred overnight to completion and then diluted with water and DCM. The layers were separated, and the aqueous layer was extracted with DCM (2 x 25 mL). The combined organic layers were dried over MgSO₄ and the crude product was purified by medium pressure chromatography (silica, 0 to 100% EtOAc:Heptanes) to give 5-oxo-5,8-dihydro-6H-pyrano[3,4-b]pyridine 1-oxide (16.0 mg, 0.097 mmol, 18.1% yield). *m*/*z* (ESI): 166.0 (M+H)⁺.

Step 2. 5-oxo-5,8-dihydro-6H-pyrano[3,4-b]pyridine 1-oxide (16.0 mg, 0.097 mmol, 1.0 equiv) was dissolved in dichloromethane (969 µL) and dimethylcarbamoyl chloride (17.8 µL, 0.194 mmol, 2.0 equiv, Sigma-Aldrich Corporation) was added followed by trimethylsilyl cyanide (26.0 µL, 0.194 mmol, 2.0 equiv, Sigma-Aldrich Corporation). The resulting solution was stirred at rt for 4.5 days to near completion. The mixture was then directly loaded onto a column for medium pressure chromatography (silica, 0 to 75% EtOAc:Heptanes) to give 5-oxo-5,8-dihydro-6H-pyrano[3,4-b]pyridine-2-carbonitrile (7.00 mg, 0.0400 mmol, 41.5 % yield). *m*/*z* (ESI): 175.0 (M+H)⁺.

Step 3. 5-oxo-5,8-dihydro-6H-pyrano[3,4-b]pyridine-2-carbonitrile (40.0 mg, 0.230 mmol, 1.0 equiv) was dissolved in trifluoroethanol (1.53 mL) and methylamine (2.0 M in THF) (0.459 mL, 0.919 mmol, 4.0 equiv, Sigma-Aldrich Corporation) was added and the solution was stirred overnight to form the imine. Sodium borohydride (21.7 mg, 0.574 mmol, 2.5 equiv, Sigma-Aldrich Corporation) was then added and the reaction was stirred for 45 minutes to completion. The reaction was then quenched by dropwise addition of water (10 mL) and this mixture was extracted with EtOAc (2 x 30 ml). The combined organic layers were then dried over Na₂SO₄. The crude product was then purified by medium pressure chromatography (silica, 0 to 100% EtOAc:Heptanes to 40 to 100%(hold) (3:1 EtOAc:EtOH):Heptanes) to give 5-(methylamino)-5,8-dihydro-6H-pyrano[3,4-b]pyridine-2-carbonitrile (10 mg, 0.053 mmol, 23.0 % yield). (**92**). *m*/*z* (ESI): 190.2 (M+H)⁺.

### Intermediate 94: 4-(methylamino)isochromane-7-carbonitrile

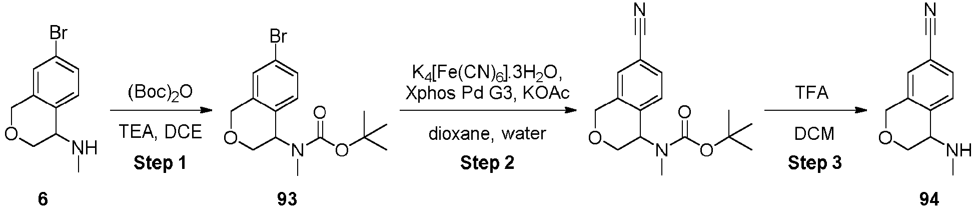

Step 1. To a 100-mL round-bottomed flask was added 7-bromo-N-methylisochroman-4-amine (6) (0.130 g, 0.537 mmol, 1.0 eq,) and di-tert-butyl dicarbonate (0.176 g,0.805 mmol, 1.50 eq. Oakwood Products) in 1,2-dichloroethane (2.68 mL). Then triethylamine (0.163 g, 0.226 mL, 1.611 mmol, 3.0 eq.Sigma-Aldrich Corporation) was added to the reaction mixture and the overall mixture was stirred at rt for 2 h. The reaction mixture was diluted with DCM (5 mL) and sat. aq. NaHCO3 (5 mL). The layers were separated, and the aqueous layer was extracted with DCM (3x). The combined organic extracts were dried over MgSO₄, filtered, and concentrated in vacuo. The crude material was absorbed onto a plug of silica gel and purified by chromatography through a silica gel column, eluting with a gradient of 0-20% EtOAc in heptane, to provide tert-butyl (7-bromoisochroman-4-yl)(methyl)carbamate (93) (0.181 g, 0.529 mmol, 99 % yield) as off-white solid. *m*/*z* (ESI): 342.0 (M+H)⁺. ¹H NMR (400 MHz, DMSO-*d*₆) δ ppm 7.47 (br d, J=8.2 Hz, 1 H), 7.39 (s, 1 H), 7.05 - 7.13 (m, 1 H), 4.93 - 5.24 (m, 1 H), 4.70 - 4.78 (m, 1 H), 4.57 - 4.65 (m, 1 H), 3.85 - 4.00 (m, 2 H), 2.53 - 2.62 (m, 3 H), 1.46 (s, 9 H).

Step 2. A glass resealable vial was charged with tert-butyl (7-bromoisochroman-4-yl)(methyl)carbamate (0.075 g, 0.219 mmol, 1.0 eq.) and potassium ferrocyanide trihydrate (0.370 g, 0.877 mmol, 4.0 eq. Toronto Research Chemicals) in a 1:1 mixture of 1,4-dioxane (1.10 mL)/water (1.10 mL). The reaction mixture was sparged with Argon (gas) for 5 min, then xphos pd g3 (0.037 g, 0.044 mmol, 0.2 eq. Sigma-Aldrich Corporation) and potassium acetate (0.065 g, 0.657 mmol, 3.0 eq. Sigma-Aldrich Corporation) were added to the reaction mixture. The resulting reaction mixture was stirred and heated at 100 °C for six hours. The reaction mixture was diluted with EtOAc, then filtered through a pad of celite. The filter cake was rinsed with MeOH:EtOAc (2: 1) and the filtrate was collected, then the combined organics were concentrated in vacuo. The crude material was absorbed onto a plug of silica gel and purified by chromatography through a silica-gel column, eluting with a gradient of 0-30% EtOAc in heptane, to provide tert-butyl (7-cyanoisochroman-4-yl)(methyl)carbamate (0.058 g, 0.201 mmol, 92 % yield) as light-yellow oil. *m*/*z* (ESI): 342.0 (M+H)⁺.

Step 3. To a 50-mL round-bottomed flask was added tert-butyl (7-cyanoisochroman-4-yl)(methyl)carbamate (0.058 g, 0.201 mmol) and trifluoroacetic acid (0.229 g, 0.150 mL, 2.011 mmol, Sigma-Aldrich Corporation) in dichloromethane (1.006 mL). The resulting reaction mixture was stirred at rt for one hour. The reaction mixture was concentrated in vacuo. The crude 4-(methylamino)isochromane-7-carbonitrile (94) was used in next step of synthesis, without further purification. *m*/*z* (ESI): 182.9 (M+H)⁺.

### Intermediate 95: N-methyl-7-(methylsulfonyl)isochroman-4-amine

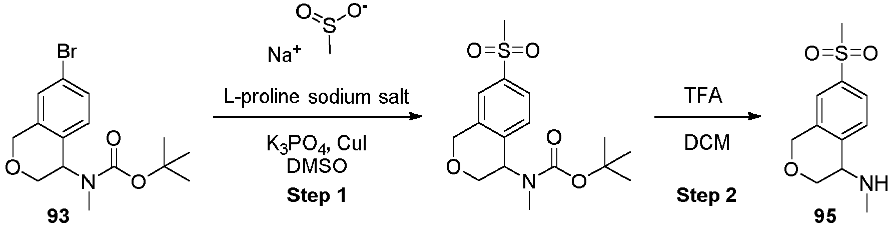

Step 1. A glass reaction vessel was charged with tert-butyl (7-bromoisochroman-4-yl)(methyl)carbamate **(93)** (0.310 g, 0.906 mmol), methanesulfinic acid, sodium salt (0.555 g, 5.43 mmol, TCI America), copper (i) iodide (0.035 g, 0.181 mmol, Alfa Aesar), (s)-pyrrolidine-2-carboxylic acid, sodium salt (0.025 g, 0.181 mmol, Combi-Blocks) and potassium phosphate tribasic (0.385 g, 1.812 mmol, Acros Organics) in dimethyl sulfoxide (4.53 mL). The atmosphere of the reaction vessel was evacuated, then backfilled with Argon (3x). The vial was sealed, then the reaction mixture was stirred and heated at 100 °C for 16 h. The reaction mixture was diluted with EtOAc and water. The layers were separated and the aqueous layer was extracted with EtOAc (3x). The combined organic extracts were dried over MgSO₄, filtered and concentrated in vacuo. The crude material was absorbed onto a plug of silica gel and purified by chromatography through a silica gel column, eluting with a gradient of 0-50% EtOAc in heptane, to provide tert-butyl methyl(7-(methylsulfonyl)isochroman-4-yl)carbamate (0.202 g, 0.592 mmol, 65.3% yield) as colorless oil. *m*/*z* (ESI): 342.1 (M+H)⁺. ¹H NMR (400 MHz, DMSO-*d*₆) δ ppm 7.83 (dd, J=8.2, 1.7 Hz, 1 H), 7.74 (br s, 1 H), 7.41 (br dd, *J*=16.6, 7.8 Hz, 1 H), 5.28 (br s, 1 H), 4.69 - 4.90 (m, 2 H), 3.89 - 4.08 (m, 2 H), 3.21 (s, 3 H), 2.57 - 2.73 (m, 3 H), 1.29 - 1.53 (m, 9 H).

Step 2. To a 50-mL round-bottomed flask was added tert-butyl methyl(7-(methylsulfonyl)isochroman-4-yl)carbamate (0.200 g, 0.586 mmol) and trifluoroacetic acid (0.668 g, 0.4 mL, 5.86 mmol, Sigma-Aldrich Corporation) in dichloromethane (6 mL). The resulting reaction mixture was stirred at rt for 4 h. The reaction mixture was concentrated in vacuo. The crude N-methyl-7-(methylsulfonyl)isochroman-4-amine **(95)** was used in the next step of the synthesis, without further purification. *m*/*z* (ESI): 242.0 (M+H)⁺.

### Intermediate 96: methyl 6-amino-2-chloro-5-(4,4,5,5-tetramethyl-1,3,2-dioxaborolan-2-yl)nicotinate

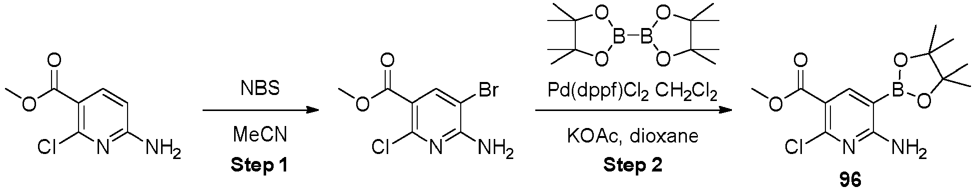

Step 1. Methyl 6-amino-2-chloronicotinate (50.0 mg, 0.268 mmol, Aurum Pharmatech) was dissolved in acetonitrile (1340 µL) and N-bromosuccinimide (52.5 mg, 0.295 mmol, Sigma-Aldrich Corporation) was added. The reaction was stirred for two hours to completion. The solution was concentrated and then water was added (15 mL). The solid was filtered and washed with water then air dried to give methyl 6-amino-5-bromo-2-chloronicotinate (48.5 mg, 0.183 mmol, 68.2% yield). *m*/*z* (ESI): 265.0, 267.0 (M+H)⁺. ¹H NMR (400 MHz, DMSO-*d*6) δ ppm 8.16 (s, 1 H), 7.14 - 7.88 (m, 2 H), 3.78 (s, 3 H).

Step 2. Methyl 6-amino-5-bromo-2-chloronicotinate (6.70 g, 25.2 mmol), bis(pinacolato)diboron (7.69 g, 30.3 mmol, Sigma-Aldrich Corporation), 1,1'-bis(diphenylphosphino)ferrocene-palladium dichloride (2.061 g, 2.52 mmol, Strem Chemicals), and potassium acetate (9.91 g, 101 mmol, Sigma-Aldrich Corporation) were added to a flask with dioxane (degassed) (84 mL). This mixture was heated at 80°C for 7.5 hours.. The reaction mixture was cooled, filtered, and washed with ethyl acetate over a pad of diatamaceous earth. The filtrate was then concentrated and then purified by medium pressure chromatography (silica, 0 to 50% EtOAc : hexanes) to give methyl 6-amino-2-chloro-5-(4,4,5,5-tetramethyl-1,3,2-dioxaborolan-2-yl)nicotinate (96) (4.50 g, 14.40 mmol, 57.1 % yield). *m*/*z* (ESI): 313.0 (M+H)⁺. ¹H NMR (400 MHz, DMSO-*d*₆) δ ppm 8.14 - 8.25 (m, 1 H), 3.78 (s, 3 H), 1.32 (s, 12 H).

### Intermediate 97: 4-Amino-1,3-dihydrofuro[3,4-c]quinoline-8-carboxylic acid.

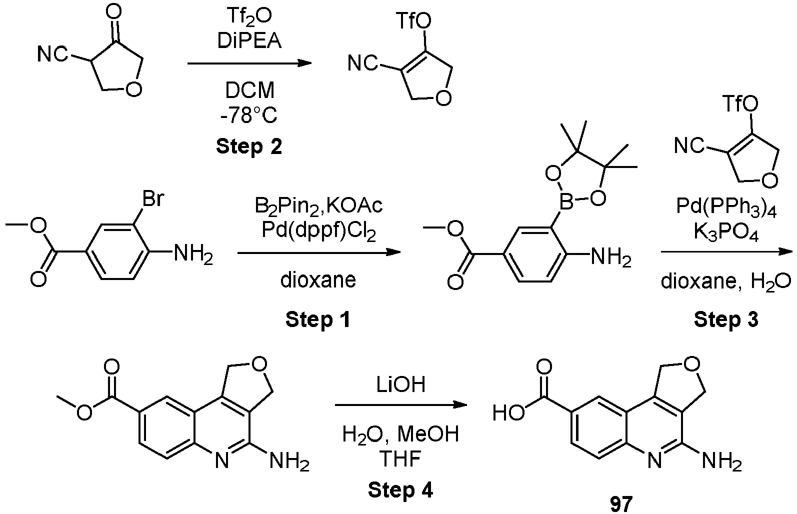

Step 1. To a 150-mL round-bottomed flask was added methyl 4-amino-3-bromobenzoate (4 g, 17.39 mmol, Combi-Blocks) and bis(pinacolato)diboron (8.83 g, 34.8 mmol, Frontier Scientific) in 1,4-dioxane (58.0 mL). To the solution was added potassium acetate (5.12 g, 52.2 mmol, Sigma-Aldrich Corporation) and the mixture was degassed by bubbling through with Argon for 5 minutes. Then, [1,1'-bis(diphenylphosphino)ferrocene]dichloropalladium (ii), complex with dichloromethane (1.420 g, 1.739 mmol, Strem Chemicals) was added. The reaction was then left stirring at 100 °C. After 18 h the reaction was cooled down and the solid filtered under vacuum and the washed with DCM. The mother liquor was concentrated to give a semisolid residue. DCM was added, and the solid formed collected by vacuum filtration. The mother liquor concentrated again, and this step was repeated. The desired methyl 4-amino-3-(4,4,5,5-tetramethyl-1,3,2-dioxaborolan-2-yl)benzoate (2.6 g, 9.38 mmol, 54.0% yield) was isolated as a grey solid. *m*/*z* (ESI): 196.1 (M+H)⁺ (boronic acid). ¹H NMR (400 MHz, CHLOROFORM-d) δ ppm 8.33 (d, J=2.1 Hz, 1 H), 7.90 (dd, J=8.6, 2.2 Hz, 1 H), 6.57 (d, J=8.5 Hz, 1 H), 5.20 (br s, 2 H), 3.87 (s, 3 H), 1.37 (s, 12 H).

Step 2. To a stirred solution of 4-oxotetrahydrofuran-3-carbonitrile (0.500 g, 4.50 mmol) in dichloromethane (5.00 mL) was added DIPEA (0.943 mL, 5.40 mmol) and the reaction mixture was cooled to -78 °C. Then, triflic anhydride (0.760 mL, 4.50 mmol) was added dropwise at -78°C for 1 min and the reaction mixture stirred at same temperature for 15 min. After completion of reaction, the reaction mixture was diluted with water, the organic layer was separated, washed with brine (2 x 10 mL), dried over sodium sulfate, and concentrated to give crude 4-cyano-2,5-dihydrofuran-3-yl trifluoromethanesulfonate (1.05 g, 4.32 mmol, 96% yield), which was used in the next step without further purification.

Step 3. To a stirred solution of 4-cyano-2,5-dihydrofuran-3-yl trifluoromethanesulfonate (10 g, 41.1 mmol) in 1,4-dioxane (200 mL) and water (20.00 mL) was added methyl 4-amino-3-(4,4,5,5-tetramethyl-1,3,2-dioxaborolan-2-yl)benzoate (9.12 g, 32.9 mmol), K₂CO₃ (17.05 g, 123 mmol), and Pd(PPh₃)₄ (4.75 g, 4.11 mmol) under nitrogen purging. Then, the reaction mixture heated at 80°C for 16 h. The reaction mixture was concentrated, then diluted with ethyl acetate (50 mL) and water (50 mL) stirred at room temperature for 30 min. Then, the solid formed was filtered and washed with ethyl acetate (50 mL) and 2% MeOH in DCM (50 mL), then dried under vacuum to give methyl 4-amino-1,3-dihydrofuro[3,4-c]quinoline-8-carboxylate (6.6 g, 27.0 mmol, 65.7% yield) as gray solid. *m*/*z* (ESI): 245.3 (M+H)⁺. ¹H NMR (400 MHz, TFA-d) δ ppm 8.59 - 8.67 (2H, m), 7.97 (1H, d, J=9.3 Hz), 5.94 (2H, t, J=3.5 Hz), 5.65 (2H, t, J=3.4 Hz), 4.24 (3H, s). Note: for some heterocycles in Table 7 Pd(dppf)Cl₂ was used in place of Pd(PPh₃)₄.

Step 4. To a stirred solution of methyl 4-amino-1,3-dihydrofuro[3,4-c]quinoline-8-carboxylate (30 g, 123 mmol) in water (300 mL):tetrahydrofuran (300 mL):methanol (300 mL) was added LiOH (11.77 g, 491 mmol) and the reaction mixture heated at 75°C for 3 h. The reaction mixture was concentrated and then the aqueous layer acidified with 1.5 N HCl up to pH 6.0. The solid obtained was filtered, washed with methanol (300 mL), and dried to give 4-amino-1,3-dihydrofuro[3,4-c]quinoline-8-carboxylic acid (97) (28 g, 122 mmol, 99% yield) as off-white solid. *m*/*z* (ESI): 231.2 (M+H)⁺. ¹H NMR (400 MHz, DMSO-d) δ ppm 12.83 (1H, s), 7.88 - 8.30 (2H, m), 7.59 (1H, d, J=8.8 Hz), 7.02 (2H, s), 5.40 (2H, t, J=3.5 Hz), 5.03 (2H, t, J=3.6 Hz).

Acids in Table 7 were prepared in a manner similar to that described for Intermediate **97.**

**Table 7**

| **Int. #** | **Chemical Structure** | **Name** | ***m*/*z* (ESI): (M+H)⁺** |
|---|---|---|---|
| **98** | 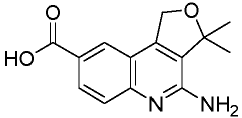 | 4-amino-3,3-dimethyl-1,3-dihydrofuro[3,4-c]quinoline-8-carboxylic acid | 259.1 |
| **99** | 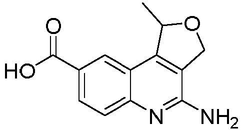 | 4-amino-1-methyl-1,3-dihydrofuro[3,4-c]quinoline-8-carboxylic acid | 245.1 |
| **100** | 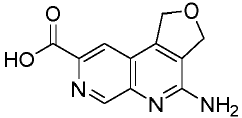 | 4-amino-1,3-dihydrofuro[3,4-c][1,7]naphthyridine-8-carboxylic acid | 232.0 |
| **101** | 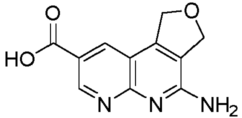 | 4-amino-1,3 -dihydrofuro [3, 4-c] [1, 8]naphthyridine-8-carboxylic acid | 232.1 |
| **102** | 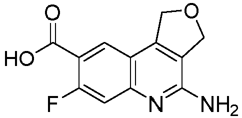 | 4-amino-7-fluoro-1,3-dihydrofuro[3,4-c]quinoline-8-carboxylic acid | 249.0 |
| **103** | 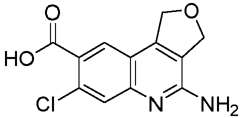 | 4-amino-7-chloro-1,3-dihydrofuro[3,4-c]quinoline-8-carboxylic acid | 264.9 |
| **104** | 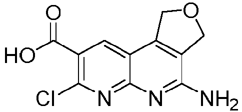 | 4-amino-7-chloro-1,3-dihydrofuro[3,4-c][1,8]naphthyridine-8-carboxylic acid | 280.2 |
| **105** | 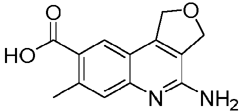 | 4-amino-7-methyl-1,3-dihydrofuro[3,4-c]quinoline-8-carboxylic acid | 245.2 |
| **106** | 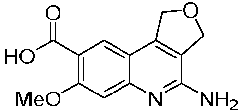 | 4-amino-7-methoxy-1,3-dihydrofuro[3,4-c]quinoline-8-carboxylic acid | 261.0 |
| **107** | 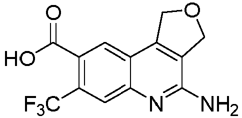 | 4-amino-7-(trifluoromethyl)-1,3-dihydrofuro[3,4-c]quinoline-8-carboxylic acid | 298.9 |
| **108** | 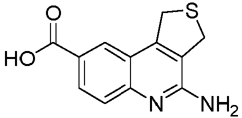 | 4-amino-1,3-dihydrothieno[3,4-c]quinoline-8-carboxylic acid | 247.1 |
| **109** | 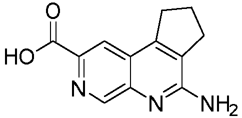 | 6-amino-8,9-dihydro-7H-cyclopenta[c][1,7]naphthyridine-2-carboxylic acid | 230.0 |
| **110** | 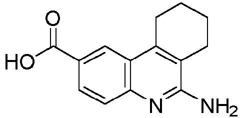 | 6-amino-7,8,9,10-tetrahydrophenanthridine-2-carboxylic acid | 243.2 |
| **111** | 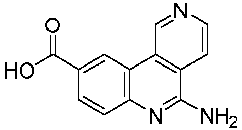 | 5-aminobenzo[c] [2,6]naphthyridine-9-carboxylic acid | 240.1 |
| **112** | 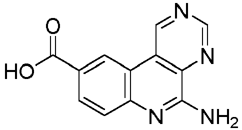 | 5-aminopyrimido[4,5-c]quinoline-9-carboxylic acid | 241.2 |
| **113** | 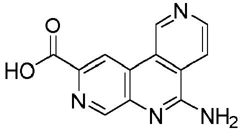 | 5-aminopyrido[4,3-c][1,7]naphthyridine-9-carboxylic acid | 241.1 |
| **114** | 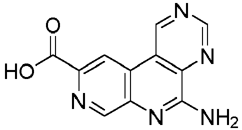 | 5-aminopyrimido[4,5-c][1,7]naphthyridine-9-carboxylic acid | 241.1 |
| **115** | 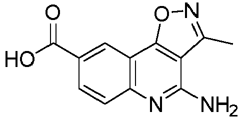 | 4-amino-3-methylisoxazolo[4,5-c]quinoline-8-carboxylic acid | 244.0 |
| **116** | 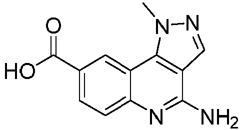 | 4-amino-1-methyl-1H-pyrazolo[4,3-c]quinoline-8-carboxylic acid | 243.0 |
| **117** | 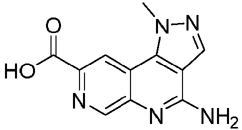 | 4-amino-1-methyl-1H-pyrazolo[4,3-c][1,7]naphthyridine-8-carboxylic acid | 244.0 |
| **118** | 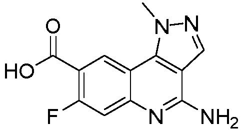 | 4-amino-7-fluoro-1-methyl-1H-pyrazolo[4,3-c]quinoline-8-carboxylic acid | 261.0 |
| **119** | 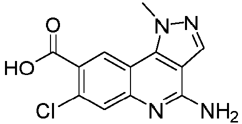 | 4-amino-7-chloro-1-methyl-1H-pyrazolo[4,3-c]quinoline-8-carboxylic acid | 277.0 |
| **120** | 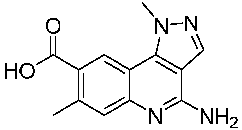 | 4-amino-1,7-dimethyl-1H-pyrazolo[4,3-c]quinoline-8-carboxylic acid | 257.0 |
| **121** | 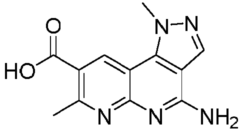 | 4-amino-1,7-dimethyl-1H-pyrazolo[4,3-c][1,8]naphthyridine-8-carboxylic acid | 258.0 |
| **122** | 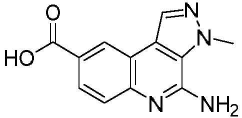 | 4-amino-3-methyl-3H-pyrazolo[3,4-c]quinoline-8-carboxylic acid | 243.1 |
| **123** | 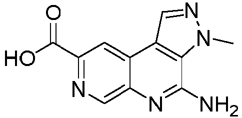 | 4-amino-3-methyl-3H-pyrazolo[3,4-c][1,7]naphthyridine-8-carboxylic acid | 244.1 |
| **124** | 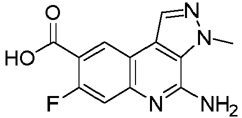 | 4-amino-7-fluoro-3-methyl-3H-pyrazolo[3,4-c]quinoline-8-carboxylic acid | 261.1 |
| **125** | 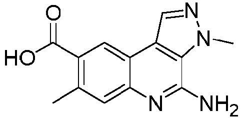 | 4-amino-3,7-dimethyl-3H-pyrazolo[3,4-c]quinoline-8-carboxylic acid | 257.1 |
| **126** | 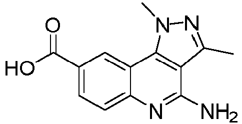 | 4-amino-1,3-dimethyl-1H-pyrazolo[4,3-c]quinoline-8-carboxylic acid | 257.0 |
| **127** | 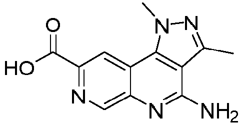 | 4-amino-1,3-dimethyl-1H-pyrazolo[4,3-c][1,7]naphthyridine-8-carboxylic acid | 258.2 |
| **128** | 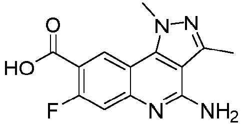 | 4-amino-7-fluoro-1,3-dimethyl-1H-pyrazolo[4,3-c]quinoline-8-carboxylic acid | 275.2 |

### Intermediate 129: 4-amino-7-cyano-1,3-dihydrofuro[3,4-c]quinoline-8-carboxylic acid hydrochloride

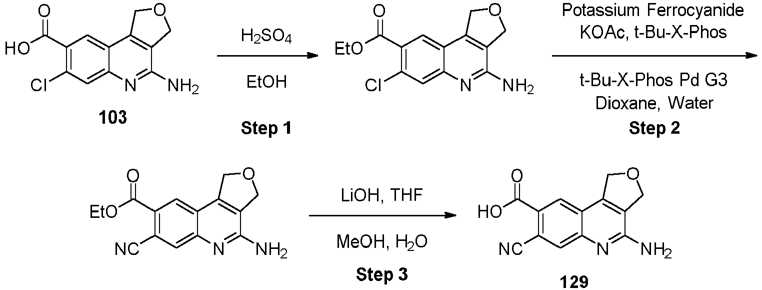

Step 1. 4-amino-7-chloro-1,3-dihydrofuro[3,4-c]quinoline-8-carboxylic acid (103) (500 mg, 1.89 mmol, 1.0 equiv) was slurried in EtOH (9.45 mL) and sulfuric acid (445 mg, 4.53 mmol, 2.4 equiv, Sigma-Aldrich Corporation) was added. The reaction mixture was stirred at reflux for 2.5 days then it was cooled, filtered and washed with EtOH to give the sulfonate salt of the desired product. The salt was slurried in 2 M ammonia in MeOH and then heated and concentrated. The residue was purified by medium pressure chromatography (silica, 0 to 100% (3:1 EtOAc:EtOH):Heptanes) to give ethyl 4-amino-7-chloro-1,3-dihydrofuro[3,4-c]quinoline-8-carboxylate (235 mg, 0.803 mmol, 42.5% yield). *m*/*z* (ESI): 293.1 (M+H)⁺.

Step 2. To a reaction vial was added methanesulfonato(2-di-t-butylphosphino-2',4',6'-tri-i-propyl-1,1'-biphenyl)(2'-amino-1,1'-biphenyl-2-yl)palladium (II) (239 mg, 0.0301 mmol, 0.4 eq, Strem Chemicals), di-tert-butyl(2',4',6'-triisopropyl-[1,1'-biphenyl]-2-yl)phosphane (128 mg, 0.0301 mmol, 0.4 eq, Strem Chemicals), K₄[Fe(CN)₆].3H₂O (159 mg, 0.376 mmol, 0.5 eq, Oakwood), and ethyl 4-amino-7-chloro-1,3-dihydrofuro[3,4-c]quinoline-8-carboxylate (220 mg, 0.752 mmol, 1 eq). The vessel was evacuated and backfilled with nitrogen (3x). Dioxane (1.25 mL), and 0.05 M KOAc (1.88 mL, 0.094 mmol, 0.125 eq, Sigma-Aldrich Corporation) in water (1.25 mL) were added to the reaction via syringe and the reaction vial was stirred at 100°C for 2.5 hours. The reaction was cooled and then extracted between EtOAc (2 x 50 mL) and brine (30 mL). The combined organic layers were dried over magnesium sulfate and the residue was purified by medium pressure chromatography (2x) (silica, 0 to 100% (3:1 EtOAc:EtOH):Heptanes) to give ethyl 4-amino-7-cyano-1,3-dihydrofuro[3,4-c]quinoline-8-carboxylate (12.0 mg, 0.042 mmol, 5.64 % yield). *m*/*z* (ESI): 284.1 (M+H)⁺.

Step 3. Lithium hydroxide, monohydrate (3.56 mg, 0.085 mmol, 2.0 equiv, Sigma-Aldrich Corporation) was added to a suspension of ethyl 4-amino-7-cyano-1,3-dihydrofuro[3,4-c]quinoline-8-carboxylate (12.0 mg, 0.042 mmol, 1.0 equiv) in MeOH (0.15 mL), THF (0.15 mL) and water (0.15 mL). The mixture was heated to 60 °C for 18 hrs then cooled to rt. Another 4.0 equiv of LiOH was added and the reaction was stirred for another 2.5 hours. The organic solvent was removed *in vacuo* and the resulting aqueous solution was taken to pH 3 with 2N HCl solution. The resulting suspension was filtered, washed with water and air dried to give 4-amino-7-cyano-1,3-dihydrofuro[3,4-c]quinoline-8-carboxylic acid hydrochloride **(129)** (10.0 mg, 0.034 mmol, 81 % yield). *m*/*z* (ESI): 256.2 (M+H)⁺.

### Intermediate 130: 6-((2,4-dimethoxybenzyl)amino)-8,9-dihydro-7H-cyclopenta[c][1,8]naphthyridine-2-carboxylic acid.

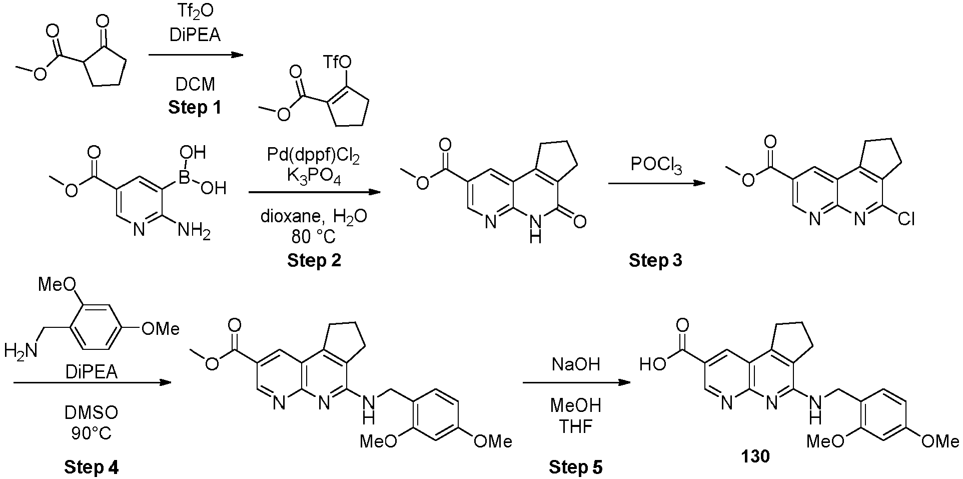

Step 1. A mixture of methyl 2-oxocyclopentanecarboxylate (1.0 g, 0.877 mL, 7.03 mmol, Matrix Scientific) and 1,1'-dimethyltriethylamine (1.000 g, 1.352 mL, 7.74 mmol, Sigma-Aldrich Corporation) in DCM (15 mL) was cooled to -78 °C and trifluoromethanesulfonic acid anhydride (7.03 mL, 7.03 mmol, Sigma-Aldrich Corporation) was added. After complete addition, the mixture was stirred at -78 °C for 5 min, then the dry ice-bath was removed and the reaction mixture was stirred at rt. After 15 min, the mixture was concentrated to afford methyl 2-(((trifluoromethyl)sulfonyl)oxy)cyclopent-1-ene-1-carboxylate with quant. yield as a light-yellow solid to be used as is. *m*/*z* (ESI): 275 (M+H)⁺.

Step 2. A mixture of methyl 2-(((trifluoromethyl)sulfonyl)oxy)cyclopent-1-ene-1-carboxylate (1.982 g, 7.23 mmol), (2-amino-5-(methoxycarbonyl)pyridin-3-yl)boronic acid (1.70 g, 8.67 mmol), potassium phosphate, tribasic (3.78 g, 21.69 mmol, Acros) and [1,1'-bis(diphenylphosphino)ferrocene]dichloropalladium (II), complex with dichloromethane (0.177 g, 0.217 mmol, Strem Chemicals) in 1,4-dioxane/water (10/0.60 mL) was heated at 80 °C for 1 h. The reaction went to completion, and was brought to rt and diluted with EtOAc. A precipitate was formed which corresponded to the desired product. It was filtered and washed with EtOAc to yield methyl 6-oxo-6,7,8,9-tetrahydro-5H-cyclopenta[c][1,8]naphthyridine-2-carboxylate as a light gray solid with quant. yield. *m*/*z* (ESI): 245 (M+H)⁺. ¹H NMR (400 MHz, DMSO-d6) δ ppm 11.93 - 12.58 (m, 1 H), 8.96 (d, J=2.1 Hz, 1 H), 8.33 (d, J=2.1 Hz, 1 H), 3.89 (s, 3 H), 3.13 (br t, J=7.6 Hz, 2 H), 2.78 (br t, J=7.3 Hz, 2 H), 2.08 - 2.18 (m, 2 H).

Step 3. A mixture of methyl 6-oxo-6,7,8,9-tetrahydro-5H-cyclopenta[c][1,8]naphthyridine-2-carboxylate (1.76 g, 7.21 mmol) in POCl₃ (24.68 g, 15 mL, 161 mmol, Sigma-Aldrich Corporation) was heated to reflux for 30 min. The reaction went to completion and was carefully added to cold-sat. aqueous NaHCO₃ to basify the reaction. After stirring for 15 min, the mixture was extracted with EtOAc and the combined organics were concentrated to afford methyl 6-chloro-8,9-dihydro-7H-cyclopenta[c][1,8]naphthyridine-2-carboxylate as a yellow solid with quant. yield. *m*/*z* (ESI): 263 (M+H)⁺.

Step 4. To a suspension of methyl 6-chloro-8,9-dihydro-7H-cyclopenta[c][1,8]naphthyridine-2-carboxylate (1.89 g, 7.19 mmol) in DMSO (15 mL) was added DIPEA (2.79 g, 3.77 mL, 21.58 mmol, Sigma-Aldrich Corporation) followed by the addition of (2,4-dimethoxyphenyl)methanamine (1.564 g, 1.405 mL, 9.35 mmol, Sigma-Aldrich Corporation). The resulting mixture was heated at 90°C overnight. The reaction was cooled to rt, diluted with water, washed with sat. NH₄Cl and extracted with EtOAc. The combined organics were dried over Na₂SO₄, filtered and concentrated to afford methyl 6-((2,4-dimethoxybenzyl)amino)-8,9-dihydro-7H-cyclopenta[c][1,8]naphthyridine-2-carboxylate (2.18 g, 5.54 mmol, 77% yield) as a yellow solid to be used as is. *m*/*z* (ESI): 394 (M+H)⁺.

Step 5. To a solution of methyl 6-((2,4-dimethoxybenzyl)amino)-8,9-dihydro-7H-cyclopenta[c][1,8]naphthyridine-2-carboxylate (2.18 g, 5.54 mmol) in THF/MeOH (10/10 mL) was added 1 N NaOH (10 mL, 10.00 mmol) and the resulting solution was heated at 70 °C for 2 h. The reaction was brought to rt and acidified with 10 mL 1M HCl. A light yellow precipitate was formed, it was filtered and azeotropically dried with toluene to afford 6-((2,4-dimethoxybenzyl)amino)-8,9-dihydro-7H-cyclopenta[c][1,8]naphthyridine-2-carboxylic acid hydrochloride **(130)** (1.44 g, 3.46 mmol, 62.5% yield) as a yellow solid. *m*/*z* (ESI): 380.2 (M+H)⁺.

Intermediates in Table **8** were prepared in a manner similar to that described for Intermediate **130.** Intermeidate **132** followed the same procedure up to step 4.

**Table 8**

| **Int. #** | **Chemical Structure** | **Name** | ***m*/*z* (ESI): (M+H)⁺** |
|---|---|---|---|
| **131** | 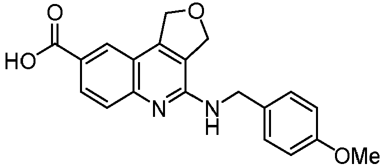 | 4-((4-methoxybenzyl)amino)-1,3-dihydrofuro[3,4-c]quinoline-8-carboxylic acid | 351.0 |
| **132** | 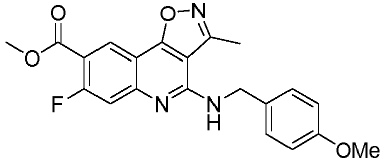 | methyl 7-fluoro-4-((4-methoxybenzyl)amino)-3-methylisoxazolo[4,5-c]quinoline-8-carboxylate | 396.1 |

### Intermediate 133: 4-amino-7-fluoro-3-methylisoxazolo[4,5-c]quinoline-8-carboxylic acid

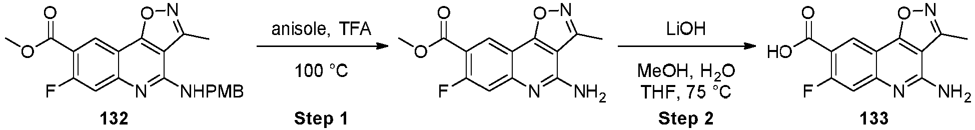

Step 1. To a mixture of methyl 7-fluoro-4-((4-methoxybenzyl)amino)-3-methylisoxazolo[4,5-c]quinoline-8-carboxylate **(132)** (17.6 g, 44.6 mmol) in TFA (210 mL) was added drop-wise anisole (209 g, 1.93 mol, 210 mL) at 100 °C. The mixture was stirred at 100 °C for 12 hrs, then reaction mixture was concentrated. The crude product was triturated with MTBE (50.0 mL) at 25 °C for 20 min, filtered and the filter cake was dried to give methyl 4-amino-7-fluoro-3-methylisoxazolo[4,5-c]quinoline-8-carboxylate (16.0 g, 43.0 mmol, 96.6% yield, TFA) as a white solid. *m*/*z* (ESI): 276.1 (M+H)⁺. ¹H NMR (400 MHz, DMSO-d6) δ ppm 8.57 (d, *J=* 7.60 Hz, 1 H), 7.92 - 7.88 (m, 1 H), 7.43 (d, *J* = 12.8 Hz, 1 H), 3.90 (s, 3 H), 2.69 (s, 3 **H).**

Step 2. To a solution of methyl 4-amino-7-fluoro-3-methylisoxazolo[4,5-c]quinoline-8-carboxylate (16.0 g, 43.0 mmol, TFA) in THF (96.0 mL), MeOH (48.0 mL) and H₂O (48.0 mL) was added LiOH•H₂O (2.93 g, 69.9 mmol) at 20°C. The mixture was stirred at 75°C for 2 hrs and then additional LiOH•H₂O (362 mg, 8.63 mmol) was added at 20°C and the mixture was stirred at 75 °C for 5 hrs. The reaction mixture was filtered and the filter caked was dried. The crude product was triturated with MeCN (120 mL) at 20°C for 30 mins. The reaction mixture was filtered and the filter cake was dried to give 4-amino-7-fluoro-3-methylisoxazolo[4,5-c]quinoline-8-carboxylic acid **(133)** (5.10 g, 19.5 mmol, 45.4% yield, 99.1% purity) as a white solid. *m*/*z* (ESI): 261.9 (M+H)⁺. ¹H NMR (400 MHz, DMSO-d6) δ ppm 8.26 (d, *J=* 7.60 Hz, 1 H), 7.12 (d, *J* =1 Hz, 1 H), 6.91 (s, 2 H), 2.66 (s, 1 H).

### Intermediate 134: 4-amino-3-methyl-1,3-dihydrofuro[3,4-c]quinoline-8-carboxylic acid

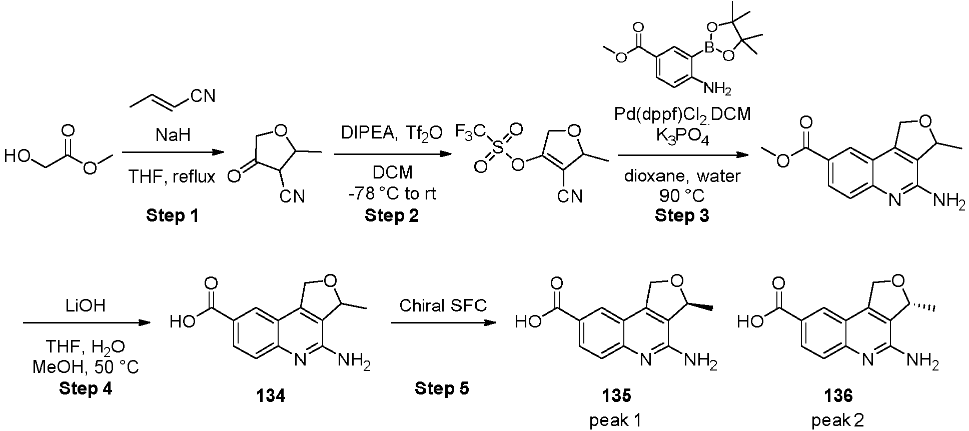

Step 1. To a suspension of sodium hydride (11.10 g, 278 mmol 0.5 equiv., 60% in mineral oil) in anhydrous tetrahydrofuran (250 mL) was added methyl 2-hydroxyacetate (42.4 mL, 555 mmol, 1.0 equiv) at room temperature under N₂ atmosphere. To the reaction mixture (E)-but-2-enenitrile (54.5 mL, 666 mmol, 1.0 equiv) was added slowly at 65 °C and stirred for 2h at same temperature. The reaction mixture was cooled and quenched with 2N NaOH solution (250 mL) and extracted with diethyl ether (500 mL). The aqueous layer was acidified with conc. HCl to adjust the pH to ~1 and extracted with dichloromethane (2 x 500 mL). The combined organic layer was washed with brine (200 mL) and dried over sodium sulfate, filtered and concentrated under reduced pressure. The crude residue was purified by column chromatography over silica gel (230-400 mesh) using 10% ethyl acetate with hexanes as an eluent to give 2-methyl-4-oxotetrahydrofuran-3-carbonitrile (22 g, 176 mmol, 32% yield) as a brown solid. *m*/*z* (ESI, Negative): 124.3 (M-H)⁻. ¹H NMR (400 MHz, Chloroform-d): δ ppm 4.40 - 4.27 (m, 2 H), 4.26 - 4.19 (m, 1 H), 3.24 - 2.99 (m, 1 H), 1.61 (dd, J=18.6, 6.2 Hz, 3 H).

Step 2. To a stirred solution of 2-methyl-4-oxotetrahydrofuran-3-carbonitrile (25.0 g, 200 mmol, 1.0 equiv) in dichloromethane (500 mL) was added DIPEA (69.8 mL, 400 mmol, 2.0 equiv) and triflic anhydride (47.1 mL, 280 mmol, 1.4 equiv) at -78 °C and stirred at same temperature for 15 min. The reaction mixture was quenched with slow addition of water (250 mL) and after attaining the room temperature was extracted with dichloromethane (2 x 500 mL). The combined organic layer was dried over sodium sulfate, filtered and concentrated under reduced pressure. The crude residue was stirred in diethyl ether and filtered. The mother liquor was concentrated under reduced pressure to give 4-cyano-5-methyl-2,5-dihydrofuran-3-yl trifluoromethanesulfonate (35.0 g, crude) as a light brown adduct. The crude material was used for next step without further purification. *m*/*z* (ESI): 257.1 [Not ionized].

Step 3. To a stirred solution of 4-cyano-5-methyl-2,5-dihydrofuran-3-yl trifluoromethanesulfonate (35 g, 136 mmol, 1.0 equiv) in 1,4-dioxane (1400 mL) and water (70.0 mL), was added methyl 4-amino-3-(4,4,5,5-tetramethyl-1,3,2-dioxaborolan-2-yl)benzoate (37.7 g, 136 mmol, 1.0 equiv) and potassium phosphate (87 g, 408 mmol, 3.0 equiv) under nitrogen atmosphere. The reaction mixture was degassed with nitrogen for 15 min and then PdCl₂(dppf)-DCM adduct (9.96 g, 13.61 mmol, 0.1 equiv) was added and the reaction mixture was heated at 90°C for 16 h. The reaction mass was concentrated under reduced pressure to get crude product. The crude residue was purified by column chromatography over silica gel (60-120 mesh) using 50% ethyl acetate with hexanes as an eluent to give methyl 4-amino-3-methyl-1,3-dihydrofuro[3,4-c]quinoline-8-carboxylate (25 g, 97 mmol, 71% yield) as a brown solid. *m*/*z* (ESI): 259.2 (M+H)⁺. ¹H NMR (400 MHz, DMSO-*d*₆): δ 8.11 (d, *J* = 2.0 Hz, 1H), 8.00 (dd, *J=* 8.8, 2.0 Hz, 1H), 7.58 (d, *J* = 8.8 Hz, 1H), 6.87 (s, 2H), 4.11 (q, *J* = 5.3 Hz, 1H), 3.87 (s, 2H), 3.17 (d, *J=* 5.3 Hz, 3H), 1.41 (d, *J* = 5.9 Hz, 3H).

Step 4. To a stirred solution of methyl 4-amino-3-methyl-1,3-dihydrofuro[3,4-c]quinoline-8-carboxylate (26.0 g, 101 mmol, 1.0 equiv) in tetrahydrofuran (130 mL), methanol (78 mL) and water (52 mL), was added lithium hydroxide (9.64 g, 403 mmol, 4.0 equiv) and stirred at 75°C for 4 h. The reaction mixture was concentrated under reduced pressure. The crude residue was dissolved in water (100 mL) and filtered to remove insoluble particles. The aqueous layer was acidified with con. HCl (pH 6 to 6.5). The precipitated solid was filtered, washed with water and dried under vacuum to get 4-amino-3-methyl-1,3-dihydrofuro[3,4-c]quinoline-8-carboxylic acid **(134)** (17.5 g, 71.6 mmol, 71% yield) as an off-white solid. *m*/*z* (ESI): 245.1 (M+H)⁺. ¹H NMR (TFA, 400 MHz): δ (ppm) 8.68 (t, J=6.2 Hz, 2H), 8.01 (dd, J=9.1, 4.2 Hz, 1H), 6.15 (s, 1H), 5.94 (m, 2H), 1.86 (t, J=5.4 Hz, 3H)

Step 5. Chiral SFC separation: 44.5 g of racemic 4-amino-3-methyl-1,3-dihydrofuro[3,4-c]quinoline-8-carboxylic acid was separated by chiral SFC to get 14 g of each isomer. Stereochemistry is assigned arbitrarily. Peak 1 was assigned as (S)-4-amino-3-methyl-1,3-dihydrofuro[3,4-c]quinoline-8-carboxylic acid **(135)** and peak 2 was assigned as (R)-4-amino-3-methyl-1,3-dihydrofuro[3,4-c]quinoline-8-carboxylic acid **(136).**

### Separation Information:

| | Key | Value |
|---|---|---|
| 1 | Instrument | SFC 200 |
| 2 | Column | ChiralPak- IC(250x30mm, 5µ) |
| 3 | Mobile Phase | Liquid CO₂: 0.5% DEA in Methanol(40:60) |
| 4 | Flow rate | 100mL/ min |
| 5 | Pressure Drop | 130bar |
| 6 | BPR | 100 bar |
| 7 | UV Detector Wavelength | 210 nm |
| 8 | Dissolution | 14.0 g dissolved in 280mL of 2% of DEA in Methanol |

Acids in Table 9 were prepared in a manner similar to that described for Intermediate **134.**

**Table 9**

| **Acids** | **SFC Conditions** | ***m*/*z* (M+H)⁺** |
|---|---|---|
| 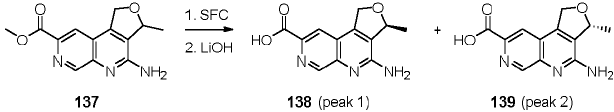 | Chiralpak IG-3 column (50 x 4.6mm I.D., 3 µm) with a mobile phase of Liquid CO₂: MeOH (0.05% isopropylamine, v/v); 95:5 →1:1; 3 min gradient | 246.0 |
| 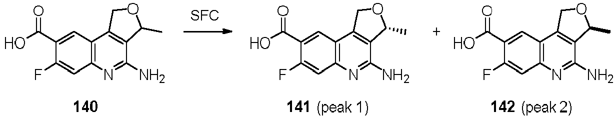 | Chiralpak IG column (250 mm x 50 mm,10 µm) with a mobile phase of 75% Liquid CO₂ and 25% MeOH with 0.3% NH₄OH using a flow rate of 200 mL/min; 100 bar | 263.1 |

### Intermediate 143: 4-amino-1-methyl-7-(trifluoromethyl)-1H-pyrazolo[4,3-c]quinoline-8-carboxylic acid

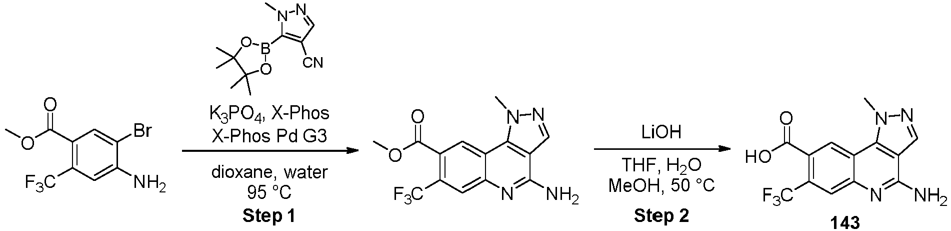

Step 1. K₃PO₄·H₂O (1.08 g, 4.70 mmol, Sigma-Aldrich Corporation), X-Phos (0.08 g, 0.16 mmol, Sigma-Aldrich Corporation), (2-dicyclohexylphosphino-2',4',6'-triisopropyl-1,1'-biphenyl)[2-(2'-amino-1,1'-biphenyl)]palladium (II) methanesulfonate (0.14 mg, 0.16 mmol, Sigma-Aldrich Corporation), 1-methyl-5-(4,4,5,5-tetramethyl-1,3,2-dioxaborolan-2-yl)-1h-pyrazole-4-carbonitrile (1.10 g, 4.70 mmol, Enamine) and methyl 4-amino-5-bromo-2-(trifluoromethyl)benzoate (0.700 g, 2.349 mmol, Combi Blocks) were suspended in a degassed mixture of water (1.0 mL) and 1,4-dioxane (5.0 mL) and stirred at 60 °C overnight and then at 90 °C for 18 h. Volatiles were removed *in vacuo* and the crude product was purified via silica column chromatography (0 to 5% MeOH/DCM + 0.5% NH₃/MeOH) to yield methyl 4-amino-1-methyl-7-(trifluoromethyl)-1H-pyrazolo[4,3-c]quinoline-8-carboxylate (0.63 g, 1.94 mmol, 83% yield) as a slight brownish solid. *m*/*z* (ESI): 324.8 (M+H)⁺. ¹H NMR (400 MHz, DMSO-d6) δ ppm 8.74 (s, 1 H), 8.35 (s, 1 H), 7.89 (s, 1 H), 7.58 (bs, 2 H), 4.45 (s, 3 H), 3.91 (s, 3 H). ¹⁹F NMR (376 MHz, DMSO-d6) δ ppm -58.06.

Step 2. Methyl 4-amino-1-methyl-7-(trifluoromethyl)-1H-pyrazolo[4,3-c]quinoline-8-carboxylate (0.62 g, 1.90 mmol) and lithium hydroxide (0.91 g, 3.79 mmol, Sigma-Aldrich Corporation) were suspended in methanol (3.0 mL), H₂O (3.0 mL) and THF (3.0 mL) and stirred at 50°C for 2 hours. Volatiles of the crude mixture were removed in vacuo and the light brown solid was co-evaporated with DCM twice, followed by co-evaporation with toluene to give lithium 4-amino-1-methyl-7-(trifluoromethyl)-1H-pyrazolo[4,3-c]quinoline-8-carboxylate hydroxide (143) (585 mg, 1.720 mmol, 91% yield) that was used in subsequent steps without further purification. *m*/*z* (ESI): 310.9 (M+H)⁺. ¹H NMR (400 MHz, DMSO-d6) δ ppm 8.33 (s, 1 H), 8.26 (s, 1 H), 7.68 (s, 1 H), 7.03 (br s, 2 H), 4.38 (s, 3 H). ¹⁹F NMR (376 MHz, DMSO-d6) δ ppm -57.47.

### Intermediate 144: 4-amino-6-fluoro-1-methyl-1H-pyrazolo[4,3-c]quinoline-8-carboxylic acid

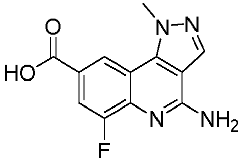

Intermediate **144** was prepared in a similar fashion to Intermediate 143 above, *m*/*z* (ESI): 261.0 (M+H)⁺.

### Intermediate 145: lithium 4-amino-1H-pyrazolo[4,3-c]quinoline-8-carboxylate hydroxide

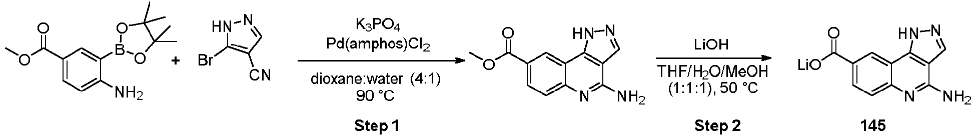

Step 1. Methyl 4-amino-3-(4,4,5-tetramethyl-1,3,2-dioxaborolan-2-yl)benzoate (886 mg, 3.2 mmol), 5-bromo-1h-pyrazole-4-carbonitrile (500 mg, 2.9 mmol), K₃PO₄ hydrate (2.68 g, 11.6 mmol) and Pd(amphos)Cl₂ (144 mg, 0.20 mmol) were suspended in degassed water (2 mL) and 1,4-dioxane (8.00 mL) and stirred at 90 °C overnight, at which time orange-beige solid formed. Water (20 mL) was added after the mixture was cooled to rt and the precipitate was filtered to yield methyl 4-amino-1H-pyrazolo[4,3-c]quinoline-8-carboxylate (230 mg, 0.949 mmol, 32.7% yield). *m*/*z* (ESI): 243.0 (M+H)⁺.

Step 2. Methyl 4-amino-1H-pyrazolo[4,3-c]quinoline-8-carboxylate (230 mg, 0.95 mmol) and lithium hydroxide hydrate (80 mg, 1.90 mmol, Sigma-Aldrich Corporation) were suspended in water (0.6 mL), methanol (0.6 mL) and tetrahydrofuran (0.6 mL) and stirred at 50°C for 90 minutes. Volatiles were removed in vacuo to yield lithium 4-amino-1H-pyrazolo[4,3-c]quinoline-8-carboxylate hydroxide **(145)** (240 mg, 0.930 mmol, 98% yield). *m*/*z* (ESI): 229.0 (M+H)⁺.

The following amines in Table 10 were prepared in a manner similar to that described for Intermediate **145.**

**Table 10**

| **Int. #** | **Chemical Structure** | **Name** | *m*/*z* (ESI: (M+H)⁺ |
|---|---|---|---|
| **146** | 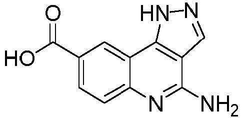 | 4-amino-6-fluoro-1-methyl-1H-pyrazolo[4,3-c]quinoline-8-carboxylic acid | 261.0 |
| **147** | 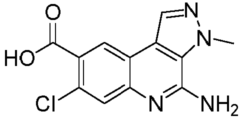 | 4-amino-7-chloro-3-methyl-3H-pyrazolo[3,4-c]quinoline-8-carboxylic acid | 277.0 |
| **148** | 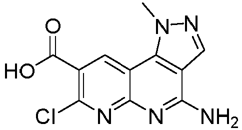 | 4-amino-7-chloro-1-methyl-1H-pyrazolo[4,3-c][1,8]naphthyridine-8-carboxylic acid | 278.1 |
| **149** | 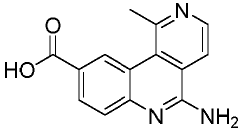 | 5-amino-1-methylbenzo[c][2,6]naphthyridine-9-carboxylic acid | 254.1 |

### Intermediate 150: 4-amino-3-methyl-1H-pyrazolo[4,3-c]quinoline-8-carboxylic acid

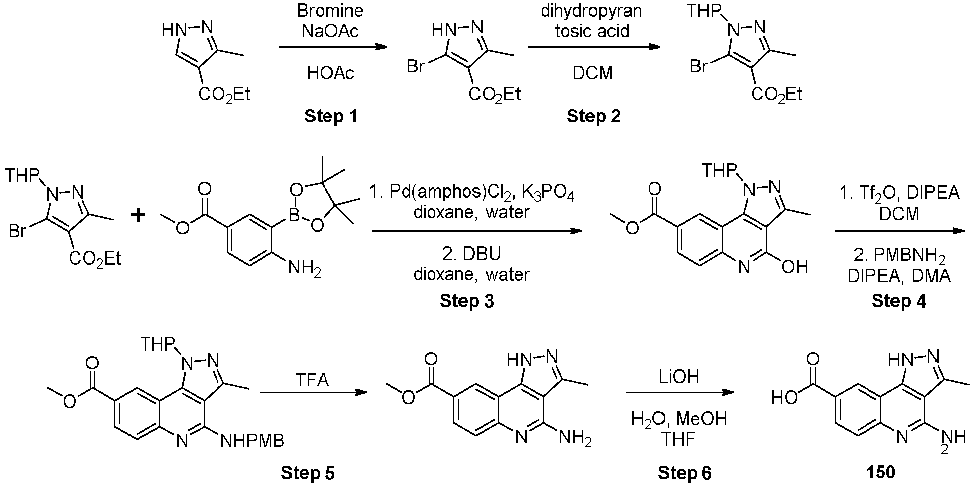

Step 1. To a solution of ethyl 5-methyl-1H-pyrazole-4-carboxylate (5.00 g, 32.4 mmol, 1.0 equiv, Combi-Blocks) in acetic acid (100 mL) was added bromine (5.01 mL, 97.0 mmol, 3.0 equiv) and sodium acetate (10.6 g, 130 mmol, 4.0 equiv.) at rt. Then the reaction mixture was stirred and heated for 16 h. The reaction was slowly quenched with sodium bicarbonate and extracted with ethyl acetate. The organic layer was dried over sodium sulphate and concentrated under reduced presure to get pure crude ethyl 3-bromo-5-methyl-1H-pyrazole-4-carboxylate (4.80 g, 20.6 mmol, 63.5% yield). *m*/*z:* 230.8, 232.9 (M+H)⁺.

Step 2. To a stirred solution of ethyl 3-bromo-5-methyl-1H-pyrazole-4-carboxylate (4.80 g, 20.6 mmol, 1.0 equiv) in dichloromethane (15 mL) was added dihydropyran (2.26 mL, 24.7 mmol, 1.2 equiv) and tosic acid (0.78 g, 4.12 mmol, 0.2 equiv) at 0 °C. The resulting reaction mixture was stirred for 16 h to completion. The reaction was quenched with water (20 mL), and extracted with ethyl acetate (20 mL x 3). The combined organic layer was washed with brine solution, dried over anhydrous sodium sulphate and concentrated to get crude material. The crude material was purified by chromatography (silica, 40% ethyl acetate in hexane) to obtain ethyl 3-bromo-5-methyl-1-(tetrahydro-2H-pyran-2-yl)-1H-pyrazole-4-carboxylate (4.80 g, 15.1 mmol, 73.5% yield) as colorless sticky liquid. *m*/*z:* 314.9, 317.0 (M+H)⁺.

Step 3. To a stirred solution of methyl 4-amino-3-(4,4,5,5-tetramethyl-1,3,2-dioxaborolan-2-yl)benzoate (7.34 g, 26.5 mmol, 1.2 equiv) in 1,4-dioxane (112 mL) and water (28.0 mL) was added ethyl 3-bromo-5-methyl-1-(tetrahydro-2H-pyran-2-yl)-1H-pyrazole-4-carboxylate (7.00 g, 22.1 mmol, 1.0 equiv), potassium phosphate, tribasic (9.36 g, 44.1 mmol, 2.0 equiv) under nitrogen purging for 10 min at room temperature. Then Pd(amphos)Cl₂ adduct (0.781 g, 1.10 mmol, 0.05 equiv) was added and the reaction mixture was heated at 90 °C for 16 h. The reaction mixture was quenched with water and extracted with ethyl acetate. The organic layer was dried over by sodium sulphate and concentrated under reduced pressure to get 7.00 grams of the crude ethyl 5-(2-amino-5-(methoxycarbonyl)phenyl)-3-methyl-1-(tetrahydro-2H-pyran-2-yl)-1H-pyrazole-4-carboxylate.

To a stirred solution of ethyl 5-(2-amino-5-(methoxycarbonyl)phenyl)-3-methyl-1-(tetrahydro-2H-pyran-2-yl)-1H-pyrazole-4-carboxylate (600 mg, 1.55 mmol, 1.0 equiv) in 1,4-dioxane (9.60 mL) and water (2.40 mL) was added DBU (2.00 mL, 13.3 mmol, 12 equiv) under nitogen at room temperature and the reaction mixture heated to 90°C for 16 h. The reaction mixture was quenched with water and extracted with ethyl acetate. The organic layer was dried over by sodium sulphate and concentrated under reduced pressure to get crude material which was purified by colunm chromatography (silica, 5% MeOH in DCM ) to get pure methyl 4-hydroxy-3-methyl-1-(tetrahydro-2H-pyran-2-yl)-1H-pyrazolo[4,3-c]quinoline-8-carboxylate (220 mg, 0.644 mmol, 41.6% yield). *m*/*z:* 377.1 (M+H)⁺ (MW -THP group).

Step 4. To a stirred solution of methyl 4-hydroxy-3-methyl-1-(tetrahydro-2H-pyran-2-yl)-1H-pyrazolo[4,3-c]quinoline-8-carboxylate (600 mg, 1.76 mmol, 1.0 equiv) in dichloromethane (3.00 mL). Then trifluoromethanesulfonic anhydride (992 mg, 3.52 mmol, 2.0 equiv) and DIPEA (921 µL, 5.27 mmol, 3.0 equiv) was added and the reaction mixture kept between 30-32°C for 16 h. The reaction mixture was concentrated under reduced pressure to get 300 mg (31% crude yield) of crude methyl 3-methyl-1-(tetrahydro-2H-pyran-2-yl)-4-(((trifluoromethyl)sulfonyl)oxy)-1H-pyrazolo[4,3-c]quinoline-8-carboxylate.

To a stirred solution of this crude methyl 3-methyl-1-(tetrahydro-2H-pyran-2-yl)-4-(((trifluoromethyl)sulfonyl)oxy)-1H-pyrazolo[4,3-c]quinoline-8-carboxylate (300 mg, 0.634 mmol, 1.0 equiv) in N, N-dimethylacetamide (2.00 mL) was added DIPEA (332 µL, 1.90 mmol, 3.0 equiv). Then (4-methoxyphenyl)methanamine (130 mg, 0.950 mmol, 1.5 equiv) was added and the reaction mixture heated at 90 °C for 4 h. The reaction mixture was quenched with water and extracted with ethyl acetate. The organic layer was dried over by sodium sulphate and concentrated under reduced pressure to get crude material which was purified by column chromatography (silica, 50% EtOAc:hexane) to get pure methyl 4-((4-methoxybenzyl)amino)-3-methyl-1-(tetrahydro-2H-pyran-2-yl)-1H-pyrazolo[4,3-c]quinoline-8-carboxylate (250 mg, 0.543 mmol, 86.0% yield). *m*/*z:* 377.1 (M+H)⁺ (MW-THP group).

Step 5. A solution of methyl 4-((4-methoxybenzyl)amino)-3-methyl-1-(tetrahydro-2H-pyran-2-yl)-1H-pyrazolo[4,3-c]quinoline-8-carboxylate (2.80 g, 6.08 mmol, 1.0 equiv) in trifluoroacetic acid (28.0 mL) was heated at 90 °C for 12 h. The reaction mixture was concentrated under reduced pressure to get crude methyl 4-amino-3-methyl-1H-pyrazolo[4,3-c]quinoline-8-carboxylate (3.50 g, 13.7 mmol, 225% crude yield). *m*/*z:* 257.3 (M+H)⁺.

Step 6. To a stirred solution of methyl 4-amino-3-methyl-1H-pyrazolo[4,3-c]quinoline-8-carboxylate (3.50 g, 13.7 mmol, 1.0 equiv) in tetrahydrofuran (35.0 mL), methanol (35.0 mL), water (35.0 mL) at room temperature was added lithium hydroxide monohydrate (4.02 g, 96.0 mmol, 7.0 equiv) and the reaction mixture was stirred at rt for 16 h. The reaction mixture was quenched with water and a solid precipitate was observed. The solid was filtered and dried under vacuum. This solid was washed with diethyl ether and dried to obtain 4-amino-3-methyl-1H-pyrazolo[4,3-c]quinoline-8-carboxylic acid (150) (1.40 g, 5.78 mmol, 42.3% yield). *m*/*z:* 243.1 (M+H)⁺.

The following amines in Table 11 were prepared in a manner similar to that described for Intermediate **150.**

**Table 11**

| **Int. #** | **Chemical Structure** | **Name** | ***m*/*z* (ESI): (M+H)⁺** |
|---|---|---|---|
| **151** | 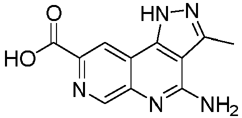 | 4-amino-3-methyl-1H-pyrazolo[4,3-c][1,7]naphthyridine-8-carboxylic acid | 258.0 (Me ester) Acid mass not observed |

### Intermediate 152: 2-amino-3-iodoquinoline-6-carboxylic acid

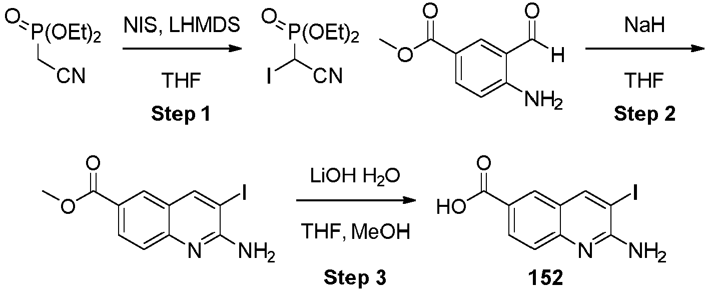

Step 1. To a stirred solution of diethyl (cyanomethyl)phosphonate (45.7 mL, 282 mmol) in tetrahydrofuran (1000 mL) was added LHMDS (423 mL, 423 mmol) at 0 °C and stirred for 30 min. To the reaction mixture N-Iodosuccinimide (95 g, 423 mmol) was added and stirred at rt for 3h. The reaction mixture was quenched with 3M HCl solution and extracted with DCM. The combined organic layer was washed with brine and dried over Na₂SO₄, filtered and concentrated under reduced pressure, to give crude diethyl (cyanoiodomethyl)phosphonate (90g, 297 mmol, 105% yield) as tan oil. *m*/*z* (ESI): 303.9 (M+H)⁺.

Step 2. To a stirred solution of sodium hydride (39.1 g, 977 mmol) in THF (500 mL) was added diethyl (cyanoiodomethyl)phosphonate (178 g, 586 mmol) in THF (500 mL) slowly at 0°C. The reaction mixture was stirred at 0°C for 30 min. Then methyl 4-amino-3-formylbenzoate (70 g, 391 mmol) in THF (500 mL) was added slowly at 0°C and stirred at RT for 16 h. After reaction completion, ice water was added. The precipitated solid was filtered and washed with diethyl ether to provide methyl 2-amino-3-iodoquinoline-6-carboxylate (90 g, 274 mmol, 70.2% yield) as a light yellow solid.

Step 3. To a stirred solution of methyl 2-amino-3-iodoquinoline-6-carboxylate (250 g, 762 mmol) in water (1000 mL):tetrahydrofuran (1000 mL):methanol (1000 mL) was added LiOH H₂O (128 g, 3048 mmol) and the reaction mixture heated at 50°C for 2 h. After completion of reaction, the reaction mixture concentrated, then diluted with water up to complete dissolution of lithium salt of the acid and the aqueous layer was acidified with 1.5 N HCl up to pH 5.0. The solid obtained was filtered, washed with water (1000 mL) and methanol (1000 mL), dried on vacuum over night to give 2-amino-3-iodoquinoline-6-carboxylic acid **(152)** (230 g, 732 mmol, 96% yield) as off white solid. *m*/*z* (ESI): 314.9 (M+H)⁺. ¹H NMR (400 MHz, DMSO-d6): 12.84 (1H, s), 8.76 (1H, s), 8.31 (1H, d, J=2.0 Hz), 7.99 (1H, dd, J=8.7, 2.0 Hz), 7.49 (1H, d, J=8.8 Hz), 6.87 (2H, s).

### Example 200: (S)-4-amino-N-cyclopropyl-7-fluoro-1-methyl-N-(6-(trifluoromethyl)-2,3-dihydrobenzofuran-3-yl)-1H-pyrazolo[4,3-c]quinoline-8-carboxamide

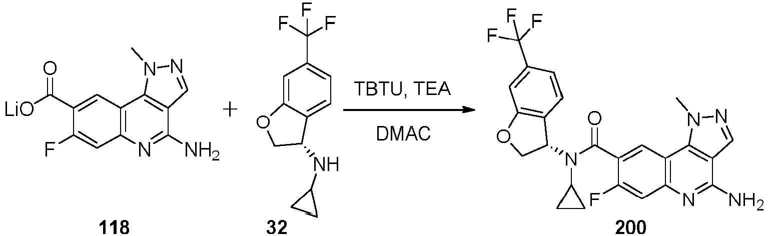

A mixture of lithium 4-amino-7-fluoro-1-methyl-1H-pyrazolo[4,3-c]quinoline-8-carboxylate (118) (0.050 g, 0.188 mmol), (*S*)-*N*-cyclopropyl-6-(trifluoromethyl)-2,3-dihydrobenzofuran-3-amine **(32)** (0.055 g, 0.225 mmol), TBTU (0.072 g, 0.225 mmol, Sigma-Aldrich Corporation), and TEA (0.079 mL, 0.564 mmol, Sigma-Aldrich Corporation) in *N,* N-dimethylacetamide (1 mL) was stirred at rt for 12 hours. The reaction mixture was purified directly on ISCO using 0-100% EtOAc/EtOH (3: 1) in heptane to afford (*S*)-4-amino-N-cyclopropyl-7-fluoro-1-methyl-*N*-(6-(trifluoromethyl)-2,3-dihydrobenzofuran-3-yl)-1H-pyrazolo[4,3-c]quinoline-8-carboxamide **(200)** (0.023 g, 0.047 mmol, 25.2 % yield). *m*/*z* (ESI): 486.3 (M+H)⁺. ¹H NMR (400 MHz, CHLOROFORM-d) δ ppm 8.15 - 8.37 (m, 1 H) 7.98 (s, 1 H) 7.50 - 7.65 (m, 1 H) 7.34 - 7.47 (m, 1 H) 7.25 - 7.28 (m, 1 H) 7.10 - 7.19 (m, 1 H) 6.11 - 6.39 (m, 1 H) 5.44 - 5.80 (m, 2 H) 4.58 - 5.01 (m, 2 H) 4.46 (s, 3 H) 2.49 - 2.85 (m, 1 H) 0.28 - 0.61 (m, 2 H) 0.06 - 0.27 (m, 2 H). ¹⁹F NMR (376 MHz, DMSO-d6) δ ppm -67.16 (s, 3 F), -117.06 (s, 1 F).

Examples in Table 12 were prepared in a manner similar to that described above for Example 200 using the indicated amide coupling reagent in the table. Compounds 220 to 224 and 279 to 282, are for reference purposes only

**Table 12**

| **Ex.** | **Structure** | **Name** | **Coupling Reagent** | **m/z (ESI): (M+H)⁺** |
|---|---|---|---|---|
| **201** | 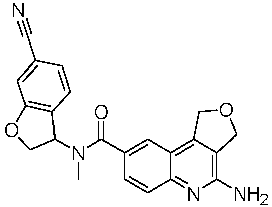 | 4-amino-N-((3R)-6-cyano-2,3-dihydro-1-benzofuran-3-yl)-N-methyl-1,3-dihydrofuro[3,4-c]quinoline-8-carboxamide and 4-amino-N-((3S)-6-cyano-2,3-dihydro-1-benzofuran-3-yl)-N-methyl-1,3-dihydrofuro[3,4-c]quinoline-8-carboxamide | PyBrop | 387.2 |
| **202** | 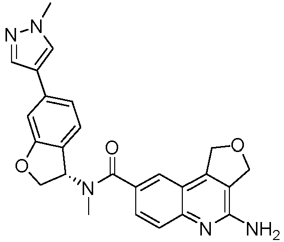 | 4-amino-N-methyl-N-((3S)-6-(1-methyl-1H-pyrazol-4-yl)-2,3-dihydro-1-benzofuran-3-yl)-1,3-dihydrofuro[3,4-c]quinoline-8-carboxamide | HATU | 442.0 |
| **203** | 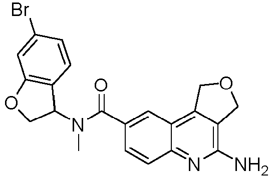 | 4-amino-N-((3R)-6-bromo-2,3-dihydro-1-benzofuran-3-yl)-N-methyl-1,3-dihydrofuro[3,4-c]quinoline-8-carboxamide and 4-amino-N-((3S)-6-bromo-2,3-dihydro-1-benzofuran-3-yl)-N-methyl-1,3-dihydrofuro[3,4-c]quinoline-8-carboxamide | HATU | 440, 442 |
| **204** | 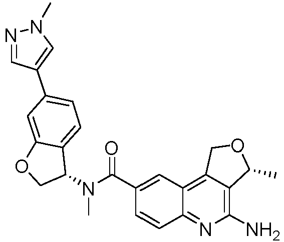 | (3R)-4-amino-N,3-dimethyl-N-((3S)-6-(1-methyl-1H-pyrazol-4-yl)-2,3-dihydro-1-benzofuran-3-yl)-1,3-dihydrofuro[3,4-c]quinoline-8-carboxamide | HATU | 456.0 |
| **205** | 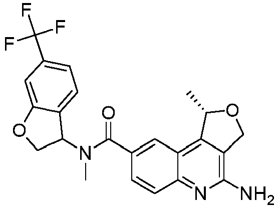 | (1R)-4-amino-N,1-dimethyl-N-((3S)-6-(trifluoromethyl)-2,3-dihydro-1-benzofuran-3-yl)-1,3-dihydrofuro[3,4-c]quinoline-8-carboxamide and (1S)-4-amino-N,1-dimethyl-N-((3S)-6-(trifluoromethyl)-2,3-dihydro-1-benzofuran-3-yl)-1,3-dihydrofuro[3,4-c]quinoline-8-carboxamide | HATU | 444.0 |
| **206** | 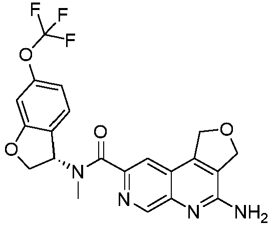 | 4-amino-N-methyl-N-((3 S)-6-(trifluoromethoxy)-2,3-dihydro-1-benzofuran-3-yl)-1,3-dihydrofuro[3,4-c][1, 7]naphthyridine-8-carboxamide | HATU | 447.0 |
| **207** | 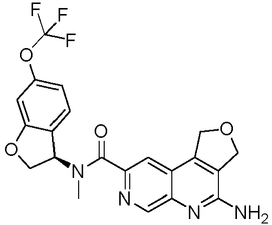 | 4-amino-N-methyl-N-((3R)-6-(trifluoromethoxy)-2,3-dihydro-1-benzofuran-3-yl)-1,3-dihydrofuro[3,4-c][1,7]naphthyridine-8-carboxamide | HATU | 447.1 |
| **208** | 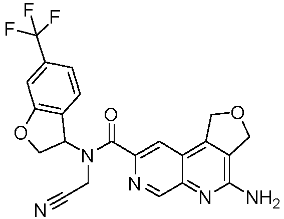 | 4-amino-N-(cyanomethyl)-N-((3R)-6-(trifluoromethyl)-2,3-dihydro-1-benzofuran-3-yl)-1,3-dihydrofuro[3,4-c][1,7]naphthyridine-8-carboxamide and 4-amino-N-(cyanomethyl)-N-((3S)-6-(trifluoromethyl)-2,3-dihydro-1-benzofuran-3-yl)-1,3-dihydrofuro[3,4-c][1,7]naphthyridine-8-carboxamide | PyBrop | 456.2 |
| **209** | 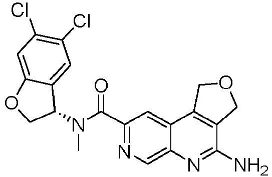 | 4-amino-N-((3S)-5,6-dichloro-2,3-dihydro-1-benzofuran-3-yl)-N-methyl-1,3-dihydrofuro[3,4-c][1,7]naphthyridine-8-carboxamide | HATU | 429.0 (M-H)- |
| **210** | 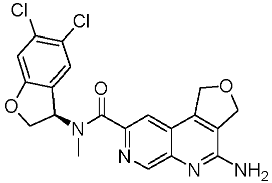 | 4-amino-N-((3R)-5,6-dichloro-2,3-dihydro-1-benzofuran-3-yl)-N-methyl-1,3-dihydrofuro[3,4-c][1,7]naphthyridine-8-carboxamide | HATU | 429.0 (M-H)- |
| **211** | 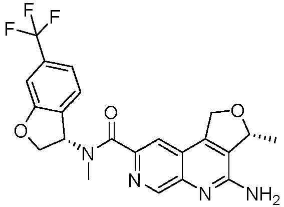 | (3R)-4-amino-N,3-dimethyl-N-((3S)-6-(trifluoromethyl)-2,3-dihydro-1-benzofuran-3-yl)-1,3-dihydrofuro[3,4-c][1,7]naphthyridine-8-carboxamide | HATU | 445.0 |
| **212** | 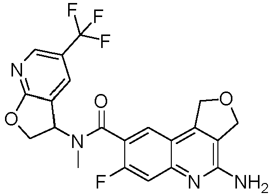 | 4-amino-7-fluoro-N-methyl-N-((3R)-5-(trifluoromethyl)-2,3-dihydrofuro[2,3-b]pyridin-3-yl)-1,3-dihydrofuro[3,4-c]quinoline-8-carboxamide and 4-amino-7-fluoro-N-methyl-N-((3S)-5-(trifluoromethyl)-2,3-dihydrofuro[2,3-b]pyridin-3-yl)-1,3-dihydrofuro[3,4-c]quinoline-8-carboxamide | HATU | 448.9 |
| **213** | 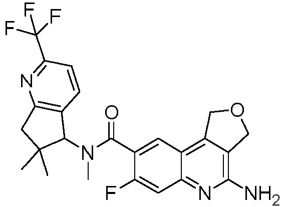 | 4-amino-N-((5R)-6,6-dimethyl-2-(trifluoromethyl)-6,7-dihydro-5H-cyclopenta[b]pyridin-5-yl)-7-fluoro-N-methyl-1,3-dihydrofuro[3,4-c]quinoline-8-carboxamide and 4-amino-N-((5S)-6,6-dimethyl-2-(trifluoromethyl)-6,7-dihydro-5H-cyclopenta[b]pyridin-5-yl)-7-fluoro-N-methyl-1,3-dihydrofuro[3,4-c]quinoline-8-carboxamide | PyBrop | 457.0 |
| **214** | 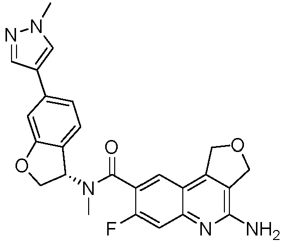 | 4-amino-7-fluoro-N-methyl-N-((3S)-6-(1-methyl-1H-pyrazol-4-yl)-2,3-dihydro-1-benzofuran-3-yl)-1,3-dihydrofuro[3,4-c]quinoline-8-carboxamide | HATU | 460.0 |
| **215** | 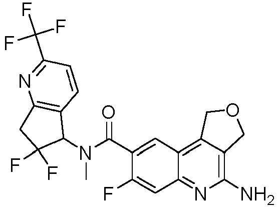 | 4-amino-N-((5R)-6,6-difluoro-2-(trifluoromethyl)-6,7-dihydro-5H-cyclopenta[b]pyridin-5-yl)-7-fluoro-N-methyl-1,3-dihydrofuro[3,4-c]quinoline-8-carboxamide and 4-amino-N-((5 S)-6,6-difluoro-2-(trifluoromethyl)-6,7-dihydro-5H-cyclopenta[b]pyridin-5-yl)-7-fluoro-N-methyl-1,3-dihydrofuro[3,4-c]quinoline-8-carboxamide | PyBrop | 483.0 |
| **216** | 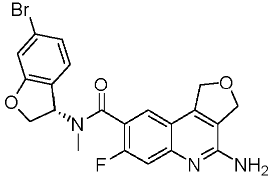 | 4-amino-N-(6-bromo-2,3-dihydrobenzofuran-3-yl)-7-fluoro-N-methyl-1,3-dihydrofuro[3,4-c] quinoline-8-carboxamide | TBTU | 458.0, 460.0 |
| **217** | 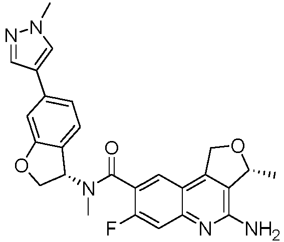 | (3R)-4-amino-7-fluoro-N,3-dimethyl-N-((3S)-6-(1-methyl-1H-pyrazol-4-yl)-2,3-dihydro-1-benzofuran-3-yl)-1,3-dihydrofuro[3,4-c]quinoline-8-carboxamide | HATU | 474.0 |
| **218** | 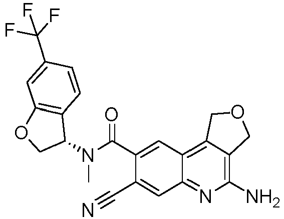 | 4-amino-7-cyano-N-methyl-N-((3S)-6-(trifluoromethyl)-2,3-dihydro-1-benzofuran-3-yl)-1,3-dihydrofuro[3,4-c]quinoline-8-carboxamide | HATU | 454.9 |
| **219** | 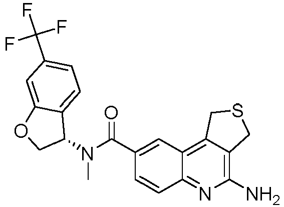 | 4-amino-N-methyl-N-((3 S)-6-(trifluoromethyl)-2,3-dihydro-1-benzofuran-3-yl)-1,3-dihydrothieno[3,4-c]quinoline-8-carboxamide | HATU | 446.1 |
| **220** | 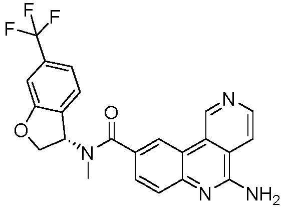 | 5-amino-N-methyl-N-((3 S)-6-(trifluoromethyl)-2,3-dihydro-1-benzofuran-3-yl)benzo[c][2,6]naphthyridine-9-carboxamide | HATU | 439.2 |
| **221** | 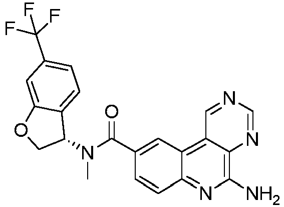 | 5-amino-N-methyl-N-((3S)-6-(trifluoromethyl)-2,3-dihydro-1-benzofuran-3-yl)pyrimido[4,5-c]quinoline-9-carboxamide | HATU | 440.3 |
| **222** | 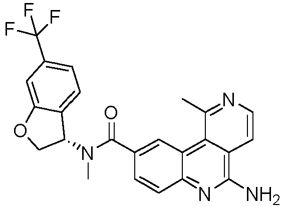 | 5-amino-N,1-dimethyl-N-((3S)-6-(trifluoromethyl)-2,3-dihydro-1-benzofuran-3-yl)benzo[c][2,6]naphthyridine-9-carboxamide | HATU | 453.1 |
| **223** | 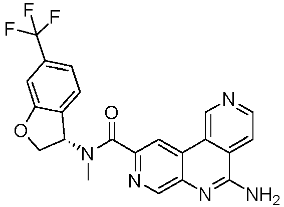 | 5-amino-N-methyl-N-((3S)-6-(trifluoromethyl)-2,3-dihydro-1-benzofuran-3-yl)pyrido[4,3-c][1,7]naphthyridine-9-carboxamide | HATU | 439.8 |
| **224** | 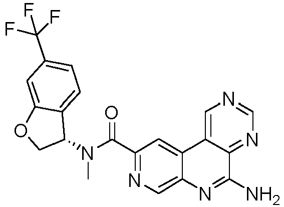 | 5-amino-N-methyl-N-((3S)-6-(trifluoromethyl)-2,3-dihydro-1-benzofuran-3-yl)pyrimido[4,5-c][1,7]naphthyridine-9-carboxamide | HATU | 442.2 |
| **225** | 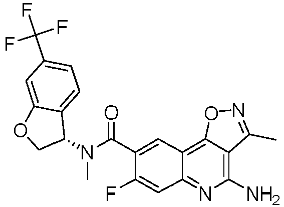 | 4-amino-7-fluoro-N,3-dimethyl-N-((3S)-6-(trifluoromethyl)-2,3-dihydro-1-benzofuran-3-yl)[1,2]oxazolo[4,5-c]quinoline-8-carboxamide | HATU | 461.0 |
| **226** | 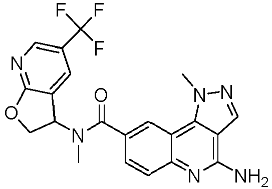 | 4-amino-N,1-dimethyl-N-((3R)-5-(trifluoromethyl)-2,3-dihydrofuro[2,3-b]pyridin-3-yl)-1H-pyrazolo[4,3-c]quinoline-8-carboxamide and 4-amino-N,1-dimethyl-N-((3S)-5-(trifluoromethyl)-2,3-dihydrofuro[2,3-b]pyridin-3-yl)-1H-pyrazolo[4,3-c]quinoline-8-carboxamide | HATU | 442.9 |
| **227** | 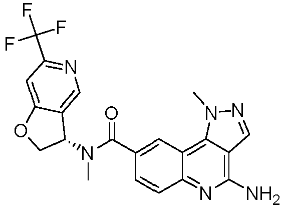 | 4-amino-N,1-dimethyl-N-((3S)-6-(trifluoromethyl)-2,3-dihydrofuro[3,2-c]pyridin-3-yl)-1H-pyrazolo[4,3-c]quinoline-8-carboxamide | HATU | 443.0 |
| **228** | 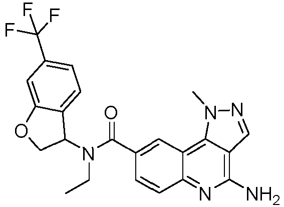 | 4-amino-N-ethyl-1-methyl-N-((3R)-6-(trifluoromethyl)-2,3-dihydro-1-benzofuran-3-yl)-1H-pyrazolo[4,3-c]quinoline-8-carboxamide and 4-amino-N-ethyl-1-methyl-N-((3S)-6-(trifluoromethyl)-2,3-dihydro-1-benzofuran-3-yl)-1H-pyrazolo[4,3-c]quinoline-8-carboxamide | HATU | 456.0 |
| **229** | 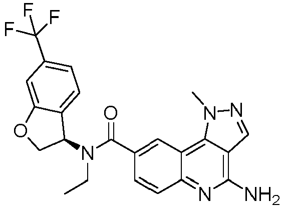 | 4-amino-N-ethyl-1-methyl-N-((3R)-6-(trifluoromethyl)-2,3-dihydro-1-benzofuran-3-yl)-1H-pyrazolo[4,3-c]quinoline-8-carboxamide | HATU | 456.0 |
| **230** | 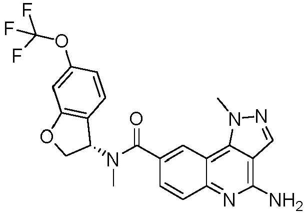 | 4-amino-N,1-dimethyl-N-((3S)-6-(trifluoromethoxy)-2,3-dihydro-1-benzofuran-3-yl)-1H-pyrazolo[4,3-c] quinoline-8-carboxamide | TBTU | 458.2 |
| **231** | 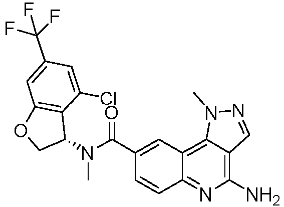 | 4-amino-N-((3 S)-4-chloro-6-(trifluoromethyl)-2,3-dihydro-1-benzofuran-3-yl)-N,1-dimethyl-1H-pyrazolo[4,3-c]quinoline-8-carboxamide | HATU | 475.8 |
| **232** | 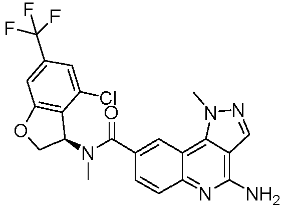 | 4-amino-N-((3R)-4-chloro-6-(trifluoromethyl)-2,3-dihydro-1-benzofuran-3-yl)-N,1-dimethyl-1H-pyrazolo[4,3-c]quinoline-8-carboxamide | HATU | 475.8 |
| **233** | 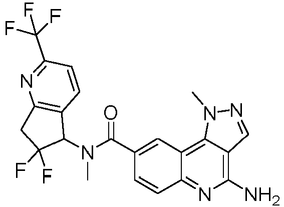 | 4-amino-N-((5R)-6,6-difluoro-2-(trifluoromethyl)-6,7-dihydro-5H-cyclopenta[b]pyridin-5-yl)-N,1-dimethyl-1H-pyrazolo[4,3-c]quinoline-8-carboxamide and 4-amino-N-((5 S)-6,6-difluoro-2-(trifluoromethyl)-6,7-dihydro-5H-cyclopenta[b]pyridin-5-yl)-N,1-dimethyl-1H-pyrazolo[4,3-c]quinoline-8-carboxamide | PyBrop | 477.0 |
| **234** | 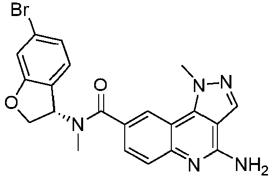 | 4-amino-N-((3S)-6-bromo-2,3-dihydro-1-benzofuran-3-yl)-N,1-dimethyl-1H-pyrazolo[4,3-c]quinoline-8-carboxamide | HATU | 452, 454 |
| **235** | 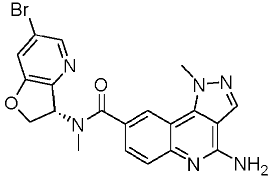 | 4-amino-N-((3 S)-6-bromo-2,3-dihydrofuro[3,2-b]pyridin-3-yl)-N,1-dimethyl-1H-pyrazolo[4,3-c]quinoline-8-carboxamide | HATU | 452.8, 454.4 |
| **236** | 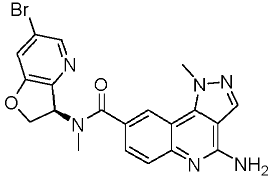 | 4-amino-N-((3R)-6-bromo-2,3-dihydrofuro[3,2-b]pyridin-3-yl)-N,1-dimethyl-1H-pyrazolo[4,3-c]quinoline-8-carboxamide | HATU | 452.8, 454.4 |
| **237** | 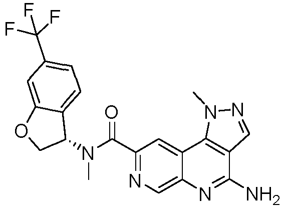 | 4-amino-N,1-dimethyl-N-((3S)-6-(trifluoromethyl)-2,3-dihydro-1-benzofuran-3-yl)-1H-pyrazolo[4,3-c][1,7]naphthyridine-8-carboxamide | PyBroP | 443.2 |
| **238** | 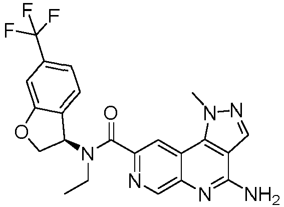 | 4-amino-N-ethyl-1-methyl-N-((3S)-6-(trifluoromethyl)-2,3-dihydro-1-benzofuran-3-yl)-1H-pyrazolo[4,3-c][1,7]naphthyridine-8-carboxamide | HATU | 457.0 |
| **239** | 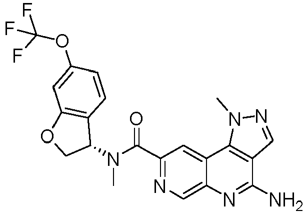 | 4-amino-N,1-dimethyl-N-((3S)-6-(trifluoromethoxy)-2,3-dihydro-1-benzofuran-3-yl)-1H-pyrazolo[4,3-c][1,7]naphthyridine-8-carboxamide | TBTU | 459.1 |
| **240** | 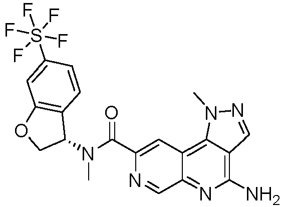 | 4-amino-N,1-dimethyl-N-((3S)-6-(pentafluoro-lambda~6~-sulfanyl)-2,3-dihydro-1-benzofuran-3-yl)-1H-pyrazolo[4,3-c][1,7]naphthyridine-8-carboxamide | TBTU | 501.1 |
| **241** | 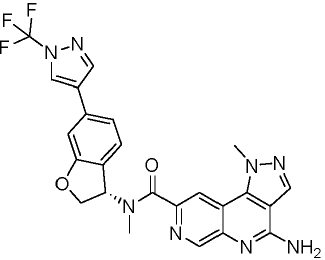 | 4-amino-N,1-dimethyl-N-((3S)-6-(1-(trifluoromethyl)-1H-pyrazol-4-yl)-2,3-dihydro-1-benzofuran-3-yl)-1H-pyrazolo[4,3-c][1, 7]naphthyridine-8-carboxamide | HATU | 509.0 |
| **242** | 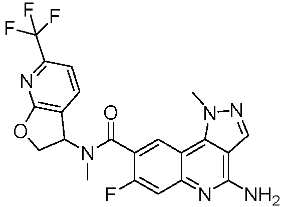 | 4-amino-7-fluoro-N,1-dimethyl-N-((3R)-6-(trifluoromethyl)-2,3-dihydrofuro[2,3-b]pyridin-3-yl)-1H-pyrazolo[4,3-c]quinoline-8-carboxamide and 4-amino-7-fluoro-N,1-dimethyl-N-((3S)-6-(trifluoromethyl)-2,3-dihydrofuro[2,3-b]pyridin-3-yl)-1H-pyrazolo[4,3-c]quinoline-8-carboxamide | PyBrop | 461.0 |
| **243** | 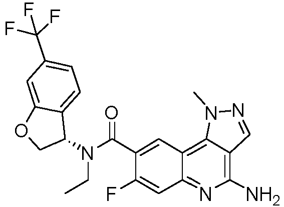 | 4-amino-N-ethyl-7-fluoro-1-methyl-N-((3S)-6-(trifluoromethyl)-2,3-dihydro-1-benzofuran-3-yl)-1H-pyrazolo[4,3-c]quinoline-8-carboxamide | HATU | 474.0 |
| **244** | 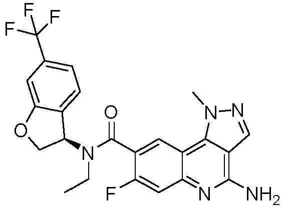 | 4-amino-N-ethyl-7-fluoro-1-methyl-N-((3R)-6-(trifluoromethyl)-2,3-dihydro-1-benzofuran-3-yl)-1H-pyrazolo[4,3-c]quinoline-8-carboxamide | HATU | 474.0 |
| **245** | 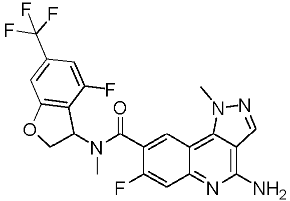 | 4-amino-7-fluoro-N-((3R)-4-fluoro-6-(trifluoromethyl)-2,3-dihydro-1-benzofuran-3-yl)-N,1-dimethyl-1H-pyrazolo [4,3-c] quinoline-8-carboxamide and 4-amino-7-fluoro-N-((3S)-4-fluoro-6-(trifluoromethyl)-2,3-dihydro-1-benzofuran-3-yl)-N,1-dimethyl-1H-pyrazolo [4,3-c] quinoline-8-carboxamide | PyBrop | 477.9 |
| **246** | 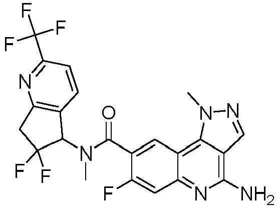 | 4-amino-N-((5R)-6,6-difluoro-2-(trifluoromethyl)-6,7-dihydro-5H-cyclopenta[b]pyridin-5-yl)-7-fluoro-N,1-dimethyl-1H-pyrazolo[4,3-c]quinoline-8-carboxamide and 4-amino-N-((5S)-6,6-difluoro-2-(trifluoromethyl)-6,7-dihydro-5H-cyclopenta[b]pyridin-5-yl)-7-fluoro-N,1-dimethyl-1H-pyrazolo[4,3-c]quinoline-8-carboxamide | PyBrop | 494.8 |
| **247** | 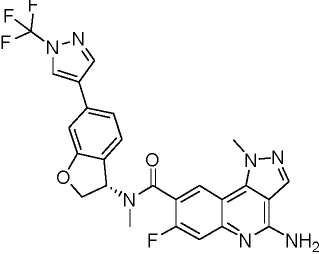 | 4-amino-7-fluoro-N,1-dimethyl-N-((3S)-6-(1-(trifluoromethyl)-1H-pyrazol-4-yl)-2,3-dihydro-1-benzofuran-3-yl)-1H-pyrazolo[4,3-c]quinoline-8-carboxamide | HATU | 526.0 |
| **248** | 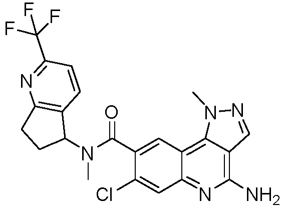 | 4-amino-7-chloro-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide and 4-amino-7-chloro-N,1-dimethyl-N-((5S)-2-(trifluoromethyl)-6,7-dihydro-5H-cyclopenta[b]pyridin-5-yl)-1H-pyrazolo[4,3-c]quinoline-8-carboxamide | PyBrop | 475.2 |
| **249** | 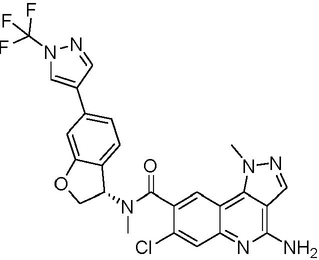 | 4-amino-7-chloro-N,1-dimethyl-N-((3S)-6-(1-(trifluoromethyl)-1H-pyrazol-4-yl)-2,3-dihydro-1-benzofuran-3-yl)-1H-pyrazolo[4,3-c]quinoline-8-carboxamide | HATU | 542.0 |
| **250** | 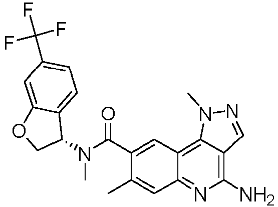 | 4-amino-N,1,7-trimethyl-N-((3S)-6-(trifluoromethyl)-2,3-dihydro-1-benzofuran-3-yl)-1H-pyrazolo[4,3-c]quinoline-8-carboxamide | TBTU | 456.2 |
| **251** | 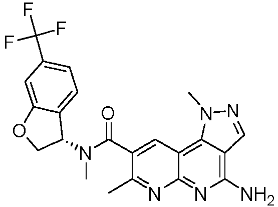 | 4-amino-N,1,7-trimethyl-N-((3S)-6-(trifluoromethyl)-2,3-dihydro-1-benzofuran-3-yl)-1H-pyrazolo[4,3-c][1,8]naphthyridine-8-carboxamide | TBTU | 457.2 |
| **252** | 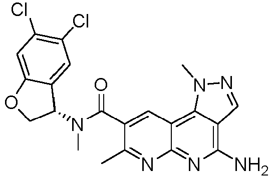 | 4-amino-N-((3S)-5,6-dichloro-2,3-dihydro-1-benzofuran-3-yl)-N,1,7-trimethyl-1H-pyrazolo[4,3-c][1,8]naphthyridine-8-carboxamide | TBTU | 457.2 |
| **253** | 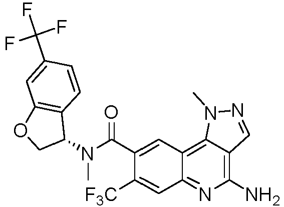 | 4-amino-N,1-dimethyl-7-(trifluoromethyl)-N-((3 S)-6-(trifluoromethyl)-2,3-dihydro-1-benzofuran-3-yl)-1H-pyrazolo[4,3-c]quinoline-8-carboxamide | TBTU | 510.2 |
| **254** | 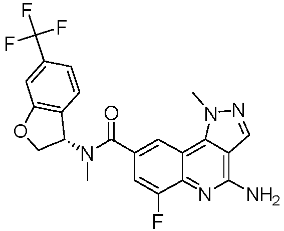 | 4-amino-6-fluoro-N,1-dimethyl-N-((3S)-6-(trifluoromethyl)-2,3-dihydro-1-benzofuran-3-yl)-1H-pyrazolo[4,3-c]quinoline-8-carboxamide | TBTU | 460.0 |
| **255** | 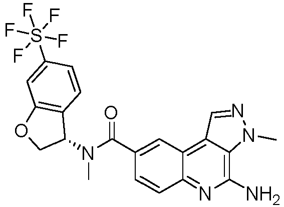 | 4-amino-N,3-dimethyl-N-((3S)-6-(pentafluoro-lambda~6~-sulfanyl)-2,3-dihydro-1-benzofuran-3-yl)-3H-pyrazolo[3,4-c]quinoline-8-carboxamide | TBTU | 500.6 |
| **256** | 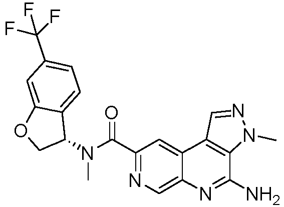 | 4-amino-N,3-dimethyl-N-((3S)-6-(trifluoromethyl)-2,3-dihydro-1-benzofuran-3-yl)-3H-pyrazolo[3,4-c][1,7]naphthyridine-8-carboxamide | HATU | 443.0 |
| **257** | 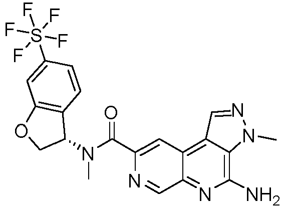 | 4-amino-N,3-dimethyl-N-((3S)-6-(pentafluoro-lambda~6~-sulfanyl)-2,3-dihydro-1-benzofuran-3-yl)-3H-pyrazolo[3,4-c][1,7]naphthyridine-8-carboxamide | TBTU | 501.6 |
| **258** | 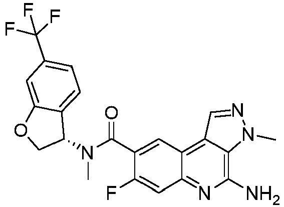 | 4-amino-7-fluoro-N,3-dimethyl-N-((3S)-6-(trifluoromethyl)-2,3-dihydro-1-benzofuran-3-yl)-3H-pyrazolo[3,4-c]quinoline-8-carboxamide | HATU | 460.0 |
| **259** | 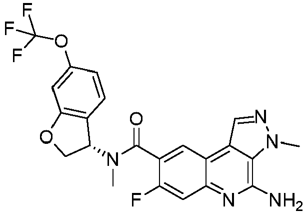 | 4-amino-7-fluoro-N,3-dimethyl-N-((3S)-6-(trifluoromethoxy)-2,3-dihydro-1-benzofuran-3-yl)-3H-pyrazolo[3,4-c]quinoline-8-carboxamide | TBTU | 476.2 |
| **260** | 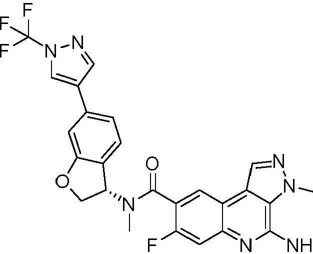 2 | 4-amino-7-fluoro-N,3-dimethyl-N-((3S)-6-(1-(trifluoromethyl)-1H-pyrazol-4-yl)-2,3-dihydro-1-benzofuran-3-yl)-3H-pyrazolo[3,4-c]quinoline-8-carboxamide | HATU | 526.0 |
| **261** | 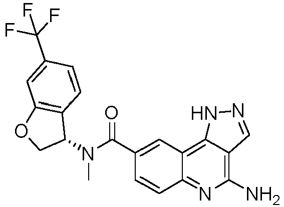 | 4-amino-N-methyl-N-((3 S)-6-(trifluoromethyl)-2,3-dihydro-1-benzofuran-3-yl)-1H-pyrazolo[4,3-c]quinoline-8-carboxamide | TBTU | 427.9 |
| **262** | 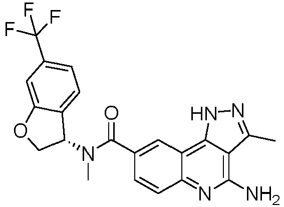 | 4-amino-N,3-dimethyl-N-((3S)-6-(trifluoromethyl)-2,3-dihydro-1-benzofuran-3-yl)-1H-pyrazolo[4,3-c]quinoline-8-carboxamide | HATU | 441.9 |
| **263** | 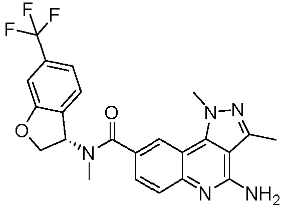 | 4-amino-N,1,3-trimethyl-N-((3S)-6-(trifluoromethyl)-2,3-dihydro-1-benzofuran-3-yl)-1H-pyrazolo[4,3-c]quinoline-8-carboxamide | HATU | 456.1 |
| **264** | 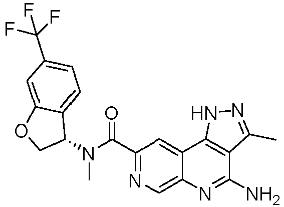 | 4-amino-N,3-dimethyl-N-((3S)-6-(trifluoromethyl)-2,3-dihydro-1-benzofuran-3-yl)-1H-pyrazolo[4,3-c][1,7]naphthyridine-8-carboxamide | HATU | 443.0 |
| **265** | 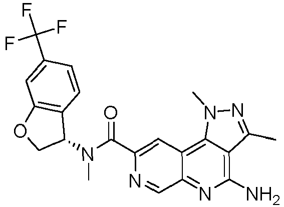 | 4-amino-N,1,3-trimethyl-N-((3S)-6-(trifluoromethyl)-2,3-dihydro-1-benzofuran-3-yl)-1H-pyrazolo[4,3-c][1,7]naphthyridine-8-carboxamide | TBTU | 457.2 |
| **266** | 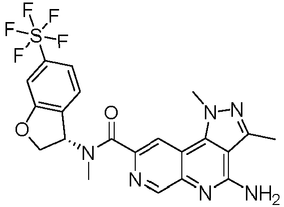 | 4-amino-N,1,3-trimethyl-N-((3S)-6-(pentafluoro-lambda~6~-sulfanyl)-2,3-dihydro-1-benzofuran-3-yl)-1H-pyrazolo[4,3-c][1,7]naphthyridine-8-carboxamide | TBTU | 515.8 |
| **267** | 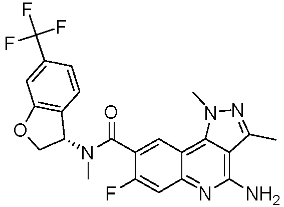 | 4-amino-7-fluoro-N,1,3-trimethyl-N-((3S)-6-(trifluoromethyl)-2,3-dihydro-1-benzofuran-3-yl)-1H-pyrazolo[4,3-c]quinoline-8-carboxamide | TBTU | 474.8 |
| **268** | 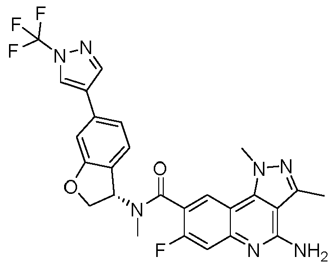 | 4-amino-7-fluoro-N,1,3-trimethyl-N-((3S)-6-(1-(trifluoromethyl)-1H-pyrazol-4-yl)-2,3-dihydro-1-benzofuran-3-yl)-1H-pyrazolo[4,3-c]quinoline-8-carboxamide | HATU | 540.0 |
| **269** | 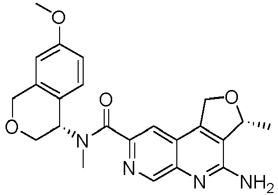 | (3R)-4-amino-N-((4S)-7-methoxy-3,4-dihydro-1H-2-benzopyran-4-yl)-N,3-dimethyl-1,3-dihydrofuro[3,4-c][1,7]naphthyridine-8-carboxamide | TBTU | 421.2 |
| **270** | 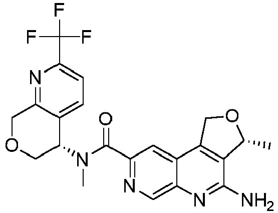 | (3R)-4-amino-N,3-dimethyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamide | TBTU | 460.2 |
| **271** | 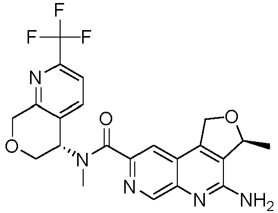 | (3S)-4-amino-N,3-dimethyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamide | TBTU | 460.2 |
| **272** | 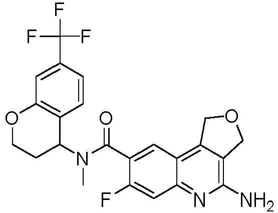 | 4-amino-7-fluoro-N-methyl-N-((4R)-7-(trifluoromethyl)-3,4-dihydro-2H-chromen-4-yl)-1,3-dihydrofuro[3,4-c]quinoline-8-carboxamide and 4-amino-7-fluoro-N-methyl-N-((4S)-7-(trifluoromethyl)-3,4-dihydro-2H-chromen-4-yl)-1,3-dihydrofuro[3,4-c]quinoline-8-carboxamide | PyBrop | 462.0 |
| **273** | 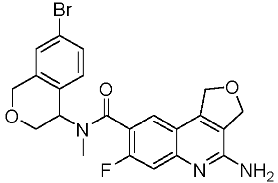 | 4-amino-N-(7-bromoisochroman-4-yl)-7-fluoro-N-methyl-1,3-dihydrofuro[3,4-c]quinoline-8-carboxamide | TBTU | 472.1 and 474.1 |
| **274** | 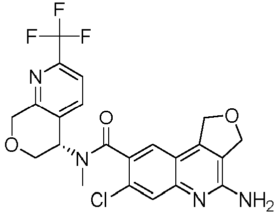 | 4-amino-7-chloro-N-methyl-N-((5S)-2-(trifluoromethyl)-5,8-dihydro-6H-pyrano[3,4-b]pyridin-5-yl)-1,3-dihydrofuro[3,4-c]quinoline-8-carboxamide | TBTU | 479.2 |
| **275** | 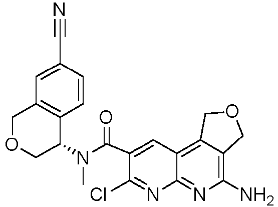 | 4-amino-7-chloro-N-((4S)-7-cyano-3,4-dihydro-1H-2-benzopyran-4-yl)-N-methyl-1,3-dihydrofuro[3,4-c][1,8]naphthyridine-8-carboxamide | PyBrop | 436.0 |
| **276** | 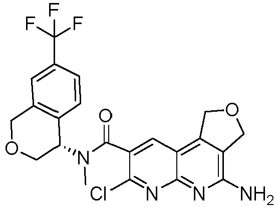 | 4-amino-7-chloro-N-methyl-N-((4S)-7-(trifluoromethyl)-3,4-dihydro-1H-2-benzopyran-4-yl)-1,3-dihydrofuro[3,4-c][1,8]naphthyridine-8-carboxamide | HATU | 479.1 |
| **277** | 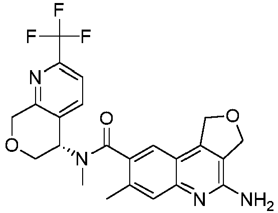 | 4-amino-N,7-dimethyl-N-((5S)-2-(trifluoromethyl)-5,8-dihydro-6H-pyrano[3,4-b]pyridin-5-yl)-1,3-dihydrofuro[3,4-c]quinoline-8-carboxamide | TBTU | 459.2 |
| **278** | 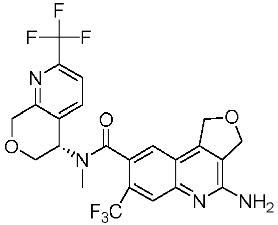 | 4-amino-N-methyl-7-(trifluoromethyl)-N-((5 S)-2-(trifluoromethyl)-5,8-dihydro-6H-pyrano[3,4-b]pyridin-5-yl)-1,3-dihydrofuro[3,4-c]quinoline-8-carboxamide | TBTU | 513.2 |
| **279** | 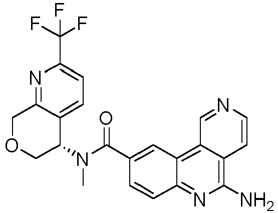 | 5-amino-N-methyl-N-((5S)-2-(trifluoromethyl)-5,8-dihydro-6H-pyrano[3,4-b]pyridin-5-yl)benzo[c][2,6]naphthyridine-9-carboxamide | HATU | 454.0 |
| **280** | 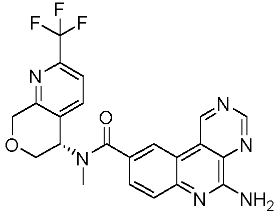 | 5-amino-N-methyl-N-((5S)-2-(trifluoromethyl)-5,8-dihydro-6H-pyrano[3,4-b]pyridin-5-yl)pyrimido[4,5-c]quinoline-9-carboxamide | HATU | 455.1 |
| **281** | 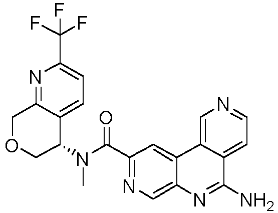 | 5-amino-N-methyl-N-((5S)-2-(trifluoromethyl)-5,8-dihydro-6H-pyrano[3,4-b]pyridin-5-yl)pyrido[4,3-c][1,7]naphthyridine-9-carboxamide | HATU | 455.1 |
| **282** | 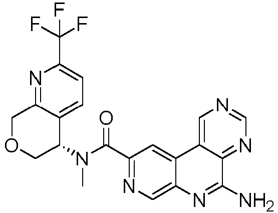 | 5-amino-N-methyl-N-((5S)-2-(trifluoromethyl)-5,8-dihydro-6H-pyrano[3,4-b]pyridin-5-yl)pyrimido[4,5-c][1,7]naphthyridine-9-carboxamide | HATU | 456.1 |
| **283** | 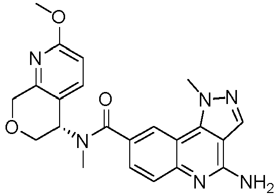 | 4-amino-N-((5S)-2-methoxy-5,8-dihydro-6H-pyrano[3,4-b]pyridin-5-yl)-N,1-dimethyl-1H-pyrazolo[4,3-c]quinoline-8-carboxamide | TBTU | 419.2 |
| **284** | 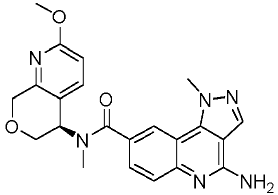 | 4-amino-N-((5R)-2-methoxy-5,8-dihydro-6H-pyrano[3,4-b]pyridin-5-yl)-N,1-dimethyl-1H-pyrazolo[4,3-c]quinoline-8-carboxamide | TBTU | 419.2 |
| **285** | 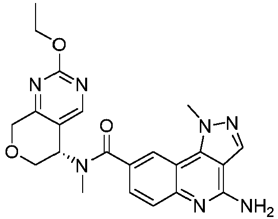 | 4-amino-N-((5S)-2-ethoxy-5,8-dihydro-6H-pyrano[3,4-d]pyrimidin-5-yl)-N,1-dimethyl-1H-pyrazolo[4,3-c]quinoline-8-carboxamide | TBTU | 434.0 |
| **286** | 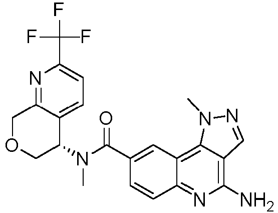 | 4-amino-N,1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide | TBTU | 457.2 |
| **287** | 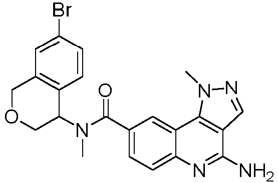 | 4-amino-N-(7-bromoisochroman-4-yl)-N,1-dimethyl-1H-pyrazolo[4,3-c]quinoline-8-carboxamide | TBTU | 466.0, 468.1 |
| **288** | 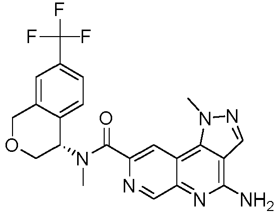 | 4-amino-N,1-dimethyl-N-((4S)-7-(trifluoromethyl)-3,4-dihydro-1H-2-benzopyran-4-yl)-1H-pyrazolo[4,3-c][1,7]naphthyridine-8-carboxamide | HATU | 457.1 |
| **289** | 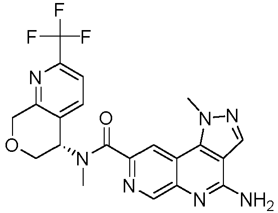 | 4-amino-N,1-dimethyl-N-((5S)-2-(trifluoromethyl)-5,8-dihydro-6H-pyrano[3,4-b]pyridin-5-yl)-1H-pyrazolo [4,3-c][1,7]naphthyridine-8-carboxamide | TBTU | 458.2 |
| **290** | 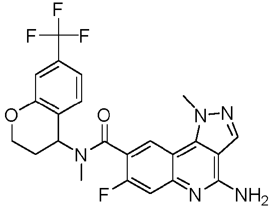 | 4-amino-7-fluoro-N,1-dimethyl-N-((4R)-7-(trifluoromethyl)-3,4-dihydro-2H-chromen-4-yl)-1H-pyrazolo[4,3-c]quinoline-8-carboxamide and 4-amino-7-fluoro-N,1-dimethyl-N-((4S)-7-(trifluoromethyl)-3,4-dihydro-2H-chromen-4-yl)-1H-pyrazolo[4,3-c]quinoline-8-carboxamide | PyBrop | 474.2 |
| **291** | 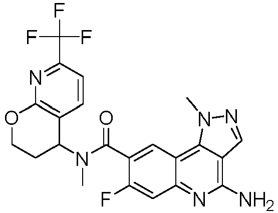 | 4-amino-7-fluoro-N,1-dimethyl-N-((4R)-7-(trifluoromethyl)-3,4-dihydro-2H-pyrano[2,3-b]pyridin-4-yl)-1H-pyrazolo[4,3-c]quinoline-8-carboxamide and 4-amino-7-fluoro-N,1-dimethyl-N-((4S)-7-(trifluoromethyl)-3,4-dihydro-2H-pyrano[2,3-b]pyridin-4-yl)-1H-pyrazolo[4,3-c]quinoline-8-carboxamide | PyBrop | 475.2 |
| **292** | 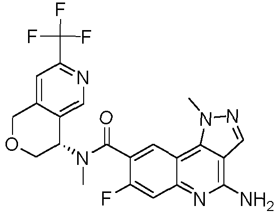 | 4-amino-7-fluoro-N,1-dimethyl-N-((4S)-7-(trifluoromethyl)-3,4-dihydro-1H-pyrano[4,3-c]pyridin-4-yl)-1H-pyrazolo[4,3-c]quinoline-8-carboxamide | TBTU | 475.2 |
| **293** | 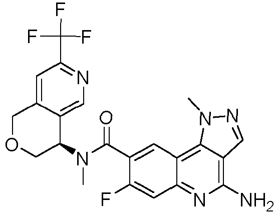 | 4-amino-7-fluoro-N,1-dimethyl-N-((4R)-7-(trifluoromethyl)-3,4-dihydro-1H-pyrano[4,3-c]pyridin-4-yl)-1H-pyrazolo[4,3-c]quinoline-8-carboxamide | TBTU | 475.2 |
| **294** | 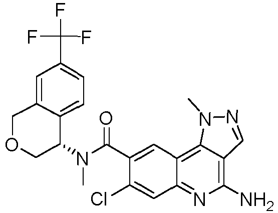 | 4-amino-7-chloro-N,1-dimethyl-N-((4S)-7-(trifluoromethyl)-3,4-dihydro-1H-2-benzopyran-4-yl)-1H-pyrazolo[4,3-c]quinoline-8-carboxamide | TBTU | 490.1 |
| **295** | 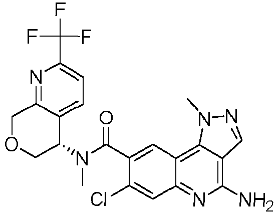 | 4-amino-7-chloro-N,1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide | TBTU | 491.2 |
| **296** | 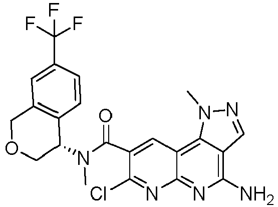 | 4-amino-7-chloro-N,1-dimethyl-N-((4S)-7-(trifluoromethyl)-3,4-dihydro-1H-2-benzopyran-4-yl)-1H-pyrazolo[4,3-c][1,8]naphthyridine-8-carboxamide | HATU | 490.9 |
| **297** | 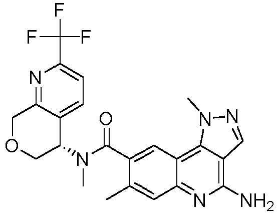 | 4-amino-N,1,7-trimethyl-N-((5S)-2-(trifluoromethyl)-5,8-dihydro-6H-pyrano[3,4-b]pyridin-5-yl)-1H-pyrazolo[4,3-c]quinoline-8-carboxamide | TBTU | 471.2 |
| **298** | 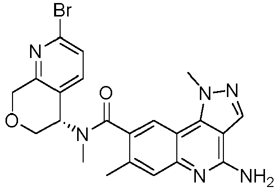 | 4-amino-N-((5S)-2-bromo-5,8-dihydro-6H-pyrano[3,4-b]pyridin-5-yl)-N,1,7-trimethyl-1H-pyrazolo[4,3-c]quinoline-8-carboxamide | TBTU | 480.8, 482.8 |
| **299** | 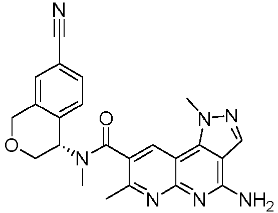 | 4-amino-N-((4S)-7-cyano-3,4-dihydro-1H-2-benzopyran-4-yl)-N,1,7-trimethyl-1H-pyrazolo[4,3-c][1,8]naphthyridine-8-carboxamide | TBTU | 428.2 |
| **300** | 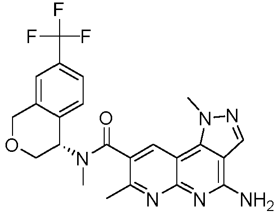 | 4-amino-N,1,7-trimethyl-N-((4S)-7-(trifluoromethyl)-3,4-dihydro-1H-2-benzopyran-4-yl)-1H-pyrazolo[4,3-c][1,8]naphthyridine-8-carboxamide | TBTU | 471.0 |
| **301** | 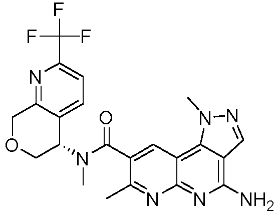 | 4-amino-N,1,7-trimethyl-N-((5S)-2-(trifluoromethyl)-5,8-dihydro-6H-pyrano[3,4-b]pyridin-5-yl)-1H-pyrazolo[4,3-c][1,8]naphthyridine-8-carboxamide | TBTU | 472.2 |
| **302** | 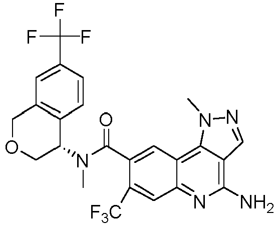 | 4-amino-N,1-dimethyl-7-(trifluoromethyl)-N-((4S)-7-(trifluoromethyl)-3,4-dihydro-1H-2-benzopyran-4-yl)-1H-pyrazolo[4,3-c]quinoline-8-carboxamide | TBTU | 524.2 |
| **303** | 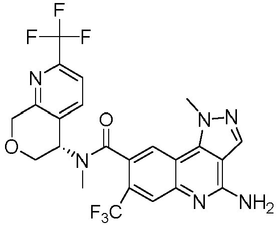 | 4-amino-N,1-dimethyl-7-(trifluoromethyl)-N-((5 S)-2-(trifluoromethyl)-5,8-dihydro-6H-pyrano[3,4-b]pyridin-5-yl)-1H-pyrazolo[4,3-c]quinoline-8-carboxamide | TBTU | 525.0 |
| **304** | 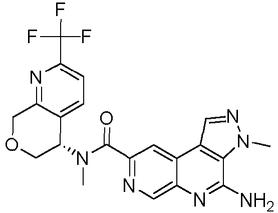 | 4-amino-N,3-dimethyl-N-((5S)-2-(trifluoromethyl)-5,8-dihydro-6H-pyrano[3,4-b]pyridin-5-yl)-3H-pyrazolo[3,4-c][1,7]naphthyridine-8-carboxamide | TBTU | 458.2 |
| **305** | 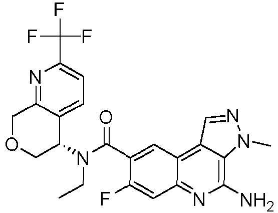 | 4-amino-N-ethyl-7-fluoro-3-methyl-N-((5 S)-2-(trifluoromethyl)-5,8-dihydro-6H-pyrano[3,4-b]pyridin-5-yl)-3H-pyrazolo[3,4-c]quinoline-8-carboxamide | TBTU | 489.2 |
| **306** | 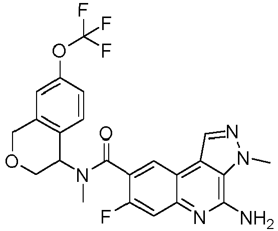 | 4-amino-7-fluoro-N,3-dimethyl-N-((4R)-7-(trifluoromethoxy)-3,4-dihydro-1H-2-benzopyran-4-yl)-3H-pyrazolo[3,4-c]quinoline-8-carboxamide and 4-amino-7-fluoro-N,3-dimethyl-N-((4S)-7-(trifluoromethoxy)-3,4-dihydro-1H-2-benzopyran-4-yl)-3H-pyrazolo[3,4-c]quinoline-8-carboxamide | HATU | 490.0 |
| **307** | 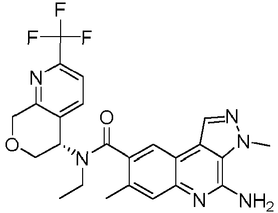 | 4-amino-N-ethyl-3,7-dimethyl-N-((5S)-2-(trifluoromethyl)-5,8-dihydro-6H-pyrano[3,4-b]pyridin-5-yl)-3H-pyrazolo[3,4-c]quinoline-8-carboxamide | TBTU | 485.2 |
| **308** | 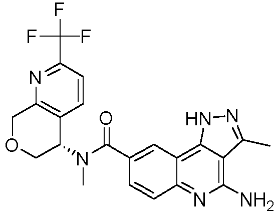 | 4-amino-N,3-dimethyl-N-((5S)-2-(trifluoromethyl)-5,8-dihydro-6H-pyrano[3,4-b]pyridin-5-yl)-1H-pyrazolo[4,3-c]quinoline-8-carboxamide | HATU | 456.8 |
| **309** | 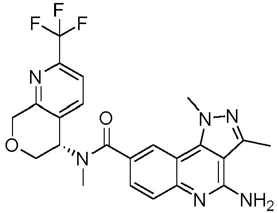 | 4-amino-N,1,3-trimethyl-N-((5S)-2-(trifluoromethyl)-5,8-dihydro-6H-pyrano[3,4-b]pyridin-5-yl)-1H-pyrazolo[4,3-c]quinoline-8-carboxamide | HATU | 471.1 |
| **310** | 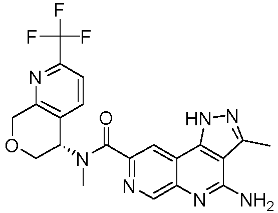 | 4-amino-N,3-dimethyl-N-((5S)-2-(trifluoromethyl)-5,8-dihydro-6H-pyrano[3,4-b]pyridin-5-yl)-1H-pyrazolo[4,3-c][1,7]naphthyridine-8-carboxamide | HATU | 458.0 |
| **311** | 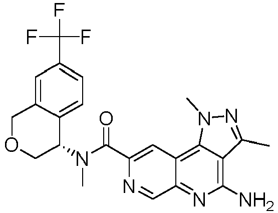 | 4-amino-N,1,3-trimethyl-N-((4S)-7-(trifluoromethyl)-3,4-dihydro-1H-2-benzopyran-4-yl)-1H-pyrazolo[4,3-c][1,7]naphthyridine-8-carboxamide | HATU | 471.2 |
| **312** | 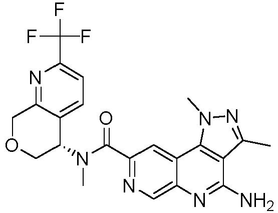 | 4-amino-N,1,3-trimethyl-N-((5S)-2-(trifluoromethyl)-5,8-dihydro-6H-pyrano[3,4-b]pyridin-5-yl)-1H-pyrazolo[4,3-c][1,7]naphthyridine-8-carboxamide | TBTU | 472.2 |
| **313** | 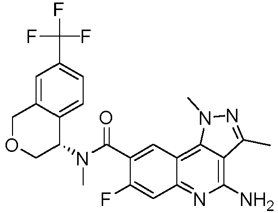 | 4-amino-7-fluoro-N,1,3-trimethyl-N-((4S)-7-(trifluoromethyl)-3,4-dihydro-1H-2-benzopyran-4-yl)-1H-pyrazolo[4,3-c]quinoline-8-carboxamide | HATU | 488.2 |
| **314** | 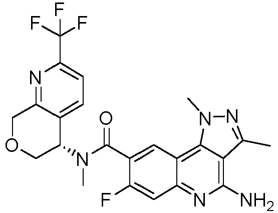 | 4-amino-7-fluoro-N, 1,3-trimethyl-N-((5S)-2-(trifluoromethyl)-5,8-dihydro-6H-pyrano[3,4-b]pyridin-5-yl)-1H-pyrazolo[4,3-c]quinoline-8-carboxamide | HATU | 489.1 |
| **315** | 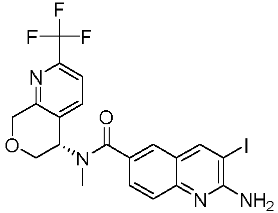 | 2-amino-3-iodo-N-methyl-N-((5S)-2-(trifluoromethyl)-5,8-dihydro-6H-pyrano[3,4-b]pyridin-5-yl)-6-quinolinecarboxamide | TBTU | 529.0 |
| **316** | 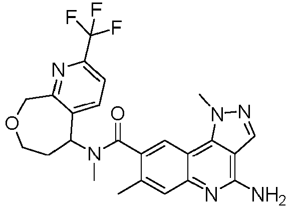 | 4-amino-N,1,7-trimethyl-N-((5R)-2-(trifluoromethyl)-5,6,7,9-tetrahydrooxepino[3,4-b]pyridin-5-yl)-1H-pyrazolo[4,3-c]quinoline-8-carboxamide and 4-amino-N,1,7-trimethyl-N-((5S)-2-(trifluoromethyl)-5,6,7,9-tetrahydrooxepino[3,4-b]pyridin-5-yl)-1H-pyrazolo[4,3-c]quinoline-8-carboxamide | TBTU | 485.2 |

### Example 317 and 318: 4-amino-N-methyl-N-(6-(trifluoromethyl)-2,3-dihydrobenzofuran-3-yl)-1,3-dihydrofuro[3,4-c]quinoline-8-carboxamide

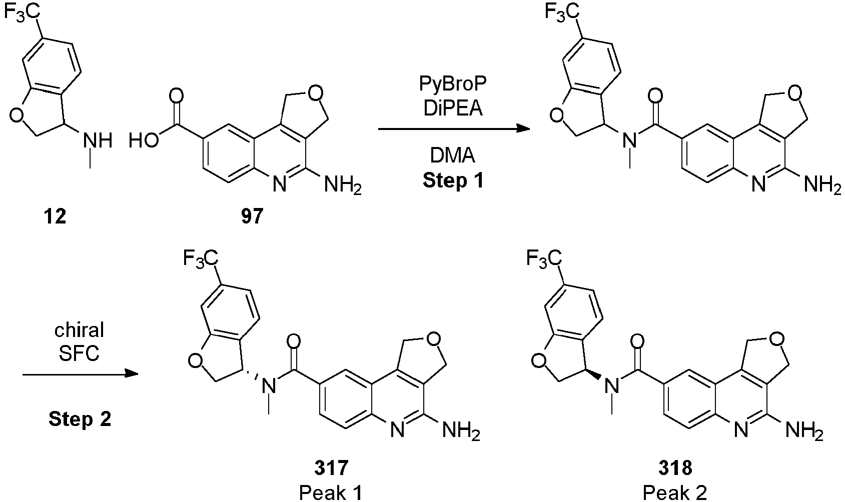

Step 1. To a stirred mixture of 4-amino-1,3-dihydrofuro[3,4-c]quinoline-8-carboxylic acid (97) (82 mg, 0.355 mmol), N-methyl-6-(trifluoromethyl)-2,3-dihydrobenzofuran-3-amine **(12)** (77 mg, 0.355 mmol), and bromotripyrrolidinophosphonium hexafluorophosphate (331 mg, 0.709 mmol, Sigma-Aldrich Corporation) in DMA (2 mL) was added N-ethyl-N-isopropylpropan-2-amine (92 mg, 0.124 mL, 0.709 mmol, Sigma-Aldrich Corporation). The resulting mixture was stirred at rt for 1.5 h. The crude mixture was directly loaded onto a silica gel precolumn (25 g) and subjected to combi-flash column chromatography on a 12-g ISCO gold column eluting with MeOH (with 0.5% ammonium hydroxide)/DCM (0 to 12%) to give 468 mg of an impure 4-amino-N-methyl-N-(6-(trifluoromethyl)-2,3-dihydrobenzofuran-3-yl)-1,3-dihydrofuro[3,4-c]quinoline-8-carboxamide as a nearly colorless film. *m*/*z* (ESI): 430.15 (M+H)⁺.

Step 2. The racemate was purified by Prep SFC using a Chiralpak AS-H column with a mobile phase of 80% Liquid CO₂ and 20% MeOH with TEA using a flow rate of 80 mL/min. The more potent (measured by IC50 in HCT116 MTAP null cell viability assay) enantiomer was assigned as the (S)-; the less potent (measured by IC50 in HCT116 MTAP null cell viability assay) enantiomer was assigned as (R)-. The 1^{st} eluting peak was (S)-4-amino-N-methyl-N-(6-(trifluoromethyl)-2,3-dihydrobenzofuran-3-yl)-1,3-dihydrofuro[3,4-c]quinoline-8-carboxamide **(317)** (62 mg, 0.144 mmol, 40.7% yield), a white solid. ¹H NMR (METHANOL-d₄, 400 MHz) δ 7.83 (s, 1H), 7.6-7.7 (m, 3H), 7.28 (br d, 1H, *J*=7.7 Hz), 7.12 (s, 1H), 5.45 (t, 2H, *J*=3.2 Hz), 5.13 (t, 2H, *J*=3.4 Hz), 4.8-4.9 (m, 1H), 4.7-4.8 (m, 2H), 2.76 (s, 3H). ¹⁹F NMR (METHANOL-d₄, 376 MHz) δ -63.86 (br s, 1F). The 2^{nd} eluting peak was (R)-4-amino-N-methyl-N-(6-(trifluoromethyl)-2,3-dihydrobenzofuran-3-yl)-1,3-dihydrofuro[3,4-c]quinoline-8-carboxamide **(318)** (57 mg, 0.133 mmol, 37.4 % yield), a white solid. ¹H NMR (METHANOL-d₄, 400 MHz) δ 7.85 (s, 1H), 7.6-7.8 (m, 3H), 7.28 (d, 1H, *J=*7.5 Hz), 7.12 (s, 1H), 5.45 (br d, 2H, *J=*3.1 Hz), 5.13 (t, 2H, *J*=3.4 Hz), 4.86 (br s, 1H), 4.6-4.8 (m, 2H), 2.77 (s, 3H). ¹⁹F NMR (METHANOL-d₄, 376 MHz) δ -63.89 (br s, 1F).

Examples in Table 13 were prepared in a manner similar to that described above for example **317** and **318** using the indicated amide coupling reagent in the table and purification conditions.

**Table 13**

| **Ex.** | **Structure** | **Name** | **Coupling Reagent** | **m/z (ESI): (M+H)⁺** | **SFC Conditions** |
|---|---|---|---|---|---|
| **319** | 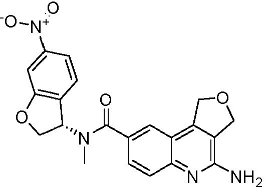 | 4-amino-N-methyl-N-((3S)-6-nitro-2,3-dihydro-1-benzofuran-3-yl)-1,3-dihydrofuro[3,4-c]quinoline-8-carboxamide | PyBrop | 407.05 | 1st peak, Chiral Technologies AS column (250 x 21 mm, 5 mm) with a mobile phase of 65% Liquid CO₂ and 35% MeOH with 0.2% TEA using a flow rate of 60 mL/min |
| **320** | 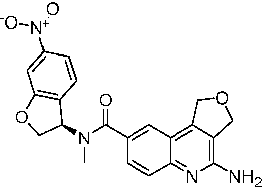 | 4-amino-N-methyl-N-((3R)-6-nitro-2,3-dihydro-1-benzofuran-3-yl)-1,3-dihydrofuro[3,4-c]quinoline-8-carboxamide | PyBrop | 407.05 | 2nd peak, Chiral Technologies AS column (250 x 21 mm, 5 mm) with a mobile phase of 65% Liquid CO₂ and 35% MeOH with 0.2% TEA using a flow rate of 60 mL/min |
| **321** | 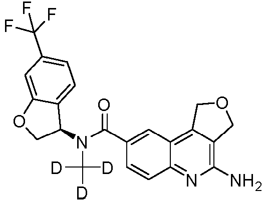 | 4-amino-N-(~2~H_3_)methyl-N-((3R)-6-(trifluoromethyl)-2,3-dihydro-1-benzofuran-3-yl)-1,3-dihydrofuro[3,4-c]quinoline-8-carboxamide | PyBrop | 433.15 | 2nd peak, Chiralpak AS-H column (250 x 21 mm, 5 µm) with a mobile phase of 85% Liquid CO₂ and 15% MeOH with 0.2% TEA using a flow rate of 80 mL/min |
| **322** | 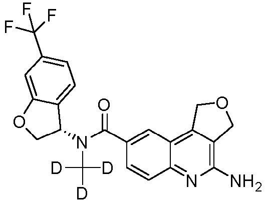 | 4-amino-N-(~2~H_3_)methyl-N-((3S)-6-(trifluoromethyl)-2,3-dihydro-1-benzofuran-3-yl)-1,3-dihydrofuro[3,4-c]quinoline-8-carboxamide | PyBrop | 433.15 | 1st peak, Chiralpak AS-H column (250 x 21 mm, 5 µm) with a mobile phase of 85% Liquid CO₂ and 15% MeOH with 0.2% TEA using a flow rate of 80 mL/min |
| **323** | 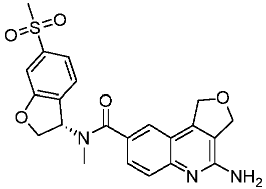 | 4-amino-N-methyl-N-((3S)-6-(methylsulfonyl)-2,3-dihydro-1-benzofuran-3-yl)-1,3-dihydrofuro[3,4-c]quinoline-8-carboxamide | PyBrop | 440.2 | 1st peak, Chiral Technologies AS column (150 x 30 mm, 5 mm) with a mobile phase of 65% Liquid CO₂ and 35% MeOH with 0.2% TEA using a flow rate of 80 mL/min |
| **324** | 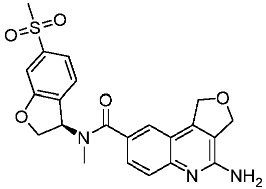 | 4-amino-N-methyl-N-((3R)-6-(methylsulfonyl)-2,3-dihydro-1-benzofuran-3-yl)-1,3-dihydrofuro[3,4-c]quinoline-8-carboxamide | PyBrop | 440.2 | 2nd peak, Chiral Technologies AS column (150 x 30 mm, 5 mm) with a mobile phase of 65% Liquid CO₂ and 35% MeOH with 0.2% TEA using a flow rate of 80 mL/min |
| **325** | 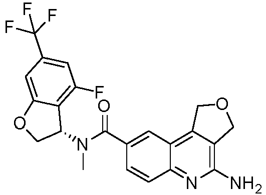 | 4-amino-N-((3S)-4-fluoro-6-(trifluoromethyl)-2,3-dihydro-1-benzofuran-3-yl)-N-methyl-1,3-dihydrofuro[3,4-c]quinoline-8-carboxamide | PyBrop | 448.2 | 1st peak, Chiralpak IF column (21 x 150 mm, 5 micron) with a mobile phase of 65% Liquid CO₂ and 35% MeOH with 0.2% diethylamine using a flow rate of 80 mL/min |
| **326** | 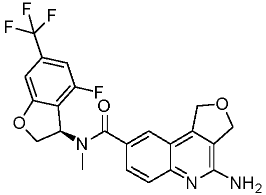 | 4-amino-N-((3R)-4-fluoro-6-(trifluoromethyl)-2,3-dihydro-1-benzofuran-3-yl)-N-methyl-1,3-dihydrofuro[3,4-c]quinoline-8-carboxamide | PyBrop | 448.2 | 2nd peak, Chiralpak IF column (21 x 150 mm, 5 micron) with a mobile phase of 65% Liquid CO₂ and 35% MeOH with 0.2% diethylamine using a flow rate of 80 mL/min |
| **327** | 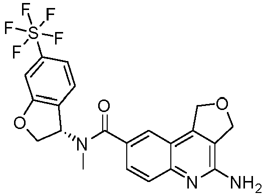 | 4-amino-N-methyl-N-((3S)-6-(pentafluoro-lambda~6~-sulfanyl)-2,3-dihydro-1-benzofuran-3-yl)-1,3-dihydrofuro[3,4-c]quinoline-8-carboxamide | TBTU | 488.8 | 2nd peak, Chiralpak AD column (21 x 150 mm, 5 micron) with a mobile phase of 50% Liquid CO₂ and 50% MeOH with 0.2% diethylamine using a flow rate of 80 mL/min |
| **328** | 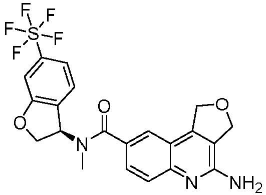 | 4-amino-N-methyl-N-((3R)-6-(pentafluoro-lambda~6~-sulfanyl)-2,3-dihydro-1-benzofuran-3-yl)-1,3-dihydrofuro[3,4-c]quinoline-8-carboxamide | TBTU | 488.8 | 1st peak, Chiralpak AD column (21 x 150 mm, 5 micron) with a mobile phase of 50% Liquid CO₂ and 50% MeOH with 0.2% diethylamine using a flow rate of 80 mL/min |
| **329** | 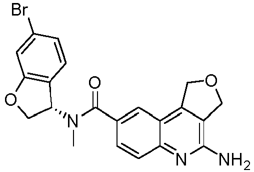 | 4-amino-N-((3S)-6-bromo-2,3-dihydro-1-benzofuran-3-yl)-N-methyl-1,3-dihydrofuro[3,4-c]quinoline-8-carboxamide | HATU | 440, 442 | 1st peak, Chiralpak AS column (250 x 21 mm, 5 µm) with a mobile phase of 65% Liquid CO₂ and 35% MeOH with 0.2% TEA using a flow rate 80 mL/min |
| **330** | 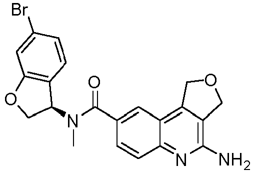 | 4-amino-N-((3R)-6-bromo-2,3-dihydro-1-benzofuran-3-yl)-N-methyl-1,3-dihydrofuro[3,4-c]quinoline-8-carboxamide | HATU | 440, 442 | 2nd peak, Chiralpak AS column (250 x 21 mm, 5 µm) with a mobile phase of 65% Liquid CO₂ and 35% MeOH with 0.2% TEA using a flow rate 80 mL/min |
| **331** | 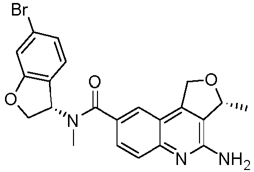 | (3R)-4-amino-N-((3S)-6-bromo-2,3-dihydro-1-benzofuran-3-yl)-N,3-dimethyl-1,3-dihydrofuro[3,4-c]quinoline-8-carboxamide | HATU | 454, 456 | 1st peak, Chiral Technologies AS column (250 x 21 mm, 5 mm) with a mobile phase of 65% Liquid CO₂ and 35% MeOH with 0.2% TEA using a flow rate of 80 mL/min |
| **332** | 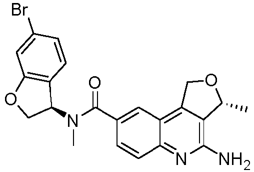 | (3R)-4-amino-N-((3R)-6-bromo-2,3-dihydro-1-benzofuran-3-yl)-N,3-dimethyl-1,3-dihydrofuro[3,4-c]quinoline-8-carboxamide | HATU | 454, 456 | 2nd peak, Chiral Technologies AS column (250 x 21 mm, 5 mm) with a mobile phase of 65% Liquid CO₂ and 35% MeOH with 0.2% TEA using a flow rate of 80 mL/min |
| **333** | 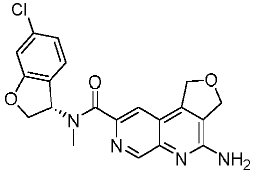 | 4-amino-N-((3S)-6-chloro-2,3-dihydro-1-benzofuran-3-yl)-N-methyl-1,3-dihydrofuro[3,4-c][1,7]naphthyridi ne-8-carboxamide | TBTU | 397.2 | 1st peak, Chiral Technologies AS column (250 x 21 mm, 5 mm) with a mobile phase of 70% Liquid CO₂ and 30% MeOH with 0.2% TEA using a flow rate of 65 mL/min |
| **334** | 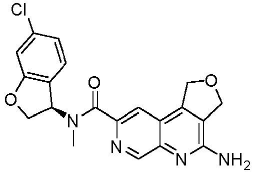 | 4-amino-N-((3R)-6-chloro-2,3-dihydro-1-benzofuran-3-yl)-N-methyl-1,3-dihydrofuro[3,4-c][1, 7]naphthyridi ne-8-carboxamide | TBTU | 397.2 | 2nd peak, Chiral Technologies AS column (250 x 21 mm, 5 mm) with a mobile phase of 70% Liquid CO₂ and 30% MeOH with 0.2% TEA using a flow rate of 65 mL/min |
| **335** | 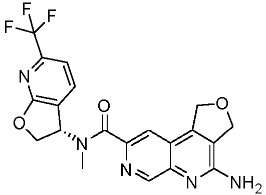 | 4-amino-N-methyl-N-((3S)-6-(trifluoromethyl)-2,3-dihydrofuro[2,3-b]pyridin-3-yl)-1,3-dihydrofuro[3,4-c][1,7]naphthyridi ne-8-carboxamide | PyBrop | 431.8 | 1st peak, Chiral Technologies AD column (250 x 21 mm, 5 mm) with a mobile phase of 65% Liquid CO₂ and 35% MeOH with 0.2% TEA using a flow rate of 80 mL/min |
| **336** | 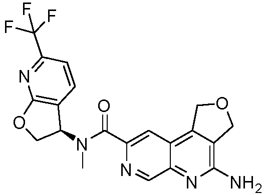 | 4-amino-N-methyl-N-((3R)-6-(trifluoromethyl)-2,3-dihydrofuro[2,3-b]pyridin-3-yl)-1,3-dihydrofuro[3,4-c][1, 7]naphthyridi ne-8-carboxamide | PyBrop | 431.8 | 2nd peak, Chiral Technologies AD column (250 x 21 mm, 5 mm) with a mobile phase of 65% Liquid CO₂ and 35% MeOH with 0.2% TEA using a flow rate of 80 mL/min |
| **337** | 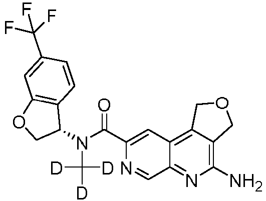 | 4-amino-N-(~2~H_3_)methyl-N-((3S)-6-(trifluoromethyl)-2,3-dihydro-1-benzofuran-3-yl)-1,3-dihydrofuro[3,4-c][1,7]naphthyridi ne-8-carboxamide | PyBrop | 433.8 | 1st peak, Chiralpak AS column (3 x 25 cm, 5 micron) with a mobile phase of 75% Liquid CO₂ and 25% MeOH with 0.2% TEA using a flow rate of 200 mL/min |
| **338** | 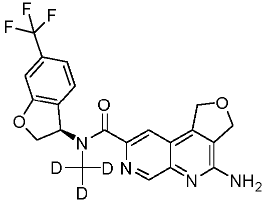 | 4-amino-N-(~2~H_3_)methyl-N-((3R)-6-(trifluoromethyl)-2,3-dihydro-1-benzofuran-3-yl)-1,3-dihydrofuro[3,4-c][1,7]naphthyridi ne-8-carboxamide | PyBrop | 433.8 | 2nd peak, Chiralpak AS column (3 x 25 cm, 5 micron) with a mobile phase of 75% Liquid CO₂ and 25% MeOH with 0.2% TEA using a flow rate of 200 mL/min |
| **339** | 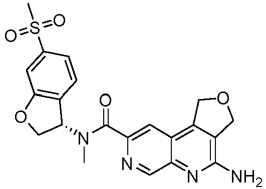 | 4-amino-N-methyl-N-((3S)-6-(methylsulfonyl)-2,3-dihydro-1-benzofuran-3-yl)-1,3-dihydrofuro[3,4-c][1, 7]naphthyridi ne-8-carboxamide | PyBroP | 440.95 | 1st peak, Chiralcel OJ column (250 x 21 mm, 5 µm) with a mobile phase of 75% Liquid CO₂ and 25% ethanol with 0.2% TEA using a flow rate 80 mL/min |
| **340** | 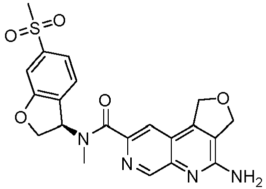 | 4-amino-N-methyl-N-((3R)-6-(methylsulfonyl)-2,3-dihydro-1-benzofuran-3-yl)-1,3-dihydrofuro[3,4-c][1, 7]naphthyridi ne-8-carboxamide | PyBroP | 440.95 | 2nd peak, Chiralcel OJ column (250 x 21 mm, 5 µm) with a mobile phase of 75% Liquid CO₂ and 25% ethanol with 0.2% TEA using a flow rate 80 mL/min |
| **341** | 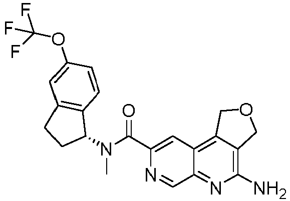 | 4-amino-N-methyl-N-((1R)-5-(trifluoromethoxy) -2,3-dihydro-1H-inden-1-yl)-1,3-dihydrofuro[3,4-c][1,7]naphthyridi ne-8-carboxamide | PyBrop | 445.15 | 1st peak, Chiralpak AS column (3 x 250 mm, 5 micron) with a mobile phase of 90% Liquid CO₂ and 10% MeOH with 0.2% TEA using a flow rate of 200 mL/min |
| **342** | 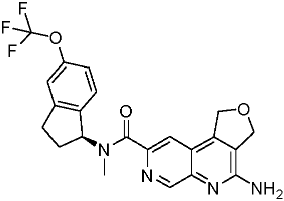 | 4-amino-N-methyl-N-((1S)-5-(trifluoromethoxy) -2,3-dihydro-1H-inden-1-yl)-1,3-dihydrofuro[3,4-c][1,7]naphthyridi ne-8-carboxamide | PyBrop | 445.15 | 2nd peak, Chiralpak AS column (3 x 250 mm, 5 micron) with a mobile phase of 90% Liquid CO₂ and 10% MeOH with 0.2% TEA using a flow rate of 200 mL/min |
| **343** | 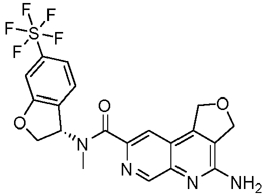 | 4-amino-N-methyl-N-((3S)-6-(pentafluoro-lambda~6~-sulfanyl)-2,3-dihydro-1-benzofuran-3-yl)-1,3-dihydrofuro[3,4-c][1,7]naphthyridi ne-8-carboxamide | TBTU | 489.8 | 1st peak, Chiralpak OD column (21 x 150 mm, 5 micron) with a mobile phase of 75% Liquid CO₂ and 25% MeOH with 0.2% diethylamine using a flow rate of 80 mL/min |
| **344** | 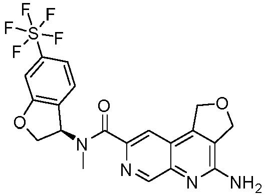 | 4-amino-N-methyl-N-((3R)-6-(pentafluoro-lambda~6~-sulfanyl)-2,3-dihydro-1-benzofuran-3-yl)-1,3-dihydrofuro[3,4-c][1,7]naphthyridi ne-8-carboxamide | TBTU | 489.8 | 2nd peak, Chiralpak OD column (21 x 150 mm, 5 micron) with a mobile phase of 75% Liquid CO₂ and 25% MeOH with 0.2% diethylamine using a flow rate of 80 mL/min |
| **345** | 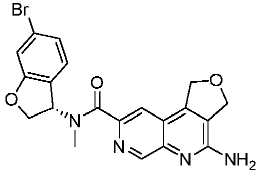 | 4-amino-N-((3S)-6-bromo-2,3-dihydro-1-benzofuran-3-yl)-N-methyl-1,3-dihydrofuro[3,4-c][1,7]naphthyridi ne-8-carboxamide | PyBrop | 441.0, 443.0 | 1st peak, Chiral Technologies AS column (250 x 30 mm, 5 mm) with a mobile phase of 65% Liquid CO₂ and 35% MeOH with 0.2% TEA using a flow rate of 150 mL/min |
| **346** | 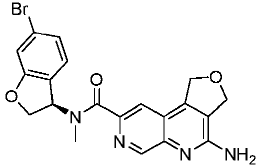 | 4-amino-N-((3R)-6-bromo-2,3-dihydro-1-benzofuran-3-yl)-N-methyl-1,3-dihydrofuro[3,4-c][1,7]naphthyridi ne-8-carboxamide | PyBrop | 441.0, 443.0 | 2nd peak, Chiral Technologies AS column (250 x 30 mm, 5 mm) with a mobile phase of 65% Liquid CO₂ and 35% MeOH with 0.2% TEA using a flow rate of 150 mL/min |
| **347** | 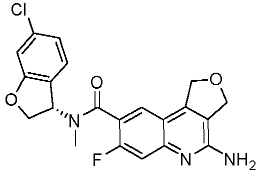 | 4-amino-N-((3S)-6-chloro-2,3-dihydro-1-benzofuran-3-yl)-7-fluoro-N-methyl-1,3-dihydrofuro[3,4-c]quinoline-8-carboxamide | TBTU | 414.0 | 1st peak, Chiral Technologies AS column (250 x 21 mm, 5 mm) with a mobile phase of 70% Liquid CO₂ and 30% MeOH with 0.2% TEA using a flow rate of 65 mL/min |
| **348** | 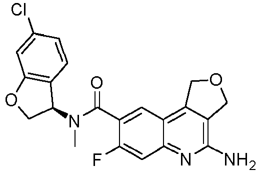 | 4-amino-N-((3R)-6-chloro-2,3-dihydro-1-benzofuran-3-yl)-7-fluoro-N-methyl-1,3-dihydrofuro[3,4-c]quinoline-8-carboxamide | TBTU | 414.0 | 2nd peak, Chiral Technologies AS column (250 x 21 mm, 5 mm) with a mobile phase of 70% Liquid CO₂ and 30% MeOH with 0.2% TEA using a flow rate of 65 mL/min |
| **349** | 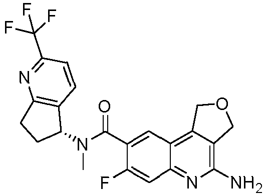 | 4-amino-7-fluoro-N-methyl-N-((5R)-2-(trifluoromethyl)-6,7-dihydro-5H-cyclopenta[b]pyrid in-5-yl)-1,3-dihydrofuro[3,4-c]quinoline-8-carboxamide | PyBrop | 447.2 | 1st peak, Chiral Technologies OD column (250 x 21 mm, 5 mm) with a mobile phase of 85% Liquid CO₂ and 15% MeOH with 0.2% TEA using a flow rate of 80 mL/min |
| **350** | 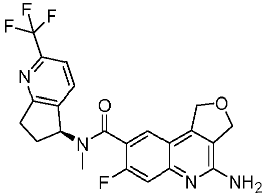 | 4-amino-7-fluoro-N-methyl-N-((5S)-2-(trifluoromethyl)-6,7-dihydro-5H-cyclopenta[b]pyrid in-5-yl)-1,3-dihydrofuro[3,4-c]quinoline-8-carboxamide | PyBrop | 447.2 | 2nd peak, Chiral Technologies OD column (250 x 21 mm, 5 mm) with a mobile phase of 85% Liquid CO₂ and 15% MeOH with 0.2% TEA using a flow rate of 80 mL/min |
| **351** | 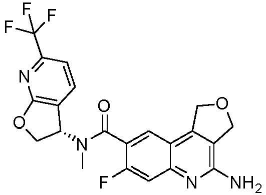 | 4-amino-7-fluoro-N-methyl-N-((3S)-6-(trifluoromethyl)-2,3-dihydrofuro[2,3-b]pyridin-3-yl)-1,3-dihydrofuro[3,4-c]quinoline-8-carboxamide | PyBroP | 449.2 | 1st peak, Chiralpak IG column (21 x 150 mm, 5 micron) with a mobile phase of 55% Liquid CO₂ and 45% MeOH with 0.2% diethylamine using a flow rate of 80 mL/min |
| **352** | 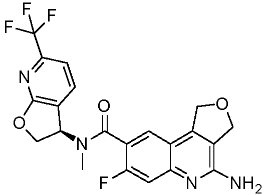 | 4-amino-7-fluoro-N-methyl-N-((3R)-6-(trifluoromethyl)-2,3-dihydrofuro[2,3-b]pyridin-3-yl)-1,3-dihydrofuro[3,4-c]quinoline-8-carboxamide | PyBroP | 449.2 | 2nd peak, Chiralpak IG column (21 x 150 mm, 5 micron) with a mobile phase of 55% Liquid CO₂ and 45% MeOH with 0.2% diethylamine using a flow rate of 80 mL/min |
| **353** | 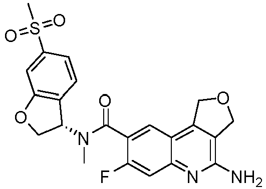 | 4-amino-7-fluoro-N-methyl-N-((3S)-6-(methylsulfonyl)-2,3-dihydro-1-benzofuran-3-yl)-1,3-dihydrofuro[3,4-c]quinoline-8-carboxamide | PyBroP | 457.75 | 1st peak, Chiralpak OJ column (21 x 150 mm, 5 micron) with a mobile phase of 55% Liquid CO₂ and 45% MeOH with 0.2% diethylamine using a flow rate of 80 mL/min |
| **354** | 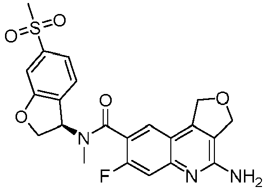 | 4-amino-7-fluoro-N-methyl-N-((3R)-6-(methylsulfonyl)-2,3-dihydro-1-benzofuran-3-yl)-1,3-dihydrofuro[3,4-c]quinoline-8-carboxamide | PyBroP | 457.75 | 2nd peak, Chiralpak OJ column (21 x 150 mm, 5 micron) with a mobile phase of 55% Liquid CO₂ and 45% MeOH with 0.2% diethylamine using a flow rate of 80 mL/min |
| **355** | 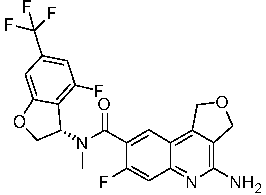 | 4-amino-7-fluoro-N-((3S)-4-fluoro-6-(trifluoromethyl)-2,3-dihydro-1-benzofuran-3-yl)-N-methyl-1,3-dihydrofuro[3,4-c]quinoline-8-carboxamide | PyBrop | 466.2 | 2nd peak, Regis (S,S) Whelk-01 column (250 x 21 mm, 5 mm) with a mobile phase of 80% Liquid CO₂ and 20% MeOH with 0.2% TEA using a flow rate of 100 mL/min |
| **356** | 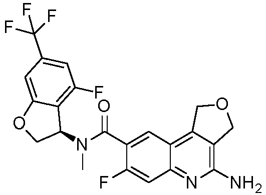 | 4-amino-7-fluoro-N-((3R)-4-fluoro-6-(trifluoromethyl)-2,3-dihydro-1-benzofuran-3-yl)-N-methyl-1,3-dihydrofuro[3,4-c]quinoline-8-carboxamide | PyBrop | 466.2 | 1st peak, Regis (S,S) Whelk-01 column (250 x 21 mm, 5 mm) with a mobile phase of 80% Liquid CO₂ and 20% MeOH with 0.2% TEA using a flow rate of 100 mL/min |
| **357** | 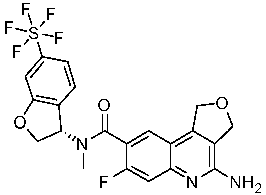 | 4-amino-7-fluoro-N-methyl-N-((3S)-6-(pentafluoro-lambda~6~-sulfanyl)-2,3-dihydro-1-benzofuran-3-yl)-1,3-dihydrofuro[3,4-c]quinoline-8-carboxamide | TBTU | 506.1 | 1st peak, Chiralcel OD column (21 x 150 mm, 5 micron) with a mobile phase of 75% Liquid CO₂ and 25% MeOH with 0.2% diethylamine using a flow rate of 80 mL/min |
| **358** | 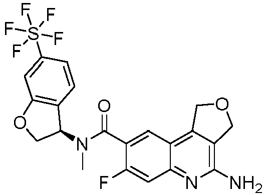 | 4-amino-7-fluoro-N-methyl-N-((3R)-6-(pentafluoro-lambda~6~-sulfanyl)-2,3-dihydro-1-benzofuran-3-yl)-1,3-dihydrofuro[3,4-c]quinoline-8-carboxamide | TBTU | 506.1 | 2nd peak, Chiralcel OD column (21 x 150 mm, 5 micron) with a mobile phase of 75% Liquid CO₂ and 25% MeOH with 0.2% diethylamine using a flow rate of 80 mL/min |
| **359** | 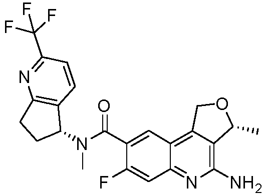 | (3R)-4-amino-7-fluoro-N,3-dimethyl-N-((5R)-2-(trifluoromethyl)-6,7-dihydro-5H-cyclopenta[b]pyrid in-5-yl)-1,3-dihydrofuro[3,4-c]quinoline-8-carboxamide | PyBroP | 461.2 | 1st peak, Chiral Technologies OD column (250 x 21 mm, 5 mm) with a mobile phase of 85% Liquid CO₂ and 15% MeOH with 0.2% TEA using a flow rate of 80 mL/min |
| **360** | 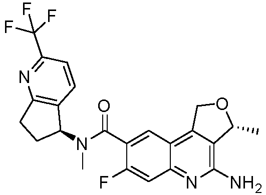 | (3R)-4-amino-7-fluoro-N,3-dimethyl-N-((5S)-2-(trifluoromethyl)-6,7-dihydro-5H-cyclopenta[b]pyrid in-5-yl)-1,3-dihydrofuro[3,4-c]quinoline-8-carboxamide | PyBroP | 461.2 | 2nd peak, Chiral Technologies OD column (250 x 21 mm, 5 mm) with a mobile phase of 85% Liquid CO₂ and 15% MeOH with 0.2% TEA using a flow rate of 80 mL/min |
| **361** | 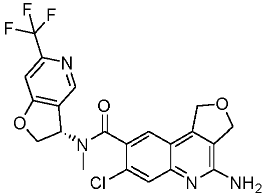 | 4-amino-7-chloro-N-methyl-N-((3S)-6-(trifluoromethyl)-2,3-dihydrofuro[3,2-c]pyridin-3-yl)-1,3-dihydrofuro[3,4-c]quinoline-8-carboxamide | TBTU | 465.2 | 1st peak, Chiralpak IC column (21 x 250 mm) with a mobile phase of 60% Liquid CO₂ and 40% MeOH with 0.2% diethylamine using a flow rate of 80 mL/min |
| **362** | 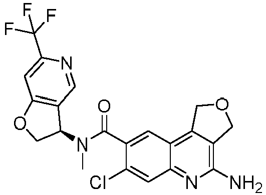 | 4-amino-7-chloro-N-methyl-N-((3R)-6-(trifluoromethyl)-2,3-dihydrofuro[3,2-c]pyridin-3-yl)-1,3-dihydrofuro[3,4-c]quinoline-8-carboxamide | TBTU | 465.2 | 2nd peak, Chiralpak IC column (21 x 250 mm) with a mobile phase of 60% Liquid CO₂ and 40% MeOH with 0.2% diethylamine using a flow rate of 80 mL/min |
| **363** | 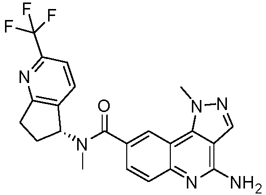 | 4-amino-N,1-dimethyl-N-((5R)-2-(trifluoromethyl)-6,7-dihydro-5H-cyclopenta [b]pyrid in-5-yl)-1H-pyrazolo[4,3-c]quinoline-8-carboxamide | PyBrop | 440.95 | 2nd peak, Chiral Technologies AD column (250 x 21 mm, 5 mm) with a mobile phase of 65% Liquid CO₂ and 35% iPrOH with 0.2% TEA using a flow rate of 70 mL/min |
| **364** | 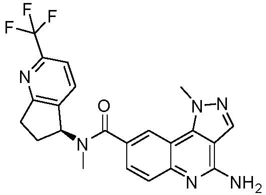 | 4-amino-N,1-dimethyl-N-((5S)-2-(trifluoromethyl)-6,7-dihydro-5H-cyclopenta [b]pyrid in-5-yl)-1H-pyrazolo[4,3-c]quinoline-8-carboxamide | PyBrop | 440.95 | 1st peak, Chiral Technologies AD column (250 x 21 mm, 5 mm) with a mobile phase of 65% Liquid CO₂ and 35% iPrOH with 0.2% TEA using a flow rate of 70 mL/min |
| **365** | 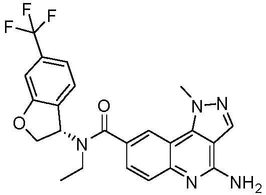 | 4-amino-N-ethyl-1-methyl-N-((3S)-6-(trifluoromethyl)-2,3-dihydro-1-benzofuran-3-yl)-1H-pyrazolo[4,3-c]quinoline-8-carboxamide | HATU | 456 | 1st peak, Chiralcel OJ column (2 x 25 cm, 5 micron) with a mobile phase of 80% Liquid CO₂ and 20% MeOH with 0.1% TEA using a flow rate of 70 mL/min |
| **366** | 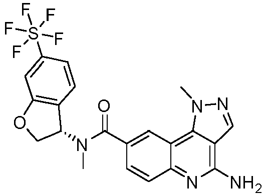 | 4-amino-N,1-dimethyl-N-((3S)-6-(pentafluoro-lambda~6~-sulfanyl)-2,3-dihydro-1-benzofuran-3-yl)-1H-pyrazolo[4,3-c]quinoline-8-carboxamide | TBTU | 500.2 | 1st peak, Chiralpak AZ column (21 x 250 mm, 5 micron) with a mobile phase of 60% Liquid CO₂ and 40% MeOH using a flow rate of 80 mL/min |
| **367** | 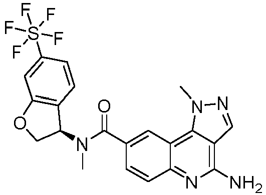 | 4-amino-N,1-dimethyl-N-((3R)-6-(pentafluoro-lambda~6~-sulfanyl)-2,3-dihydro-1-benzofuran-3-yl)-1H-pyrazolo[4,3-c]quinoline-8-carboxamide | TBTU | 500.2 | 2nd peak, Chiralpak AZ column (21 x 250 mm, 5 micron) with a mobile phase of 60% Liquid CO₂ and 40% MeOH using a flow rate of 80 mL/min |
| **368** | 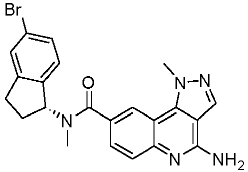 | 4-amino-N-((1R)-5-bromo-2,3-dihydro-1H-inden-1-yl)-N,1-dimethyl-1H-pyrazolo[4,3-c]quinoline-8-carboxamide | HATU | 450, 452 | 2nd peak, AD-H column (25 x 3 cm) with a mobile phase of 60% Liquid CO₂ and 40% MeOH with 0.2% diethylamine |
| **369** | 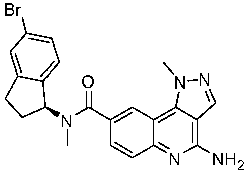 | 4-amino-N-((1S)-5-bromo-2,3-dihydro-1H-inden-1-yl)-N,1-dimethyl-1H-pyrazolo[4,3-c]quinoline-8-carboxamide | HATU | 450, 452 | 1st peak, AD-H column (25 x 3 cm) with a mobile phase of 60% Liquid CO₂ and 40% MeOH with 0.2% diethylamine |
| **370** | 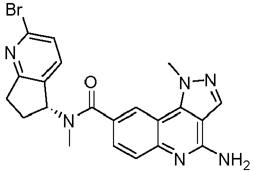 | 4-amino-N-((5R)-2-bromo-6,7-dihydro-5H-cyclopenta [b]pyrid in-5-yl)-N,1-dimethyl-1H-pyrazolo[4,3-c]quinoline-8-carboxamide | HATU | 451, 453 | 1st peak, Chiralcel OJ column (2 x 25 cm, 5 micron) with a mobile phase of 75% Liquid CO₂ and 25% MeOH with 0.1% diethylamine using a flow rate of 55 mL/min |
| **371** | 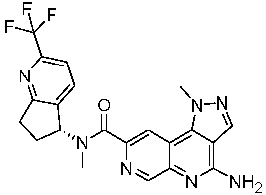 | 4-amino-N,1-dimethyl-N-((5R)-2-(trifluoromethyl)-6,7-dihydro-5H-cyclopenta[b]pyrid in-5-yl)-1H-pyrazolo[4,3-c][1,7]naphthyridi ne-8-carboxamide | PyBrop | 442.2 | 1st peak, Chiralcel OD column (21 x 150 mm, 5 micron) with a mobile phase of 65% Liquid CO₂ and 35% MeOH with 0.2% diethylamine using a flow rate of 45 mL/min |
| **372** | 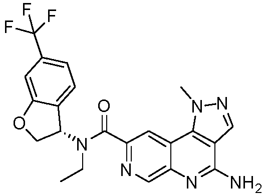 | 4-amino-N-ethyl-1-methyl-N-((3R)-6-(trifluoromethyl)-2,3-dihydro-1-benzofuran-3-yl)-1H-pyrazolo[4,3-c][1,7]naphthyridi ne-8-carboxamide | HATU | 457 | 2nd peak, Chiralcel OJ column (21 x 150 mm, 5 micron) with a mobile phase of 90% Liquid CO₂ and 10% MeOH with 0.2% diethylamine using a flow rate of 80 mL/min |
| **373** | 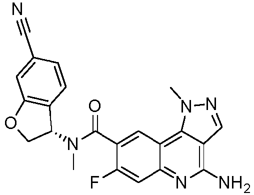 | 4-amino-N-((3S)-6-cyano-2,3-dihydro-1-benzofuran-3-yl)-7-fluoro-N,1-dimethyl-1H-pyrazolo[4,3-c]quinoline-8-carboxamide | TBTU | 417.1 | 1st peak, Chiralpak AS column (2 x 25 cm, 5 micron) with a mobile phase of 80% Liquid CO₂ and 20% MeOH with 0.1% diethylamine using a flow rate of 65 mL/min |
| **374** | 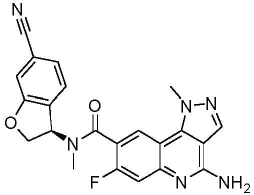 | 4-amino-N-((3R)-6-cyano-2,3-dihydro-1-benzofuran-3-yl)-7-fluoro-N,1-dimethyl-1H-pyrazolo[4,3-c]quinoline-8-carboxamide | TBTU | 417.1 | 2nd peak, Chiralpak AS column (2 x 25 cm, 5 micron) with a mobile phase of 80% Liquid CO₂ and 20% MeOH with 0.1% diethylamine using a flow rate of 65 mL/min |
| **375** | 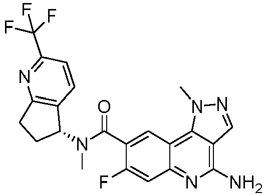 | 4-amino-7-fluoro-N,1-dimethyl-N-((5R)-2-(trifluoromethyl)-6,7-dihydro-5H-cyclopenta [b]pyrid in-5-yl)-1H-pyrazolo[4,3-c]quinoline-8-carboxamide | PyBrop | 458.8 | 2nd peak, Chiral Technologies AD column (250 x 21 mm, 5 mm) with a mobile phase of 75% Liquid CO₂ and 25% iPrOH with 0.2% TEA using a flow rate of 80 mL/min |
| **376** | 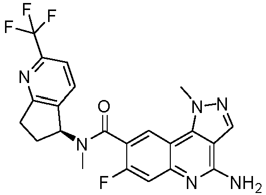 | 4-amino-7-fluoro-N,1-dimethyl-N-((5S)-2-(trifluoromethyl)-6,7-dihydro-5H-cyclopenta [b]pyrid in-5-yl)-1H-pyrazolo[4,3-c]quinoline-8-carboxamide | PyBrop | 458.8 | 1st peak, Chiral Technologies AD column (250 x 21 mm, 5 mm) with a mobile phase of 75% Liquid CO₂ and 25% iPrOH with 0.2% TEA using a flow rate of 80 mL/min |
| **377** | 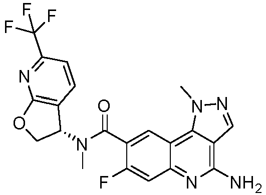 | 4-amino-7-fluoro-N,1-dimethyl-N-((3S)-6-(trifluoromethyl)-2,3-dihydrofuro[2,3-b]pyridin-3-yl)-1H-pyrazolo[4,3-c]quinoline-8-carboxamide | PyBrop | 461 | 2nd peak, Chiralpak IC column (21 x 150 mm, 5 micron) with a mobile phase of 55% Liquid CO₂ and 45% MeOH with 0.2% diethylamine using a flow rate of 80 mL/min |
| **378** | 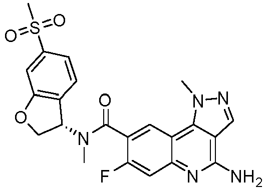 | 4-amino-7-fluoro-N,1-dimethyl-N-((3S)-6-(methylsulfonyl)-2,3-dihydro-1-benzofuran-3-yl)-1H-pyrazolo[4,3-c]quinoline-8-carboxamide | TBTU | 469.8 | 1st peak, Chiralpak AS-H column (250 x 21 mm, 5 um) with a mobile phase of 70% Liquid CO₂ and 30% MeOH with 0.2% TEA using a flow rate of 80 mL/min |
| **379** | 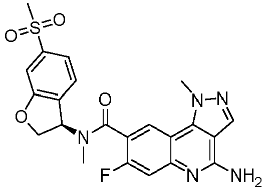 | 4-amino-7-fluoro-N,1-dimethyl-N-((3R)-6-(methylsulfonyl)-2,3-dihydro-1-benzofuran-3-yl)-1H-pyrazolo[4,3-c]quinoline-8-carboxamide | TBTU | 469.9 | 2nd peak, Chiralpak AS-H column (250 x 21 mm, 5 um) with a mobile phase of 70% Liquid CO₂ and 30% MeOH with 0.2% TEA using a flow rate of 80 mL/min |
| **380** | 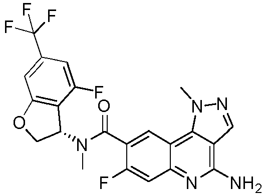 | 4-amino-7-fluoro-N-((3S)-4-fluoro-6-(trifluoromethyl)-2,3-dihydro-1-benzofuran-3-yl)-N,1-dimethyl-1H-pyrazolo[4,3-c]quinoline-8-carboxamide | PyBrop | 477.9 | 1st peak, Chiralpak IG column (21 x 500 mm) with a mobile phase of 75% Liquid CO₂ and 25% isopropanol with 0.2% diethylamine using a flow rate of 40 mL/min |
| **381** | 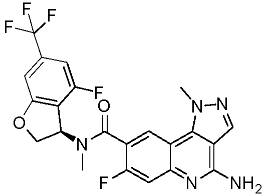 | 4-amino-7-fluoro-N-((3R)-4-fluoro-6-(trifluoromethyl)-2,3-dihydro-1-benzofuran-3-yl)-N,1-dimethyl-1H-pyrazolo[4,3-c]quinoline-8-carboxamide | PyBrop | 477.9 | 2nd peak, Chiralpak IG column (21 x 500 mm) with a mobile phase of 75% Liquid CO₂ and 25% isopropanol with 0.2% diethylamine using a flow rate of 40 mL/min |
| **382** | 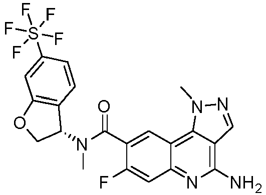 | 4-amino-7-fluoro-N,1-dimethyl-N-((3S)-6-(pentafluoro-lambda~6~-sulfanyl)-2,3-dihydro-1-benzofuran-3-yl)-1H-pyrazolo[4,3-c]quinoline-8-carboxamide | TBTU | 518.2 | 1st peak, Chiralcel OJ column (21 x 550 mm, 5 micron) with a mobile phase of 75% Liquid CO₂ and 25% MeOH with 0.2% diethylamine using a flow rate of 40 mL/min |
| **383** | 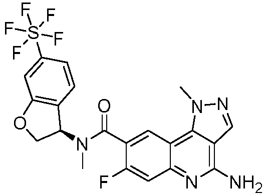 | 4-amino-7-fluoro-N,1-dimethyl-N-((3R)-6-(pentafluoro-lambda~6~-sulfanyl)-2,3-dihydro-1-benzofuran-3-yl)-1H-pyrazolo[4,3-c]quinoline-8-carboxamide | TBTU | 518.2 | 2nd peak, Chiralcel OJ column (21 x 550 mm, 5 micron) with a mobile phase of 75% Liquid CO₂ and 25% MeOH with 0.2% diethylamine using a flow rate of 40 mL/min |
| **384** | 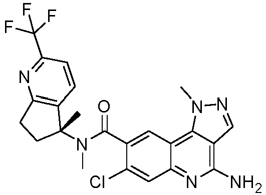 | 4-amino-7-chloro-N,1-dimethyl-N-((5R)-5-methyl-2-(trifluoromethyl)-6,7-dihydro-5H-cyclopenta [b]pyrid in-5-yl)-1H-pyrazolo[4,3-c]quinoline-8-carboxamide | PyBrop | 459 | 1st peak, Chiralpak IG column (21 x 250 mm) with a mobile phase of 65% Liquid CO₂ and 35% MeOH with 0.2% diethylamine using a flow rate of 80 mL/min |
| **385** | 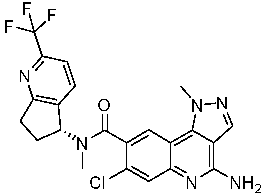 | 4-amino-7-chloro-N,1-dimethyl-N-((5R)-2-(trifluoromethyl)-6,7-dihydro-5H-cyclopenta [b]pyrid in-5-yl)-1H-pyrazolo[4,3-c]quinoline-8-carboxamide | PyBrop | 475.2 | 1st peak, Chiralcel OD column (21 x 500 mm, 5 micron) with a mobile phase of 75% Liquid CO₂ and 25% (1:1) acetonitrile:MeO H with 0.2% diethylamine using a flow rate of 70 mL/min |
| **386** | 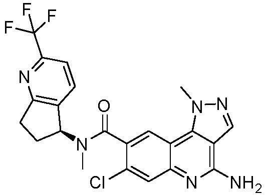 | 4-amino-7-chloro-N,1-dimethyl-N-((5S)-2-(trifluoromethyl)-6,7-dihydro-5H-cyclopenta [b]pyrid in-5-yl)-1H-pyrazolo[4,3-c]quinoline-8-carboxamide | PyBrop | 475.2 | 2nd peak, Chiralcel OD column (21 x 500 mm, 5 micron) with a mobile phase of 75% Liquid CO₂ and 25% (1:1) acetonitrile:MeO H with 0.2% diethylamine using a flow rate of 70 mL/min |
| **387** | 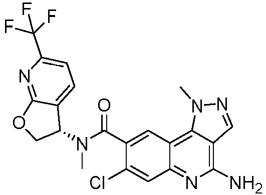 | 4-amino-7-chloro-N,1-dimethyl-N-((3S)-6-(trifluoromethyl)-2,3-dihydrofuro[2,3-b]pyridin-3-yl)-1H-pyrazolo[4,3-c]quinoline-8-carboxamide | PyBrop | 477.2 | 2nd peak, Chiralpak IC column (21 x 500 mm, 5 micron) with a mobile phase of 50% Liquid CO₂ and 50% MeOH with 0.2% diethylamine using a flow rate of 45 mL/min |
| **388** | 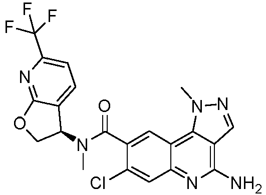 | 4-amino-7-chloro-N,1-dimethyl-N-((3R)-6-(trifluoromethyl)-2,3-dihydrofuro[2,3-b]pyridin-3-yl)-1H-pyrazolo[4,3-c]quinoline-8-carboxamide | PyBrop | 477.2 | 1st peak, Chiralpak IC column (21 x 500 mm, 5 micron) with a mobile phase of 50% Liquid CO₂ and 50% MeOH with 0.2% diethylamine using a flow rate of 45 mL/min |
| **389** | 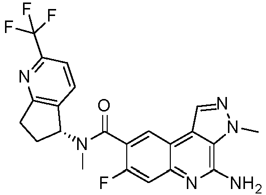 | 4-amino-7-fluoro-N,3-dimethyl-N-((5R)-2-(trifluoromethyl)-6,7-dihydro-5H-cyclopenta [b]pyrid in-5-yl)-3H-pyrazolo[3,4-c]quinoline-8-carboxamide | PyBroP | 459.2 | 1st peak, Chiral Technologies OD column (250 x 21 mm, 5 mm) with a mobile phase of 75% Liquid CO₂ and 25% MeOH with 0.2% TEA using a flow rate of 80 mL/min |
| **390** | 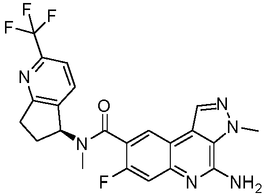 | 4-amino-7-fluoro-N,3-dimethyl-N-((5S)-2-(trifluoromethyl)-6,7-dihydro-5H-cyclopenta [b]pyrid in-5-yl)-3H-pyrazolo[3,4-c]quinoline-8-carboxamide | PyBroP | 459.2 | 2nd peak, Chiral Technologies OD column (250 x 21 mm, 5 mm) with a mobile phase of 75% Liquid CO₂ and 25% MeOH with 0.2% TEA using a flow rate of 80 mL/min |
| **391** | 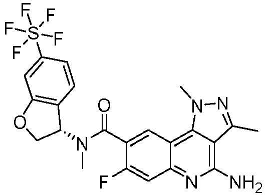 | 4-amino-7-fluoro-N,1,3-trimethyl-N-((3S)-6-(pentafluoro-lambda~6~-sulfanyl)-2,3-dihydro-1-benzofuran-3-yl)-1H-pyrazolo[4,3-c]quinoline-8-carboxamide | TBTU | 532 | 2nd peak, Chiralpak AD column (21 x 150 mm, 5 micron) with a mobile phase of 65% Liquid CO₂ and 35% MeOH with 0.2% diethylamine using a flow rate of 80 mL/min |
| **392** | 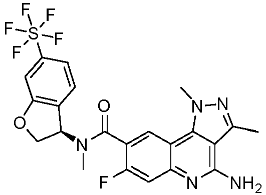 | 4-amino-7-fluoro-N,1,3-trimethyl-N-((3R)-6-(pentafluoro-lambda~6~-sulfanyl)-2,3-dihydro-1-benzofuran-3-yl)-1H-pyrazolo[4,3-c]quinoline-8-carboxamide | TBTU | 532 | 1st peak, Chiralpak AD column (21 x 150 mm, 5 micron) with a mobile phase of 65% Liquid CO₂ and 35% MeOH with 0.2% diethylamine using a flow rate of 80 mL/min |
| **393** | 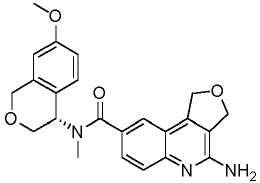 | 4-amino-N-((4S)-7-methoxy-3,4-dihydro-1H-2-benzopyran-4-yl)-N-methyl-1,3-dihydrofuro[3,4-c]quinoline-8-carboxamide | TBTU | 406 | 2nd peak, Chiralpak AS column (21 x 250 mm, 5 micron) with a mobile phase of 70% Liquid CO₂ and 30% MeOH with 0.2% diethylamine using a flow rate of 80 mL/min |
| **394** | 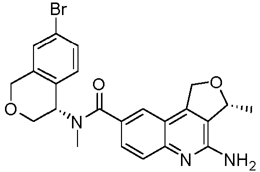 | (3R)-4-amino-N-((4S)-7-bromo-3,4-dihydro-1H-2-benzopyran-4-yl)-N,3-dimethyl-1,3-dihydrofuro[3,4-c]quinoline-8-carboxamide | PyBrop | 468.10, 470.05 | 1st peak, Chiral Technologies AS column (250 x 21 mm, 5 mm) with a mobile phase of 70% Liquid CO₂ and 30% MeOH with 0.2% TEA using a flow rate of 80 mL/min |
| **395** | 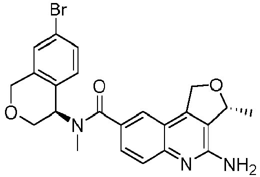 | (3R)-4-amino-N-((4R)-7-bromo-3,4-dihydro-1H-2-benzopyran-4-yl)-N,3-dimethyl-1,3-dihydrofuro[3,4-c]quinoline-8-carboxamide | PyBrop | 468.10, 470.05 | 2nd peak, Chiral Technologies AS column (250 x 21 mm, 5 mm) with a mobile phase of 70% Liquid CO₂ and 30% MeOH with 0.2% TEA using a flow rate of 80 mL/min |
| **396** | 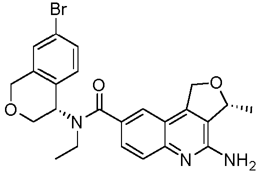 | (3R)-4-amino-N-((4S)-7-bromo-3,4-dihydro-1H-2-benzopyran-4-yl)-N-ethyl-3-methyl-1,3-dihydrofuro[3,4-c]quinoline-8-carboxamide | PyBrop | 482.05, 484.00 | 1st peak, Chiralcel OJ-H column (250 x 21 mm, 5 µm) DAS2587 with a mobile phase of 75% Liquid CO₂ and 25% MeOH with 0.2% TEA using a flow rate of 80 mL/min |
| **397** | 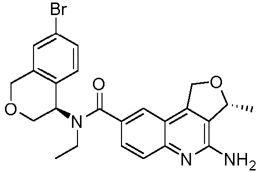 | (3R)-4-amino-N-((4R)-7-bromo-3,4-dihydro-1H-2-benzopyran-4-yl)-N-ethyl-3-methyl-1,3-dihydrofuro[3,4-c]quinoline-8-carboxamide | PyBrop | 482.05, 484.00 | 2nd peak, Chiralcel OJ-H column (250 x 21 mm, 5 µm) DAS2587 with a mobile phase of 75% Liquid CO₂ and 25% MeOH with 0.2% TEA using a flow rate of 80 mL/min |
| **398** | 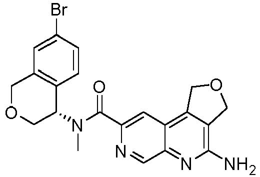 | 4-amino-N-((4S)-7-bromo-3,4-dihydro-1H-2-benzopyran-4-yl)-N-methyl-1,3-dihydrofuro[3,4-c][1,7]naphthyridi ne-8-carboxamide | PyBrop | 455.05, 457.00 | 2nd peak, Chiral Technologies OJ column (250 x 21 mm, 5 mm) with a mobile phase of 60% Liquid CO₂ and 40% MeOH with 0.2% TEA using a flow rate of 80 mL/min |
| **399** | 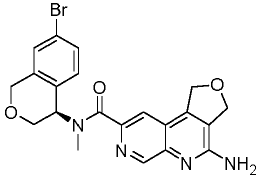 | 4-amino-N-((4R)-7-bromo-3,4-dihydro-1H-2-benzopyran-4-yl)-N-methyl-1,3-dihydrofuro[3,4-c] [1, 7]naphthyridi ne-8-carboxamide | PyBrop | 455.05, 457.00 | 1st peak, Chiral Technologies OJ column (250 x 21 mm, 5 mm) with a mobile phase of 60% Liquid CO₂ and 40% MeOH with 0.2% TEA using a flow rate of 80 mL/min |
| **400** | 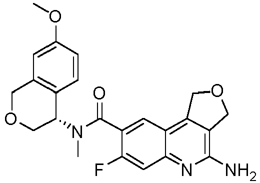 | 4-amino-7-fluoro-N-((4S)-7-methoxy-3,4-dihydro-1H-2-benzopyran-4-yl)-N-methyl-1,3-dihydrofuro[3,4-c]quinoline-8-carboxamide | HATU | 424 | 2nd peak, Chiralpak IG column (2 x 25 cm, 5 micron) with a mobile phase of 60% Liquid CO₂ and 40% isopropanol with 0.1% diethylamine using a flow rate of 60 mL/min |
| **401** | 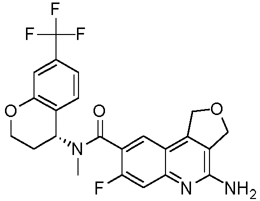 | 4-amino-7-fluoro-N-methyl-N-((4R)-7-(trifluoromethyl)-3,4-dihydro-2H-chromen-4-yl)-1,3-dihydrofuro[3,4-c]quinoline-8-carboxamide | PyBrop | 462 | 1st peak, Chiralpak ID column (30 x 250 mm) with a mobile phase of 70% Liquid CO₂ and 30% MeOH with 0.2% diethylamine using a flow rate of 80 mL/min |
| **402** | 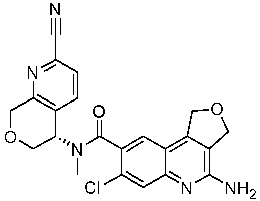 | 4-amino-7-chloro-N-((5S)-2-cyano-5,8-dihydro-6H-pyrano[3,4-b]pyridin-5-yl)-N-methyl-1,3-dihydrofuro[3,4-c]quinoline-8-carboxamide | Pybrop | 436.2 | 2nd peak, Chiralpak IE column (21 x 250 mm, 5 micron) with a mobile phase of 50% Liquid CO₂ and 50% MeOH using a flow rate of 80 mL/min |
| **403** | 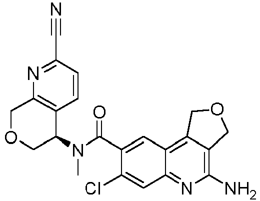 | 4-amino-7-chloro-N-((5R)-2-cyano-5,8-dihydro-6H-pyrano[3,4-b]pyridin-5-yl)-N-methyl-1,3-dihydrofuro[3,4-c]quinoline-8-carboxamide | Pybrop | 436.2 | 1st peak, Chiralpak IE column (21 x 250 mm, 5 micron) with a mobile phase of 50% Liquid CO₂ and 50% MeOH using a flow rate of 80 mL/min |
| **404** | 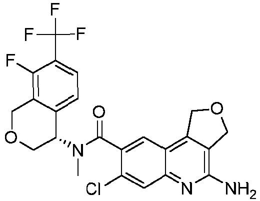 | 4-amino-7-chloro-N-((4S)-8-fluoro-7-(trifluoromethyl)-3,4-dihydro-1H-2-benzopyran-4-yl)-N-methyl-1,3-dihydrofuro[3,4-c]quinoline-8-carboxamide | TBTU | 496.1 | 1st peak, Chiralpak IE column (21 x 250 mm, 5 micron) with a mobile phase of 50% Liquid CO₂ and 50% MeOH with 0.2% diethylamine using a flow rate of 60 mL/min |
| **405** | 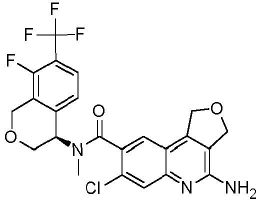 | 4-amino-7-chloro-N-((4R)-8-fluoro-7-(trifluoromethyl)-3,4-dihydro-1H-2-benzopyran-4-yl)-N-methyl-1,3-dihydrofuro[3,4-c]quinoline-8-carboxamide | TBTU | 496.1 | 2nd peak, Chiralpak IE column (21 x 250 mm, 5 micron) with a mobile phase of 50% Liquid CO₂ and 50% MeOH with 0.2% diethylamine using a flow rate of 60 mL/min |
| **406** | 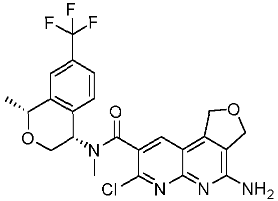 | 4-amino-7-chloro-N-methyl-N-((1R,4S)-1-methyl-7-(trifluoromethyl)-3,4-dihydro-1H-2-benzopyran-4-yl)-1,3-dihydrofuro[3,4-c] [1,8]naphthyridi ne-8-carboxamide | Pybrop | 493 | 1st peak, Chiralpak IE column (21 x 250 mm, 5 micron) with a mobile phase of 45% Liquid CO₂ and 55% MeOH with 0.2% diethylamine using a flow rate of 60 mL/min |
| **407** | 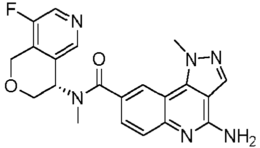 | 4-amino-N-((4S)-8-fluoro-3,4-dihydro-1H-pyrano[4,3-c]pyridin-4-yl)-N,1-dimethyl-1H-pyrazolo[4,3-c]quinoline-8-carboxamide | TBTU | 407.2 | 1st peak, AS-H column (25 x 2 cm) with a mobile phase of 80% Liquid CO₂ and 20% MeOH with 0.2% TEA using a flow rate of 60 mL/min |
| **408** | 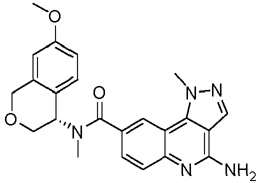 | 4-amino-N-((4S)-7-methoxy-3,4-dihydro-1H-2-benzopyran-4-yl)-N,1-dimethyl-1H-pyrazolo[4,3-c]quinoline-8-carboxamide | TBTU | 418.1 | 2nd peak, Chiralpak IG column (21 x 250 mm, 5 micron) with a mobile phase of 50% Liquid CO₂ and 50% MeOH with 0.2% diethylamine using a flow rate of 80 mL/min |
| **409** | 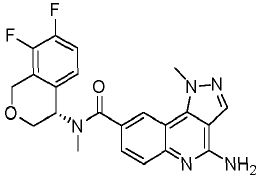 | 4-amino-N-((4S)-7,8-difluoro-3,4-dihydro-1H-2-benzopyran-4-yl)-N,1-dimethyl-1H-pyrazolo[4,3-c]quinoline-8-carboxamide | TBTU | 424.2 | 1st peak, Chiral Technologies AS column (250 x 21 mm, 5 mm) with a mobile phase of 75% Liquid CO₂ and 25% MeOH with 0.2% TEA using a flow rate of 80 mL/min |
| **410** | 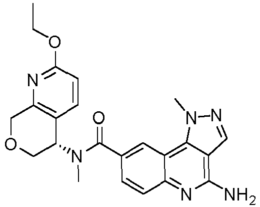 | 4-amino-N-((5S)-2-ethoxy-5,8-dihydro-6H-pyrano[3,4-b]pyridin-5-yl)-N,1-dimethyl-1H-pyrazolo[4,3-c]quinoline-8-carboxamide | HATU | 433.3 | 2nd peak, Chiralpak IG column (2 x 25 cm, 5 micron) with a mobile phase of 55% Liquid CO₂ and 45% MeOH with 0.1% diethylamine using a flow rate of 60 mL/min |
| **411** | 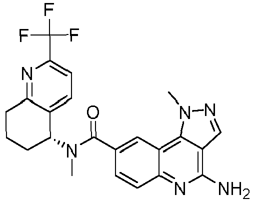 | 4-amino-N,1-dimethyl-N-((5R)-2-(trifluoromethyl)-5,6,7,8-tetrahydro-5-quinolinyl)-1H-pyrazolo[4,3-c]quinoline-8-carboxamide | TBTU | 455.2 | 1st peak, ChromegaChiral CC4 column (21 x 500 mm, 5 micron) with a mobile phase of 65% Liquid CO₂ and 35% MeOH with 0.2% diethylamine using a flow rate of 60 mL/min |
| **412** | 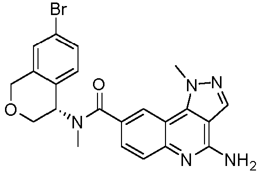 | 4-amino-N-((4S)-7-bromo-3,4-dihydro-1H-2-benzopyran-4-yl)-N,1-dimethyl-1H-pyrazolo[4,3-c]quinoline-8-carboxamide | TBTU | 466.1 | 1st Peak, Chiralpak AS column (21 x 250 mm) with a mobile phase of 80% Liquid CO₂ and 20% MeOH with 0.2% diethylamine using a flow rate of 80 mL/min |
| **413** | 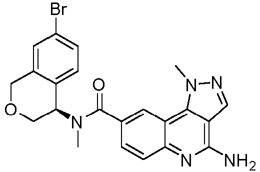 | 4-amino-N-((4R)-7-bromo-3,4-dihydro-1H-2-benzopyran-4-yl)-N,1-dimethyl-1H-pyrazolo[4,3-c]quinoline-8-carboxamide | TBTU | 466.1 | 2nd Peak, Chiralpak AS column (21 x 250 mm) with a mobile phase of 80% Liquid CO₂ and 20% MeOH with 0.2% diethylamine using a flow rate of 80 mL/min |
| **414** | 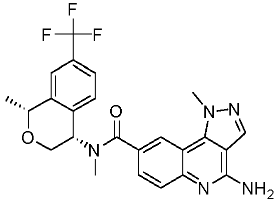 | 4-amino-N, 1-dimethyl-N-((1R,4S)-1-methyl-7-(trifluoromethyl)-3,4-dihydro-1H-2-benzopyran-4-yl)-1H-pyrazolo[4,3-c]quinoline-8-carboxamide | TBTU | 470.2 | 2nd peak, Chiralcel OD column (2 x 25 cm, 5 micron) with a mobile phase of 80% Liquid CO₂ and 20% MeOH with 0.1% diethylamine using a flow rate of 50 mL/min |
| **415** | 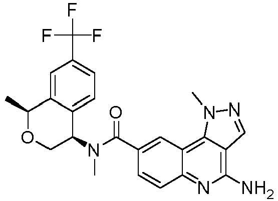 | 4-amino-N,1-dimethyl-N-((1S,4R)-1-methyl-7-(trifluoromethyl)-3,4-dihydro-1H-2-benzopyran-4-yl)-1H-pyrazolo[4,3-c]quinoline-8-carboxamide | TBTU | 470.2 | 1st peak, Chiralcel OD column (2 x 25 cm, 5 micron) with a mobile phase of 80% Liquid CO₂ and 20% MeOH with 0.1% diethylamine using a flow rate of 50 mL/min |
| **416** | 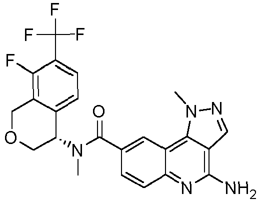 | 4-amino-N-((4S)-8-fluoro-7-(trifluoromethyl)-3,4-dihydro-1H-2-benzopyran-4-yl)-N,1-dimethyl-1H-pyrazolo[4,3-c]quinoline-8-carboxamide | TBTU | 474 | 1st peak, Chiralpak AS column (2 x 15 cm, 5 micron) with a mobile phase of 88% Liquid CO₂ and 12% MeOH with 0.1% diethylamine using a flow rate of 60 mL/min |
| **417** | 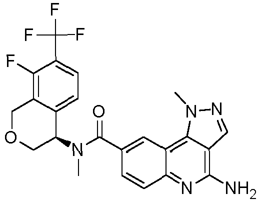 | 4-amino-N-((4R)-8-fluoro-7-(trifluoromethyl)-3,4-dihydro-1H-2-benzopyran-4-yl)-N,1-dimethyl-1H-pyrazolo[4,3-c]quinoline-8-carboxamide | TBTU | 474 | 2nd peak, Chiralpak AS column (2 x 15 cm, 5 micron) with a mobile phase of 88% Liquid CO₂ and 12% MeOH with 0.1% diethylamine using a flow rate of 60 mL/min |
| **418** | 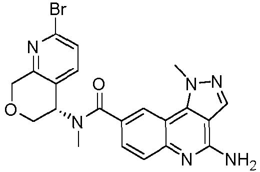 | 4-amino-N-((5S)-2-bromo-5,8-dihydro-6H-pyrano[3,4-b]pyridin-5-yl)-N,1-dimethyl-1H-pyrazolo[4,3-c]quinoline-8-carboxamide | TBTU | 467.0, 469.0 | 2nd peak, AD column (250 x 21 mm, 5 mm) with a mobile phase of 70% Liquid CO₂ and 30% iPrOH with 0.2% diethylamine using a flow rate of 80 mL/min |
| **419** | 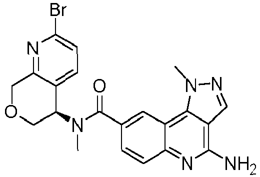 | 4-amino-N-((5R)-2-bromo-5,8-dihydro-6H-pyrano[3,4-b]pyridin-5-yl)-N,1-dimethyl-1H-pyrazolo[4,3-c]quinoline-8-carboxamide | TBTU | 467.0, 469.0 | 1st peak, AD column (250 x 21 mm, 5 mm) with a mobile phase of 70% Liquid CO₂ and 30% iPrOH with 0.2% diethylamine using a flow rate of 80 mL/min |
| **420** | 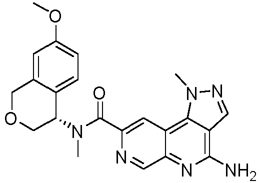 | 4-amino-N-((4S)-7-methoxy-3,4-dihydro-1H-2-benzopyran-4-yl)-N,1-dimethyl-1H-pyrazolo[4,3-c] [1, 7]naphthyridi ne-8-carboxamide | TBTU | 419.2 | 1st peak, Lux Cellulose 2 column (21 x 150 mm) with a mobile phase of 45% Liquid CO₂ and 55% MeOH with 0.2% diethylamine using a flow rate of 60 mL/min |
| **421** | 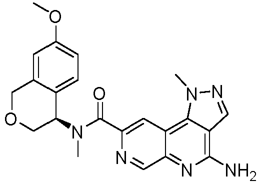 | 4-amino-N-((4R)-7-methoxy-3,4-dihydro-1H-2-benzopyran-4-yl)-N,1-dimethyl-1H-pyrazolo[4,3-c] [1, 7]naphthyridi ne-8-carboxamide | TBTU | 419.2 | 2nd peak, Lux Cellulose 2 column (21 x 150 mm) with a mobile phase of 45% Liquid CO₂ and 55% MeOH with 0.2% diethylamine using a flow rate of 60 mL/min |
| **422** | 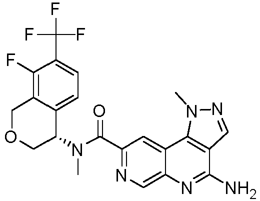 | 4-amino-N-((4S)-8-fluoro-7-(trifluoromethyl)-3,4-dihydro-1H-2-benzopyran-4-yl)-N,1-dimethyl-1H-pyrazolo[4,3-c][1,7]naphthyridi ne-8-carboxamide | TBTU | 475.2 | 2nd peak, Chiralcel OJ column (2 x 25 cm, 5 micron) with a mobile phase of 87% Liquid CO₂ and 13% MeOH with 0.1% diethylamine, using a flow rate of 65 mL/min |
| **423** | 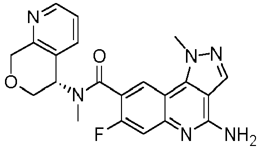 | 4-amino-N-((5S)-5,8-dihydro-6H-pyrano[3,4-b]pyridin-5-yl)-7-fluoro-N,1-dimethyl-1H-pyrazolo[4,3-c]quinoline-8-carboxamide | HATU | 407.2 | 1st peak, Chiralpak IC column (21 x 150 mm) with a mobile phase of 45% Liquid CO₂ and 55% (1:1) MeOH: acetonitrile with 0.2% diethylamine using a flow rate of 80 mL/min |
| **424** | 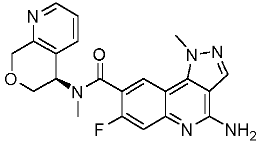 | 4-amino-N-((5R)-5,8-dihydro-6H-pyrano[3,4-b]pyridin-5-yl)-7-fluoro-N,1-dimethyl-1H-pyrazolo[4,3-c]quinoline-8-carboxamide | HATU | 407.2 | 2nd peak, Chiralpak IC column (21 x 150 mm) with a mobile phase of 45% Liquid CO₂ and 55% (1:1) MeOH: acetonitrile with 0.2% diethylamine using a flow rate of 80 mL/min |
| **425** | 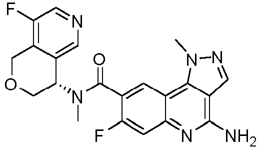 | 4-amino-7-fluoro-N-((4S)-8-fluoro-3,4-dihydro-1H-pyrano[4,3-c]pyridin-4-yl)-N,1-dimethyl-1H-pyrazolo[4,3-c]quinoline-8-carboxamide | TBTU | 425.2 | 1st peak, Chiralpak AS column (21 x 250 mm) with a mobile phase of 85% Liquid CO₂ and 15% MeOH with 0.2% TEA using a flow rate of 80 mL/min |
| **426** | 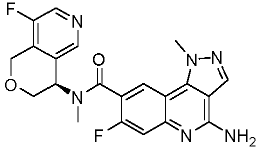 | 4-amino-7-fluoro-N-((4R)-8-fluoro-3,4-dihydro-1H-pyrano[4,3-c]pyridin-4-yl)-N,1-dimethyl-1H-pyrazolo[4,3-c]quinoline-8-carboxamide | TBTU | 425.2 | 2nd peak, Chiralpak AS column (21 x 250 mm) with a mobile phase of 85% Liquid CO₂ and 15% MeOH with 0.2% TEA using a flow rate of 80 mL/min |
| **427** | 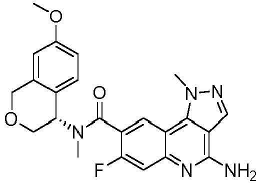 | 4-amino-7-fluoro-N-((4S)-7-methoxy-3,4-dihydro-1H-2-benzopyran-4-yl)-N,1-dimethyl-1H-pyrazolo[4,3-c]quinoline-8-carboxamide | TBTU | 436.3 | 2nd peak, Chiralpak AZ column (21 x 250 mm) with a mobile phase of 60% Liquid CO₂ and 40% isopropanol with 0.2% diethylamine using a flow rate of 60 mL/min |
| **428** | 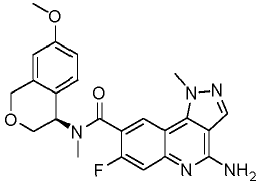 | 4-amino-7-fluoro-N-((4R)-7-methoxy-3,4-dihydro-1H-2-benzopyran-4-yl)-N,1-dimethyl-1H-pyrazolo[4,3-c]quinoline-8-carboxamide | TBTU | 436.3 | 1st peak, Chiralpak AZ column (21 x 250 mm) with a mobile phase of 60% Liquid CO₂ and 40% isopropanol with 0.2% diethylamine using a flow rate of 60 mL/min |
| **429** | 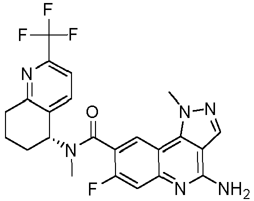 | 4-amino-7-fluoro-N,1-dimethyl-N-((5R)-2-(trifluoromethyl)-5,6,7,8-tetrahydro-5-quinolinyl)-1H-pyrazolo[4,3-c]quinoline-8-carboxamide | TBTU | 473.2 | 2nd peak, Chiralpak IC column (21 x 150 mm) with a mobile phase of 45% Liquid CO₂ and 55% MeOH with 0.2% diethylamine using a flow rate of 60 mL/min |
| **430** | 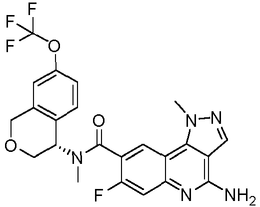 | 4-amino-7-fluoro-N,1-dimethyl-N-((4S)-7-(trifluoromethoxy) -3,4-dihydro-1H-2-benzopyran-4-yl)-1H-pyrazolo[4,3-c]quinoline-8-carboxamide | Pybrop | 490.2 | 2nd peak, Chiralpak IC column (21 x 250 mm) with a mobile phase of 55% Liquid CO₂ and 45% MeOH with 0.2% diethylamine using a flow rate of 80 mL/min |
| **431** | 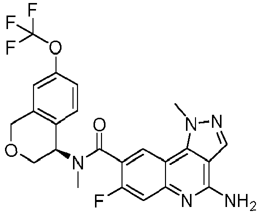 | 4-amino-7-fluoro-N,1-dimethyl-N-((4R)-7-(trifluoromethoxy) -3,4-dihydro-1H-2-benzopyran-4-yl)-1H-pyrazolo[4,3-c]quinoline-8-carboxamide | Pybrop | 490.2 | 1st peak, Chiralpak IC column (21 x 250 mm) with a mobile phase of 55% Liquid CO₂ and 45% MeOH with 0.2% diethylamine using a flow rate of 80 mL/min |
| **432** | 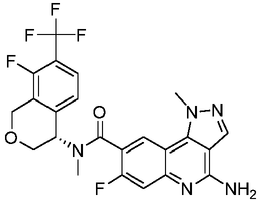 | 4-amino-7-fluoro-N-((4S)-8-fluoro-7-(trifluoromethyl)-3,4-dihydro-1H-2-benzopyran-4-yl)-N,1-dimethyl-1H-pyrazolo[4,3-c]quinoline-8-carboxamide | TBTU | 492.2 | 2nd peak, Chiralpak IC column (21 x 250 mm) with a mobile phase of 55% Liquid CO₂ and 45% MeOH with 0.2% diethylamine using a flow rate of 80 mL/min |
| **433** | 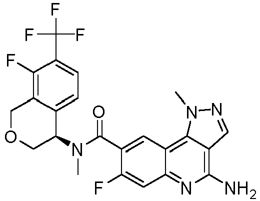 | 4-amino-7-fluoro-N-((4R)-8-fluoro-7-(trifluoromethyl)-3,4-dihydro-1H-2-benzopyran-4-yl)-N,1-dimethyl-1H-pyrazolo[4,3-c]quinoline-8-carboxamide | TBTU | 492.2 | 1st peak, Chiralpak IC column (21 x 250 mm) with a mobile phase of 55% Liquid CO₂ and 45% MeOH with 0.2% diethylamine using a flow rate of 80 mL/min |
| **434** | 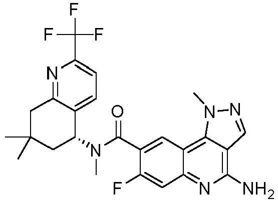 | 4-amino-N-((5R)-7,7-dimethyl-2-(trifluoromethyl)-5,6,7,8-tetrahydro-5-quinolinyl)-7-fluoro-N,1-dimethyl-1H-pyrazolo[4,3-c]quinoline-8-carboxamide | TBTU | 501.2 | 1st peak, Chiralcel OD column (21 x 250 mm) with a mobile phase of 70% Liquid CO₂ and 30% MeOH with 0.2% diethylamine using a flow rate of 80 mL/min |
| **435** | 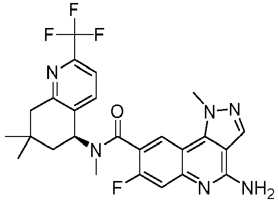 | 4-amino-N-((5S)-7,7-dimethyl-2-(trifluoromethyl)-5,6,7,8-tetrahydro-5-quinolinyl)-7-fluoro-N,1-dimethyl-1H-pyrazolo[4,3-c]quinoline-8-carboxamide | TBTU | 501.2 | 2nd peak, Chiralcel OD column (21 x 250 mm) with a mobile phase of 70% Liquid CO₂ and 30% MeOH with 0.2% diethylamine using a flow rate of 80 mL/min |
| **436** | 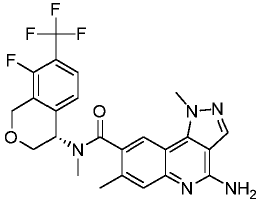 | 4-amino-N-((4S)-8-fluoro-7-(trifluoromethyl)-3,4-dihydro-1H-2-benzopyran-4-yl)-N,1,7-trimethyl-1H-pyrazolo[4,3-c]quinoline-8-carboxamide | TBTU | 488.2 | 1st peak, Chiralpak IG column (2 x 25 cm, 5 micron) with a mobile phase of 78% Liquid CO₂ and 22% ethanol with 0.1% diethylamine using a flow rate of 60 mL/min |
| **437** | 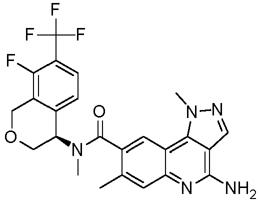 | 4-amino-N-((4R)-8-fluoro-7-(trifluoromethyl)-3,4-dihydro-1H-2-benzopyran-4-yl)-N,1,7-trimethyl-1H-pyrazolo[4,3-c]quinoline-8-carboxamide | TBTU | 488.2 | 2nd peak, Chiralpak IG column (2 x 25 cm, 5 micron) with a mobile phase of 78% Liquid CO₂ and 22% ethanol with 0.1% diethylamine using a flow rate of 60 mL/min |
| **438** | 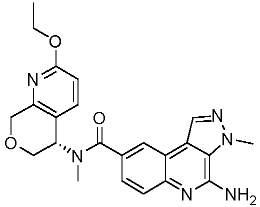 | 4-amino-N-((5S)-2-ethoxy-5,8-dihydro-6H-pyrano[3,4-b]pyridin-5-yl)-N,3-dimethyl-3H-pyrazolo[3,4-c]quinoline-8-carboxamide | HATU | 433.1 | 2nd peak, Chiralcel OJ column (2 x 25 cm, 5 micron) with a mobile phase of 65% Liquid CO₂ and 35% MeOH with 0.1% diethylamine using a flow rate of 60 mL/min |
| **439** | 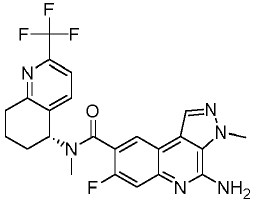 | 4-amino-7-fluoro-N,3-dimethyl-N-((5R)-2-(trifluoromethyl)-5,6,7,8-tetrahydro-5-quinolinyl)-3H-pyrazolo[3,4-c]quinoline-8-carboxamide | TBTU | 473.2 | 1st peak, Chiralpak IG column (2 x 25 cm, 5 micron) with a mobile phase of 70% Liquid CO₂ and 30% MeOH with 0.1% diethylamine using a flow rate of 70 mL/min |
| **440** | 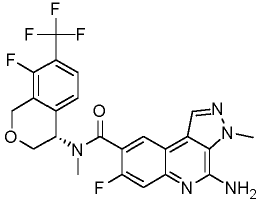 | 4-amino-7-fluoro-N-((4S)-8-fluoro-7-(trifluoromethyl)-3,4-dihydro-1H-2-benzopyran-4-yl)-N,3-dimethyl-3H-pyrazolo[3,4-c]quinoline-8-carboxamide | TBTU | 492.2 | 2nd peak, Chiralpak IC column (2 x 15 cm, 5 micron) with a mobile phase of 70% Liquid CO₂ and 30% MeOH with 0.1% diethylamine using a flow rate of 60 mL/min |
| **441** | 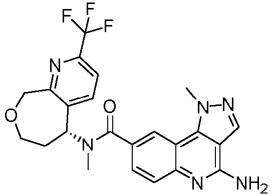 | 4-amino-N,1-dimethyl-N-((5R)-2-(trifluoromethyl)-5,6,7,9-tetrahydrooxepino [3,4-b]pyridin-5-yl)-1H-pyrazolo[4,3-c]quinoline-8-carboxamide | TBTU | 471.2 | 1st peak, OD-H column (25 x 2 cm) with a mobile phase of 65% Liquid CO₂ and 35% MeOH with 0.1% diethylamine using a flow rate of 55 mL/min |

### Example 442: (S)-4-amino-7-chloro-N-cyclopropyl-N-(6-(trifluoromethyl)-2,3-dihydrobenzofuran-3-yl)-1,3-dihydrofuro[3,4-c]quinoline-8-carboxamide

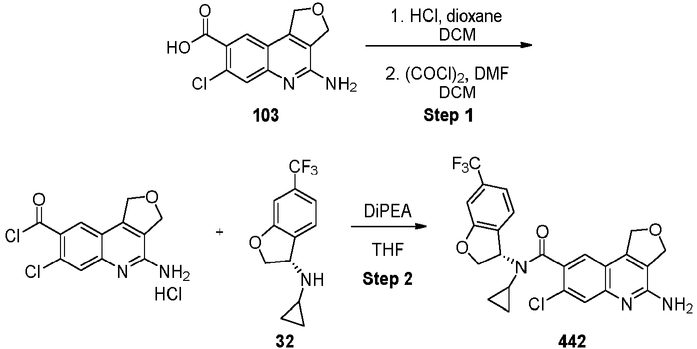

Step 1. To a stirred suspension of 4-amino-7-chloro-1,3-dihydrofuro[3,4-c]quinoline-8-carboxylic acid (103) (2.00 g, 7.56 mmol) in DCM (20.0 mL) was added 4 M HCl in 1,4-dioxane (5.67 mL, 22.67 mmol) and the resulting suspension was allowed to stir at room temperature for 30 min. The mixture was concentrated under reduced pressure, then co-evaporated with toluene (2 x 10 mL). The obtained crude material was re-suspended in dichloromethane (80.0 mL), cooled to 0°C, and treated with oxalyl chloride (2 M in DCM, 15.11 mL, 30.2 mmol) followed by DMF (15 drops). The reaction vessel was flushed with nitrogen and the reaction mixture was allowed to stir at room temperature under nitrogen overnight. After 16 h, the reaction mixture was concentrated under reduced pressure, and the obtained crude residue was rinsed with heptane (30 mL) and dried in vacuo to give 4-amino-7-chloro-1,3-dihydrofuro[3,4-c]quinoline-8-carbonyl chloride hydrochloride (2.42 g, 7.56 mmol, quant. yield) as a tan solid. *m*/*z* (ESI): 279.1 (M+H)⁺ was observed for the corresponding methyl ester after quenching of an aliquot with MeOH.

Step 2. A mixture of 4-amino-7-chloro-1,3-dihydrofuro[3,4-c]quinoline-8-carbonyl chloride hydrochloride (0.140 g, 0.438 mmol), (S)-N-cyclopropyl-6-(trifluoromethyl)-2,3-dihydrobenzofuran-3-amine (32) (0.071 g, 0.292 mmol), and diisopropylethylamine (0.204 mL, 1.168 mmol, Sigma-Aldrich Corporation) in THF (3 mL) was stirred at rt for 2 hours. The reaction was concentrated and the crude mixture purified by column chromatography using 0-20% MeOH in DCM to afford (*S*)-4-amino-7-chloro-N-cyclopropyl-N-(6-(trifluoromethyl)-2,3-dihydrobenzofuran-3-yl)-1,3-dihydrofuro[3,4-c]quinoline-8-carboxamide (442) (0.035 g, 0.071 mmol, 24.48% yield). *m*/*z* (ESI): 490.11 (M+H)⁺. ¹H NMR (400 MHz, DMSO-d6) δ ppm 7.52 - 7.73 (m, 3 H) 7.30 (d, J=7.73 Hz, 1 H) 7.21 (s, 1 H) 6.87 (br s, 2 H) 5.94 - 6.16 (m, 1 H) 5.33 (br s, 2 H) 5.01 (t, J=3.24 Hz, 2 H) 4.81 - 4.95 (m, 1 H) 4.62 - 4.79 (m, 1 H) 2.68 (br d, J=1.88 Hz, 1 H) 0.02 - 0.51 (m, 4 H). ¹⁹F NMR (376 MHz, DMSO-d6) δ ppm -60.68 (s, 3 F).

Examples in Table 14 were prepared in a manner similar to that described for Example 442. Enantiopure analogs were synthesized using chiral starting materials.

**Table 14**

| **Ex.** | **Structure** | **Name** | **m/z (ESI): (M+H)⁺** |
|---|---|---|---|
| **443** | 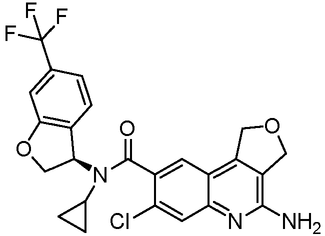 | 4-amino-7-chloro-N-cyclopropyl-N-((3R)-6-(trifluoromethyl)-2,3-dihydro-1-benzofuran-3-yl)-1,3-dihydrofuro[3,4-c] quinoline-8-carboxamide | 490.1 |
| **444** | 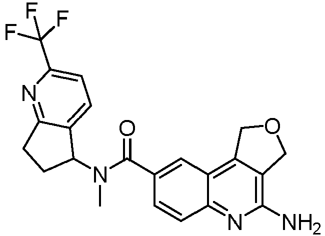 | 4-amino-N-methyl-N-((5R)-2-(trifluoromethyl)-6,7-dihydro-5H-cyclopenta[b]pyridin-5-yl)-1,3-dihydrofuro[3,4-c]quinoline-8-carboxamide and 4-amino-N-methyl-N-((5S)-2-(trifluoromethyl)-6,7-dihydro-5H-cyclopenta[b]pyridin-5-yl)-1,3-dihydrofuro[3,4-c]quinoline-8-carboxamide | 428.8 |
| **445** | 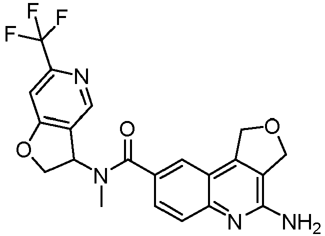 | 4-amino-N-methyl-N-((3R)-6-(trifluoromethyl)-2,3-dihydrofuro[3,2-c]pyridin-3-yl)-1,3-dihydrofuro[3,4-c] quinoline-8-carboxamide and 4-amino-N-methyl-N-((3S)-6-(trifluoromethyl)-2,3-dihydrofuro[3,2-c]pyridin-3-yl)-1,3-dihydrofuro[3,4-c] quinoline-8-carboxamide | 431.1 |
| **446** | 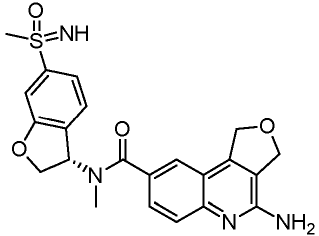 | 4-amino-N-methyl-N-((3S)-6-(S-methylsulfonimidoyl)-2,3-dihydro-1-benzofuran-3-yl)-1,3-dihydrofuro[3,4-c] quinoline-8-carboxamide | 439.1 |
| **447** | 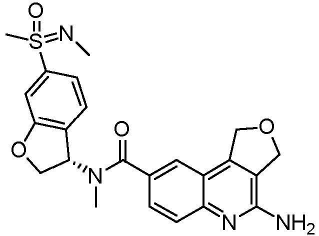 | 4-amino-N-((3S)-6-((R)-N,S-dimethylsulfonimidoyl)-2,3-dihydro-1-benzofuran-3-yl)-N-methyl-1,3-dihydrofuro[3,4-c]quinoline-8-carboxamide and 4-amino-N-((3 S)-6-((S)-N,S-dimethylsulfonimidoyl)-2,3-dihydro-1-benzofuran-3-yl)-N-methyl-1,3-dihydrofuro[3,4-c]quinoline-8-carboxamide | 453.3 |
| **448** | 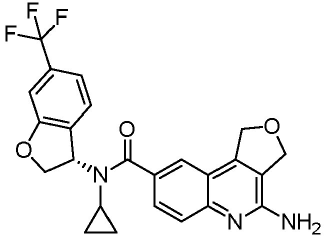 | 4-amino-N-cyclopropyl-N-((3S)-6-(trifluoromethyl)-2,3-dihydro-1-benzofuran-3-yl)-1,3-dihydrofuro[3,4-c] quinoline-8-carboxamide | 456.2 |
| **449** | 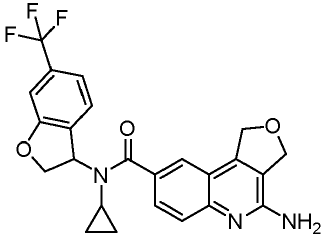 | 4-amino-N-cyclopropyl-N-((3R)-6-(trifluoromethyl)-2,3-dihydro-1-benzofuran-3-yl)-1,3-dihydrofuro[3,4-c] quinoline-8-carboxamide and 4-amino-N-cyclopropyl-N-((3S)-6-(trifluoromethyl)-2,3-dihydro-1-benzofuran-3-yl)-1,3-dihydrofuro[3,4-c] quinoline-8-carboxamide | 456.2 |
| **450** | 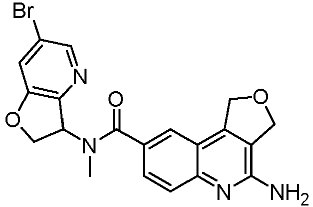 | 4-amino-N-((3R)-6-bromo-2,3-dihydrofuro[3,2-b]pyridin-3-yl)-N-methyl-1,3-dihydrofuro[3,4-c]quinoline-8-carboxamide and 4-amino-N-((3S)-6-bromo-2,3-dihydrofuro[3,2-b]pyridin-3-yl)-N-methyl-1,3-dihydrofuro[3,4-c]quinoline-8-carboxamide | 441.1, 443.1 |
| **451** | 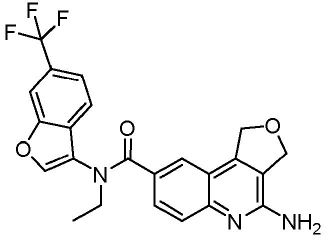 | 4-amino-N-ethyl-N-(6-(trifluoromethyl)-1-benzofuran-3-yl)-1,3-dihydrofuro[3,4-c] quinoline-8-carboxamide | 441.8 |
| **452** | 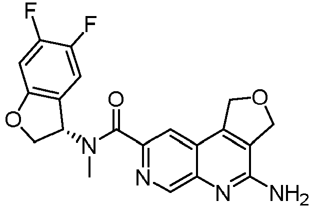 | 4-amino-N-((3S)-5,6-difluoro-2,3-dihydro-1-benzofuran-3-yl)-N-methyl-1,3-dihydrofuro[3,4-c][1,7]naphthyridine-8-carboxamide | 399.1 |
| **453** | 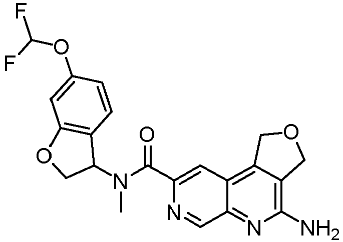 | 4-amino-N-((3R)-6-(difluoromethoxy)-2,3-dihydro-1-benzofuran-3-yl)-N-methyl-1,3-dihydrofuro[3,4-c][1,7]naphthyridine-8-carboxamide and 4-amino-N-((3 S)-6-(difluoromethoxy)-2,3-dihydro-1-benzofuran-3-yl)-N-methyl-1,3-dihydrofuro[3,4-c][1,7]naphthyridine-8-carboxamide | 429.3 |
| **454** | 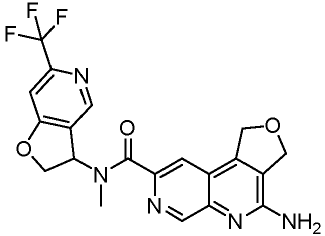 | 4-amino-N-methyl-N-((3R)-6-(trifluoromethyl)-2,3-dihydrofuro[3,2-c]pyridin-3-yl)-1,3-dihydrofuro[3,4-c][1,7]naphthyridine-8-carboxamide and 4-amino-N-methyl-N-((3S)-6-(trifluoromethyl)-2,3-dihydrofuro[3,2-c]pyridin-3-yl)-1,3-dihydrofuro[3,4-c][1,7]naphthyridine-8-carboxamide | 432.3 |
| **455** | 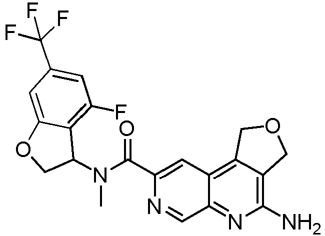 | 4-amino-N-((3R)-4-fluoro-6-(trifluoromethyl)-2,3-dihydro-1-benzofuran-3-yl)-N-methyl-1,3-dihydrofuro[3,4-c][1,7]naphthyridine-8-carboxamide and 4-amino-N-((3 S)-4-fluoro-6-(trifluoromethyl)-2,3-dihydro-1-benzofuran-3-yl)-N-methyl-1,3-dihydrofuro[3,4-c][1,7]naphthyridine-8-carboxamide | 449.0 |
| **456** | 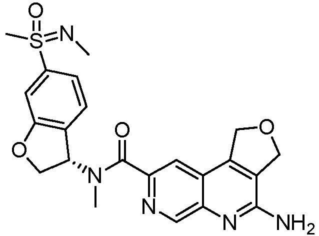 | 4-amino-N-((3S)-6-((R)-N,S-dimethylsulfonimidoyl)-2,3-dihydro-1-benzofuran-3-yl)-N-methyl-1,3-dihydrofuro[3,4-c][1,7]naphthyridine-8-carboxamide and 4-amino-N-((3 S)-6-((S)-N,S-dimethylsulfonimidoyl)-2,3-dihydro-1-benzofuran-3-yl)-N-methyl-1,3-dihydrofuro[3,4-c][1,7]naphthyridine-8-carboxamide | 454.1 |
| **457** | 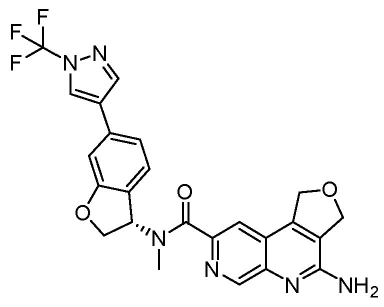 | 4-amino-N-methyl-N-((3S)-6-(1-(trifluoromethyl)-1H-pyrazol-4-yl)-2,3-dihydro-1-benzofuran-3-yl)-1,3-dihydrofuro[3,4-c][1,7]naphthyridine-8-carboxamide | 497.0 |
| **458** | 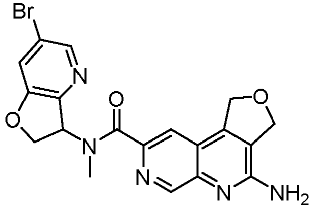 | 4-amino-N-((3R)-6-bromo-2,3-dihydrofuro[3,2-b]pyridin-3-yl)-N-methyl-1,3-dihydrofuro[3,4-c][1,7]naphthyridine-8-carboxamide and 4-amino-N-((3S)-6-bromo-2,3-dihydrofuro[3,2-b]pyridin-3-yl)-N-methyl-1,3-dihydrofuro[3,4-c][1,7]naphthyridine-8-carboxamide | 442.1, 444.0 |
| **459** | 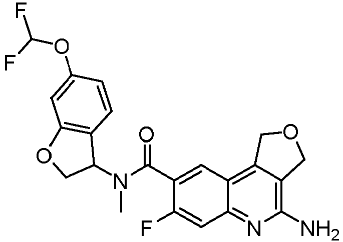 | 4-amino-N-((3R)-6-(difluoromethoxy)-2,3-dihydro-1-benzofuran-3-yl)-7-fluoro-N-methyl-1,3-dihydrofuro[3,4-c] quinoline-8-carboxamide and 4-amino-N-((3 S)-6-(difluoromethoxy)-2,3-dihydro-1-benzofuran-3-yl)-7-fluoro-N-methyl-1,3-dihydrofuro[3,4-c] quinoline-8-carboxamide | 446.1 |
| **460** | 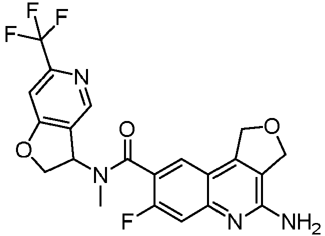 | 4-amino-7-fluoro-N-methyl-N-((3R)-6-(trifluoromethyl)-2,3-dihydrofuro[3,2-c]pyridin-3-yl)-1,3-dihydrofuro[3,4-c] quinoline-8-carboxamide and 4-amino-7-fluoro-N-methyl-N-((3S)-6-(trifluoromethyl)-2,3-dihydrofuro[3,2-c]pyridin-3-yl)-1,3-dihydrofuro[3,4-c] quinoline-8-carboxamide | 449.1 |
| **461** | 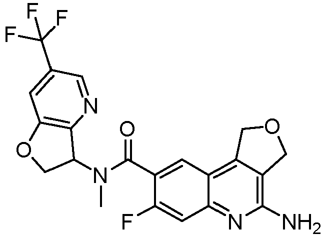 | 4-amino-7-fluoro-N-methyl-N-((3R)-6-(trifluoromethyl)-2,3-dihydrofuro[3,2-b]pyridin-3-yl)-1,3-dihydrofuro[3,4-c] quinoline-8-carboxamide and 4-amino-7-fluoro-N-methyl-N-((3S)-6-(trifluoromethyl)-2,3-dihydrofuro[3,2-b]pyridin-3-yl)-1,3-dihydrofuro[3,4-c] quinoline-8-carboxamide | 449.2 |
| **462** | 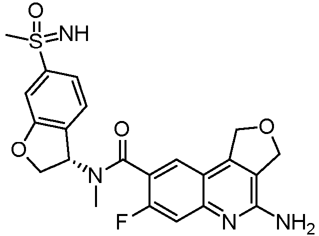 | 4-amino-7-fluoro-N-methyl-N-((3S)-6-(S-methylsulfonimidoyl)-2,3-dihydro-1-benzofuran-3-yl)-1,3-dihydrofuro[3,4-c] quinoline-8-carboxamide | 457.1 |
| **463** | 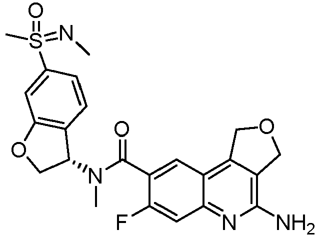 | 4-amino-N-((3S)-6-((R)-N,S-dimethylsulfonimidoyl)-2,3-dihydro-1-benzofuran-3-yl)-7-fluoro-N-methyl-1,3-dihydrofuro[3,4-c]quinoline-8-carboxamide and 4-amino-N-((3 S)-6-((S)-N,S-dimethylsulfonimidoyl)-2,3-dihydro-1-benzofuran-3-yl)-7-fluoro-N-methyl-1,3-dihydrofuro[3,4-c]quinoline-8-carboxamide | 471.1 |
| **464** | 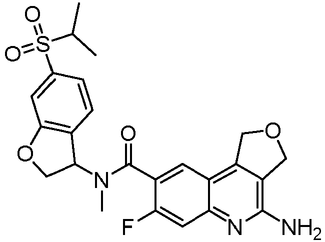 | 4-amino-7-fluoro-N-methyl-N-((3R)-6-(2-propanylsulfonyl)-2,3-dihydro-1-benzofuran-3-yl)-1,3-dihydrofuro[3,4-c] quinoline-8-carboxamide and 4-amino-7-fluoro-N-methyl-N-((3S)-6-(2-propanylsulfonyl)-2,3-dihydro-1-benzofuran-3-yl)-1,3-dihydrofuro[3,4-c] quinoline-8-carboxamide | 486.1 |
| **465** | 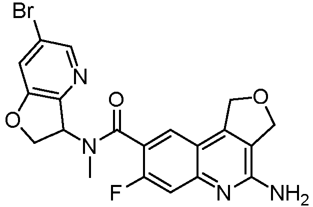 | 4-amino-N-((3R)-6-bromo-2,3-dihydrofuro[3,2-b]pyridin-3-yl)-7-fluoro-N-methyl-1,3-dihydrofuro[3,4-c] quinoline-8-carboxamide and 4-amino-N-((3S)-6-bromo-2,3-dihydrofuro[3,2-b]pyridin-3-yl)-7-fluoro-N-methyl-1,3-dihydrofuro[3,4-c] quinoline-8-carboxamide | 459.0, 461.1 |
| **466** | 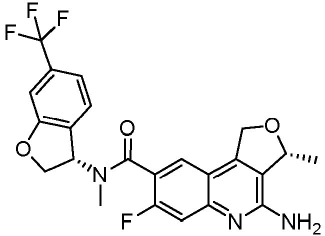 | (3R)-4-amino-7-fluoro-N,3-dimethyl-N-((3S)-6-(trifluoromethyl)-2,3-dihydro-1-benzofuran-3-yl)-1,3-dihydrofuro[3,4-c] quinoline-8-carboxamide | 462.2 |
| **467** | 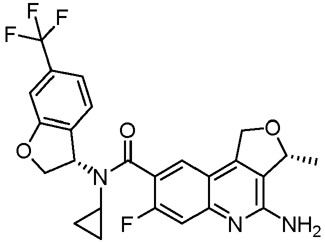 | (3R)-4-amino-N-cyclopropyl-7-fluoro-3-methyl-N-((3S)-6-(trifluoromethyl)-2,3-dihydro-1-benzofuran-3-yl)-1,3-dihydrofuro[3,4-c]quinoline-8-carboxamide | 488.1 |
| **468** | 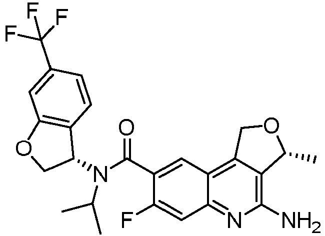 | (3R)-4-amino-7-fluoro-3-methyl-N-(2-propanyl)-N-((3S)-6-(trifluoromethyl)-2,3-dihydro-1-benzofuran-3-yl)-1,3-dihydrofuro[3,4-c]quinoline-8-carboxamide | 490.1 |
| **469** | 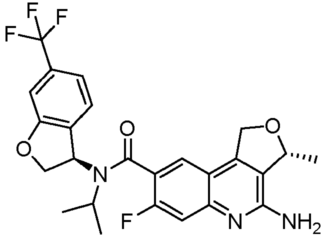 | (3R)-4-amino-7-fluoro-3-methyl-N-(2-propanyl)-N-((3R)-6-(trifluoromethyl)-2,3-dihydro-1-benzofuran-3-yl)-1,3-dihydrofuro[3,4-c]quinoline-8-carboxamide | 490.1 |
| **470** | 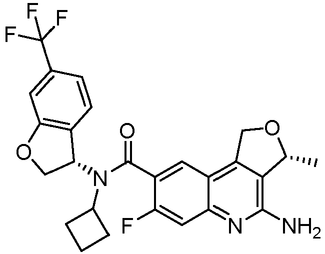 | (3R)-4-amino-N-cyclobutyl-7-fluoro-3-methyl-N-((3S)-6-(trifluoromethyl)-2,3-dihydro-1-benzofuran-3-yl)-1,3-dihydrofuro[3,4-c]quinoline-8-carboxamide | 502.1 |
| **471** | 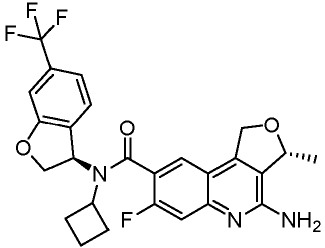 | (3R)-4-amino-N-cyclobutyl-7-fluoro-3-methyl-N-((3R)-6-(trifluoromethyl)-2,3-dihydro-1-benzofuran-3-yl)-1,3-dihydrofuro[3,4-c]quinoline-8-carboxamide | 502.1 |
| **472** | 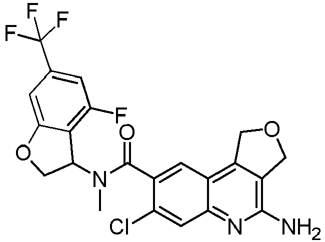 | 4-amino-7-chloro-N-((3R)-4-fluoro-6-(trifluoromethyl)-2,3-dihydro-1-benzofuran-3-yl)-N-methyl-1,3-dihydrofuro[3,4-c]quinoline-8-carboxamide and 4-amino-7-chloro-N-((3S)-4-fluoro-6-(trifluoromethyl)-2,3-dihydro-1-benzofuran-3-yl)-N-methyl-1,3-dihydrofuro[3,4-c]quinoline-8-carboxamide | 482.0 |
| **473** | 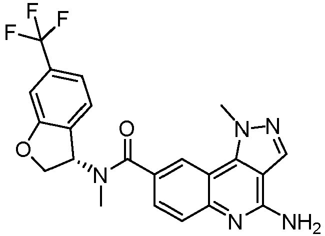 | 4-amino-N,1-dimethyl-N-((3S)-6-(trifluoromethyl)-2,3-dihydro-1-benzofuran-3-yl)-1H-pyrazolo[4,3-c] quinoline-8-carboxamide | 442.2 |
| **474** | 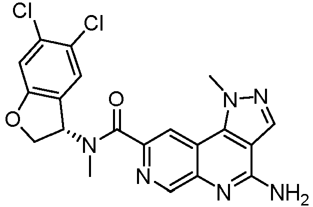 | 4-amino-N-((3S)-5,6-dichloro-2,3-dihydro-1-benzofuran-3-yl)-N,1-dimethyl-1H-pyrazolo[4,3-c][1,7]naphthyridine-8-carboxamide | 443 |
| **475** | 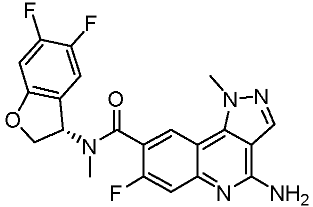 | 4-amino-N-((3S)-5,6-difluoro-2,3-dihydro-1-benzofuran-3-yl)-7-fluoro-N,1-dimethyl-1H-pyrazolo[4,3-c] quinoline-8-carboxamide | 428.1 |
| **476** | 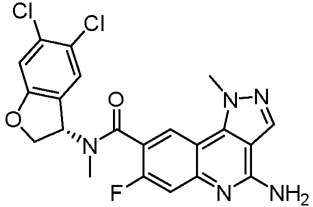 | 4-amino-N-((3 S)-5,6-dichloro-2,3-dihydro-1-benzofuran-3-yl)-7-fluoro-N,1-dimethyl-1H-pyrazolo[4,3-c] quinoline-8-carboxamide | 460 |
| **477** | 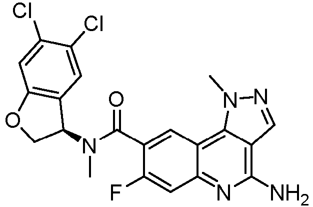 | 4-amino-N-((3R)-5,6-dichloro-2,3-dihydro-1-benzofuran-3-yl)-7-fluoro-N,1-dimethyl-1H-pyrazolo[4,3-c] quinoline-8-carboxamide | 460.1 |
| **478** | 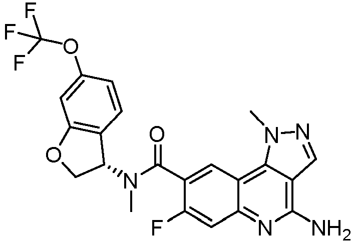 | 4-amino-7-fluoro-N, 1-dimethyl-N-((3S)-6-(trifluoromethoxy)-2,3-dihydro-1-benzofuran-3-yl)-1H-pyrazolo[4,3-c] quinoline-8-carboxamide | 476.1 |
| **479** | 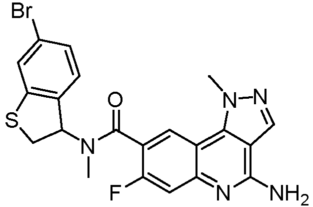 | 4-amino-N-((3R)-6-bromo-2,3-dihydro-1-benzothiophen-3-yl)-7-fluoro-N,1-dimethyl-1H-pyrazolo[4,3-c]quinoline-8-carboxamide and 4-amino-N-((3S)-6-bromo-2,3-dihydro-1-benzothiophen-3-yl)-7-fluoro-N,1-dimethyl-1H-pyrazolo[4,3-c]quinoline-8-carboxamide | 486.1, 488.1 |
| **480** | 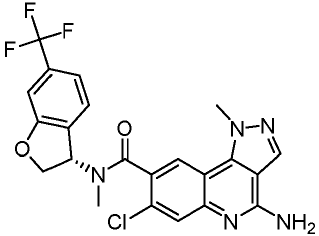 | 4-amino-7-chloro-N, 1-dimethyl-N-((3 S)-6-(trifluoromethyl)-2,3-dihydro-1-benzofuran-3-yl)-1H-pyrazolo[4,3-c] quinoline-8-carboxamide | 475.8 |
| **481** | 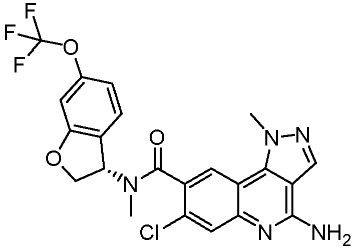 | 4-amino-7-chloro-N, 1-dimethyl-N-((3 S)-6-(trifluoromethoxy)-2,3-dihydro-1-benzofuran-3-yl)-1H-pyrazolo[4,3-c] quinoline-8-carboxamide | 492.1 |
| **482** | 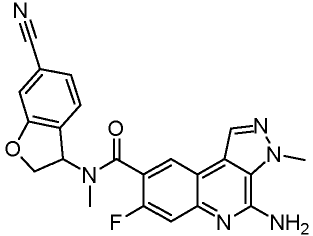 | 4-amino-N-((3R)-6-cyano-2,3-dihydro-1-benzofuran-3-yl)-7-fluoro-N,3-dimethyl-3H-pyrazolo[3,4-c]quinoline-8-carboxamide and 4-amino-N-((3S)-6-cyano-2,3-dihydro-1-benzofuran-3-yl)-7-fluoro-N,3-dimethyl-3H-pyrazolo[3,4-c]quinoline-8-carboxamide | 417.1 |
| **483** | 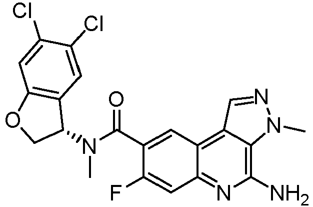 | 4-amino-N-((3S)-5,6-dichloro-2,3-dihydro-1-benzofuran-3-yl)-7-fluoro-N,3-dimethyl-3H-pyrazolo[3,4-c] quinoline-8-carboxamide | 460 |
| **484** | 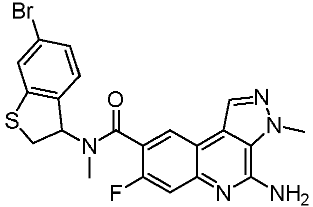 | 4-amino-N-((3R)-6-bromo-2,3-dihydro-1-benzothiophen-3-yl)-7-fluoro-N,3-dimethyl-3H-pyrazolo[3,4-c]quinoline-8-carboxamide and 4-amino-N-((3S)-6-bromo-2,3-dihydro-1-benzothiophen-3-yl)-7-fluoro-N,3-dimethyl-3H-pyrazolo[3,4-c]quinoline-8-carboxamide | 486.1, 488.1 |
| 485 | 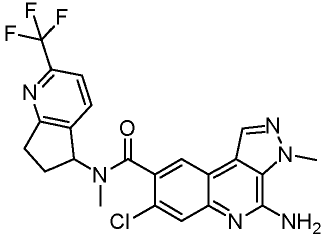 | 4-amino-7-chloro-N,3-dimethyl-N-((5R)-2-(trifluoromethyl)-6,7-dihydro-5H-cyclopenta[b]pyridin-5-yl)-3H-pyrazolo[3,4-c]quinoline-8-carboxamide and 4-amino-7-chloro-N,3-dimethyl-N-((5S)-2-(trifluoromethyl)-6,7-dihydro-5H-cyclopenta[b]pyridin-5-yl)-3H-pyrazolo[3,4-c]quinoline-8-carboxamide | 474.9 |
| 486 | 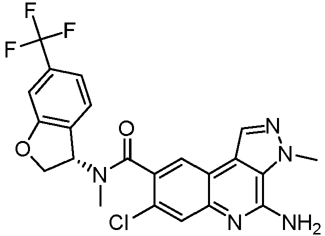 | 4-amino-7-chloro-N,3-dimethyl-N-((3S)-6-(trifluoromethyl)-2,3-dihydro-1-benzofuran-3-yl)-3H-pyrazolo[3,4-c] quinoline-8-carboxamide | 475.8 |
| 487 | 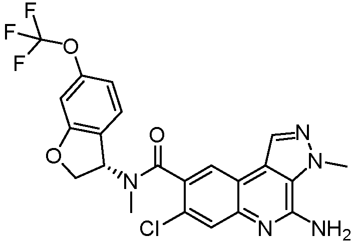 | 4-amino-7-chloro-N,3-dimethyl-N-((3S)-6-(trifluoromethoxy)-2,3-dihydro-1-benzofuran-3-yl)-3H-pyrazolo[3,4-c] quinoline-8-carboxamide | 492.1 |
| 488 | 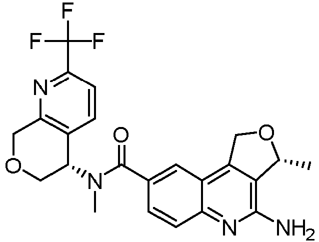 | (3R)-4-amino-N,3-dimethyl-N-((5S)-2-(trifluoromethyl)-5,8-dihydro-6H-pyrano[3,4-b]pyridin-5-yl)-1,3-dihydrofuro[3,4-c]quinoline-8-carboxamide | 459.2 |
| **489** | 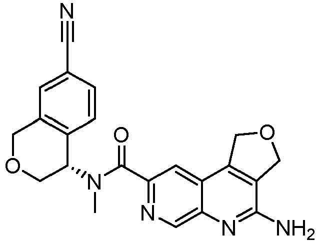 | 4-amino-N-((4S)-7-cyano-3,4-dihydro-1H-2-benzopyran-4-yl)-N-methyl-1,3-dihydrofuro[3,4-c][1,7]naphthyridine-8-carboxamide | 402.2 |
| **490** | 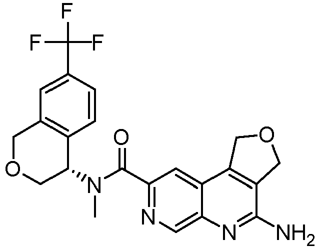 | 4-amino-N-methyl-N-((4S)-7-(trifluoromethyl)-3,4-dihydro-1H-2-benzopyran-4-yl)-1,3-dihydrofuro[3,4-c][1,7]naphthyridine-8-carboxamide | 445.0 |
| **491** | 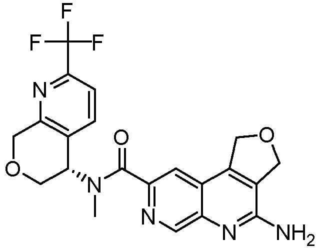 | 4-amino-N-methyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamide | 446.0 |
| **492** | 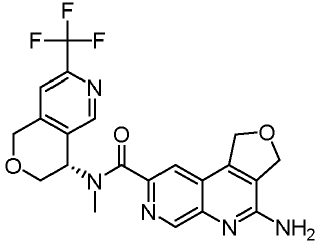 | 4-amino-N-methyl-N-((4S)-7-(trifluoromethyl)-3,4-dihydro-1H-pyrano[4,3-c]pyridin-4-yl)-1,3-dihydrofuro[3,4-c][1,7]naphthyridine-8-carboxamide | 446.0 |
| **493** | 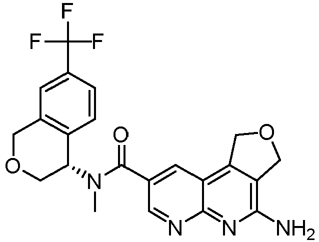 | 4-amino-N-methyl-N-((4S)-7-(trifluoromethyl)-3,4-dihydro-1H-2-benzopyran-4-yl)-1,3-dihydrofuro[3,4-c] [1,8]naphthyridine-8-carboxamide | 445.0 |
| **494** | 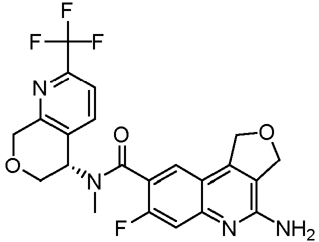 | 4-amino-7-fluoro-N-methyl-N-((5S)-2-(trifluoromethyl)-5,8-dihydro-6H-pyrano[3,4-b]pyridin-5-yl)-1,3-dihydrofuro[3,4-c]quinoline-8-carboxamide | 463.2 |
| **495** | 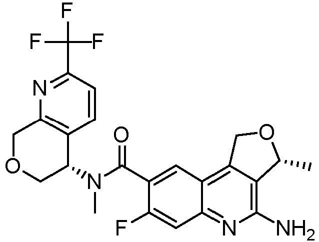 | (3R)-4-amino-7-fluoro-N,3-dimethyl-N-((5S)-2-(trifluoromethyl)-5,8-dihydro-6H-pyrano[3,4-b]pyridin-5-yl)-1,3-dihydrofuro[3,4-c]quinoline-8-carboxamide | 477.2 |
| **496** | 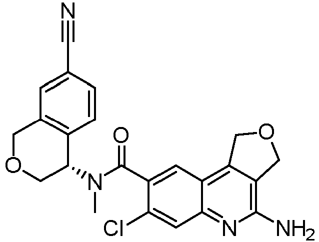 | 4-amino-7-chloro-N-((4S)-7-cyano-3,4-dihydro-1H-2-benzopyran-4-yl)-N-methyl-1,3-dihydrofuro[3,4-c]quinoline-8-carboxamide | 435.2 |
| **497** | 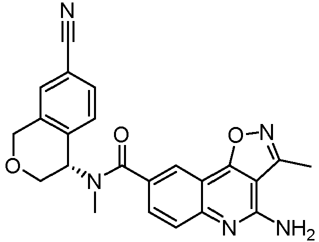 | 4-amino-N-((4S)-7-cyano-3,4-dihydro-1H-2-benzopyran-4-yl)-N,3-dimethyl[1,2]oxazolo[4,5-c]quinoline-8-carboxamide | 414.0 |
| **498** | 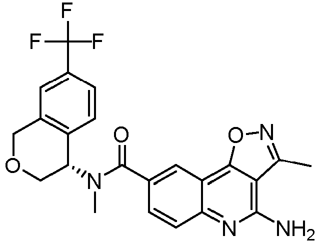 | 4-amino-N,3-dimethyl-N-((4S)-7-(trifluoromethyl)-3,4-dihydro-1H-2-benzopyran-4-yl)[1,2]oxazolo[4,5-c] quinoline-8-carboxamide | 457.0 |
| **499** | 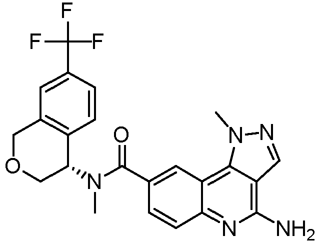 | 4-amino-N,1-dimethyl-N-((4S)-7-(trifluoromethyl)-3,4-dihydro-1H-2-benzopyran-4-yl)-1H-pyrazolo[4,3-c] quinoline-8-carboxamide | 456.2 |
| **500** | 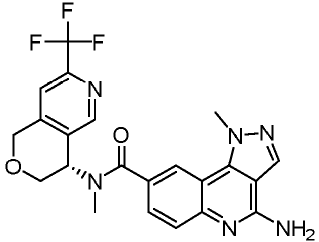 | 4-amino-N,1-dimethyl-N-((4S)-7-(trifluoromethyl)-3,4-dihydro-1H-pyrano[4,3-c]pyridin-4-yl)-1H-pyrazolo[4,3-c]quinoline-8-carboxamide | 457.0 |
| **501** | 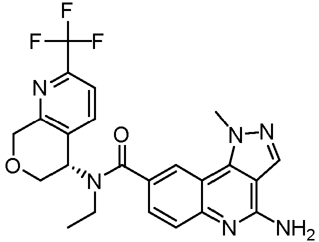 | 4-amino-N-ethyl-1-methyl-N-((5S)-2-(trifluoromethyl)-5,8-dihydro-6H-pyrano[3,4-b]pyridin-5-yl)-1H-pyrazolo[4,3-c]quinoline-8-carboxamide | 471.2 |
| **502** | 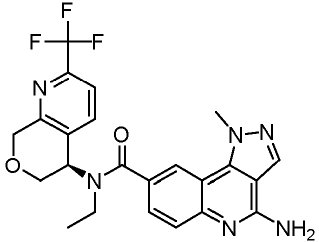 | 4-amino-N-ethyl-1-methyl-N-((5R)-2-(trifluoromethyl)-5,8-dihydro-6H-pyrano[3,4-b]pyridin-5-yl)-1H-pyrazolo[4,3-c]quinoline-8-carboxamide | 471.2 |
| **503** | 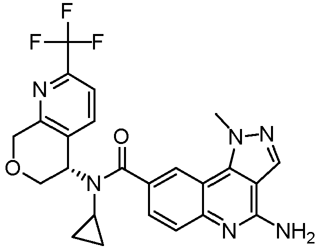 | 4-amino-N-cyclopropyl-1-methyl-N-((5S)-2-(trifluoromethyl)-5,8-dihydro-6H-pyrano[3,4-b]pyridin-5-yl)-1H-pyrazolo[4,3-c]quinoline-8-carboxamide | 483.2 |
| **504** | 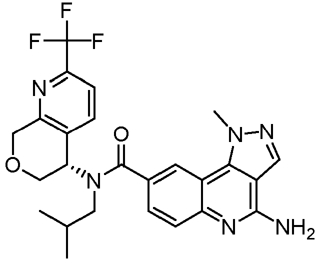 | 4-amino-1-methyl-N-(2-methylpropyl)-N-((55)-2-(trifluoromethyl)-5,8-dihydro-6H-pyrano[3,4-b]pyridin-5-yl)-1H-pyrazolo[4,3-c]quinoline-8-carboxamide | 499.2 |
| **505** | 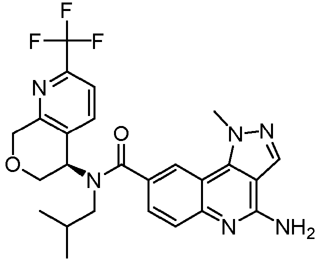 | 4-amino-1-methyl-N-(2-methylpropyl)-N-((5R)-2-(trifluoromethyl)-5,8-dihydro-6H-pyrano[3,4-b]pyridin-5-yl)-1H-pyrazolo[4,3-c]quinoline-8-carboxamide | 499.2 |
| **506** | 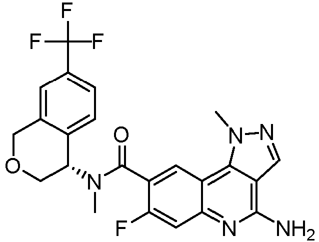 | 4-amino-7-fluoro-N,1-dimethyl-N-((4S)-7-(trifluoromethyl)-3,4-dihydro-1H-2-benzopyran-4-yl)-1H-pyrazolo[4,3-c] quinoline-8-carboxamide | 474.2 |
| **507** | 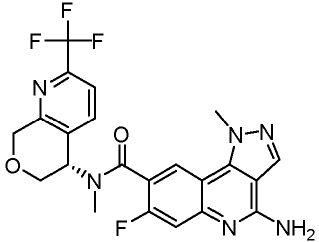 | 4-amino-7-fluoro-N, 1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide | 475.0 |
| **508** | 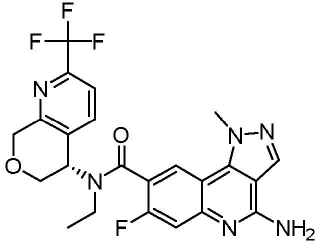 | 4-amino-N-ethyl-7-fluoro-1-methyl-N-((5S)-2-(trifluoromethyl)-5,8-dihydro-6H-pyrano[3,4-b]pyridin-5-yl)-1H-pyrazolo[4,3-c]quinoline-8-carboxamide | 489.2 |
| **509** | 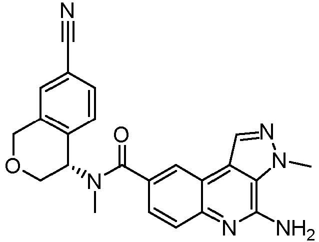 | 4-amino-N-((4S)-7-cyano-3,4-dihydro-1H-2-benzopyran-4-yl)-N,3-dimethyl-3H-pyrazolo[3,4-c]quinoline-8-carboxamide | 413.0 |
| **510** | 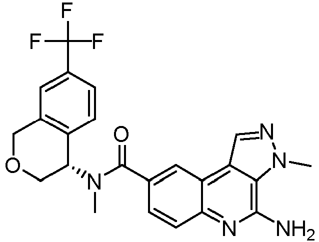 | 4-amino-N,3-dimethyl-N-((4S)-7-(trifluoromethyl)-3,4-dihydro-1H-2-benzopyran-4-yl)-3H-pyrazolo[3,4-c] quinoline-8-carboxamide | 456.0 |
| **511** | 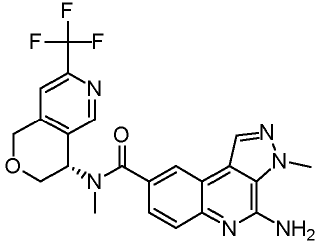 | 4-amino-N,3-dimethyl-N-((4S)-7-(trifluoromethyl)-3,4-dihydro-1H-pyrano[4,3-c]pyridin-4-yl)-3H-pyrazolo[3,4-c]quinoline-8-carboxamide | 457.0 |
| **512** | 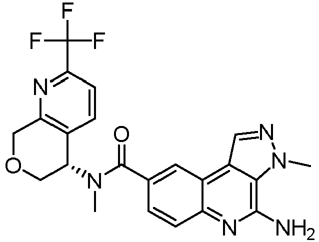 | 4-amino-N,3-dimethyl-N-((5S)-2-(trifluoromethyl)-5,8-dihydro-6H-pyrano [3,4-b]pyridin-5-yl)-3H-pyrazolo[3,4-c]quinoline-8-carboxamide | 457.0 |
| **513** | 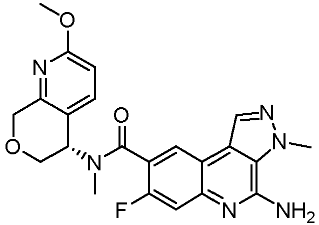 | 4-amino-7-fluoro-N-((5S)-2-methoxy-5,8-dihydro-6H-pyrano[3,4-b]pyridin-5-yl)-N,3-dimethyl-3H-pyrazolo[3,4-c] quinoline-8-carboxamide | 437.0 |
| **514** | 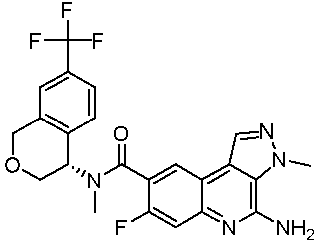 | 4-amino-7-fluoro-N,3-dimethyl-N-((4S)-7-(trifluoromethyl)-3,4-dihydro-1H-2-benzopyran-4-yl)-3H-pyrazolo[3,4-c] quinoline-8-carboxamide | 474.0 |
| **515** | 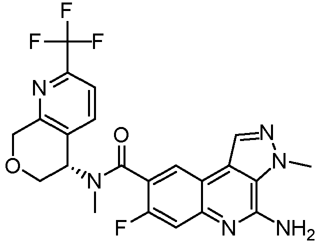 | 4-amino-7-fluoro-N,3-dimethyl-N-((5S)-2-(trifluoromethyl)-5,8-dihydro-6H-pyrano [3,4-b]pyridin-5-yl)-3H-pyrazolo[3,4-c]quinoline-8-carboxamide | 475.0 |
| **516** | 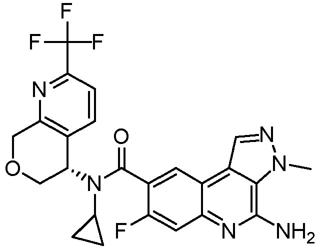 | 4-amino-N-cyclopropyl-7-fluoro-3-methyl-N-((5 S)-2-(trifluoromethyl)-5,8-dihydro-6H-pyrano[3,4-b]pyridin-5-yl)-3H-pyrazolo[3,4-c]quinoline-8-carboxamide | 501.2 |
| **517** | 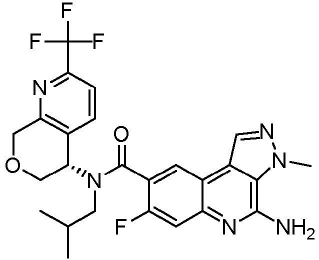 | 4-amino-7-fluoro-3-methyl-N-(2-methylpropyl)-N-((5 S)-2-(trifluoromethyl)-5,8-dihydro-6H-pyrano [3,4-b]pyridin-5-yl)-3H-pyrazolo[3,4-c]quinoline-8-carboxamide | 517.2 |
| **518** | 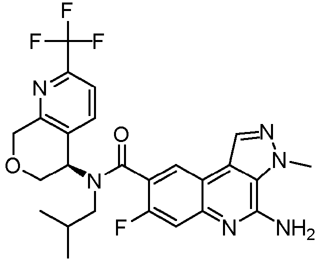 | 4-amino-7-fluoro-3-methyl-N-(2-methylpropyl)-N-((5R)-2-(trifluoromethyl)-5,8-dihydro-6H-pyrano [3,4-b]pyridin-5-yl)-3H-pyrazolo[3,4-c]quinoline-8-carboxamide | 517.2 |
| **519** | 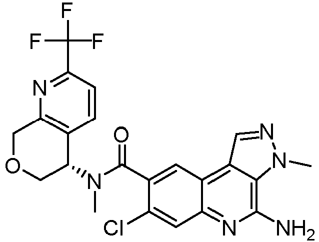 | 4-amino-7-chloro-N,3-dimethyl-N-((5S)-2-(trifluoromethyl)-5,8-dihydro-6H-pyrano [3,4-b]pyridin-5-yl)-3H-pyrazolo[3,4-c]quinoline-8-carboxamide | 491 |
| **520** | 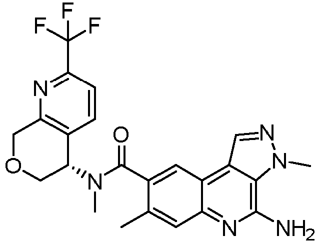 | 4-amino-N,3,7-trimethyl-N-((5S)-2-(trifluoromethyl)-5,8-dihydro-6H-pyrano [3,4-b]pyridin-5-yl)-3H-pyrazolo[3,4-c]quinoline-8-carboxamide | 471.2 |
| **521** | 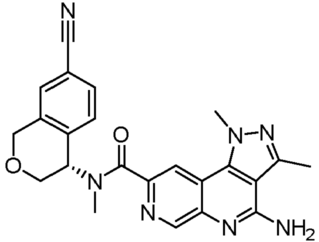 | 4-amino-N-((4S)-7-cyano-3,4-dihydro-1H-2-benzopyran-4-yl)-N,1,3-trimethyl-1H-pyrazolo[4,3-c][1,7]naphthyridine-8-carboxamide | 428.0 |

### Examples 522 and 523: 4-amino-N-methyl-N-(6-(trifluoromethyl)-2,3-dihydrobenzofuran-3-yl)-1,3-dihydrofuro[3,4-c][1,7]naphthyridine-8-carboxamide

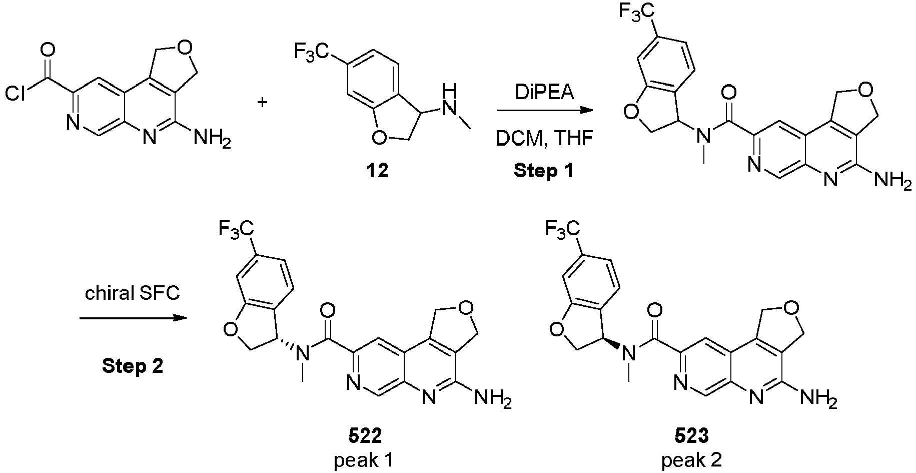

The acid chloride used in Step 1 was synthesized in the same manner as in Step 1 towards the synthesis of 442.

Step 1. To a stirred ice-cooled solution of N-methyl-6-(trifluoromethyl)-2,3-dihydrobenzofuran-3-amine (12) (70.2 mg, 0.323 mmol) in DCM (1.5 mL) and THF (1.5 mL) was added 4-amino-1,3-dihydrofuro[3,4-c][1,7]naphthyridine-8-carbonyl chloride (85 mg, 0.340 mmol) followed by N-ethyl-N-isopropylpropan-2-amine (88 mg, 0.119 mL, 0.681 mmol, Sigma-Aldrich Corporation). The resulting mixture was stirred at 0 °C for 5 min and at rt for 1 h. The crude mixture was directly loaded onto a silica gel precolumn (25 g) and subjected to combi-flash column chromatography on a 24-g ISCO gold column eluting with MeOH (with 0.5% ammonium hydroxide)/DCM (0 to 16%) to give 4-amino-N-methyl-N-(6-(trifluoromethyl)-2,3-dihydrobenzofuran-3-yl)-1,3-dihydrofuro[3,4-c][1,7]naphthyridine-8-carboxamide (92 mg, 0.214 mmol, 62.8% yield) as a white solid. *m*/*z* (ESI): 431.05 (M+H)⁺. ¹H NMR (METHANOL-d₄, 400 MHz) δ 8.8-9.0 (m, 1H), 7.8-8.0 (m, 1H), 7.6-7.7 (m, 1H), 7.2-7.3 (m, 1H), 7.0-7.1 (m, 1H), 6.0-6.6 (m, 1H), 5.45 (br s, 2H), 5.15 (t, 2H, J=3.4 Hz), 4.75 (br s, 2H), 2.80 (br d, 3H, J=10.2 Hz).

Step 2. 4-Amino-N-methyl-N-(6-(trifluoromethyl)-2,3-dihydrobenzofuran-3-yl)-1,3-dihydrofuro[3,4-c][1,7]naphthyridine-8-carboxamide was resolved via preparative SFC using a Chiral Technologies AS column (250 x 21 mm, 5 mm) with a mobile phase of 80% Liquid CO₂ and 20% MeOH with 0.2% TEA using a flow rate of 80 mL/min to generate (S)-4-amino-N-methyl-N-(6-(trifluoromethyl)-2,3-dihydrobenzofuran-3-yl)-1,3-dihydrofuro[3,4-c][1,7]naphthyridine-8-carboxamide (42.6 mg, 0.099 mmol, 29.1 % yield) (522) as the first eluting enantiomer and (R)-4-amino-N-methyl-N-(6-(trifluoromethyl)-2,3-dihydrobenzofuran-3-yl)-1,3-dihydrofuro[3,4-c][1,7]naphthyridine-8-carboxamide (44.7 mg, 0.104 mmol, 30.5 % yield) (523) as the second eluting enantiomer, each as a white solid with > 99% ee. ¹H NMR (DMSO-d₆, 500 MHz) δ 8.8-9.0 (m, 1H), 7.8-7.9 (m, 1H), 7.5-7.8 (m, 1H), 7.32 (br d, 1H, J=7.3 Hz), 7.2-7.3 (m, 1H), 7.05 (br d, 2H, *J=13.0* Hz), 5.9-6.5 (m, 1H), 5.38 (br s, 2H), 5.05 (br s, 2H), 4.6-4.9 (m, 2H), 2.6-2.8 (m, 3H).

Examples in Table 15 were prepared in a manner similar to that described for Example 522 and 523.

**Table 15**

| **Ex.** | **Structure** | **Name** | **m/z (ESI): (M+H)⁺** | **SFC Conditions** |
|---|---|---|---|---|
| **524** | 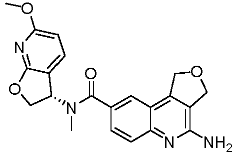 | 4-amino-N-((3S)-6-methoxy-2,3-dihydrofuro[2,3-b]pyridin-3-yl)-N-methyl-1,3-dihydrofuro[3,4-c]quinoline-8-carboxamide | 393.1 | 1st peak, Chiral Technologies AS column (250 x 21 mm, 5 mm) with a mobile phase of 82% Liquid CO₂ and 18% MeOH with 0.2% TEA using a flow rate of 80 mL/min |
| **525** | 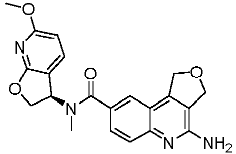 | 4-amino-N-((3R)-6-methoxy-2,3-dihydrofuro[2,3-b]pyridin-3-yl)-N-methyl-1,3-dihydrofuro[3,4-c]quinoline-8-carboxamide | 393.1 | 2nd peak, Chiral Technologies AS column (250 x 21 mm, 5 mm) with a mobile phase of 82% Liquid CO₂ and 18% MeOH with 0.2% TEA using a flow rate of 80 mL/min |
| **526** | 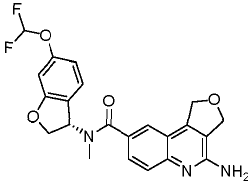 | 4-amino-N-((3S)-6-(difluoromethoxy)-2,3-dihydro-1-benzofuran-3-yl)-N-methyl-1,3-dihydrofuro[3,4-c]quinoline-8-carboxamide | 428.2 | 1st peak, SFC using a ChiralcelOJ column (3 x 15 cm, 5 micron) with a mobile phase of 75% Liquid CO₂ and 25% MeOH with 0.1% diethylamine using a flow rate of 60 mL/min |
| **527** | 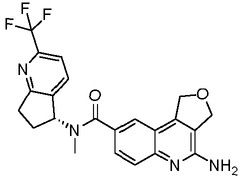 | 4-amino-N-methyl-N-((5R)-2-(trifluoromethyl)-6,7-dihydro-5H-cyclopenta[b]pyridin-5-yl)-1,3-dihydrofuro[3,4-c]quinoline-8-carboxamide | 428.8 | 2nd peak, Chiral Technologies AD column (250 x 21 mm, 5 mm) with a mobile phase of 75% Liquid CO₂ and 25% iPrOH with 0.2% TEA using a flow rate of 100 mL/min |
| **528** | 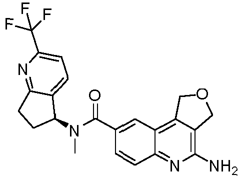 | 4-amino-N-methyl-N-((5 S)-2-(trifluoromethyl)-6,7-dihydro-5H-cyclopenta[b]pyridin-5-yl)-1,3-dihydrofuro[3,4-c]quinoline-8-carboxamide | 428.8 | 1st peak, Chiral Technologies AD column (250 x 21 mm, 5 mm) with a mobile phase of 75% Liquid CO₂ and 25% iPrOH with 0.2% TEA using a flow rate of 100 mL/min |
| **529** | 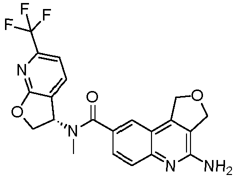 | 4-amino-N-methyl-N-((3 S)-6-(trifluoromethyl)-2,3-dihydrofuro[2,3-b]pyridin-3-yl)-1,3-dihydrofuro[3,4-c]quinoline-8-carboxamide | 430.8 | 1st peak, Chiral Technologies IG column (250 x 21 mm, 5 mm) x 2 with a mobile phase of 60% Liquid CO₂ and 40% MeOH:ACN 1:1 using a flow rate of 50 mL/min |
| **530** | 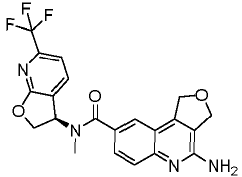 | 4-amino-N-methyl-N-((3R)-6-(trifluoromethyl)-2,3-dihydrofuro[2,3-b]pyridin-3-yl)-1,3-dihydrofuro[3,4-c]quinoline-8-carboxamide | 430.8 | 2nd peak, Chiral Technologies IG column (250 x 21 mm, 5 mm) x 2 with a mobile phase of 60% Liquid CO₂ and 40% MeOH:ACN 1:1 using a flow rate of 50 mL/min |
| **531** | 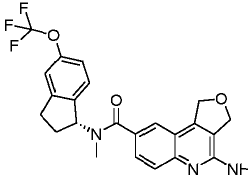 | 4-amino-N-methyl-N-((1R)-5-(trifluoromethoxy)-2,3-dihydro-1H-inden-1-yl)-1,3-dihydrofuro[3,4-c]quinoline-8-carboxamide | 443.8 | 1st peak, Chiral Technologies AS column (250 x 21 mm, 5 mm) with a mobile phase of 85% Liquid CO₂ and 15% MeOH with 0.2% TEA using a flow rate of 100 mL/min |
| **532** | 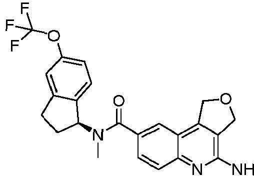 | 4-amino-N-methyl-N-((1S)-5-(trifluoromethoxy)-2,3-dihydro-1H-inden-1-yl)-1,3-dihydrofuro[3,4-c]quinoline-8-carboxamide | 443.8 | 2nd peak, Chiral Technologies AS column (250 x 21 mm, 5 mm) with a mobile phase of 85% Liquid CO₂ and 15% MeOH with 0.2% TEA using a flow rate of 100 mL/min |
| **533** | 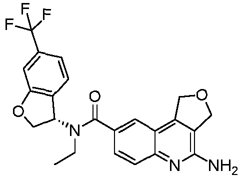 | 4-amino-N-ethyl-N-((3S)-6-(trifluoromethyl)-2,3-dihydro-1-benzofuran-3-yl)-1,3-dihydrofuro[3,4-c]quinoline-8-carboxamide | 444.2 | 1st peak, Chiral Technologies AD column (250 x 21 mm, 5 mm) with a mobile phase of 75% Liquid CO₂ and 25% MeOH with 0.2% TEA using a flow rate of 100 mL/min |
| **534** | 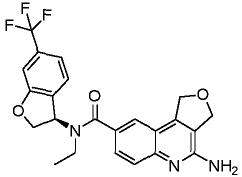 | 4-amino-N-ethyl-N-((3R)-6-(trifluoromethyl)-2,3-dihydro-1-benzofuran-3-yl)-1,3-dihydrofuro[3,4-c]quinoline-8-carboxamide | 444.2 | 2nd peak, Chiral Technologies AD column (250 x 21 mm, 5 mm) with a mobile phase of 75% Liquid CO₂ and 25% MeOH with 0.2% TEA using a flow rate of 100 mL/min |
| **535** | 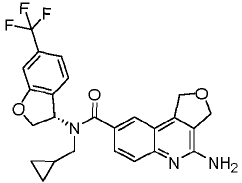 | (4S,6R)-4-(3-chloro-5-fluorophenyl)-1-(2-hydroxyethyl)-6-(3-methylphenyl)-2-piperidinone | 470.2 | 1st peak, Chiral Technologies OJ column (250 x 21 mm, 5 mm) with a mobile phase of 70% Liquid CO₂ and 30% MeOH with 0.2% TEA using a flow rate of 80 mL/min |
| **536** | 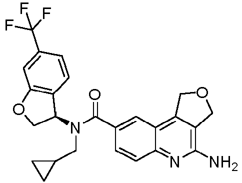 | N-(6,8-dichloro-2-(3-chloro-4-(1,2,4-oxadiazol-5-yl)phenyl)imidazo[1,2-b]pyridazin-3-yl)-2,2,2-trifluoroacetamide | 470.2 | 2nd peak, Chiral Technologies OJ column (250 x 21 mm, 5 mm) with a mobile phase of 70% Liquid CO₂ and 30% MeOH with 0.2% TEA using a flow rate of 80 mL/min |
| **537** | 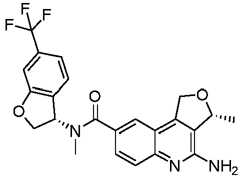 | (3R)-4-amino-N,3-dimethyl-N-((3S)-6-(trifluoromethyl)-2,3-dihydro-1-benzofuran-3-yl)-1,3-dihydrofuro[3,4-c]quinoline-8-carboxamide | 444.2 | 1st peak, Chiral Technologies AS column (250 x 21 mm, 5 mm) with a mobile phase of 85% Liquid CO₂ and 15% MeOH with 0.2% TEA using a flow rate of 80 mL/min |
| **538** | 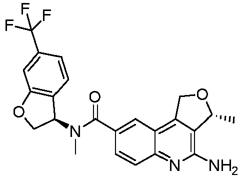 | (3R)-4-amino-N,3-dimethyl-N-((3R)-6-(trifluoromethyl)-2,3-dihydro-1-benzofuran-3-yl)-1,3-dihydrofuro[3,4-c]quinoline-8-carboxamide | 444.2 | 2nd peak, Chiral Technologies AS column (250 x 21 mm, 5 mm) with a mobile phase of 85% Liquid CO₂ and 15% MeOH with 0.2% TEA using a flow rate of 80 mL/min |
| **539** | 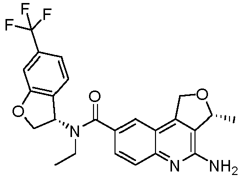 | (3R)-4-amino-N-ethyl-3-methyl-N-((3S)-6-(trifluoromethyl)-2,3-dihydro-1-benzofuran-3-yl)-1,3-dihydrofuro[3,4-c]quinoline-8-carboxamide | 445.2 | 1st peak, Chiral Technologies AD column (250 x 21 mm, 5 mm) with a mobile phase of 80% Liquid CO₂ and 20% MeOH with 0.2% TEA using a flow rate of 80 mL/min |
| **540** | 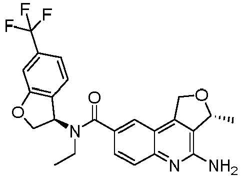 | (3R)-4-amino-N-ethyl-3-methyl-N-((3R)-6-(trifluoromethyl)-2,3-dihydro-1-benzofuran-3-yl)-1,3-dihydrofuro[3,4-c]quinoline-8-carboxamide | 445.2 | 2nd peak, Chiral Technologies AD column (250 x 21 mm, 5 mm) with a mobile phase of 80% Liquid CO₂ and 20% MeOH with 0.2% TEA using a flow rate of 80 mL/min |
| **541** | 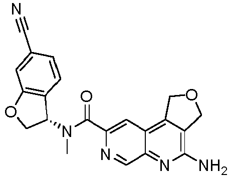 | 4-amino-N-((3S)-6-cyano-2,3-dihydro-1-benzofuran-3-yl)-N-methyl-1,3-dihydrofuro[3,4-c][1,7]naphthyridine-8-carboxamide | 388.2 | 1st peak, SFC using a Chiralpak AD column (2 x 25 cm, 5 micron) with a mobile phase of 70% Liquid CO₂ and 30% MeOH with 0.1% diethylamine using a flow rate of 70 mL/min |
| **542** | 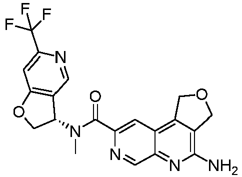 | 4-amino-N-methyl-N-((3S)-6-(trifluoromethyl)-2,3-dihydrofuro[3,2-c]pyridin-3-yl)-1,3-dihydrofuro[3,4-c][1,7]naphthyridine-S-carboxamide | 432.2 | 1st peak, SFC using a Chiralcel OX column (21 x250 mm, 5 micron) with a mobile phase of 60% Liquid CO₂ and 40% MeOH with 0.2% diethylamine using a flow rate of 70 mL/min |
| **543** | 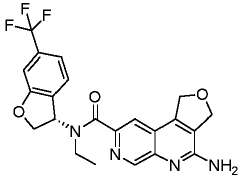 | 4-amino-N-ethyl-N-((3S)-6-(trifluoromethyl)-2,3-dihydro-1-benzofuran-3-yl)-1,3-dihydrofuro[3,4-c][1,7]naphthyridine-8-carboxamide | 445.2 | 1st peak, Chiral Technologies AD column (250 x 21 mm, 5 mm) with a mobile phase of 80% Liquid CO₂ and 20% MeOH with 0.2% TEA using a flow rate of 80 mL/min |
| **544** | 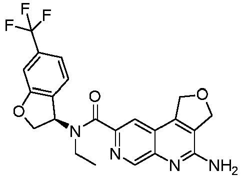 | 4-amino-N-ethyl-N-((3R)-6-(trifluoromethyl)-2,3-dihydro-1-benzofuran-3-yl)-1,3-dihydrofuro[3,4-c][1,7]naphthyridine-8-carboxamide | 445.2 | 2nd peak, Chiral Technologies AD column (250 x 21 mm, 5 mm) with a mobile phase of 80% Liquid CO₂ and 20% MeOH with 0.2% TEA using a flow rate of 80 mL/min |
| **545** | 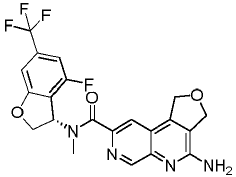 | 4-amino-N-((3S)-4-fluoro-6-(trifluoromethyl)-2,3-dihydro-1-benzofuran-3-yl)-N-methyl-1,3-dihydrofuro[3,4-c][1,7]naphthyridine-8-carboxamide | 449 | 1st peak, Chiralpak AD column (21 x 150 mm, 5 micron) with a mobile phase of 65% Liquid CO₂ and 35% MeOH with 0.2% diethylamine using a flow rate of 80 mL/min |
| **546** | 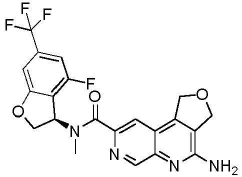 | 4-amino-N-((3R)-4-fluoro-6-(trifluoromethyl)-2,3-dihydro-1-benzofuran-3-yl)-N-methyl-1,3-dihydrofuro[3,4-c][1,7]naphthyridine-8-carboxamide | 449 | 2nd peak, Chiralpak AD column (21 x 150 mm, 5 micron) with a mobile phase of 65% Liquid CO₂ and 35% MeOH with 0.2% diethylamine using a flow rate of 80 mL/min |
| **547** | 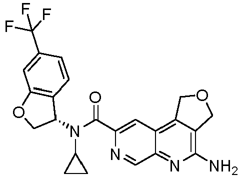 | 4-amino-N-cyclopropyl-N-((3S)-6-(trifluoromethyl)-2,3-dihydro-1-benzofuran-3-yl)-1,3-dihydrofuro[3,4-c][1,7]naphthyridine-S-carboxamide | 457.15 | 2nd peak, SFC Chiral Technologies OJ column (250 x 21 mm, 5 mm) with a mobile phase of 80% Liquid CO₂ and 20% MeOH with 0.2% TEA using a flow rate of 80 mL/min |
| **548** | 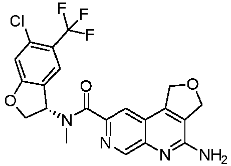 | 4-amino-N-((3S)-6-chloro-5-(trifluoromethyl)-2,3-dihydro-1-benzofuran-3-yl)-N-methyl-1,3-dihydrofuro[3,4-c][1,7]naphthyridine-8-carboxamide | 464.9 | 1st peak, Chiralpak AD-H column (250 x 30 mm, 5 micron) with a mobile phase 60% Liquid CO₂ and 40% MeOH using a flow rate of 120 mL/ min |
| **549** | 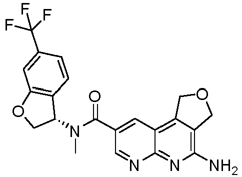 | 4-amino-N-methyl-N-((3S)-6-(trifluoromethyl)-2,3-dihydro-1-benzofuran-3-yl)-1,3-dihydrofuro[3,4-c][1,8]naphthyridine-8-carboxamide | 431.2 | 1st peak, Chiral Technologies AS column (250 x 21 mm, 5 mm) with a mobile phase of 85% Liquid CO₂ and 15% MeOH with 0.2% TEA using a flow rate of 80 mL/min |
| **550** | 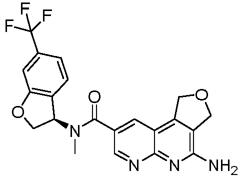 | 4-amino-N-methyl-N-((3R)-6-(trifluoromethyl)-2,3-dihydro-1-benzofuran-3-yl)-1,3-dihydrofuro[3,4-c][1,8]naphthyridine-8-carboxamide | 431.2 | 2nd peak, Chiral Technologies AS column (250 x 21 mm, 5 mm) with a mobile phase of 85% Liquid CO₂ and 15% MeOH with 0.2% TEA using a flow rate of 80 mL/min |
| **551** | 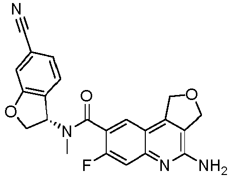 | 4-amino-N-((3S)-6-cyano-2,3-dihydro-1-benzofuran-3-yl)-7-fluoro-N-methyl-1,3-dihydrofuro[3,4-c]quinoline-8-carboxamide | 404.1 | 1st peak, SFC using a Chiralcel OJ column (2 x 25 cm, 5 micron) with a mobile phase of 70% Liquid CO₂ and 30% MeOH with 0.1% diethylamine using a flow rate of 65 mL/min |
| **552** | 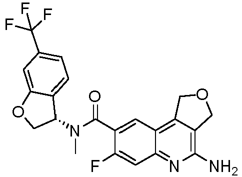 | 4-amino-7-fluoro-N-methyl-N-((3S)-6-(trifluoromethyl)-2,3-dihydro-1-benzofuran-3-yl)-1,3-dihydrofuro[3,4-c]quinoline-8-carboxamide | 448.15 | 1st peak, Chiral Technologies OD column (250 x 21 mm, 5 mm) with a mobile phase of 90% Liquid CO₂ and 10% MeOH with 0.2% TEA using a flow rate of 80 mL/min |
| **553** | 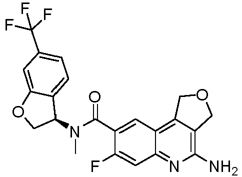 | 4-amino-7-fluoro-N-methyl-N-((3R)-6-(trifluoromethyl)-2,3-dihydro-1-benzofuran-3-yl)-1,3-dihydrofuro[3,4-c]quinoline-8-carboxamide | 448.15 | 2nd peak, Chiral Technologies OD column (250 x 21 mm, 5 mm) with a mobile phase of 90% Liquid CO₂ and 10% MeOH with 0.2% TEA using a flow rate of 80 mL/min |
| **554** | 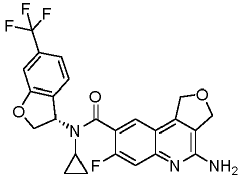 | 4-amino-N-cyclopropyl-7-fluoro-N-((3S)-6-(trifluoromethyl)-2,3-dihydro-1-benzofuran-3-yl)-1,3-dihydrofuro[3,4-c]quinoline-8-carboxamide | 474.1 | 2nd peak, Whelk-O-SS column (250 x 30 mm, 5 µm) x 2 with a mobile phase of 65% Liquid CO₂ and 35% MeOH with 0.2% TEA using a flow rate of 110 mL/min |
| **555** | 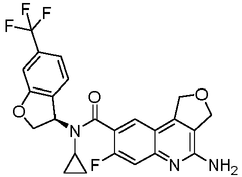 | 4-amino-N-cyclopropyl-7-fluoro-N-((3R)-6-(trifluoromethyl)-2,3-dihydro-1-benzofuran-3-yl)-1,3-dihydrofuro[3,4-c]quinoline-8-carboxamide | 474.1 | 1st peak, Whelk-O-SS column (250 x 30 mm, 5 µm) x 2 with a mobile phase of 65% Liquid CO₂ and 35% MeOH with 0.2% TEA using a flow rate of 110 mL/min |
| **556** | 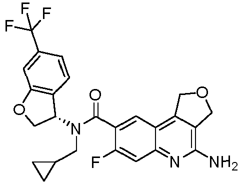 | 4-amino-N-(cyclopropylmethyl)-7-fluoro-N-((3S)-6-(trifluoromethyl)-2,3-dihydro-1-benzofuran-3-yl)-1,3-dihydrofuro[3,4-c]quinoline-8-carboxamide | 488.2 | 1st peak, Chiralcel OJ column (21 x 150mm 5 micron) with a mobile phase of 75% Liquid CO₂ and 25% MeOH with 0.2% diethylamine using a flow rate of 80 mL/min |
| **557** | 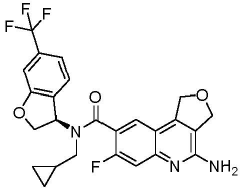 | 4-amino-N-(cyclopropylmethyl)-7-fluoro-N-((3R)-6-(trifluoromethyl)-2,3-dihydro-1-benzofuran-3-yl)-1,3-dihydrofuro[3,4-c]quinoline-8-carboxamide | 488.2 | 2nd peak, Chiralcel OJ column (21 x 150mm 5 micron) with a mobile phase of 75% Liquid CO₂ and 25% MeOH with 0.2% diethylamine using a flow rate of 80 mL/min |
| **558** | 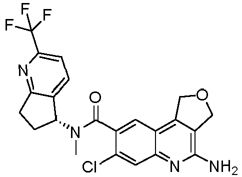 | 4-amino-7-chloro-N-methyl-N-((5R)-2-(trifluoromethyl)-6,7-dihydro-5H-cyclopenta[b]pyridin-5-yl)-1,3-dihydrofuro[3,4-c]quinoline-8-carboxamide | 462.8 | 2nd peak, 2 x Chiralpak IC column (21 x 150 mm, 5 micron) with a mobile phase of 70% Liquid CO₂ and 30% ethanol with 0.2% diethylamine using a flow rate of 80 mL/min |
| **559** | 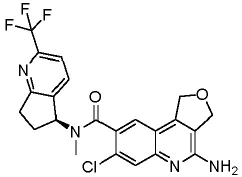 | 4-amino-7-chloro-N-methyl-N-((5S)-2-(trifluoromethyl)-6,7-dihydro-5H-cyclopenta[b]pyridin-5-yl)-1,3-dihydrofuro[3,4-c] quinoline-8-carboxamide | 462.8 | 1st peak, 2 x Chiralpak IC column (21 x 150 mm, 5 micron) with a mobile phase of 70% Liquid CO₂ and 30% ethanol with 0.2% diethylamine using a flow rate of 80 mL/min |
| **560** | 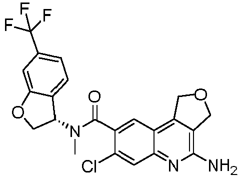 | 4-amino-7-chloro-N-methyl-N-((3S)-6-(trifluoromethyl)-2,3-dihydro-1-benzofuran-3-yl)-1,3-dihydrofuro[3,4-c]quinoline-8-carboxamide | 464.05 | 1st peak, Chiral Technologies OD column (250 x 21 mm, 5 mm) and OD (150 x 21 mm, 5 mm) with a mobile phase of 90% Liquid CO₂ and 10% MeOH with 0.2% TEA using a flow rate of 80 mL/min |
| **561** | 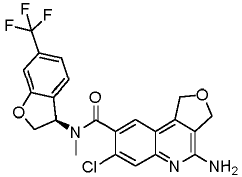 | 4-amino-7-chloro-N-methyl-N-((3R)-6-(trifluoromethyl)-2,3-dihydro-1-benzofuran-3-yl)-1,3-dihydrofuro[3,4-c]quinoline-8-carboxamide | 464.05 | 2nd peak, Chiral Technologies OD column (250 x 21 mm, 5 mm) and OD (150 x 21 mm, 5 mm) with a mobile phase of 90% Liquid CO₂ and 10% MeOH with 0.2% TEA using a flow rate of 80 mL/min |
| **562** | 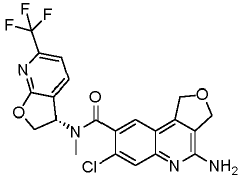 | 4-amino-7-chloro-N-methyl-N-((3S)-6-(trifluoromethyl)-2,3-dihydrofuro[2,3-b]pyridin-3-yl)-1,3-dihydrofuro[3,4-c]quinoline-8-carboxamide | 464.6 | 1st peak, Chiral Technologies AD column (150 x 21 mm, 5 mm) with a mobile phase of 65% Liquid CO₂ and 35% MeOH with 0.2% TEA using a flow rate of 80 mL/min |
| **563** | 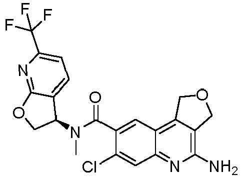 | 4-amino-7-chloro-N-methyl-N-((3R)-6-(trifluoromethyl)-2,3-dihydrofuro[2,3-b]pyridin-3-yl)-1,3-dihydrofuro[3,4-c]quinoline-8-carboxamide | 464.6 | 2nd peak, Chiral Technologies AD column (150 x 21 mm, 5 mm) with a mobile phase of 65% Liquid CO₂ and 35% MeOH with 0.2% TEA using a flow rate of 80 mL/min |
| **564** | 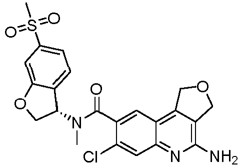 | 4-amino-7-chloro-N-methyl-N-((3S)-6-(methylsulfonyl)-2,3-dihydro-1-benzofuran-3-yl)-1,3-dihydrofuro[3,4-c]quinoline-8-carboxamide | 474 | 1st peak, Chiralpak AS column (21 x 250 mm, 5 micron) with a mobile phase 70% Liquid CO₂ and 30% MeOH with 0.2% diethylamine using a flow rate of 80 mL/min |
| **565** | 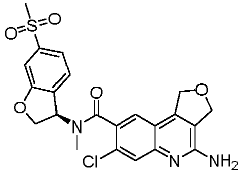 | 4-amino-7-chloro-N-methyl-N-((3R)-6-(methylsulfonyl)-2,3-dihydro-1-benzofuran-3-yl)-1,3-dihydrofuro[3,4-c]quinoline-8-carboxamide | 474 | 2nd peak, Chiralpak AS column (21 x 250 mm, 5 micron) with a mobile phase 70% Liquid CO₂ and 30% MeOH with 0.2% diethylamine using a flow rate of 80 mL/min |
| **566** | 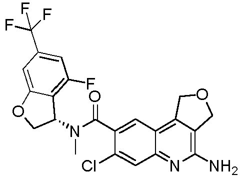 | 4-amino-7-chloro-N-((3S)-4-fluoro-6-(trifluoromethyl)-2,3-dihydro-1-benzofuran-3-yl)-N-methyl-1,3-dihydrofuro[3,4-c]quinoline-8-carboxamide | 482 | 1st peak, ChromegaChiral CCC column (21 x 250 mm, 5 micron) with a mobile phase of 65% Liquid CO₂ and 35% MeOH with 0.2% diethylamine using a flow rate of 80 mL/min |
| **567** | 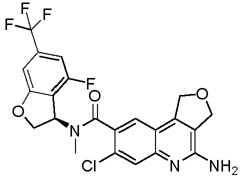 | 4-amino-7-chloro-N-((3R)-4-fluoro-6-(trifluoromethyl)-2,3-dihydro-1-benzofuran-3-yl)-N-methyl-1,3-dihydrofuro[3,4-c]quinoline-8-carboxamide | 482 | 2nd peak, ChromegaChiral CCC column (21 x 250 mm, 5 micron) with a mobile phase of 65% Liquid CO₂ and 35% MeOH with 0.2% diethylamine using a flow rate of 80 mL/min |
| **568** | 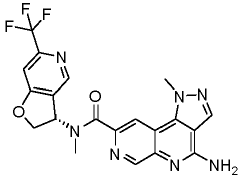 | 4-amino-N,1-dimethyl-N-((3 S)-6-(trifluoromethyl)-2,3-dihydrofuro[3,2-c]pyridin-3-yl)-1H-pyrazolo[4,3-c][1,7]naphthyridine-8-carboxamide | 444.2 | 1st peak, SFC using a Chiralpak IC column (21x150 mm) with a mobile phase of 45% Liquid CO₂ and 55% MeOH with 0.2% diethylamine using a flow rate of 80 mL/min |
| **569** | 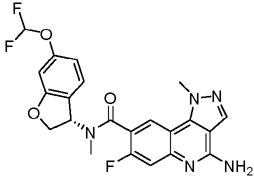 | 4-amino-N-((3S)-6-(difluoromethoxy)-2,3-dihydro-1-benzofuran-3-yl)-7-fluoro-N,1-dimethyl-1H-pyrazolo[4,3-c]quinoline-8-carboxamide | 458.2 | 1st peak, SFC using Chiralcel OJ column (3 x 15 cm, 5 micron) with mobile phase of 75% Liquid CO₂ and 25% MeOH with 0.1% diethylamine using a flow rate of 60 mL/min |
| **570** | 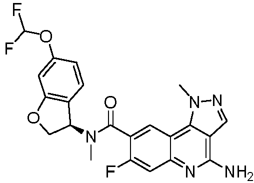 | 4-amino-N-((3R)-6-(difluoromethoxy)-2,3-dihydro-1-benzofuran-3-yl)-7-fluoro-N,1-dimethyl-1H-pyrazolo[4,3-c]quinoline-8-carboxamide | 458.2 | 2nd peak, SFC using Chiralcel OJ column (3 x 15 cm, 5 micron) with mobile phase of 75% Liquid CO₂ and 25% MeOH with 0.1% diethylamine using a flow rate of 60 mL/min |
| **571** | 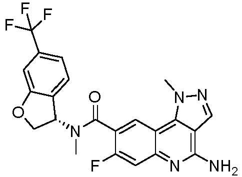 | 4-amino-7-fluoro-N,1-dimethyl-N-((3S)-6-(trifluoromethyl)-2,3-dihydro-1-benzofuran-3-yl)-1H-pyrazolo[4,3-c] quinoline-8-carboxamide | 459.8 | 1st peak, Chiral Technologies AD column (250 x 21 mm, 5 mm) with a mobile phase of 75% Liquid CO₂ and 25% iPrOH with 0.2% TEA using a flow rate of 100 mL/min |
| **572** | 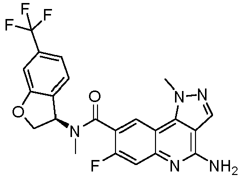 | 4-amino-7-fluoro-N,1-dimethyl-N-((3R)-6-(trifluoromethyl)-2,3-dihydro-1-benzofuran-3-yl)-1H-pyrazolo[4,3-c] quinoline-8-carboxamide | 459.8 | 1st peak, Chiral Technologies AD column (250 x 21 mm, 5 mm) with a mobile phase of 75% Liquid CO₂ and 25% iPrOH with 0.2% TEA using a flow rate of 100 mL/min |
| **573** | 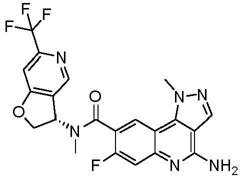 | 4-amino-7-fluoro-N,1-dimethyl-N-((3S)-6-(trifluoromethyl)-2,3-dihydrofuro[3,2-c]pyridin-3-yl)-1H-pyrazolo[4,3-c]quinoline-8-carboxamide | 461.2 | 2nd peak, SFC using a Chiralpak AD column (21 x 150 mm, 5 micron) with a mobile phase of 55% Liquid CO₂ and 45% MeOH with 0.2% diethylamine using a flow rate of 80 mL/min |
| **574** | 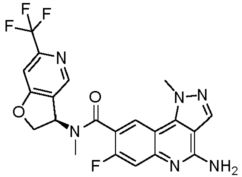 | 4-amino-7-fluoro-N,1-dimethyl-N-((3R)-6-(trifluoromethyl)-2,3-dihydrofuro[3,2-c]pyridin-3-yl)-1H-pyrazolo[4,3-c]quinoline-8-carboxamide | 461.2 | 1st peak, SFC using a Chiralpak AD column (21 x 150 mm, 5 micron) with a mobile phase of 55% Liquid CO₂ and 45% MeOH with 0.2% diethylamine using a flow rate of 80 mL/min |
| **575** | 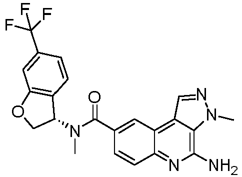 | 4-amino-N,3-dimethyl-N-((3S)-6-(trifluoromethyl)-2,3-dihydro-1-benzofuran-3-yl)-3H-pyrazolo[3,4-c]quinoline-8-carboxamide | 442.2 | 1st peak, Chiral Technologies AS column (250 x 21mm, 5 mm) with a mobile phase of 75% Liquid CO₂ and 25% MeOH with 0.2% TEA using a flow rate of 70 mL/min |
| **576** | 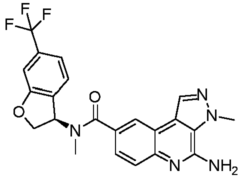 | 4-amino-N,3-dimethyl-N-((3R)-6-(trifluoromethyl)-2,3-dihydro-1-benzofuran-3-yl)-3H-pyrazolo[3,4-c]quinoline-8-carboxamide | 442.2 | 2nd peak, Chiral Technologies AS column (250 x 21mm, 5 mm) with a mobile phase of 75% Liquid CO₂ and 25% MeOH with 0.2% TEA using a flow rate of 70 mL/min |
| **577** | 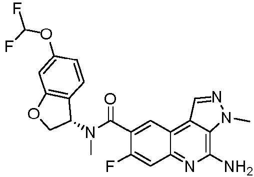 | 4-amino-N-((3S)-6-(difluoromethoxy)-2,3-dihydro-1-benzofuran-3-yl)-7-fluoro-N,3-dimethyl-3H-pyrazolo[3,4-c]quinoline-8-carboxamide | 458.2 | 2nd peak, SFC using a (S,S) Whelk-O 1 column (21 x 250 mm) with a mobile phase of 60% Liquid CO₂ and 40% MeOH with 0.2% diethylamine using a flow rate of 80 mL/min |
| **578** | 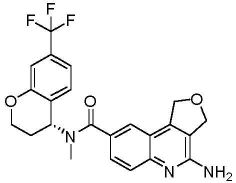 | 4-amino-N-methyl-N-((4R)-7-(trifluoromethyl)-3,4-dihydro-2H-chromen-4-yl)-1,3-dihydrofuro[3,4-c]quinoline-8-carboxamide | 444.1 | 1st peak, Chiral Technologies AS column (250 x 21 mm, 5 mm) with a mobile phase of 75% Liquid CO₂ and 25% MeOH with 0.2% TEA using a flow rate of 80 mL/min |
| **579** | 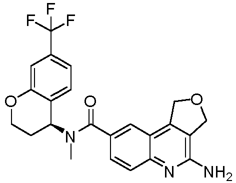 | 4-amino-N-methyl-N-((4S)-7-(trifluoromethyl)-3,4-dihydro-2H-chromen-4-yl)-1,3-dihydrofuro[3,4-c]quinoline-8-carboxamide | 444.1 | 2nd peak, Chiral Technologies AS column (250 x 21 mm, 5 mm) with a mobile phase of 75% Liquid CO₂ and 25% MeOH with 0.2% TEA using a flow rate of 80 mL/min |
| **580** | 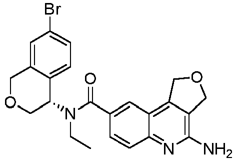 | 4-amino-N-((4S)-7-bromo-3,4-dihydro-1H-2-benzopyran-4-yl)-N-ethyl-1,3-dihydrofuro[3,4-c]quinoline-8-carboxamide | 467.95, 470.05 | 2nd peak, Chiral Technologies AD column (250 x 21 mm, 5 mm) with a mobile phase of 80% Liquid CO₂ and 20% MeOH with 0.2% TEA using a flow rate of 110 mL/min |
| **581** | 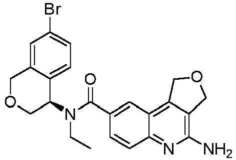 | 4-amino-N-((4R)-7-bromo-3,4-dihydro-1H-2-benzopyran-4-yl)-N-ethyl-1,3-dihydrofuro[3,4-c]quinoline-8-carboxamide | 467.95, 470.05 | 1st peak, Chiral Technologies AD column (250 x 21 mm, 5 mm) with a mobile phase of 80% Liquid CO₂ and 20% MeOH with 0.2% TEA using a flow rate of 110 mL/min |
| **582** | 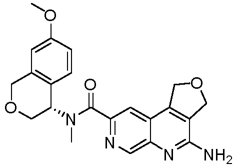 | 4-amino-N-((4S)-7-methoxy-3,4-dihydro-1H-2-benzopyran-4-yl)-N-methyl-1,3-dihydrofuro[3,4-c][1,7]naphthyridine-8-carboxamide | 407.2 | 1st peak, SFC using a Lux Cellulose 2 column (21 x 150 mm) with a mobile phase of 40% Liquid CO₂ and 60% MeOH with 0.2% diethylamine using a flow rate of 60 mL/min |
| **583** | 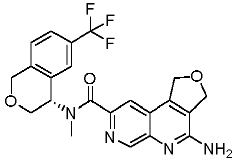 | 4-amino-N-methyl-N-((4S)-6-(trifluoromethyl)-3,4-dihydro-1H-2-benzopyran-4-yl)-1,3-dihydrofuro[3,4-c][1, 7]naphthyridine-8-carboxamide | 445 | 2nd peak, Chiral Technologies OX column (250 x 21 mm, 5 mm) with a mobile phase of 70% Liquid CO₂ and 30% MeOH with 0.2% TEA using a flow rate of 70 mL/min |
| **584** | 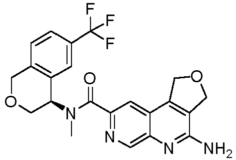 | 4-amino-N-methyl-N-((4R)-6-(trifluoromethyl)-3,4-dihydro-1H-2-benzopyran-4-yl)-1,3-dihydrofuro[3,4-c][1, 7]naphthyridine-8-carboxamide | 445 | 1st peak, Chiral Technologies OX column (250 x 21 mm, 5 mm) with a mobile phase of 70% Liquid CO₂ and 30% MeOH with 0.2% TEA using a flow rate of 70 mL/min |
| **585** | 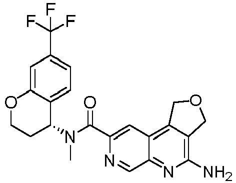 | 4-amino-N-methyl-N-((4R)-7-(trifluoromethyl)-3,4-dihydro-2H-chromen-4-yl)-1,3-dihydrofuro[3,4-c][1,7]naphthyridine-8-carboxamide | 445.15 | 1st peak, Chiralcel OD-H column (250 x 21 mm, 5 µm) with a mobile phase of 70% Liquid CO₂ and 30% MeOH with 0.2% TEA using a flow rate of 80 mL/min |
| **586** | 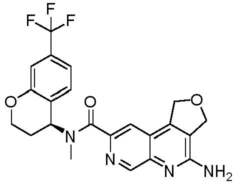 | 4-amino-N-methyl-N-((4S)-7-(trifluoromethyl)-3,4-dihydro-2H-chromen-4-yl)-1,3-dihydrofuro[3,4-c][1,7]naphthyridine-8-carboxamide | 445.15 | 2nd peak, Chiralcel OD-H column (250 x 21 mm, 5 µm) with a mobile phase of 70% Liquid CO₂ and 30% MeOH with 0.2% TEA using a flow rate of 80 mL/min |
| **587** | 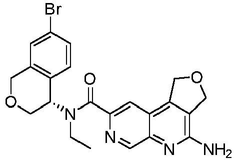 | 4-amino-N-((4S)-7-bromo-3,4-dihydro-1H-2-benzopyran-4-yl)-N-ethyl-1,3-dihydrofuro[3,4-c][1,7]naphthyridine-8-carboxamide | 469.00, 470.95 | 2nd peak, column Whelk-O SS column (250 x 21 mm, 5 µm), DAS2548, with a mobile phase of 70% Liquid CO₂ and 30% MeOH with TEA using a flow rate of 80 mL/min |
| **588** | 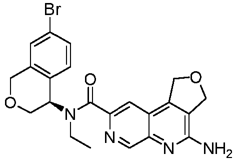 | 4-amino-N-((4R)-7-bromo-3,4-dihydro-1H-2-benzopyran-4-yl)-N-ethyl-1,3-dihydrofuro[3,4-c][1,7]naphthyridine-S-carboxamide | 469.00, 470.95 | 1st peak, column Whelk-O SS column (250 x 21 mm, 5 µm), DAS2548, with a mobile phase of 70% Liquid CO₂ and 30% MeOH with TEA using a flow rate of 80 mL/min |
| **589** | 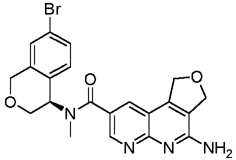 | 4-amino-N-((4R)-7-bromo-3,4-dihydro-1H-2-benzopyran-4-yl)-N-methyl-1,3-dihydrofuro[3,4-c][1,8]naphthyridine-8-carboxamide | 455.05, 457.00 | 2nd peak, Chiral Technologies AS column (250 x 21 mm, 5 mm) with a mobile phase of 75% Liquid CO₂ and 25% MeOH with 0.2% TEA using a flow rate of 75 mL/min |
| **590** | 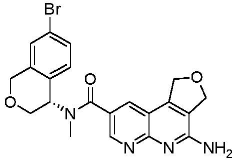 | 4-amino-N-((4S)-7-bromo-3,4-dihydro-1H-2-benzopyran-4-yl)-N-methyl-1,3-dihydrofuro[3,4-c][1,8]naphthyridine-8-carboxamide | 455.05, 457.00 | 1st peak, Chiral Technologies AS column (250 x 21 mm, 5 mm) with a mobile phase of 75% Liquid CO₂ and 25% MeOH with 0.2% TEA using a flow rate of 75 mL/min |
| **591** | 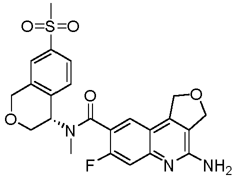 | 4-amino-7-fluoro-N-methyl-N-((4S)-7-(methylsulfonyl)-3,4-dihydro-1H-2-benzopyran-4-yl)-1,3-dihydrofuro[3,4-c]quinoline-8-carboxamide | 472.1 | 2nd peak, Chiralpak OJ column (2 x 25 cm, 5 micron) with a mobile phase of 65% Liquid CO₂ and 35% MeOH with 0.1% TEA using a flow rate of 60 mL/min |
| **592** | 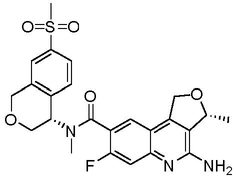 | (3R)-4-amino-7-fluoro-N,3-dimethyl-N-((4S)-7-(methylsulfonyl)-3,4-dihydro-1H-2-benzopyran-4-yl)-1,3-dihydrofuro[3,4-c]quinoline-8-carboxamide | 486.1 | 1st peak, Chiralpak AS column (2 x 25 cm, 5 micron) with a mobile phase of 78% Liquid CO₂ and 22% MeOH with 0.1% diethylamine using a flow rate of 70 mL/min |
| **593** | 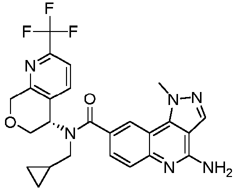 | 4-amino-N-(cyclopropylmethyl)-1-methyl-N-((5S)-2-(trifluoromethyl)-5,8-dihydro-6H-pyrano[3,4-b]pyridin-5-yl)-1H-pyrazolo[4,3-c]quinoline-8-carboxamide | 497.2 | 2nd peak, SFC Chiralpak AD column (21 x 150 mm) with a mobile phase of 65% Liquid CO₂ and 35% isopropanol with 0.2% diethylamine using a flow rate of 80 mL/min |
| **594** | 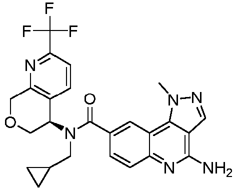 | 4-amino-N-(cyclopropylmethyl)-1-methyl-N-((5R)-2-(trifluoromethyl)-5,8-dihydro-6H-pyrano[3,4-b]pyridin-5-yl)-1H-pyrazolo[4,3-c]quinoline-8-carboxamide | 497.2 | 1st peak, SFC Chiralpak AD column (21 x 150 mm) with a mobile phase of 65% Liquid CO₂ and 35% isopropanol with 0.2% diethylamine using a flow rate of 80 mL/min |
| **595** | 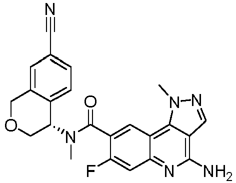 | 4-amino-N-((4S)-7-cyano-3,4-dihydro-1H-2-benzopyran-4-yl)-7-fluoro-N,1-dimethyl-1H-pyrazolo[4,3-c]quinoline-8-carboxamide | 431.1 | 2nd peak, Chiralcel OD column (21 x 250 mm) with a mobile phase of 70% Liquid CO₂ and 30% MeOH with 0.2% diethylamine using a flow rate of 80 mL/min |
| **596** | 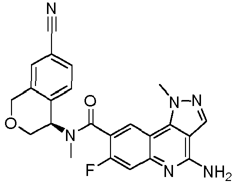 | 4-amino-N-((4R)-7-cyano-3,4-dihydro-1H-2-benzopyran-4-yl)-7-fluoro-N,1-dimethyl-1H-pyrazolo[4,3-c]quinoline-8-carboxamide | 431.1 | 1st peak, Chiralcel OD column (21 x 250 mm) with a mobile phase of 70% Liquid CO₂ and 30% MeOH with 0.2% diethylamine using a flow rate of 80 mL/min |
| **597** | 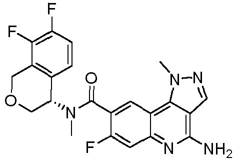 | 4-amino-N-((4S)-7,8-difluoro-3,4-dihydro-1H-2-benzopyran-4-yl)-7-fluoro-N,1-dimethyl-1H-pyrazolo[4,3-c]quinoline-8-carboxamide | 442.2 | 1st peak, SFC Chiralpak AS column (2 x 25 cm, 5 micron) with a mobile phase of 80% Liquid CO₂ and 20% ethanol with 0.1% diethylamine, using a flow rate of 60 mL/min |
| **598** | 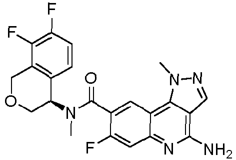 | 4-amino-N-((4R)-7,8-difluoro-3,4-dihydro-1H-2-benzopyran-4-yl)-7-fluoro-N,1-dimethyl-1H-pyrazolo[4,3-c]quinoline-8-carboxamide | 442.2 | 2nd peak, SFC Chiralpak AS column (2 x 25 cm, 5 micron) with a mobile phase of 80% Liquid CO₂ and 20% ethanol with 0.1% diethylamine, using a flow rate of 60 mL/min |
| **599** | 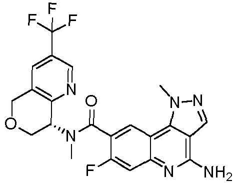 | 4-amino-7-fluoro-N,1-dimethyl-N-((8S)-3-(trifluoromethyl)-7,8-dihydro-5H-pyrano[4,3-b]pyridin-8-yl)-1H-pyrazolo[4,3-c]quinoline-8-carboxamide | 475 | 2nd peak, Chiralpak IG column (21 x 250 mm, 5 micron) x 2 with a mobile phase of 65% Liquid CO₂ and 35% MeOH with 0.2% diethylamine using a flow rate of 70 mL/min |
| **600** | 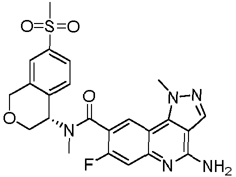 | 4-amino-7-fluoro-N,1-dimethyl-N-((4S)-7-(methylsulfonyl)-3,4-dihydro-1H-2-benzopyran-4-yl)-1H-pyrazolo[4,3-c]quinoline-8-carboxamide | 484.1 | 1st peak, Lux Cellulose-4 column (2 x 25 cm, 5 micron) with a mobile phase of 35% Liquid CO₂ and 65% isopropanol with 0.2% triethylamine using a flow rate of 45 mL/min |
| **601** | 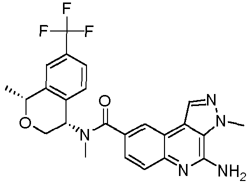 | 4-amino-N,3-dimethyl-N-((1R,4S)-1-methyl-7-(trifluoromethyl)-3,4-dihydro-1H-2-benzopyran-4-yl)-3H-pyrazolo[3,4-c]quinoline-8-carboxamide | 470.2 | 2nd peak, (S,S) Whelk-O 1 column (150 x 21 mm, 5 micron) with a mobile phase of 60% Liquid CO₂ and 40% MeOH with 0.2% diethylamine using a flow rate of 80 mL/min |
| **602** | 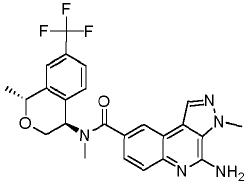 | 4-amino-N,3-dimethyl-N-((1R,4R)-1-methyl-7-(trifluoromethyl)-3,4-dihydro-1H-2-benzopyran-4-yl)-3H-pyrazolo[3,4-c]quinoline-8-carboxamide | 470.2 | 1st peak, Chiralpak AD column (250 x 21 mm, 5 micron) with a mobile phase of 75% Liquid CO₂ and 25% IPA with 0.2% diethylamine using a flow rate of 80 mL/min |
| **603** | 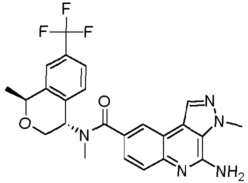 | 4-amino-N,3-dimethyl-N-((1S,4S)-1-methyl-7-(trifluoromethyl)-3,4-dihydro-1H-2-benzopyran-4-yl)-3H-pyrazolo[3,4-c]quinoline-8-carboxamide | 470.2 | 2nd peak, Chiralpak AD column (250 x 21 mm, 5 micron) with a mobile phase of 75% Liquid CO₂ and 25% IPA with 0.2% diethylamine using a flow rate of 80 mL/min |
| **604** | 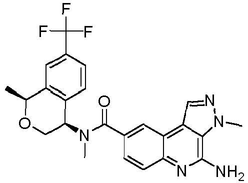 | 4-amino-N,3-dimethyl-N-((1S,4R)-1-methyl-7-(trifluoromethyl)-3,4-dihydro-1H-2-benzopyran-4-yl)-3H-pyrazolo[3,4-c]quinoline-8-carboxamide | 470.2 | 1st peak, (S,S) Whelk-O 1 column (150 x 21 mm, 5 micron) with a mobile phase of 60% Liquid CO₂ and 40% MeOH with 0.2% diethylamine using a flow rate of 80 mL/min |
| **605** | 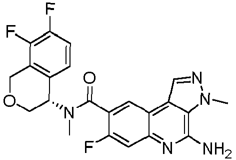 | 4-amino-N-((4S)-7,8-difluoro-3,4-dihydro-1H-2-benzopyran-4-yl)-7-fluoro-N,3-dimethyl-3H-pyrazolo[3,4-c]quinoline-8-carboxamide | 442.2 | 1st peak, SFC Chiralpak AS column (2 x 25 cm, 5 micron) with a mobile phase of 70% Liquid CO₂ and 30% MeOH with 0.1% diethylamine, using a flow rate of 65 mL/min |
| **606** | 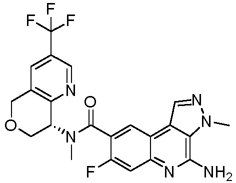 | 4-amino-7-fluoro-N,3-dimethyl-N-((8S)-3-(trifluoromethyl)-7,8-dihydro-5H-pyrano[4,3-b]pyridin-8-yl)-3H-pyrazolo[3,4-c]quinoline-8-carboxamide | 475.2 | 2nd peak, (S,S) Whelk-O1 column (21 x 250 mm, 5 micron) with a mobile phase of 65% Liquid CO₂ and 35% MeOH with 0.2% diethylamine using a flow rate of 80 mL/min |
| **607** | 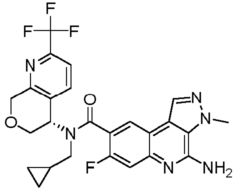 | 4-amino-N-(cyclopropylmethyl)-7-fluoro-3-methyl-N-((5S)-2-(trifluoromethyl)-5,8-dihydro-6H-pyrano[3,4-b]pyridin-5-yl)-3H-pyrazolo[3,4-c]quinoline-8-carboxamide | 515.2 | 2nd Peak, SFC AD-H column (2 x 25 cm) with a mobile phase of 70% Liquid CO₂ and 30% isopropanol using a flow rate of 80 mL/min |

### Examples 608 and 609: 4-amino-N-(7-bromoisochroman-4-yl)-N-methyl-1,3-dihydrofuro[3,4-c] quinoline-8-carboxamide

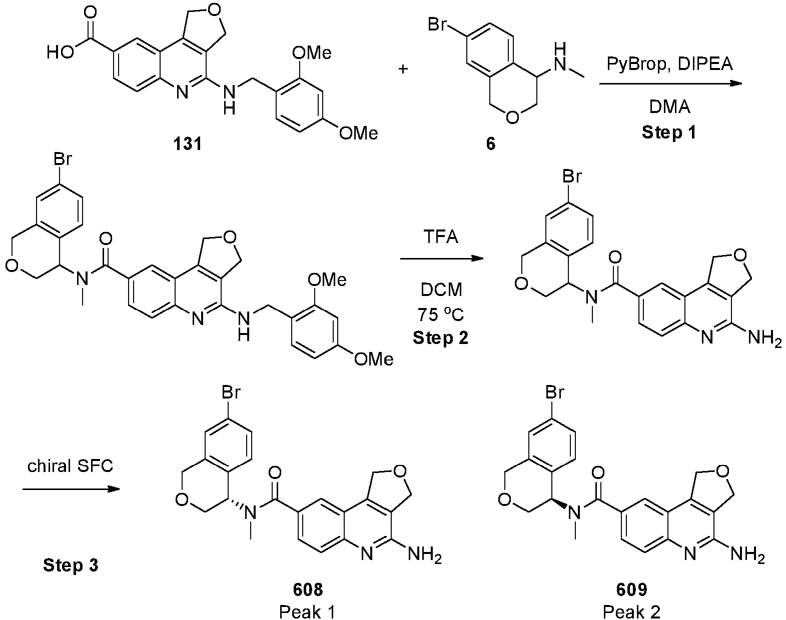

Step 1. To a stirred mixture of 4-((2,4-dimethoxybenzyl)amino)-1,3-dihydrofuro[3,4-c]quinoline-8-carboxylic acid **(131)** (189 mg, 0.496 mmol), 7-bromo-N-methylisochroman-4-amine (6) (120 mg, 0.496 mmol), and bromotripyrrolidinophosphonium hexafluorophosphate (462 mg, 0.991 mmol, Sigma-Aldrich Corporation) in DMA (1.5 mL) was added N-ethyl-N-isopropylpropan-2-amine (128 mg, 0.173 mL, 0.991 mmol, Sigma-Aldrich Corporation). The resulting mixture was stirred at rt for 1.5 h. The crude mixture was directly loaded onto a silica gel precolumn (25 g) and subjected to combi-flash column chromatography on a 12-g ISCO gold column eluting with MeOH (with 0.5% ammonium hydroxide)/DCM (0 to 12%) to give 200 mg of impure N-(7-bromoisochroman-4-yl)-4-((2,4-dimethoxybenzyl)amino)-N-methyl-1,3-dihydrofuro[3,4-c]quinoline-8-carboxamide as a nearly colorless film. It was taken onto the next step without further purification. *m*/*z* (ESI): 604.15 and 606.10 (M+H)⁺.

Step 2. To a stirred solution/suspension of N-(7-bromoisochroman-4-yl)-4-((2,4-dimethoxybenzyl)amino)-N-methyl-1,3-dihydrofuro[3,4-c]quinoline-8-carboxamide (150 mg, 0.248 mmol) in DCM (6 mL) in a 20-mL microwave reaction vessel was added dropwise at rt 1,1,1-trifluoroacetic acid (7650 mg, 5 mL, 67.1 mmol, Sigma-Aldrich Corporation). The resulting mixture was stirred at rt for 5 min before the vessel was sealed and subjected to microwave reaction condition (75 °C, 40 min). The volatiles were removed and the residue was dissolved in MeOH/TFA and subjected to preparative reverse-phase HPLC (Gemini^{™} Prep C18 10 µm column; Phenomenex; gradient elution of 10 to 85% MeCN in water, where both solvents contain 0.1% TFA 15 min in a 24-min method) to give, after lyophilization, 55 mg of 4-amino-N-(7-bromoisochroman-4-yl)-N-methyl-1,3-dihydrofuro[3,4-c]quinoline-8-carboxamide as a white solid as the TFA salt. *m*/*z* (ESI): 454.00 and 456.10 (M+H)⁺. ¹H NMR (METHANOL-d₄, 400 MHz) δ 7.9-8.0 (m, 2H), 7.8-7.9 (m, 1H), 7.49 (br d, 1H, *J*=8.6 Hz), 7.2-7.4 (m, 2H), 5.77 (br s, 1H), 5.54 (br d, 2H, *J*=2.9 Hz), 5.20 (t, 2H, *J*=3.6 Hz), 4.78 (br d, 1H, *J*=5.4 Hz), 4.5-4.7 (m, 1H), 3.9-4.4 (m, 2H), 2.7-3.0 (m, 3H).

Step 3. 4-Amino-N-(7-bromoisochroman-4-yl)-N-methyl-1,3-dihydrofuro[3,4-c]quinoline-8-carboxamide from Step 2 was resolved via preparative SFC using a Chiral Technologies AS column (250 x 21 mm, 5 mm) with a mobile phase of 55% Liquid CO₂ and 45% MeOH with 0.2% TEA using a flow rate of 60 mL/min to generate (S)-4-amino-N-(7-bromoisochroman-4-yl)-N-methyl-1,3-dihydrofuro[3,4-c]quinoline-8-carboxamide **(608)** (20 mg, 0.044 mmol, 17.74 % yield) as the first eluting enantiomer and (R)-4-amino-N-(7-bromoisochroman-4-yl)-N-methyl-1,3-dihydrofuro[3,4-c]quinoline-8-carboxamide (609) (22 mg, 0.048 mmol, 19.51 % yield) as the second eluting enantiomer, each as an off-white solid with > 99% ee. ¹H NMR (METHANOL-d₄, 400 MHz) δ 7.6-7.8 (m, 3H), 7.47 (br d, 1H, *J*=7.5 Hz), 7.1-7.4 (m, 2H), 4.9-5.8 (m, 5H), 4.5-4.8 (m, 2H), 4.0-4.3 (m, 2H), 2.8-2.9 (m, 3H).

### Examples 610 and 611: 4-amino-N-(6-cyclopropyl-2,3-dihydrobenzofuran-3-yl)-N-methyl-1,3-dihydrofuro[3,4-c]quinoline-8-carboxamide

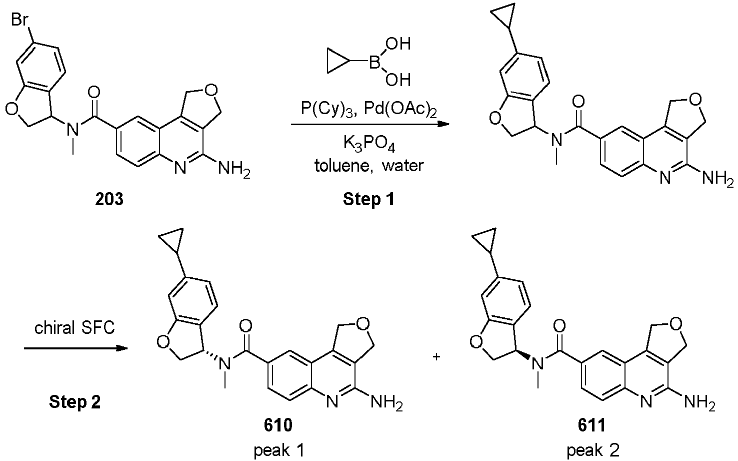

Step 1. To a mixture of 4-amino-N-(6-bromo-2,3-dihydrobenzofuran-3-yl)-N-methyl-1,3-dihydrofuro[3,4-c]quinoline-8-carboxamide **(203)** (0.100 g, 0.227 mmol), cyclopropylboronic acid (0.098 g, 1.136 mmol, Combi-Blocks) and toluene (2 mL) purged with Ar, potassium phosphate tribasic monohydrate (0.157 g, 0.681 mmol, Sigma-Aldrich Corporation) and water (0.222 mL) were added and stirred for 10 min at rt. Then, tricyclohexylphosphine (0.013 g, 0.045 mmol, Strem Chemicals) and palladium (II) acetate (5.10 mg, 0.023 mmol, Sigma-Aldrich Corporation) were added. The mixture was stirred in a sealed vial at 90°C overnight. The mixture was filtered through celite and concentrated in vacuo. The crude material was purified by chromatography through a silica gel column, eluting with 0-100% 3/1 EtOAc/EtOH in heptane. The pure 4-amino-N-(6-cyclopropyl-2,3-dihydrobenzofuran-3-yl)-N-methyl-1,3-dihydrofuro[3,4-c]quinoline-8-carboxamide (0.073 g, 0.182 mmol, 80% yield) was obtained as a white solid. *m*/*z* (ESI): 402 (M+H)⁺. ¹H NMR (400 MHz, METHANOL-*d*₄) δ ppm 7.57 - 7.81 (m, 3 H), 7.06 - 7.33 (m, 1 H), 6.73 (br d, *J*=7.9 Hz, 1 H), 6.48 - 6.62 (m, 1 H), 5.45 (br s, 2 H), 5.13 (t, *J*=3.3 Hz, 2 H), 4.46 - 4.72 (m, 3 H), 2.62 - 2.78 (m, 3 H), 1.84 - 1.94 (m, 1 H), 0.91 - 1.01 (m, 2 H), 0.57 - 0.74 (m, 2 H).

Step 2. 70 mg of 4-amino-N-(6-cyclopropyl-2,3-dihydrobenzofuran-3-yl)-N-methyl-1,3-dihydrofuro[3,4-c]quinoline-8-carboxamide was dissolved in 7 mL DCM:MeOH and purified by Prep SFC using Chiralpak AS column (250 x 21 mm, 5 µm) with a mobile phase of 75% Liquid CO₂ and 25% methanol with 0.2% TEA using a flow rate 90 mL/min to yield 28.2 mg of peak 1, (S)-4-amino-N-(6-cyclopropyl-2,3-dihydrobenzofuran-3-yl)-N-methyl-1,3-dihydrofuro[3,4-c]quinoline-8-carboxamide **(610),** with an ee of >99% (chemical purity > 99%) and 28.9 mg of peak 2, (R)-4-amino-N-(6-cyclopropyl-2,3-dihydrobenzofuran-3-yl)-N-methyl-1,3-dihydrofuro[3,4-c]quinoline-8-carboxamide **(611),** with an ee of 98.06 % (chemical purity> 99%).

Examples in Table 16 were prepared in a manner similar to that described above for Examples **610** and **611** using the indicated purification conditions.

**Table 16**

| **Ex.** | **Structure** | **Name** | **m/z (ESI): (M+H)⁺** | **SFC Conditions** |
|---|---|---|---|---|
| **612** | 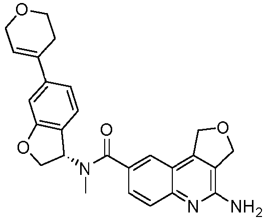 | 4-amino-N-((3S)-6-(3,6-dihydro-2H-pyran-4-yl)-2,3-dihydro-1-benzofuran-3-yl)-N-methyl-1,3-dihydrofuro[3,4-c]quinoline-8-carboxamide | 444.0 | 1st peak, Chiral Technologies OD column (250 x 21 mm, 5 mm) with a mobile phase of 70% Liquid CO₂ and 30% MeOH with 0.2% TEA using a flow rate of 80 mL/min |
| **613** | 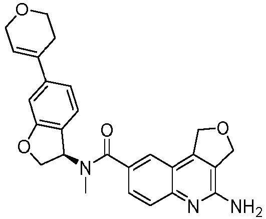 | 4-amino-N-((3R)-6-(3,6-dihydro-2H-pyran-4-yl)-2,3-dihydro-1-benzofuran-3-yl)-N-methyl-1,3-dihydrofuro[3,4-c]quinoline-8-carboxamide | 444.0 | 2nd peak, Chiral Technologies OD column (250 x 21 mm, 5 mm) with a mobile phase of 70% Liquid CO₂ and 30% MeOH with 0.2% TEA using a flow rate of 80 mL/min |

Examples in Table 16 were prepared in a manner similar to that described above for Examples **610** and **611** through Step 1. No chiral SFC was used as these compounds were either isolated as racemates **(614)** or enantiopure starting materials were used **(615-621)**

**Table 17**

| **Ex.** | **Structure** | **Name** | **m/z (ESI): (M+H)⁺** |
|---|---|---|---|
| **614** | 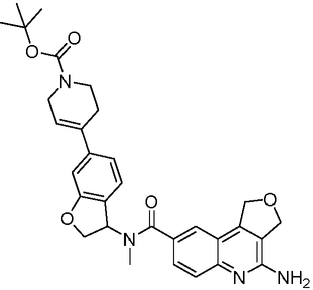 | 2-methyl-2-propanyl 4-((3R)-3-(((4-amino-1,3-dihydrofuro[3,4-c]quinolin-8-yl)carbonyl)(methyl)amino)-2,3-dihydro-1-benzofuran-6-yl)-3,6-dihydro-1(2H)-pyridinecarboxylate and 2-methyl-2-propanyl 4-((3S)-3-(((4-amino-1,3-dihydrofuro[3,4-c]quinolin-8-yl)carbonyl)(methyl)amino)-2,3-dihydro-1-benzofuran-6-yl)-3,6-dihydro-1(2H)-pyridinecarboxylate | 543.0 |
| **615** | 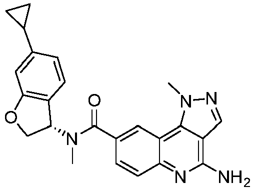 | 4-amino-N-((3 S)-6-cyclopropyl-2,3-dihydro-1-benzofuran-3-yl)-N,1-dimethyl-1H-pyrazolo[4,3-c]quinoline-8-carboxamide | 414 |
| **616** | 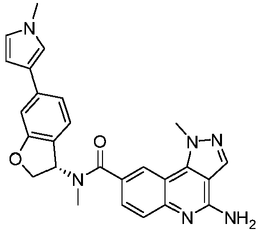 | 4-amino-N, 1-dimethyl-N-((3S)-6-(1-methyl-1H-pyrrol-3-yl)-2,3-dihydro-1-benzofuran-3-yl)-1H-pyrazolo[4,3-c]quinoline-8-carboxamide | 453 |
| **617** | 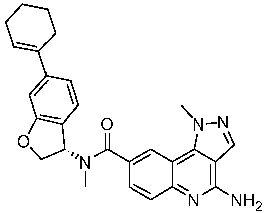 | 4-amino-N-((3S)-6-(1-cyclohexen-1-yl)-2,3-dihydro-1-benzofuran-3-yl)-N,1-dimethyl-1H-pyrazolo[4,3-c]quinoline-8-carboxamide | 454 |
| 618 | 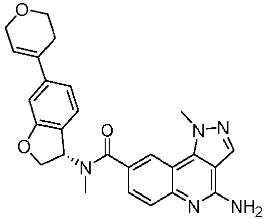 | 4-amino-N-((3S)-6-(3,6-dihydro-2H-pyran-4-yl)-2,3-dihydro-1-benzofuran-3-yl)-N,1-dimethyl-1H-pyrazolo[4,3-c]quinoline-8-carboxamide | 456 |
| **619** | 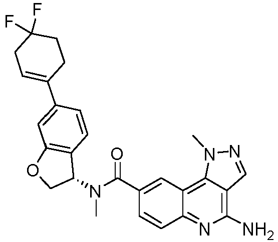 | 4-amino-N-((3 S)-6-(4,4-difluoro-1-cyclohexen-1-yl)-2,3-dihydro-1-benzofuran-3-yl)-N,1-dimethyl-1H-pyrazolo[4,3-c]quinoline-8-carboxamide | 490 |
| **620** | 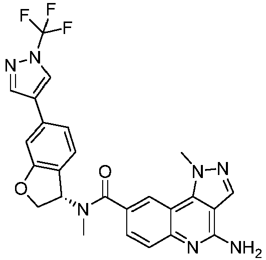 | 4-amino-N,1-dimethyl-N-((3S)-6-(1-(trifluoromethyl)-1H-pyrazol-4-yl)-2,3-dihydro-1-benzofuran-3-yl)-1H-pyrazolo[4,3-c] quinoline-8-carboxamide | 508 |
| **621** | 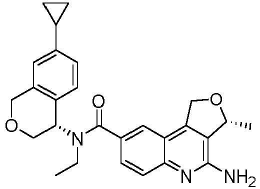 | (3R)-4-amino-N-((4S)-7-cyclopropyl-3,4-dihydro-1H-2-benzopyran-4-yl)-N-ethyl-3-methyl-1,3-dihydrofuro[3,4-c]quinoline-8-carboxamide | 444.2 |

### Intermediate 622: (S)-N-(6-(1H-pyrazol-4-yl)-2,3-dihydrobenzofuran-3-yl)-4-amino-N,1-dimethyl-1H-pyrazolo[4,3-c]quinoline-8-carboxamide

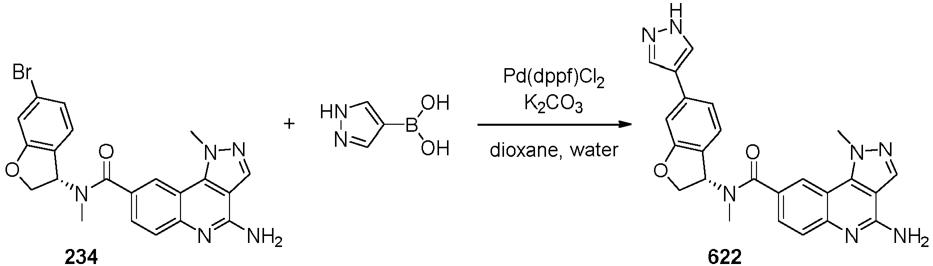

A mixture of (S)-4-amino-N-(6-bromo-2,3-dihydrobenzofuran-3-yl)-N,1-dimethyl-1H-pyrazolo[4,3-c]quinoline-8-carboxamide **(234)** (0.030 g, 0.066 mmol), (1H-pyrazol-4-yl)boronic acid (0.015 g, 0.133 mmol, AA Blocks), dichloro[1,1'-bis(diphenylphosphino)ferrocene]palladium (II) dichloromethane adduct (9.71 mg, 0.013 mmol, Strem Chemicals), potassium carbonate (0.027 g, 0.199 mmol, Sigma-Aldrich Corporation), 1,4-dioxane (1.5 mL) and water (0.167 mL) was purged with argon, then was stirred in a sealed vial at 95 °C overnight. The crude product was diluted with ethyl acetate, filtered through celite and concentrated in vacuo. The crude product was dissolved in DMF and purified by HPLC using an XBridge column (19 x 100mm, 5 µm) with 0.1% NH₄OH in H₂O and ACN as mobile phase, to obtain (S)-N-(6-(1H-pyrazol-4-yl)-2,3-dihydrobenzofuran-3-yl)-4-amino-N,1-dimethyl-1H-pyrazolo[4,3-c]quinoline-8-carboxamide **(622)** (1.8 mg, 4.10 µmol, 6.18% yield). *m*/*z* (ESI): 440.2 (M+H)⁺. ¹H NMR (500 MHz, DMSO-*d*₆) δ ppm 8.31 (s, 1 H), 8.26 (s, 1 H), 7.84 - 8.17 (m, 2 H), 7.57 - 7.68 (m, 3 H), 7.30 - 7.44 (m, 1 H), 7.22 (br d, *J*=7.7 Hz, 1 H), 7.09 - 7.17 (m, 3 H), 5.63 - 6.44 (m, 1 H), 4.68 (br s, 2 H), 4.42 (s, 3 H), 2.68 (s, 3 H).

Examples in Table 18 were prepared in a manner similar to that described above for Example **622** using the indicated purification conditions.

**Table 18**

| **Ex.** | **Structure** | **Name** | **m/z (ESI): (M+H)⁺** | **SFC Conditions** |
|---|---|---|---|---|
| **623** | 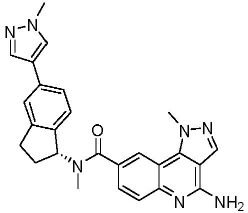 | 4-amino-N,1-dimethyl-N-((1R)-5-(1-methyl-1H-pyrazol-4-yl)-2,3-dihydro-1H-inden-1-yl)-1H-pyrazolo[4,3-c]quinoline-8-carboxamide | 452 | 1st peak, OJ-H column (25 x 2 cm) with a mobile phase of 65% Liquid CO₂ and 35% MeOH with 0.1% diethylamine using a flow rate of 60 mL/min |
| **624** | 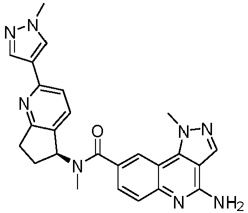 | 4-amino-N,1-dimethyl-N-((5S)-2-(1-methyl-1H-pyrazol-4-yl)-6,7-dihydro-5H-cyclopenta[b]pyridin-5-yl)-1H-pyrazolo[4,3-c]quinoline-8-carboxamide | 453 | 1st peak, Chiralcel OJ column (21 x 150, 5 µm) with a mobile phase of 55% Liquid CO₂ and 45% methanol with 0.2% diethylamine using a flow rate of 80 mL/min |

Examples in Table 19 were prepared in a manner similar to that described above for Example **622** using chiral starting materials.

**Table 19**

| **Ex.** | **Structure** | **Name** | **m/z (ESI): (M+H)⁺** |
|---|---|---|---|
| **625** | 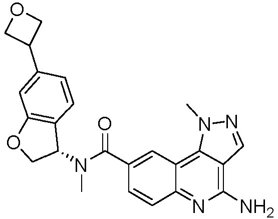 | 4-amino-N,1-dimethyl-N-((3S)-6-(3-oxetanyl)-2,3-dihydro-1-benzofuran-3-yl)-1H-pyrazolo[4,3-c]quinoline-8-carboxamide | 430 |
| **626** | 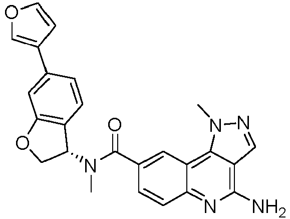 | 4-amino-N-((3S)-6-(3-furanyl)-2,3-dihydro-1-benzofuran-3-yl)-N,1-dimethyl-1H-pyrazolo [4,3-c]quinoline-8-carboxamide | 440 |
| **627** | 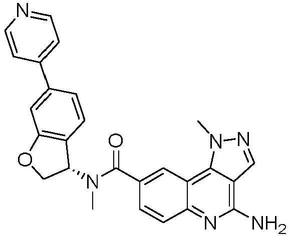 | 4-amino-N,1-dimethyl-N-((3 S)-6-(4-pyridinyl)-2,3-dihydro-1-benzofuran-3-yl)-1H-pyrazolo[4,3-c]quinoline-8-carboxamide | 451 |
| **628** | 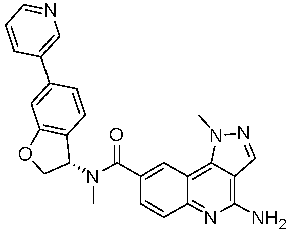 | 4-amino-N,1-dimethyl-N-((3S)-6-(3-pyridinyl)-2,3-dihydro-1-benzofuran-3-yl)-1H-pyrazolo[4,3-c]quinoline-8-carboxamide | 451 |
| **629** | 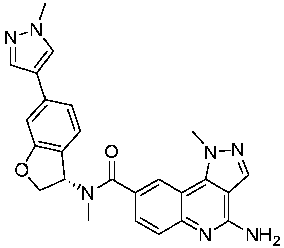 | 4-amino-N,1-dimethyl-N-((3S)-6-(1-methyl-1H-pyrazol-4-yl)-2,3-dihydro-1-benzofuran-3-yl)-1H-pyrazolo[4,3-c] quinoline-8-carboxamide | 454 |
| **630** | 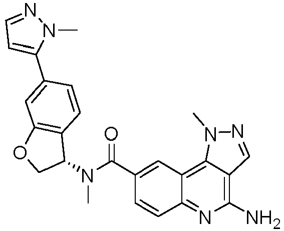 | 4-amino-N,1-dimethyl-N-((3S)-6-(1-methyl-1H-pyrazol-5-yl)-2,3-dihydro-1-benzofuran-3-yl)-1H-pyrazolo[4,3-c] quinoline-8-carboxamide | 454 |
| **631** | 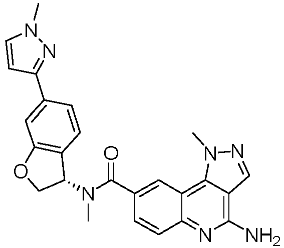 | 4-amino-N,1-dimethyl-N-((3S)-6-(1-methyl-1H-pyrazol-3-yl)-2,3-dihydro-1-benzofuran-3-yl)-1H-pyrazolo[4,3-c] quinoline-8-carboxamide | 454 |
| **632** | 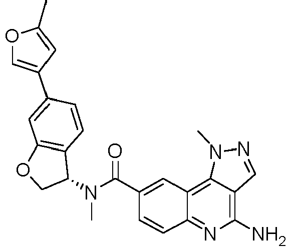 | 4-amino-N,1-dimethyl-N-((3S)-6-(5-methyl-3-furanyl)-2,3-dihydro-1-benzofuran-3-yl)-1H-pyrazolo[4,3-c]quinoline-8-carboxamide | 454 |
| **633** | 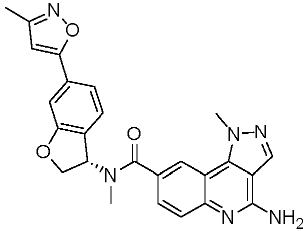 | 4-amino-N,1-dimethyl-N-((3S)-6-(3-methyl-1,2-oxazol-5-yl)-2,3-dihydro-1-benzofuran-3-yl)-1H-pyrazolo[4,3-c]quinoline-8-carboxamide | 455 |
| **634** | 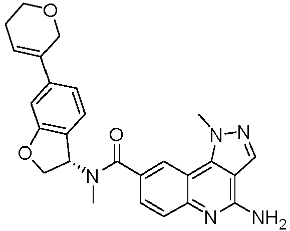 | 4-amino-N-((3S)-6-(5,6-dihydro-2H-pyran-3-yl)-2,3-dihydro-1-benzofuran-3-yl)-N,1-dimethyl-1H-pyrazolo[4,3-c]quinoline-8-carboxamide | 456.1 |
| **635** | 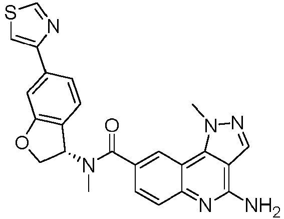 | 4-amino-N,1-dimethyl-N-((3S)-6-(1,3-thiazol-4-yl)-2,3-dihydro-1-benzofuran-3-yl)-1H-pyrazolo[4,3-c]quinoline-8-carboxamide | 457 |
| **636** | 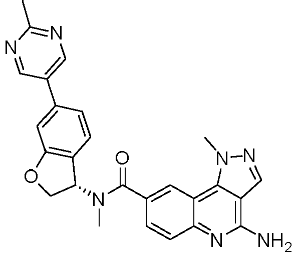 | 4-amino-N,1-dimethyl-N-((3S)-6-(2-methyl-5-pyrimidinyl)-2,3-dihydro-1-benzofuran-3-yl)-1H-pyrazolo[4,3-c]quinoline-8-carboxamide | 466 |
| **637** | 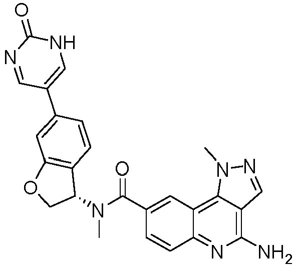 | 4-amino-N,1-dimethyl-N-((3S)-6-(2-oxo-1,2-dihydro-5-pyrimidinyl)-2,3-dihydro-1-benzofuran-3-yl)-1H-pyrazolo[4,3-c]quinoline-8-carboxamide | 468 |
| **638** | 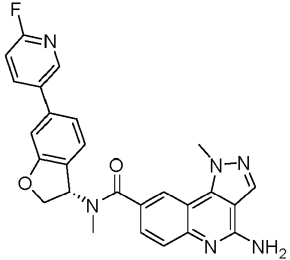 | 4-amino-N-((3S)-6-(6-fluoro-3-pyridinyl)-2,3-dihydro-1-benzofuran-3-yl)-N,1-dimethyl-1H-pyrazolo[4,3-c]quinoline-8-carboxamide | 469 |
| **639** | 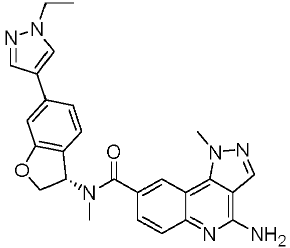 | 4-amino-N-((3S)-6-(1-ethyl-1H-pyrazol-4-yl)-2,3-dihydro-1-benzofuran-3-yl)-N,1-dimethyl-1H-pyrazolo[4,3-c]quinoline-8-carboxamide | 469 |
| **640** | 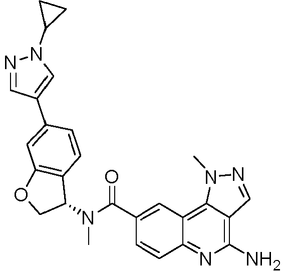 | 4-amino-N-((3S)-6-(1-cyclopropyl-1H-pyrazol-4-yl)-2,3-dihydro-1-benzofuran-3-yl)-N, 1-dimethyl-1H-pyrazolo[4,3-c] quinoline-8-carboxamide | 480 |
| **641** | 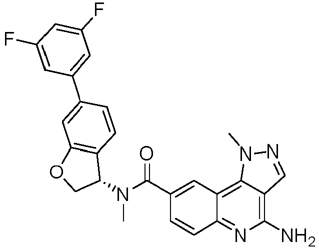 | 4-amino-N-((3 S)-6-(3,5-difluorophenyl)-2,3-dihydro-1-benzofuran-3-yl)-N,1-dimethyl-1H-pyrazolo[4,3-c]quinoline-8-carboxamide | 486 |
| **642** | 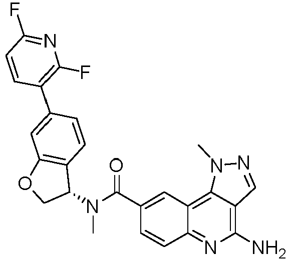 | 4-amino-N-((3S)-6-(2,6-difluoro-3-pyridinyl)-2,3-dihydro-1-benzofuran-3-yl)-N,1-dimethyl-1H-pyrazolo[4,3-c]quinoline-8-carboxamide | 487 |
| **643** | 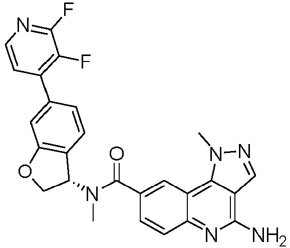 | 4-amino-N-((3S)-6-(2,3-difluoro-4-pyridinyl)-2,3-dihydro-1-benzofuran-3-yl)-N,1-dimethyl-1H-pyrazolo[4,3-c]quinoline-8-carboxamide | 487 |
| **644** | 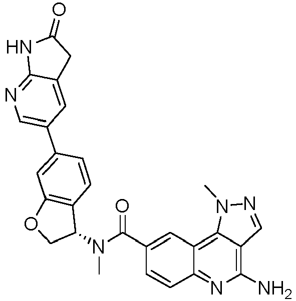 | 4-amino-N,1-dimethyl-N-((3 S)-6-(2-oxo-2,3-dihydro-1H-pyrrolo[2,3-b]pyridin-5-yl)-2,3-dihydro-1-benzofuran-3-yl)-1H-pyrazolo[4,3-c]quinoline-8-carboxamide | 506 |
| **645** | 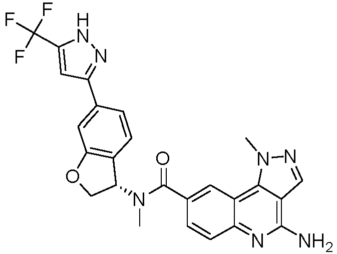 | 4-amino-N,1-dimethyl-N-((3S)-6-(5-(trifluoromethyl)-1H-pyrazol-3-yl)-2,3-dihydro-1-benzofuran-3-yl)-1H-pyrazolo[4,3-c]quinoline-8-carboxamide | 508 |
| **646** | 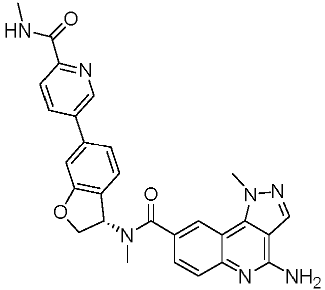 | 4-amino-N,1-dimethyl-N-((3S)-6-(6-(methylcarbamoyl)-3-pyridinyl)-2,3-dihydro-1-benzofuran-3-yl)-1H-pyrazolo[4,3-c]quinoline-8-carboxamide | 508 |
| 647 | 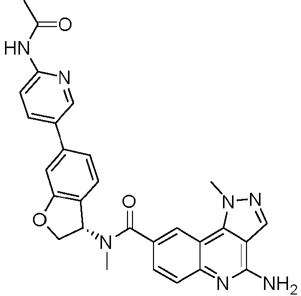 | N-((3S)-6-(6-acetamido-3-pyridinyl)-2,3-dihydro-1-benzofuran-3-yl)-4-amino-N,1-dimethyl-1H-pyrazolo[4,3-c]quinoline-8-carboxamide | 508 |
| **648** | 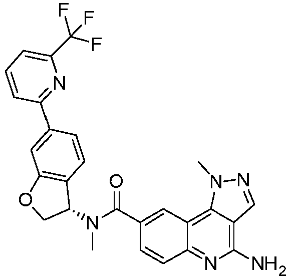 | 4-amino-N,1-dimethyl-N-((3S)-6-(6-(trifluoromethyl)-2-pyridinyl)-2,3-dihydro-1-benzofuran-3-yl)-1H-pyrazolo[4,3-c]quinoline-8-carboxamide | 519 |
| **649** | 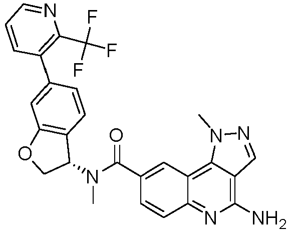 | 4-amino-N,1-dimethyl-N-((3S)-6-(2-(trifluoromethyl)-3-pyridinyl)-2,3-dihydro-1-benzofuran-3-yl)-1H-pyrazolo[4,3-c]quinoline-8-carboxamide | 519 |
| **650** | 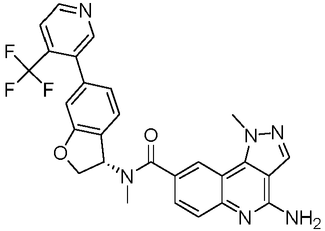 | 4-amino-N,1-dimethyl-N-((3 S)-6-(4-(trifluoromethyl)-3-pyridinyl)-2,3-dihydro-1-benzofuran-3-yl)-1H-pyrazolo[4,3-c]quinoline-8-carboxamide | 519 |
| **651** | 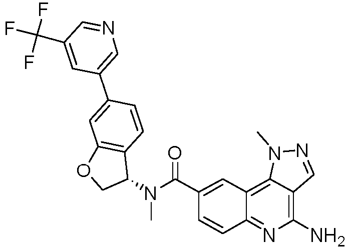 | 4-amino-N,1-dimethyl-N-((3S)-6-(5-(trifluoromethyl)-3-pyridinyl)-2,3-dihydro-1-benzofuran-3-yl)-1H-pyrazolo[4,3-c]quinoline-8-carboxamide | 519 |
| **652** | 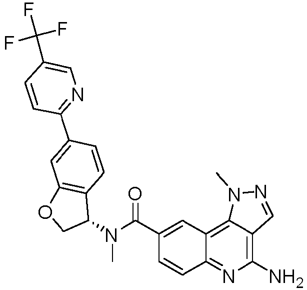 | 4-amino-N,1-dimethyl-N-((3S)-6-(5-(trifluoromethyl)-2-pyridinyl)-2,3-dihydro-1-benzofuran-3-yl)-1H-pyrazolo[4,3-c]quinoline-8-carboxamide | 519 |
| **653** | 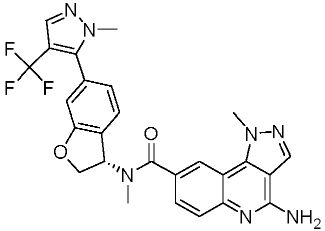 | 4-amino-N,1-dimethyl-N-((3S)-6-(1-methyl-4-(trifluoromethyl)-1H-pyrazol-5-yl)-2,3-dihydro-1-benzofuran-3-yl)-1H-pyrazolo[4,3-c]quinoline-8-carboxamide | 522 |
| **654** | 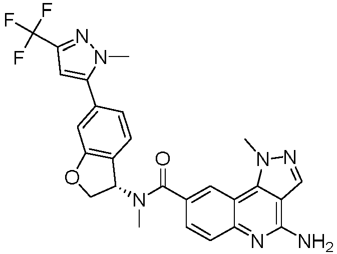 | 4-amino-N,1-dimethyl-N-((3S)-6-(1-methyl-3-(trifluoromethyl)-1H-pyrazol-5-yl)-2,3-dihydro-1-benzofuran-3-yl)-1H-pyrazolo[4,3-c]quinoline-8-carboxamide | 522 |
| **655** | 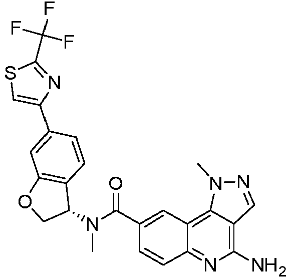 | 4-amino-N,1-dimethyl-N-((3S)-6-(2-(trifluoromethyl)-1,3-thiazol-4-yl)-2,3-dihydro-1-benzofuran-3-yl)-1H-pyrazolo[4,3-c]quinoline-8-carboxamide | 525 |
| **656** | 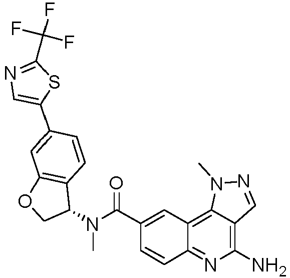 | 4-amino-N,1-dimethyl-N-((3S)-6-(2-(trifluoromethyl)-1,3-thiazol-5-yl)-2,3-dihydro-1-benzofuran-3-yl)-1H-pyrazolo[4,3-c]quinoline-8-carboxamide | 525 |
| **657** | 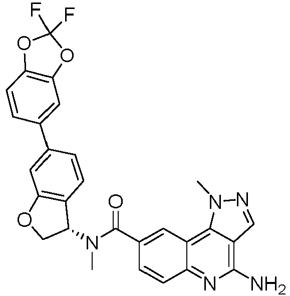 | 4-amino-N-((3S)-6-(2,2-difluoro-1,3-benzodioxol-5-yl)-2,3-dihydro-1-benzofuran-3-yl)-N,1-dimethyl-1H-pyrazolo[4,3-c]quinoline-8-carboxamide | 530 |
| **658** | 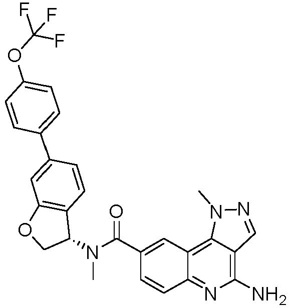 | 4-amino-N, 1-dimethyl-N-((3 S)-6-(4-(trifluoromethoxy)phenyl)-2,3-dihydro-1-benzofuran-3-yl)-1H-pyrazolo[4,3-c] quinoline-8-carboxamide | 534 |
| **659** | 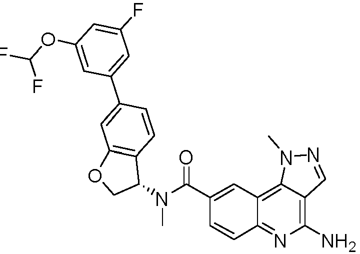 | 4-amino-N-((3S)-6-(3-(difluoromethoxy)-5-fluorophenyl)-2,3-dihydro-1-benzofuran-3-yl)-N,1-dimethyl-1H-pyrazolo[4,3-c]quinoline-8-carboxamide | 534 |
| **660** | 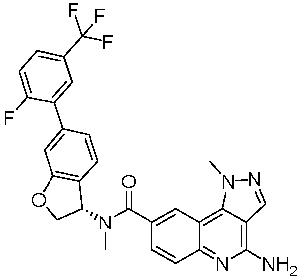 | 4-amino-N-((3S)-6-(2-fluoro-5-(trifluoromethyl)phenyl)-2,3-dihydro-1-benzofuran-3-yl)-N,1-dimethyl-1H-pyrazolo[4,3-c]quinoline-8-carboxamide | 536 |
| **661** | 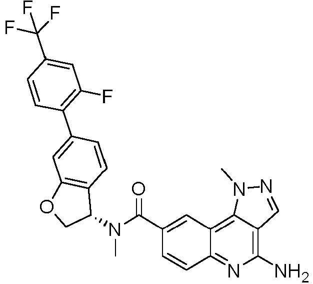 | 4-amino-N-((3S)-6-(2-fluoro-4-(trifluoromethyl)phenyl)-2,3-dihydro-1-benzofuran-3-yl)-N,1-dimethyl-1H-pyrazolo[4,3-c]quinoline-8-carboxamide | 536 |
| **662** | 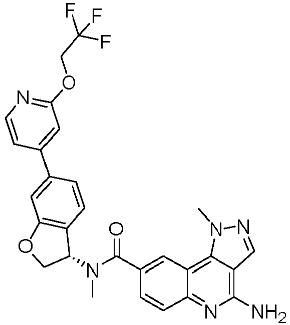 | 4-amino-N,1-dimethyl-N-((3S)-6-(2-(2,2,2-trifluoroethoxy)-4-pyridinyl)-2,3-dihydro-1-benzofuran-3-yl)-1H-pyrazolo[4,3-c]quinoline-8-carboxamide | 549 |

Intermediates **663** and **664:** (S)-4-amino-N,1-dimethyl-N-(7-(1-(trifluoromethyl)-1H-pyrazol-4-yl)isochroman-4-yl)-1H-pyrazolo[4,3-c]quinoline-8-carboxamide and (R)-4-amino-N,1-dimethyl-N-(7-(1-(trifluoromethyl)-1H-pyrazol-4-yl)isochroman-4-yl)-1H-pyrazolo[4,3-c]quinoline-8-carboxamide

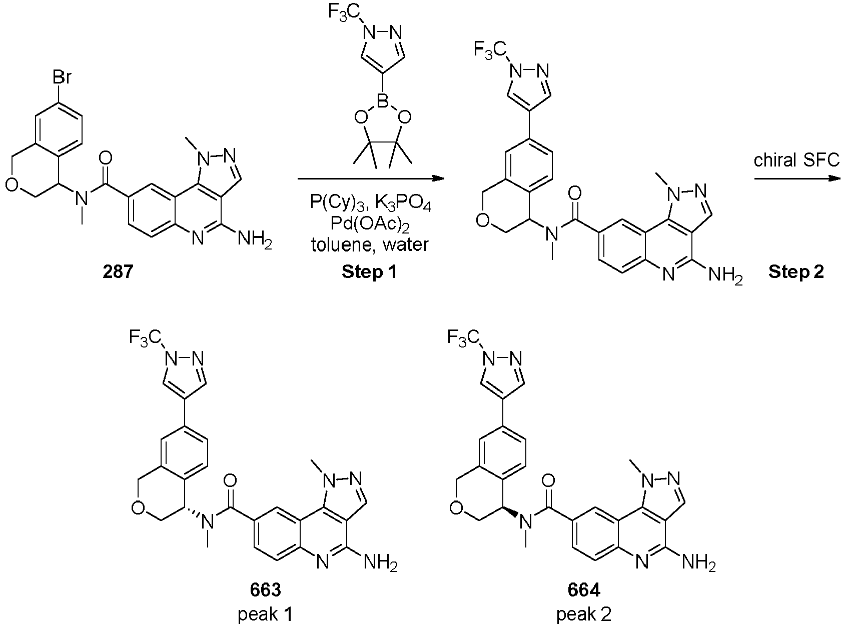

Step 1. To a resealable vial, was added 4-amino-N-(7-bromoisochroman-4-yl)-N,1-dimethyl-1H-pyrazolo[4,3-c]quinoline-8-carboxamide **(287)** (0.080 g, 0.172 mmol), 4-(4,4,5,5-tetramethyl-1,3,2-dioxaborolan-2-yl)-1-(trifluoromethyl)-1h-pyrazole (0.090 g, 0.090 mL, 0.343 mmol, Enamine) and potassium phosphate tribasic (0.109 g, 0.515 mmol, Sigma-Aldrich Corporation) in toluene (0.772 mL)/water (0.086 mL). The reaction mixture was sparged with Argon (gas) for 5 min, then tricyclohexylphosphine (0.019 g, 0.069 mmol, Strem Chemicals), followed by palladium (II) acetate (7.70 mg, 0.034 mmol, Sigma-Aldrich Corporation) were added to the reaction mixture and the vial was sealed. The reaction mixture was stirred and heated at 90°C for 16 h. Then, the reaction mixture was cooled to rt, and diluted with EtOAc and brine. The layers were separated and the aqueous layer was extracted with EtOAc (3x). The combined organic extracts were dried over MgSO₄, filtered and concentrated in vacuo. The crude material was diluted with DMSO (0.8 mL) and absorbed directly on a C18 column, then purified by chromatography, eluting with a gradient of 0-40% MeCN in water (0.1% TFA), to provide 4-amino-N,1-dimethyl-N-(7-(1-(trifluoromethyl)-1H-pyrazol-4-yl)isochroman-4-yl)-1H-pyrazolo[4,3-c]quinoline-8-carboxamide 2,2,2-trifluoroacetate (0.060 g, 0.094 mmol, 55.0 % yield) as white solid. ¹H NMR (400 MHz, METHANOL-*d*₄) δ ppm 8.61 (br s, 1 H), 8.55 (s, 1 H), 8.50 (s, 1 H), 8.25 (br s, 1 H), 7.93 (br d, *J*=8.4 Hz, 1 H), 7.87 (br s, 1 H), 7.66 (br d, *J*=7.1 Hz, 1 H), 7.48 - 7.56 (m, 1 H), 7.45 (br s, 1 H), 5.87 (br s, 1 H), 4.87 - 5.10 (m, 2 H), 4.57 (br s, 3 H), 4.37 (br d, *J*=11.7 Hz, 1 H), 4.09 - 4.28 (m, 1 H), 2.85 - 3.02 (m, 3 H). *m*/*z* (ESI): 522.1 (M+H)⁺.

Step 2. 4-amino-N,1-dimethyl-N-(7-(1-(trifluoromethyl)-1H-pyrazol-4-yl)isochroman-4-yl)-1H-pyrazolo[4,3-c]quinoline-8-carboxamide 2,2,2-trifluoroacetate from Step 1 was resolved via preparative SFC using an AS-H column (25 x 2 cm) with a mobile phase of 77% Liquid CO₂ and 23% MeOH with 0.2% TEA using a flow rate of 60 mL/min to yield (S)-4-amino-N,1-dimethyl-N-(7-(1-(trifluoromethyl)-1H-pyrazol-4-yl)isochroman-4-yl)-1H-pyrazolo[4,3-c]quinoline-8-carboxamide **(663)** (21 mg, 0.040 mmol, 42.9% yield) as the first eluting peak with >99% *ee* and (R)-4-amino-N,1-dimethyl-N-(7-(1-(trifluoromethyl)-1H-pyrazol-4-yl)isochroman-4-yl)-1H-pyrazolo[4,3-c]quinoline-8-carboxamide **(664)** (20 mg, 0.038 mmol, 40.8% yield) as the second eluting peak with 97.9% *ee.*

### Intermediates 665 and 666: (R)-4-amino-7-fluoro-N-methyl-N-(7-(4-(trifluoromethyl)phenyl)isochroman-4-yl)-1,3-dihydrofuro[3,4-c]quinoline-8-carboxamide and (S)-4-amino-7-fluoro-N-methyl-N-(7-(4-(trifluoromethyl)phenyl)isochroman-4-yl)-1,3-dihydrofuro[3,4-c] quinoline-8-carboxamide

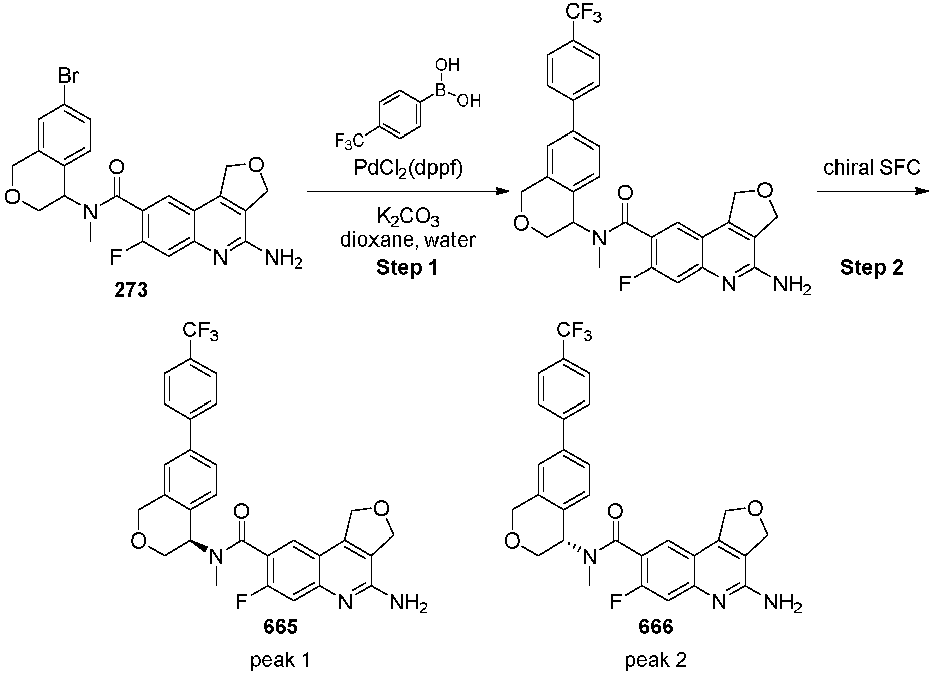

Step 1. A resealable vial was charged with 4-amino-N-(7-bromoisochroman-4-yl)-7-fluoro-N-methyl-1,3-dihydrofuro[3,4-c]quinoline-8-carboxamide **(273)** (0.110 g, 0.233 mmol), boronic acid, b-[4-(trifluoromethyl)phenyl]- (0.049 g, 0.256 mmol, AA Blocks) and potassium carbonate (0.097 g, 0.699 mmol, Oakwood Chemical) in 1,4-dioxane (2 mL) and water (0.2 mL). The reaction mixture was sparged with Argon for 5 min. Then (1,1'-bis(diphenylphosphino) ferrocene) dichloropalladium (0.034 g, 0.047 mmol, Combi-Blocks) was added to the reaction mixture and the vial was sealed. The reaction mixture was stirred and heated at 90 °C overnight before it was concentrated in vacuo. The crude material was absorbed onto a plug of silica gel and purified by chromatography through a Redi-Sep pre-packed silica gel column (40 g), eluting with a gradient of 0-25% EtOAc:EtOH (3:1) in heptane, to provide 4-amino-7-fluoro-N-methyl-N-(7-(4-(trifluoromethyl)phenyl)isochroman-4-yl)-1,3-dihydrofuro[3,4-c]quinoline-8-carboxamide (0.075 g, 0.140 mmol, 59.9 % yield) as off-white solid.

Step 2. 4-amino-7-fluoro-N-methyl-N-(7-(4-(trifluoromethyl)phenyl)isochroman-4-yl)-1,3-dihydrofuro[3,4-c]quinoline-8-carboxamide from Step 1 was resolved via preparative SFC using a Chiralpak IC column (3 x 15 cm, 5 micron) with a mobile phase of 70% Liquid CO₂ and 30% ethanol with 0.1% DEA using a flow rate of 80 mL/min to generate peak 1, (R)-4-amino-7-fluoro-N-methyl-N-(7-(4-(trifluoromethyl)phenyl)isochroman-4-yl)-1,3-dihydrofuro[3,4-c]quinoline-8-carboxamide **(665)** (35 mg, 0.065 mmol, 46.7% yield), with an ee of >99% and peak 2, (S)-4-amino-7-fluoro-N-methyl-N-(7-(4-(trifluoromethyl)phenyl)isochroman-4-yl)-1,3-dihydrofuro[3,4-c]quinoline-8-carboxamide **(666)** (33 mg, 0.061 mmol, 44.0% yield), with an ee of >99%.

Examples in Table 20 were prepared in a manner similar to that described above for Examples 665 and 666 using the indicated purification conditions or enantiopure intermediates.

**Table 20**

| **Ex.** | **Structure** | **Name** | **m/z (ESI): (M+H)⁺** | **SFC Conditions** |
|---|---|---|---|---|
| **667** | 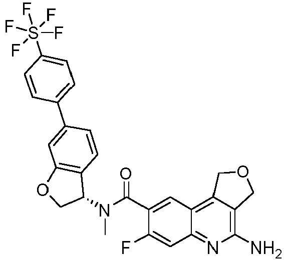 | 4-amino-7-fluoro-N-methyl-N-((3S)-6-(4-(pentafluoro-lambda~6~-sulfanyl)phenyl)-2,3-dihydro-1-benzofuran-3-yl)-1,3-dihydrofuro[3,4-c]quinoline-8-carboxamide | 582.1 | |
| **668** | 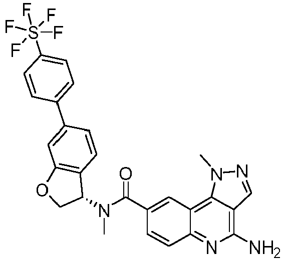 | 4-amino-N, 1-dimethyl-N-((3S)-6-(4-(pentafluoro-lambda~6~-sulfanyl)phenyl)-2,3-dihydro-1-benzofuran-3-yl)-1H-pyrazolo[4,3-c]quinoline-8-carboxamide | 576.1 | |
| **669** | 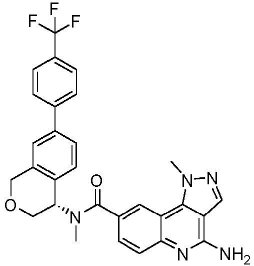 | 4-amino-N, 1-dimethyl-N-((4S)-7-(4-(trifluoromethyl)phenyl )-3,4-dihydro-1H-2-benzopyran-4-yl)-1H-pyrazolo[4,3-c]quinoline-8-carboxamide | 532.2 | 1st peak, AS column (21 x 250, 5 micron) with a mobile phase of 70% Liquid CO₂ and 30% methanol with 0.2% diethylamine using a flow rate of 80 mL/min |
| **670** | 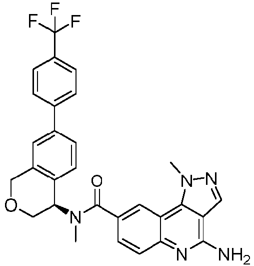 | 4-amino-N, 1-dimethyl-N-((4R)-7-(4-(trifluoromethyl)phenyl )-3,4-dihydro-1H-2-benzopyran-4-yl)-1H-pyrazolo[4,3-c]quinoline-8-carboxamide | 532.2 | 2nd peak, AS column (21 x 250, 5 micron) with a mobile phase of 70% Liquid CO₂ and 30% methanol with 0.2% diethylamine using a flow rate of 80 mL/min |

### Intermediate 671: (S)-4-amino-N-(6-methoxy-2,3-dihydrobenzofuran-3-yl)-N,1-dimethyl-1H-pyrazolo[4,3-c]quinoline-8-carboxamide

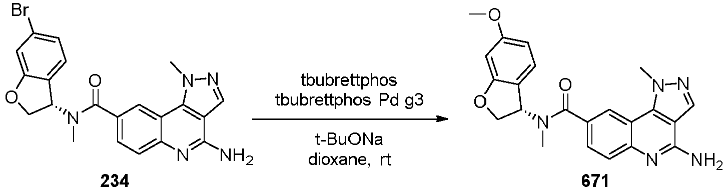

A red-capped vial with a Teflon-coated magnetic stir bar was charged with tbubrettphos (0.051 g, 0.105 mmol, Sigma-Aldrich Corporation), sodium tert-butoxide (0.017 g, 0.176 mmol, Sigma-Aldrich Corporation), and (S)-4-amino-N-(6-bromo-2,3-dihydrobenzofuran-3-yl)-N,1-dimethyl-1H-pyrazolo[4,3-c]quinoline-8-carboxamide **(234)** (0.053 g, 0.117 mmol), t-bu-brettphos Pd G3 (0.090 g, 0.105 mmol, Sigma-Aldrich Corporation), 1,4-dioxane (1 mL), and methanol (0.131 g, 0.166 mL, 4.10 mmol, Sigma-Aldrich Corporation). The mixture was purged with argon, sealed and was stirred at room temperature overnight. The crude product was diluted with ethyl acetate, filtered through celite and concentrated in vacuo. The residue was purified by silica gel flash column chromatography using 0-100% EtOAc/EtOH (3/1) in heptane. The crude product was obtained as orange solid. The product was dissolved in DMF and subjected to purification was performed by HPLC using an XBridge column (19 x 100mm, 5 µm) with 0.1% NH₄OH in H₂O and ACN as mobile phase to obtain pure (S)-4-amino-N-(6-methoxy-2,3-dihydrobenzofuran-3-yl)-N,1-dimethyl-1H-pyrazolo[4,3-c]quinoline-8-carboxamide (671) (13.1 mg, 0.032 mmol, 27.7% yield). *m*/*z* (ESI): 404 (M+H)⁺. ¹H NMR (500 MHz, DMSO-*d*₆) δ ppm 8.29 (s, 1 H), 8.25 (s, 1 H), 7.61 (s, 2 H), 7.21 - 7.35 (m, 1 H), 7.12 (s, 2 H), 6.53 (br d, *J*=8.2 Hz, 1 H), 6.48 (d, J=1.6 Hz, 1 H), 5.75 (s, 1 H), 4.67 (br s, 2 H), 4.41 (s, 3 H), 3.73 (s, 3 H), 3.31 (s, 1 H), 2.63 - 2.66 (m, 2 H).

### Intermediates 672 and 673: (S)-4-amino-N-(2,3-dihydrobenzofuran-3-yl)-N,1-dimethyl-1H-pyrazolo[4,3-c]quinoline-8-carboxamide 2,2,2-trifluoroacetate and (S)-4-amino-N-(2,3-dihydrobenzofuran-3-yl)-N,1-dimethyl-1H-pyrazolo[4,3-c]quinoline-8-carboxamide

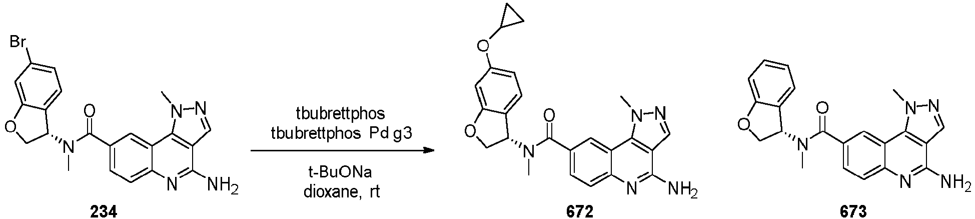

A red-capped vial with a Teflon-coated magnetic stir bar was charged with t-bu-brettphos (8.57 mg, 0.018 mmol, Sigma-Aldrich Corporation) , sodium tert-butoxide (6.37 mg, 0.066 mmol, Sigma-Aldrich Corporation), and (S)-4-amino-N-(6-bromo-2,3-dihydrobenzofuran-3-yl)-N,1-dimethyl-1H-pyrazolo[4,3-c]quinoline-8-carboxamide **(234)** (0.020 g, 0.044 mmol, 128130-16-1), t-bu-brettphos Pd G3 (0.015 g, 0.018 mmol, Sigma-Aldrich Corporation), 1,4-dioxane (1 mL), and cyclopropanol (0.090 g, 0.098 mL, 1.548 mmol, Combi-Blocks). The mixture was purged with argon, sealed and was stirred at room temperature overnight. The crude product was diluted with ethyl acetate, filtered through celite, concentrated in vacuo and purified by silica gel flash column chromatography using 0-100% EtOAc/EtOH (3/1) in heptane. The two products were obtained with impurities. The solid was dissolved in DMF and purified by reverse phase prep HPLC using a mobile phase of 10-70% water in CH₃CN with 0.1% TFA. The second eluting peak was (S)-4-amino-N-(6-cyclopropoxy-2,3-dihydrobenzofuran-3-yl)-N,1-dimethyl-1H-pyrazolo[4,3-c]quinoline-8-carboxamide 2,2,2-trifluoroacetate **(672)** (9.9 mg, 0.018 mmol, 41.2% yield), which was obtained as a white solid. *m*/*z* (ESI): 430 (M+H)⁺. ¹H NMR (400 MHz, METHANOL-*d*₄) δ ppm 8.48 - 8.53 (m, 2 H), 7.79 - 7.93 (m, 2 H), 7.17 - 7.37 (m, 1 H), 6.57 - 6.70 (m, 2 H), 5.53 - 6.40 (m, 1 H), 4.66 - 4.77 (m, 2 H), 4.53 - 4.59 (m, 3 H), 3.76 (br s, 1 H), 2.68 - 2.85 (m, 3 H), 0.75 - 0.83 (m, 2 H), 0.64 - 0.71 (m, 2 H). The first eluting peak was the side product (S)-4-amino-N-(2,3-dihydrobenzofuran-3-yl)-N,1-dimethyl-1H-pyrazolo[4,3-c]quinoline-8-carboxamide 2,2,2-trifluoroacetate **(673)** (6.7 mg, 0.014 mmol, 31.1% yield). *m*/*z* (ESI): 374 (M+H)⁺. ¹H NMR (400 MHz, METHANOL-*d*₄) δ ppm 8.53 (d, *J=*1.5 Hz, 1 H), 8.49 (s, 1 H), 7.87 - 7.94 (m, 1 H), 7.84 (br s, 1 H), 7.45 (br s, 1 H), 7.29 (t, *J*=7.6 Hz, 1 H), 7.00 (br t, *J*=7.5 Hz, 1 H), 6.87 (br d, *J*=7.3 Hz, 1 H), 6.35 - 6.52 (m, 1 H), 4.68 (br d, *J*=6.5 Hz, 2 H), 4.55 (s, 3 H), 2.70 - 2.85 (m, 3 H).

Examples in Table 21 were prepared in a manner similar to that described above for Examples **671-673.**

**Table 21**

| **Ex.** | **Structure** | **Name** | **m/z (ESI): (M+H)⁺** |
|---|---|---|---|
| **674** | 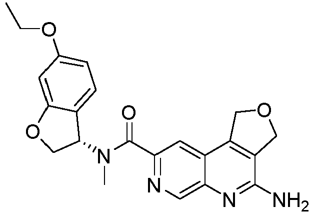 | 4-amino-N-((3S)-6-ethoxy-2,3-dihydro-1-benzofuran-3-yl)-N-methyl-1,3-dihydrofuro[3,4-c][1,7]naphthyridine-8-carboxamide | 407 |
| **675** | 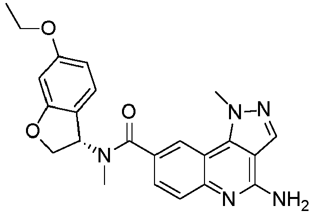 | 4-amino-N-((3S)-6-ethoxy-2,3-dihydro-1-benzofuran-3-yl)-N,1-dimethyl-1H-pyrazolo[4,3-c]quinoline-8-carboxamide | 418 |
| **676** | 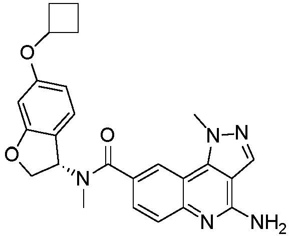 | 4-amino-N-((3S)-6-(cyclobutyloxy)-2,3-dihydro-1-benzofuran-3-yl)-N,1-dimethyl-1H-pyrazolo[4,3-c]quinoline-8-carboxamide | 444 |
| **677** | 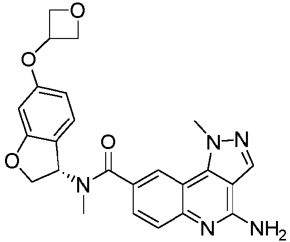 | 4-amino-N,1-dimethyl-N-((3S)-6-(3-oxetanyloxy)-2,3-dihydro-1-benzofuran-3-yl)-1H-pyrazolo[4,3-c]quinoline-8-carboxamide | 446 |
| **678** | 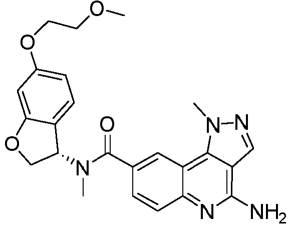 | 4-amino-N-((3S)-6-(2-methoxyethoxy)-2,3-dihydro-1-benzofuran-3-yl)-N,1-dimethyl-1H-pyrazolo[4,3-c]quinoline-8-carboxamide | 448 |

### Intermediate 679: (S)-4-amino-N,1-dimethyl-N-(2-methyl-5,8-dihydro-6H-pyrano[3,4-b]pyridin-5-yl)-1H-pyrazolo[4,3-c]quinoline-8-carboxamide

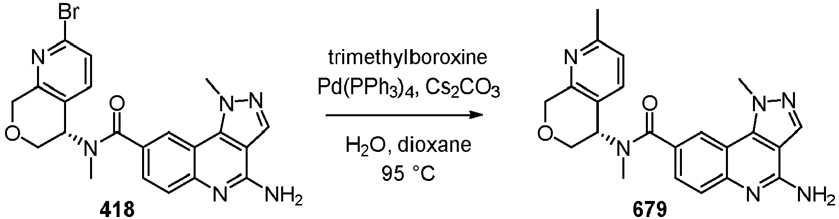

A solution of (S)-4-amino-N-(2-bromo-5,8-dihydro-6H-pyrano[3,4-b]pyridin-5-yl)-N,1-dimethyl-1H-pyrazolo[4,3-c]quinoline-8-carboxamide **(418)** (25 mg, 0.053 mmol), trimethylboroxine (13.43 mg, 14.99 µL, 0.107 mmol, Sigma-Aldrich Corporation), Pd(PPh₃)₄ (6.80 mg, 5.88 µmol, Sigma-Aldrich Corporation) and cesium carbonate (33.1 mg, 0.102 mmol, Sigma-Aldrich Corporation) in 1,4-dioxane (708 µL) and water (79 µL) was purged with argon and stirred at 95°C overnight. Then, the solution was filtered through a syringe filter, and diluted with DCM. The organics were washed with NaHCO₃ and extracted with DCM. The organics were dried over MgSO₄, filtered, and concentrated. The crude material was loaded onto an SCX column and washed with MeOH. Then, the crude product was eluted with NH₃ in MeOH (2M) and concentrated. HPLC purification was performed on an XBridge column (19 x 100mm, 5 µm) with 0.1% NH₄OH in H₂O and ACN as the mobile phase to give (S)-4-amino-N,1-dimethyl-N-(2-methyl-5,8-dihydro-6H-pyrano[3,4-b]pyridin-5-yl)-1H-pyrazolo[4,3-c]quinoline-8-carboxamide **(679)** (6.698 mg, 0.017 mmol, 31.1 % yield). *m*/*z* (ESI): 403.2 (M+H)⁺. ¹H NMR (500 MHz, DMSO-*d*₆) δ ppm 8.32 (d, *J*=1.6 Hz, 1 H), 8.25 (s, 1 H), 7.71 (br d, *J*=6.5 Hz, 1 H), 7.58 - 7.67 (m, 2 H), 7.22 (br d, *J*=7.5 Hz, 1 H), 7.12 (s, 2 H), 5.63 - 5.84 (m, 1 H), 4.46 - 4.79 (m, 2 H), 4.39 (s, 3 H), 4.01 - 4.23 (m, 2 H), 2.76 (s, 3 H), 2.40 - 2.55 (m, 2 H).

Examples in Table 22 were prepared in a manner similar to that described above for Example **679** using the indicated purification conditions. Example **681** was a side product in the reaction to make Example **680.**

**Table 22**

| **Ex.** | **Structure** | **Name** | **m/z (ESI): (M+H)⁺** | **SFC Conditions** |
|---|---|---|---|---|
| **680** | 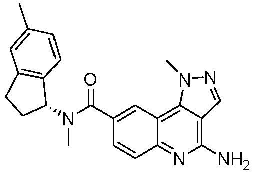 | 4-amino-N,1-dimethyl-N-((1R)-5-methyl-2,3-dihydro-1H-inden-1-yl)-1H-pyrazolo[4,3-c]quinoline-8-carboxamide | 386 | 1st peak, Chiralcel OD column (21 x 250 mm) with a mobile phase of 60% Liquid CO₂ and 40% MeOH with 0.2% diethylamine using a flow rate of 80 mL/min |
| **681** | 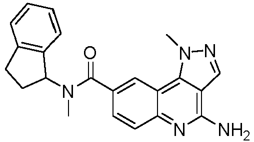 | 4-amino-N-((1R)-2,3-dihydro-1H-inden-1-yl)-N,1-dimethyl-1H-pyrazolo[ 4,3-c]quinoline-8-carboxamide and 4-amino-N-((1S)-2,3-dihydro-1H-inden-1-yl)-N,1-dimethyl-1H-pyrazolo[4,3-c]quinoline-8-carboxamide | 372 | |
| **682** | 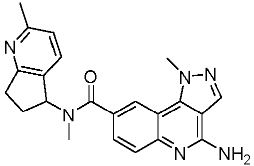 | 4-amino-N,1-dimethyl-N-((5R)-2-methyl-6,7-dihydro-SH-cyclopenta[b]pyridin-5-yl)-1H-pyrazolo[4,3-c] quinoline-8-carboxamide and 4-amino-N,1-dimethyl-N-((5S)-2-methyl-6,7-dihydro-5H-cyclopenta[b]pyridin-5-yl)-1H-pyrazolo[4,3-c]quinoline-8-carboxamide | 387 | |
| **683** | 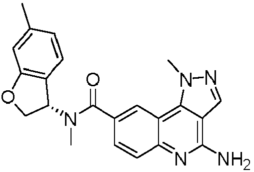 | 4-amino-N,1-dimethyl-N-((3S)-6-methyl-2,3-dihydro-1-benzofuran-3-yl)-1H-pyrazolo[4,3-c]quinoline-8-carboxamide | 388 | |
| **684** | 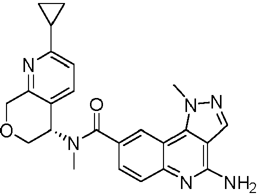 | 4-amino-N-((5S)-2-cyclopropyl-5,8-dihydro-6H-pyrano [3,4-b]pyridin-5-yl)-N,1-dimethyl-1H-pyrazolo[4,3-c]quinoline-8-carboxamide | 429.2 | |

### Intermediates 685 and 686: methyl (S)-3-(4-amino-N,1-dimethyl-1H-pyrazolo[4,3-c]quinoline-8-carboxamido)-2,3-dihydrobenzofuran-6-carboxylate and (S)-4-amino-N-(6-(hydroxymethyl)-2,3-dihydrobenzofuran-3-yl)-N,1-dimethyl-1H-pyrazolo[4,3-c]quinoline-8-carboxamide

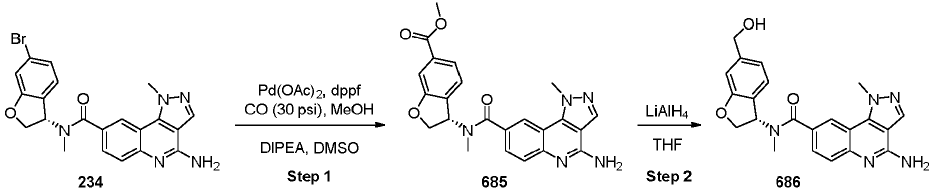

Step 1. A tube with a stir bar was charged with (S)-4-amino-N-(6-bromo-2,3-dihydrobenzofuran-3-yl)-N,1-dimethyl-1H-pyrazolo[4,3-c]quinoline-8-carboxamide **(234)** (0.1553 g, 0.343 mmol), dimethyl sulfoxide (1.3 mL), dppf (0.029 g, 0.052 mmol, Sigma-Aldrich Corporation), palladium diacetate (9.25 mg, 0.041 mmol, Sigma-Aldrich Corporation), and methanol (0.660 g, 0.833 mL, 20.60 mmol, Sigma-Aldrich Corporation). The mixture was purged with CO (30 psi), and was stirred at 80°C for 20 h. The mixture was diluted with EtOAc, and washed with water and brine. The organic phase was dried over Na₂SO₄ and concentrated in vacuo. The crude was purified by silica gel chromatography using 0-100% EtOAc/EtOH (3/1) in heptane. Methyl (S)-3-(4-amino-N,1-dimethyl-1H-pyrazolo[4,3-c]quinoline-8-carboxamido)-2,3-dihydrobenzofuran-6-carboxylate (75 mg, 0.174 mmol, 50.6% yield) was obtained as off-white solid. *m*/*z* (ESI): 432 (M+H)⁺. ¹H NMR (400 MHz, CHLOROFORM-*d*) δ ppm 8.41 (d, *J*=1.7 Hz, 1 H), 7.98 (s, 1 H), 7.90 (br dd, *J*=11.0, 8.0 Hz, 2 H), 7.81 (d, *J*=8.6 Hz, 1 H), 7.71 (dd, *J*=7.8, 1.1 Hz, 1 H), 7.63 (dd, *J*=8.6, 1.9 Hz, 1 H), 7.53 (s, 1 H), 5.16 - 5.28 (m, 2 H), 4.74 - 4.85 (m, 1 H), 4.60 (dd, *J=*10.5, 4.2 Hz, 1 H), 4.50 (s, 3 H), 3.92 (s, 3 H), 2.74 - 2.83 (m, 3 H).

Step 2. To methyl (S)-3-(4-amino-N,1-dimethyl-1H-pyrazolo[4,3-c]quinoline-8-carboxamido)-2,3-dihydrobenzofuran-6-carboxylate (0.012 g, 0.028 mmol) in THF (1.5 mL) in ice bath was added LAH, 2.0 M in THF (0.028 mL, 0.056 mmol, Sigma-Aldrich Corporation) dropwise. After 30 min, the reaction was quenched with sodium sulfate decahydrate and was diluted with EtOAc. The solid was filtered and the filtrate was concentrated in vacuo. The crude was purified by HPLC using an XBridge column (19 x 100mm, 5 µm) with 0.1% NH₄OH in H₂O and ACN as the mobile phase to yield (S)-4-amino-N-(6-(hydroxymethyl)-2,3-dihydrobenzofuran-3-yl)-N,1-dimethyl-1H-pyrazolo[4,3-c]quinoline-8-carboxamide **(686)** (0.001 g, 0.002 mmol, 8.91% yield). *m*/*z* (ESI): 404 (M+H)⁺. ¹H NMR (400 MHz, METHANOL-*d*₄) δ ppm 8.41 - 8.47 (m, 1 H), 8.24 (s, 1 H), 7.67 - 7.79 (m, 2 H), 7.28 - 7.47 (m, 1 H), 6.99 (br d, *J*=7.7 Hz, 1 H), 6.84 - 6.92 (m, 1 H), 4.54 - 4.73 (m, 5 H), 4.49 (s, 3 H), 2.74 - 2.83 (m, 3 H).

### Intermediate 687: (S)-4-amino-N-(6-chloro-2,3-dihydrobenzofuran-3-yl)-N,1-dimethyl-1H-pyrazolo[4,3-c]quinoline-8-carboxamide 2,2,2-trifluoroacetate

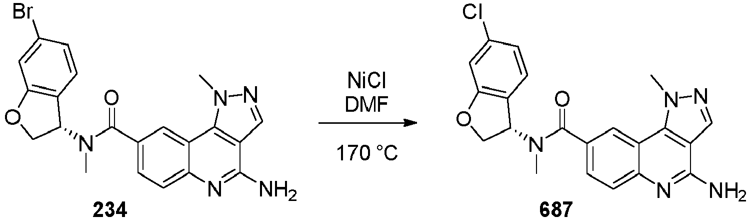

To a mixture of (S)-4-amino-N-(6-bromo-2,3-dihydrobenzofuran-3-yl)-N,1-dimethyl-1H-pyrazolo[4,3-c]quinoline-8-carboxamide **(234)** (0.0113 g, 0.025 mmol) and N, N-dimethylformamide (0.5 mL) in a microwave vial was added nickel (II) chloride (9.71 mg, 0.075 mmol, Sigma-Aldrich Corporation) in a dry box. The vial was sealed and heated in a microwave reactor at 170°C for 5 min. More nickel (II) chloride (9.71 mg, 0.075 mmol, Sigma-Aldrich Corporation) was added and the reaction was reset at 170 °C for 1 h. The crude product was filtered and purified by reverse phase prep HPLC uisng 10-90% water in MeCN with 0.1% TFA. (S)-4-amino-N-(6-chloro-2,3-dihydrobenzofuran-3-yl)-N,1-dimethyl-1H-pyrazolo[4,3-c]quinoline-8-carboxamide 2,2,2-trifluoroacetate **(687)** (7.5 mg, 0.014 mmol, 57.5% yield) was obtained as a white solid. *m*/*z* (ESI): 408 (M+H)⁺. ¹H NMR (400 MHz, METHANOL-*d*₄) δ ppm 8.53 (s, 1 H), 8.51 (s, 1 H), 7.91 - 7.94 (m, 1 H), 7.85 (br d, *J*=8.5 Hz, 1 H), 7.35 - 7.50 (m, 1 H), 6.97 - 7.05 (m, 1 H), 6.93 (br s, 1 H), 6.37 - 6.48 (m, 1 H), 4.93 - 4.95 (m, 1 H), 4.73 - 4.75 (m, 1 H), 4.56 (s, 3 H), 2.80 (br s, 3 H). ¹⁹F NMR (377 MHz, METHANOL-*d*₄) δ ppm -77.03 (m, 3 F).

### Intermediate 688: (S)-4-amino-N,1-dimethyl-N-(6-(tetrahydro-2H-pyran-4-yl)-2,3-dihydrobenzofuran-3-yl)-1H-pyrazolo[4,3-c]quinoline-8-carboxamide

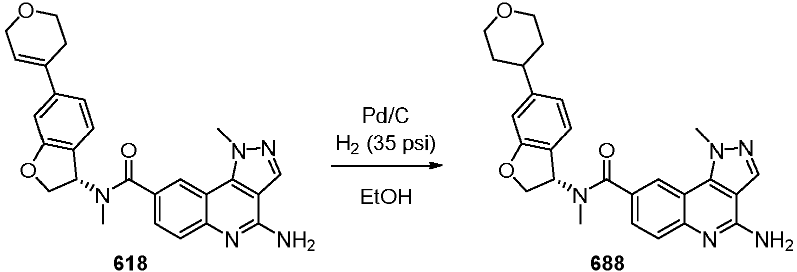

A mixture of 10% palladium on carbon (0.102 g, 0.095 mmol, Sigma-Aldrich Corporation), (S)-4-amino-N-(6-(3,6-dihydro-2H-pyran-4-yl)-2,3-dihydrobenzofuran-3-yl)-N,1-dimethyl-1H-pyrazolo[4,3-c]quinoline-8-carboxamide **(618)** (0.0435 g, 0.095 mmol) and ethanol (4 mL) was purged with N₂, then with H₂. The mixture was stirred in a sealed vial with H₂ pressure at 35 psi overnight. The reaction was reset at 35 psi and stirred for 4 h. The stirring was stopped and the reaction was allowed to stand at rt for 2 days. Then, the catalyst was filtered through celite, the solid was washed with EtOAc/EtOH (3/1), and the filtrate was concentrated in vacuo. The crude product was purified by silica gel chromatography using 0-30%-100% EtOAc/EtOH (3/1) in heptane. (S)-4-amino-N,1-dimethyl-N-(6-(tetrahydro-2H-pyran-4-yl)-2,3-dihydrobenzofuran-3-yl)-1H-pyrazolo[4,3-c]quinoline-8-carboxamide **(688)** (20.8 mg, 0.045 mmol, 47.6% yield) was isolated as off-white solid. *m*/*z* (ESI): 458 (M+H)⁺. ¹H NMR (400 MHz, CHLOROFORM-d) δ ppm 8.39 (d, *J*=1.7 Hz, 1 H), 7.94 - 7.99 (m, 1 H), 7.79 (d, *J*=8.6 Hz, 1 H), 7.62 (dd, *J*=8.6, 1.9 Hz, 1 H), 7.26 - 7.34 (m, 1 H), 6.85 (dd, *J*=7.7, 1.0 Hz, 1 H), 6.75 (s, 1 H), 5.13 - 5.26 (m, 2 H), 4.63 - 4.80 (m, 1 H), 4.55 (d, *J*=4.0 Hz, 1 H), 4.52 (d, *J*=4.0 Hz, 1 H), 4.49 (s, 3 H), 4.08 - 4.11 (m, 1 H), 4.06 (br d, *J*=2.9 Hz, 1 H), 3.48 - 3.56 (m, 2 H), 2.77 - 2.83 (m, 3 H), 2.71 - 2.76 (m, 1 H), 1.73 - 1.86 (m, 4 H).

### Example 689: N-(6-(1-acetyl-1,2,3,6-tetrahydropyridin-4-yl)-2,3-dihydrobenzofuran-3-yl)-4-amino-N-methyl-1,3-dihydrofuro[3,4-c]quinoline-8-carboxamide

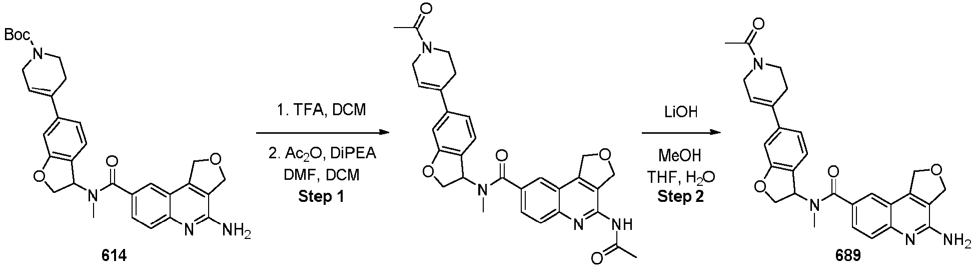

Step 1. To a mixture of tert-butyl 4-(3-(4-amino-N-methyl-1,3-dihydrofuro[3,4-c]quinoline-8-carboxamido)-2,3-dihydrobenzofuran-6-yl)-3,6-dihydropyridine-1(2H)-carboxylate **(614)** (0.070 g, 0.129 mmol) and DCM (2 mL) was added TFA (0.5 mL). The mixture was stirred at rt for 1 h. The mixture was concentrated in vacuo to afford the TFA salt of the product as off-white solid. *m*/*z* (ESI): 443 (M+H)⁺.

To a mixture of 4-amino-N-methyl-N-(6-(1,2,3,6-tetrahydropyridin-4-yl)-2,3-dihydrobenzofuran-3-yl)-1,3-dihydrofuro[3,4-c]quinoline-8-carboxamide (3.00 mg, 6.78 µmol), DIPEA (70.1 mg, 95.0 µL, 0.542 mmol, Sigma-Aldrich Corporation), DCM (1 mL) and DMF (0.1 mL) was added acetic anhydride (8.31 mg, 7.68 µL, 0.081 mmol, Sigma-Aldrich Corporation). The mixture was stirred at rt for 3 h, concentrated in vacuo, and used directly in the next step. *m*/*z* (ESI): 527 (M+H)⁺.

Step 2. The crude 4-acetamido-N-(6-(1-acetyl-1,2,3,6-tetrahydropyridin-4-yl)-2,3-dihydrobenzofuran-3-yl)-N-methyl-1,3-dihydrofuro[3,4-c]quinoline-8-carboxamide was diluted with THF (0.9 mL), MeOH (0.9 mL) and water (0.4 mL) and treated with lithium hydroxide hydrate (17.1 mg, 0.406 mmol, Sigma-Aldrich Corporation). The mixture was stirred at rt overnight. The mixture was diluted with Na₂CO₃ and EtOAc. The organic phase was washed with water and brine, dried over Na₂SO₄ and concentrated in vacuo. The residue was purified by silica gel chromatography, using a mobile phase of EtOAc/EtOH (3/1) in heptane (0-100%) to give N-(6-(1-acetyl-1,2,3,6-tetrahydropyridin-4-yl)-2,3-dihydrobenzofuran-3-yl)-4-amino-N-methyl-1,3-dihydrofuro[3,4-c]quinoline-8-carboxamide (689) (1.9 mg, 3.92 µmol, 57.8% yield) as a white solid. *m*/*z* (ESI): 485 (M+H)⁺. ¹H NMR (400 MHz, METHANOL-*d*₄) δ ppm 7.63 - 7.75 (m, 3 H), 7.28 - 7.43 (m, 1 H), 7.08 (br d, *J*=7.7 Hz, 1 H), 6.84 - 6.98 (m, 1 H), 6.05 - 6.18 (m, 1 H), 5.45 (br s, 2 H), 5.13 (t, *J*=3.4 Hz, 2 H), 4.53 - 4.68 (m, 2 H), 4.16 - 4.24 (m, 2 H), 4.02 - 4.12 (m, 1 H), 3.69 - 3.82 (m, 2 H), 2.69 - 2.81 (m, 3 H), 2.61 (br s, 1 H), 2.49 - 2.57 (m, 1 H), 2.16 (d, *J*=14.5 Hz, 3 H).

### HCT116 Proliferation Activity

To assess selective anti-proliferative activity of compounds of the invention in cells that have loss expression of MTAP, an HCT-116 isogenic cell line pair was utilized where one cell line was engineered to genetically knockout both MTAP alleles. Cell viability was then assesed in both the parent HCT-116 cell line and the MTAP null cell line after 6 days of treatment with compounds of the present invention. Selective anti-proliferative activity in the MTAP null cell line indicates MTA-cooperative inhibition of PRMT5 and ability to inhibit growth of cancer cells that have loss of MTAP.

HCT116 MTAP null and WT cells were seeded in 96-well tissue culture plates in RPMI 1640 media + 10% fetal bovine serum. Plates were incubated overnight at 37°C and 5% CO₂. Cells were then treated with an 8- or 9-point serial dilution of compound, using a top concentration of 1, or 10 µM, 1:3 serial dilution steps and, a DMSO-only control. Cells were incubated in the presence of drug for 6 days. Effects on cell viability were measured with the CellTiter-Glo^{®} Luminescent Cell Viability Assay (Promega) per manufacturer's recommendation. Assay plates were read on an EnVision^{™} Multilabel Reader using the Ultra-Sensitive luminescence module. IC₅₀ values were calculated with GraphPad Prism v 5.01 using symmetrical sigmoidal dose-response least squares fit with Hill slope fixed to -1 and top constrain to 100% or GeneData Screener using a 4-parameter logistic model to fit dose response curves.

Alternatively, compounds could be assayed with a 384 well plate format:
Compounds were pre-spotted into 384 well plates with a 22-point serial dilution of compound, using a top concentration of 10 or 50 µM, 1:2 serial dilution steps and, a DMSO-only control. HCT116 MTAP null and WT cells were then seeded as above and after 6 days effects on cell viability were measured with the Cell Titer-Glo^{®} Luminescent Cell Viability Assay (Promega). Assay plates were read as above and IC₅₀ values were calculated with GeneData Screener using a 4-parameter logistic model to fit dose response curves. The reported IC₅₀ represents the value where the curve transits 50% of control.

**Table 23. HCT116-MTAP null and WT cell line proliferation**

| **Ex.** | **HCT-116 MTAP null IC₅₀ (µM)** | **HCT-116 WT IC₅₀ (µM)** |
|---|---|---|
| **200** | 0.008 | 0.112 |
| **201** | 0.149 | 5.820 |
| **202** | 0.083 | 11.400 |
| **203** | 0.093 | 4.590 |
| **204** | 0.031 | 4.390 |
| **205** | 0.360 | 12.300 |
| **206** | 0.100 | 9.790 |
| **207** | 6.060 | 23.200 |
| **208** | 0.553 | 16.600 |
| **209** | 0.061 | 3.000 |
| **210** | 2.370 | 25.300 |
| **211** | 0.028 | 1.463 |
| **212** | 0.495 | 10.700 |
| **213** | 0.365 | 12.900 |
| **214** | 0.029 | 2.060 |
| **215** | 0.427 | 15.000 |
| **216** | | |
| **217** | 0.010 | 1.020 |
| **218** | 0.013 | 0.838 |
| **219** | 0.105 | 2.770 |
| **220** | 0.018 | 0.432 |
| **221** | 0.590 | 4.290 |
| **222** | 0.280 | 3.910 |
| **223** | 0.023 | 0.437 |
| **224** | 0.424 | 3.600 |
| **225** | 0.403 | 4.940 |
| **226** | 0.038 | 2.150 |
| **227** | 0.019 | 1.480 |
| **228** | 0.007 | 0.683 |
| **229** | 0.265 | 7.740 |
| **230** | 0.007 | 0.530 |
| **231** | 0.215 | 2.060 |
| **232** | 0.222 | 2.490 |
| **233** | 0.723 | 19.000 |
| **234** | 0.008 | 0.613 |
| **235** | 0.219 | 10.000 |
| **236** | 0.786 | 24.200 |
| **237** | 0.012 | 0.192 |
| **238** | 0.215 | 13.000 |
| **239** | 0.010 | 0.168 |
| **240** | 0.013 | 0.250 |
| **241** | 0.009 | 0.388 |
| **242** | 0.005 | 0.533 |
| **243** | 0.009 | 0.082 |
| **244** | 0.182 | 7.440 |
| **245** | 0.019 | 0.880 |
| **246** | 0.610 | 8.480 |
| **247** | 0.005 | 0.304 |
| **248** | 0.018 | 0.635 |
| **249** | 0.003 | 0.066 |
| **250** | 0.002 | 0.046 |
| **251** | 0.007 | 0.217 |
| **252** | 0.019 | 0.234 |
| **253** | 0.035 | 2.100 |
| **254** | 0.308 | 20.200 |
| **255** | 0.047 | 2.400 |
| **256** | 0.143 | 7.500 |
| **257** | 0.059 | 2.020 |
| **258** | 0.054 | 4.030 |
| **259** | 0.062 | 3.240 |
| **260** | 0.082 | 10.500 |
| **261** | 0.015 | 0.703 |
| **262** | 0.065 | 1.950 |
| **263** | 0.058 | 2.010 |
| **264** | 0.070 | 5.110 |
| **265** | 0.151 | 3.850 |
| **266** | 0.070 | 2.660 |
| **267** | 0.036 | 1.980 |
| **268** | 0.047 | 8.563 |
| **269** | 0.039 | 2.930 |
| **270** | 0.017 | 0.740 |
| **271** | 0.956 | 31.400 |
| **272** | 0.206 | 5.390 |
| **273** | | |
| **274** | 0.004 | 0.229 |
| **275** | 0.083 | 2.670 |
| **276** | 0.016 | 0.417 |
| **277** | 0.005 | 0.202 |
| **278** | 0.056 | 4.290 |
| **279** | 0.017 | 0.706 |
| **280** | 0.461 | 5.050 |
| **281** | 0.023 | 0.611 |
| **282** | 0.475 | 5.970 |
| **283** | 0.021 | 1.365 |
| **284** | 0.674 | 15.600 |
| **285** | 0.974 | 31.300 |
| **286** | 0.008 | 0.619 |
| **287** | | |
| **288** | 0.006 | 0.072 |
| **289** | 0.016 | 0.357 |
| **290** | 0.061 | 2.460 |
| **291** | 0.169 | 10.500 |
| **292** | 0.007 | 0.224 |
| **293** | 0.305 | 7.210 |
| **294** | 0.003 | 0.012 |
| **295** | 0.002 | 0.034 |
| **296** | 0.008 | 0.159 |
| **297** | 0.006 | 0.162 |
| **298** | 0.001 | 0.016 |
| **299** | 0.037 | 1.520 |
| **300** | 0.016 | 0.225 |
| **301** | 0.028 | 1.250 |
| **302** | 0.006 | 0.161 |
| **303** | 0.014 | 0.902 |
| **304** | 0.198 | 9.230 |
| **305** | 0.020 | 0.429 |
| **306** | 0.111 | 5.060 |
| **307** | 0.019 | 0.471 |
| **308** | 0.076 | 4.130 |
| **309** | 0.150 | 9.590 |
| **310** | 0.110 | 7.380 |
| **311** | 0.030 | 1.020 |
| **312** | 0.103 | 9.480 |
| **313** | 0.014 | 0.523 |
| **314** | 0.041 | 2.630 |
| **315** | 0.508 | 13.400 |
| **316** | 0.026 | 1.720 |
| **317** | 0.024 | 1.165 |
| **318** | 1.360 | 10.000 |
| **319** | 0.048 | 2.210 |
| **320** | 3.665 | 36.450 |
| **321** | 2.660 | 20.100 |
| **322** | 0.041 | 1.390 |
| **323** | 0.076 | 3.447 |
| **324** | 5.315 | 50.000 |
| **325** | 0.068 | 4.160 |
| **326** | 1.785 | 20.550 |
| **327** | 0.011 | 0.457 |
| **328** | 2.160 | 10.100 |
| **329** | 0.046 | 1.820 |
| **330** | 1.610 | 22.900 |
| **331** | 0.026 | 2.240 |
| **332** | 0.920 | 22.300 |
| **333** | 0.173 | 8.483 |
| **334** | 11.900 | 50.000 |
| **335** | 0.272 | 10.900 |
| **336** | 11.900 | 50.000 |
| **337** | 0.041 | 2.093 |
| **338** | 4.675 | 37.500 |
| **339** | 0.237 | 8.780 |
| **340** | 7.320 | 50.000 |
| **341** | 0.184 | 6.240 |
| **342** | 4.445 | 28.000 |
| **343** | 0.019 | 0.581 |
| **344** | 1.060 | 19.400 |
| **345** | 0.091 | 5.443 |
| **346** | 6.010 | 37.000 |
| **347** | 0.086 | 3.220 |
| **348** | 1.900 | 23.450 |
| **349** | 0.039 | 2.243 |
| **350** | 3.165 | 50.000 |
| **351** | 0.083 | 2.847 |
| **352** | 3.247 | 50.000 |
| **353** | 0.032 | 1.377 |
| **354** | 3.870 | 50.000 |
| **355** | 0.026 | 1.180 |
| **356** | 1.040 | 11.300 |
| **357** | 0.012 | 0.208 |
| **358** | 1.200 | 8.850 |
| **359** | 0.017 | 1.233 |
| **360** | 0.810 | 42.300 |
| **361** | 0.029 | 1.340 |
| **362** | 0.744 | 35.600 |
| **363** | 0.017 | 2.503 |
| **364** | 2.857 | 34.875 |
| **365** | 0.005 | 0.212 |
| **366** | 0.006 | 0.155 |
| **367** | 0.485 | 5.270 |
| **368** | 0.037 | 2.530 |
| **369** | 0.519 | 4.130 |
| **370** | 0.029 | 1.800 |
| **371** | 0.027 | 1.623 |
| **372** | 0.010 | 0.114 |
| **373** | 0.004 | 0.235 |
| **374** | 0.272 | 22.350 |
| **375** | 0.009 | 0.568 |
| **376** | 0.882 | 12.755 |
| **377** | 0.010 | 0.595 |
| **378** | 0.010 | 0.239 |
| **379** | 0.490 | 13.500 |
| **380** | 0.023 | 1.540 |
| **381** | 0.195 | 11.100 |
| **382** | 0.004 | 0.063 |
| **383** | 0.380 | 5.660 |
| **384** | 0.869 | 17.200 |
| **385** | 0.007 | 0.297 |
| **386** | 0.552 | 13.600 |
| **387** | 0.005 | 0.136 |
| **388** | 0.136 | 7.540 |
| **389** | 0.185 | 17.200 |
| **390** | 6.550 | 25.000 |
| **391** | 0.038 | 1.040 |
| **392** | 0.995 | 8.300 |
| **393** | 0.136 | 4.070 |
| **394** | 0.025 | 1.440 |
| **395** | 0.696 | 10.000 |
| **396** | 0.010 | 0.272 |
| **397** | 0.599 | 18.700 |
| **398** | 0.020 | 0.911 |
| **399** | 1.330 | 10.000 |
| **400** | 0.050 | 2.108 |
| **401** | 0.148 | 6.570 |
| **402** | 0.008 | 0.436 |
| **403** | 0.463 | 25.800 |
| **404** | 0.009 | 0.250 |
| **405** | 0.696 | 14.700 |
| **406** | 0.011 | 0.322 |
| **407** | 0.554 | 23.300 |
| **408** | 0.024 | 1.710 |
| **409** | 0.048 | 3.910 |
| **410** | 0.021 | 1.470 |
| **411** | 0.083 | 6.270 |
| **412** | 0.004 | 0.228 |
| **413** | 0.360 | 7.990 |
| **414** | 0.001 | 0.019 |
| **415** | 0.078 | 2.630 |
| **416** | 0.006 | 0.414 |
| **417** | 0.841 | 6.105 |
| **418** | 0.003 | 0.421 |
| **419** | 0.545 | 26.400 |
| **420** | 0.015 | 0.439 |
| **421** | 0.792 | 35.200 |
| **422** | 0.010 | 0.241 |
| **423** | 0.039 | 1.610 |
| **424** | 0.593 | 18.600 |
| **425** | 0.158 | 13.400 |
| **426** | 0.355 | 27.900 |
| **427** | 0.008 | 0.247 |
| **428** | 0.275 | 8.340 |
| **429** | 0.049 | 2.350 |
| **430** | 0.003 | 0.075 |
| **431** | 0.305 | 5.880 |
| **432** | 0.009 | 0.127 |
| **433** | 0.418 | 3.280 |
| **434** | 0.223 | 11.500 |
| **435** | 0.320 | 13.400 |
| **436** | 0.002 | 0.023 |
| **437** | 0.041 | 0.687 |
| **438** | 0.193 | 25.200 |
| **439** | 0.192 | 9.460 |
| **440** | 0.048 | 3.010 |
| **441** | 0.181 | 9.310 |
| **442** | 0.009 | 0.200 |
| **443** | 0.433 | 3.460 |
| **444** | 0.348 | 12.050 |
| **445** | 0.316 | 12.600 |
| **446** | 0.864 | 36.000 |
| **447** | 0.724 | 24.900 |
| **448** | 0.058 | 3.710 |
| **449** | 0.096 | 3.348 |
| **450** | 2.070 | 37.800 |
| **451** | 5.183 | 9.517 |
| **452** | 0.173 | 13.400 |
| **453** | 0.246 | 13.300 |
| **454** | 3.555 | 50.000 |
| **455** | 0.122 | 5.620 |
| **456** | 0.842 | 24.100 |
| **457** | 0.063 | 3.770 |
| **458** | 5.050 | 38.500 |
| **459** | 0.091 | 5.540 |
| **460** | 0.162 | 10.800 |
| **461** | 0.871 | 38.300 |
| **462** | 0.237 | 11.700 |
| **463** | 0.134 | 14.700 |
| **464** | 0.022 | 1.880 |
| **465** | 1.420 | 32.200 |
| **466** | 0.009 | 0.245 |
| **467** | 0.010 | 0.418 |
| **468** | 0.114 | 2.690 |
| **469** | 2.730 | 19.700 |
| **470** | 0.027 | 0.256 |
| **471** | 0.574 | 13.900 |
| **472** | 0.022 | 1.145 |
| **473** | 0.015 | 0.349 |
| **474** | 0.008 | 0.174 |
| **475** | 0.008 | 0.199 |
| **476** | 0.006 | 0.125 |
| **477** | 0.092 | 2.530 |
| **478** | 0.005 | 0.133 |
| **479** | 0.023 | 0.710 |
| **480** | 0.004 | 0.043 |
| **481** | 0.005 | 0.045 |
| **482** | 0.166 | 17.500 |
| **483** | 0.081 | 5.280 |
| **484** | 0.221 | 5.590 |
| **485** | 0.125 | 11.700 |
| **486** | 0.078 | 4.880 |
| **487** | 0.047 | 2.870 |
| **488** | 0.013 | 0.682 |
| **489** | 0.049 | 2.220 |
| **490** | 0.033 | 1.030 |
| **491** | 0.043 | 2.055 |
| **492** | 0.706 | 31.400 |
| **493** | 0.092 | 2.050 |
| **494** | 0.023 | 0.671 |
| **495** | 0.015 | 0.386 |
| **496** | 0.005 | 0.205 |
| **497** | 0.350 | 19.700 |
| **498** | 0.185 | 9.110 |
| **499** | 0.004 | 0.152 |
| **500** | 0.010 | 0.501 |
| **501** | 0.004 | 0.141 |
| **502** | 0.414 | 11.800 |
| **503** | 0.018 | 1.255 |
| **504** | 0.004 | 0.144 |
| **505** | 0.244 | 12.100 |
| **506** | 0.004 | 0.034 |
| **507** | 0.008 | 0.162 |
| **508** | 0.005 | 0.043 |
| **509** | 0.107 | 8.110 |
| **510** | 0.048 | 4.588 |
| **511** | 0.374 | 17.400 |
| **512** | 0.123 | 11.080 |
| **513** | 0.168 | 10.200 |
| **514** | 0.068 | 1.525 |
| **515** | 0.037 | 3.648 |
| **516** | 0.105 | 6.520 |
| **517** | 0.018 | 0.342 |
| **518** | 0.965 | 9.330 |
| **519** | 0.027 | 1.390 |
| **520** | 0.025 | 2.415 |
| **521** | 0.168 | 14.100 |
| **522** | 0.063 | 2.474 |
| **523** | 2.720 | 10.000 |
| **524** | 0.396 | 24.500 |
| **525** | 4.165 | 50.000 |
| **526** | 0.052 | 3.820 |
| **527** | 0.143 | 7.520 |
| **528** | 5.280 | 50.000 |
| **529** | 0.089 | 5.590 |
| **530** | 2.510 | 40.200 |
| **531** | 0.113 | 4.860 |
| **532** | 2.510 | 6.680 |
| **533** | 0.017 | 0.816 |
| **534** | 1.730 | 23.900 |
| **535** | 0.007 | 0.198 |
| **536** | 0.728 | 11.700 |
| **537** | 0.021 | 0.881 |
| **538** | 1.845 | 23.650 |
| **539** | 0.015 | 0.663 |
| **540** | 0.722 | 18.600 |
| **541** | 0.077 | 3.800 |
| **542** | 0.785 | 46.000 |
| **543** | 0.049 | 1.460 |
| **544** | 5.230 | 15.200 |
| **545** | 0.068 | 3.250 |
| **546** | 9.590 | 30.700 |
| **547** | 0.122 | 2.457 |
| **548** | 0.058 | 3.060 |
| **549** | 0.231 | 5.595 |
| **550** | 3.325 | 23.050 |
| **551** | 0.017 | 1.345 |
| **552** | 0.034 | 1.224 |
| **553** | 1.380 | 11.400 |
| **554** | 0.021 | 1.396 |
| **555** | 1.620 | 13.300 |
| **556** | 0.011 | 0.133 |
| **557** | 0.665 | 10.500 |
| **558** | 0.017 | 0.941 |
| **559** | 1.275 | 41.400 |
| **560** | 0.012 | 0.594 |
| **561** | 0.657 | 10.830 |
| **562** | 0.026 | 1.817 |
| **563** | 1.960 | 25.100 |
| **564** | 0.019 | 0.925 |
| **565** | 0.858 | 15.000 |
| **566** | 0.013 | 0.447 |
| **567** | 0.313 | 9.870 |
| **568** | 0.097 | 5.800 |
| **569** | 0.006 | 0.222 |
| **570** | 0.134 | 9.210 |
| **571** | 0.010 | 0.116 |
| **572** | 0.758 | 11.350 |
| **573** | 0.012 | 0.685 |
| **574** | 0.415 | 40.100 |
| **575** | 0.179 | 7.607 |
| **576** | 4.915 | 13.250 |
| **577** | 0.132 | 16.133 |
| **578** | 0.222 | 5.880 |
| **579** | 2.370 | 4.510 |
| **580** | 0.019 | 0.976 |
| **581** | 0.790 | 17.900 |
| **582** | 0.076 | 5.580 |
| **583** | 0.403 | 8.860 |
| **584** | 6.990 | 50.000 |
| **585** | 0.256 | 12.550 |
| **586** | 3.020 | 10.000 |
| **587** | 0.021 | 0.573 |
| **588** | 1.980 | 10.000 |
| **589** | 7.950 | 34.700 |
| **590** | 0.129 | 4.503 |
| **591** | 0.026 | 1.605 |
| **592** | 0.021 | 1.020 |
| **593** | 0.005 | 0.201 |
| **594** | 0.228 | 19.350 |
| **595** | 0.005 | 0.275 |
| **596** | 0.185 | 17.100 |
| **597** | 0.015 | 0.795 |
| **598** | 0.416 | 10.100 |
| **599** | 0.064 | 3.820 |
| **600** | 0.010 | 0.226 |
| **601** | 0.021 | 2.100 |
| **602** | 4.260 | 21.300 |
| **603** | 0.327 | 16.000 |
| **604** | 2.305 | 19.100 |
| **605** | 0.285 | 26.400 |
| **606** | 0.724 | 13.500 |
| **607** | 0.021 | 0.825 |
| **608** | 0.026 | 1.041 |
| **609** | 0.598 | 7.800 |
| **610** | 0.108 | 5.510 |
| **611** | 1.690 | 10.500 |
| **612** | 0.033 | 2.640 |
| **613** | 0.860 | 2.630 |
| **614** | 0.127 | 1.360 |
| **615** | 0.014 | 1.310 |
| **616** | 0.032 | 3.670 |
| **617** | 0.055 | 1.650 |
| **618** | 0.010 | 0.786 |
| **619** | 0.017 | 0.649 |
| **620** | 0.006 | 0.634 |
| **621** | 0.025 | 1.040 |
| **622** | 0.014 | 0.877 |
| **623** | 0.064 | 3.490 |
| **624** | 0.075 | 6.510 |
| **625** | 0.029 | 2.320 |
| **626** | 0.006 | 0.384 |
| **627** | 0.004 | 0.136 |
| **628** | 0.005 | 0.511 |
| **629** | 0.004 | 0.691 |
| **630** | 0.003 | 0.222 |
| **631** | 0.023 | 2.533 |
| **632** | 0.026 | 0.841 |
| **633** | 0.008 | 0.672 |
| **634** | 0.008 | 0.524 |
| **635** | 0.009 | 0.613 |
| **636** | 0.013 | 1.948 |
| **637** | 0.339 | 32.133 |
| **638** | 0.006 | 0.591 |
| **639** | 0.015 | 1.400 |
| **640** | 0.016 | 2.050 |
| **641** | 0.042 | 1.600 |
| **642** | 0.005 | 0.535 |
| **643** | 0.005 | 0.228 |
| **644** | 0.027 | 3.557 |
| **645** | 0.024 | 1.470 |
| **646** | 0.003 | 0.329 |
| **647** | 0.008 | 0.582 |
| **648** | 0.030 | 1.170 |
| **649** | 0.018 | 1.040 |
| **650** | 0.008 | 0.447 |
| **651** | 0.007 | 0.724 |
| **652** | 0.006 | 0.291 |
| **653** | 0.019 | 0.735 |
| **654** | 0.010 | 1.000 |
| **655** | 0.028 | 0.946 |
| **656** | 0.006 | 0.511 |
| **657** | 0.037 | 1.710 |
| **658** | 0.042 | 0.630 |
| **659** | 0.025 | 1.280 |
| **660** | 0.015 | 0.657 |
| **661** | 0.014 | 0.481 |
| **662** | 0.021 | 1.120 |
| **663** | 0.010 | 0.922 |
| **664** | 0.319 | 1.770 |
| **665** | 0.875 | 2.510 |
| **666** | 0.022 | 1.100 |
| **667** | 0.383 | 7.065 |
| **668** | 0.085 | 2.920 |
| **669** | 0.016 | 1.001 |
| **670** | 0.536 | 1.117 |
| **671** | 0.034 | 4.285 |
| **672** | 0.044 | 3.525 |
| **673** | 0.120 | 13.107 |
| **674** | 0.156 | 8.610 |
| **675** | 0.025 | 2.753 |
| **676** | 0.040 | 2.590 |
| **677** | 0.137 | 9.810 |
| **678** | 0.029 | 2.780 |
| **679** | 0.026 | 2.270 |
| **680** | 0.168 | 7.960 |
| **681** | 0.530 | 9.330 |
| **682** | 0.297 | 21.000 |
| **683** | 0.038 | 3.577 |
| **684** | 0.006 | 0.227 |
| **685** | 0.022 | 1.520 |
| **686** | 0.129 | 11.400 |
| **687** | 0.012 | 0.846 |
| **688** | 0.005 | 0.431 |
| **689** | 0.101 | 4.540 |

## Claims

1. A compound of Formula I 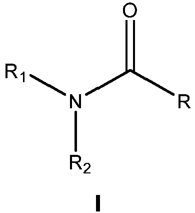
a tautomer thereof, a stereoisomer thereof, or a pharmaceutically acceptable salt of any of the foregoing;
wherein R is a tricycle independently selected from the formula IA: 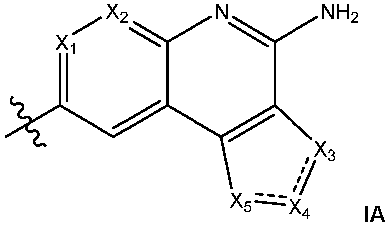
wherein 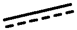 is a single or double bond;
X¹ and X² are in each instance independently selected from optionally substituted N and C, wherein substituents are independently selected from C₁₋₃ alkyl;
wherein both X¹ and X² cannot be N at the same time;
wherein if X¹ is C, it can be optionally substituted with halo, halo C₁₋₃ alkyl or -CN;
X³, X⁴ and X⁵ are at each instance independently selected from optionally substituted C, O and N, wherein the substituents are independently selected from C₁₋₃ alkyl, and C₁₋₃ alkyl(OH), wherein alkyl can be optionally substituted with halo;
wherein R¹ is a bicycle independently selected from the formulae IB, IC and ID, optionally substituted with R⁴; 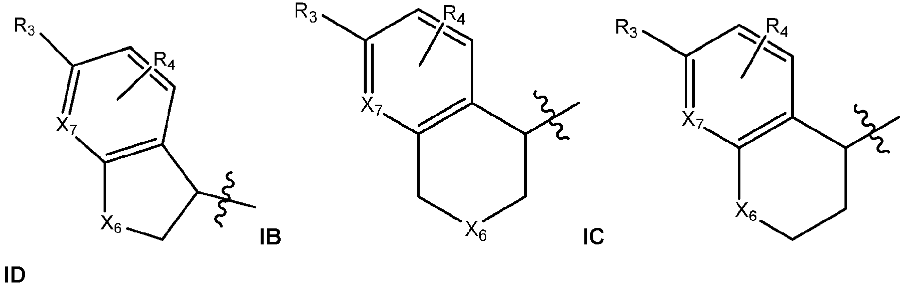
wherein X⁶ is in each instance independently selected from O and C;
wherein X⁷ is in each instance independently selected from N and C;
wherein R² is in each instance independently selected from an optionally substituted C₁₋₆ alkyl or optionally substituted C₁₋₆ cycloalkyl wherein the substituents are selected from -CN or C₁₋₆ cycloalkyl;
wherein R³ is in each instance independently selected from C₁₋₆ alkyl, C₁₋₆ cycloalkyl, halo, C₁₋₆ haloalkyl, -S(=O)₂C₁₋₆ alkyl, -S(O)(NH) C₁₋₆ alkyl, -S(O)(N-C₁₋₃ alkyl)C₁₋₆ alkyl, -CN, -OC₁₋₆ alkyl, -OC₁₋₆ haloalkyl, -N(=O)-OC₁₋₆ alkyl, -C(O)C₁₋₆ alkyl, -C(O)C₁₋₆ haloalkyl, 3,6-dihydro-2H-pyranyl and pentafluorosulfanyl;
wherein R⁴ is in each instance independently selected from C₁₋₆ alkyl, halo, and C₁₋₆ haloalkyl.

2. The compound of claim 1, the tautomer thereof, the stereoisomer thereof, or the pharmaceutically acceptable salt of any of the foregoing, wherein R is

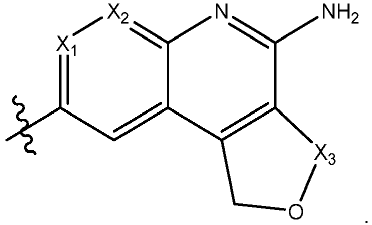

3. The compound of claim 2, the tautomer thereof, the stereoisomer thereof, or the pharmaceutically acceptable salt of any of the foregoing, wherein X¹ is C, optionally substituted with halo; or wherein X¹ is N.

4. The compound of claim 2, the tautomer thereof, the stereoisomer thereof, or the pharmaceutically acceptable salt of any of the foregoing, wherein X³ is optionally substituted C.

5. The compound of claim 1, the tautomer thereof, the stereoisomer thereof, or the pharmaceutically acceptable salt of any of the foregoing, wherein R is 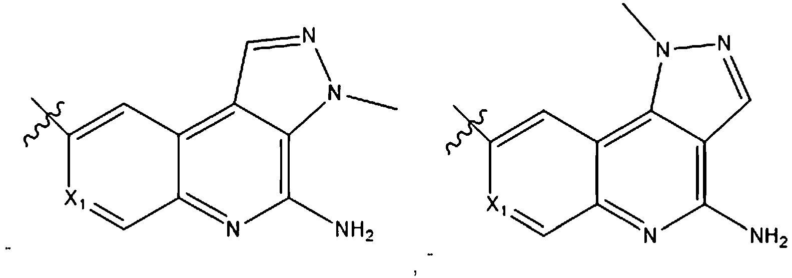 or

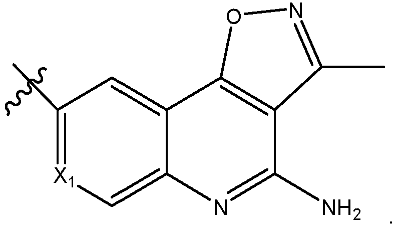

6. The compound of claim 5, the tautomer thereof, the stereoisomer thereof, or the pharmaceutically acceptable salt of any of the foregoing, wherein X¹ is C, optionally substituted, preferably with halo.

7. The compound of any of the claims 1, or 5, the tautomer thereof, the stereoisomer thereof, or the pharmaceutically acceptable salt of any of the foregoing, wherein R' is IB, IC or ID.

8. The compound of claim 7, the tautomer thereof, the stereoisomer thereof, or the pharmaceutically acceptable salt of any of the foregoing, wherein R¹ is substituted with R⁴.

9. The compound of claim 8, the tautomer thereof, the stereoisomer thereof, or the pharmaceutically acceptable salt of any of the foregoing, wherein R⁴ is halo.

10. The compound of claim 1, the tautomer thereof, the stereoisomer thereof, or the pharmaceutically acceptable salt of any of the foregoing, wherein
a) R³ is in each instance independently selected from C₁₋₆ alkyl, halo, and C₁₋₆ haloalkyl; or
b) R³ is in each instance independently selected from -S(=O)₂C₁₋₆ alkyl and -CN.

11. The compound of any of the claims 1 or 5, the tautomer thereof, the stereoisomer thereof, or the pharmaceutically acceptable salt of any of the foregoing, wherein R² is independently selected from methyl, ethyl and cyclopropyl.

12. The compound, the tautomer thereof, the stereoisomer thereof, or the pharmaceutically acceptable salt of claim 1, wherein the compound is selected from:
4-amino-7-chloro-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-N-((4S)-8-fluoro-7-(trifluoromethyl)-3,4-dihydro-1H-2-benzopyran-4-yl)-N,1-dimethyl-1H-pyrazolo[4,3-c]quinoline-8-carboxamide,
4-amino-N-((5S)-2-bromo-5,8-dihydro-6H-pyrano[3,4-b]pyridin-5-yl)-N,1-dimethyl-1H-pyrazolo[4,3-c]quinoline-8-carboxamide,
4-amino-7-chloro-N,1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-7-fluoro-N-methyl-N-((5R)-2-(trifluoromethyl)-6,7-dihydro-5H-cyclopenta[b]pyridin-5-yl)-1,3-dihydrofuro[3,4-c]quinoline-8-carboxamide,
(3R)-4-amino-7-fluoro-N,3-dimethyl-N-((5R)-2-(trifluoromethyl)-6,7-dihydro-5H-cyclopenta[b]pyridin-5-yl)-1,3-dihydrofuro[3,4-c]quinoline-8-carboxamide,
4-amino-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-N,1-dimethyl-N-((3S)-6-(pentafluoro-lambda~6~-sulfanyl)-2,3-dihydro-1-benzofuran-3-yl)-1H-pyrazolo[4,3-c]quinoline-8-carboxamide,
4-amino-N,1-dimethyl-N-((5R)-2-(trifluoromethyl)-6,7-dihydro-5H-cyclopenta[b]pyridin-5-yl)-1H-pyrazolo[4,3-c][1,7]naphthyridine-8-carboxamide,
4-amino-7-fluoro-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-7-fluoro-N,1-dimethyl-N-((3S)-6-(trifluoromethyl)-2,3-dihydrofuro[2,3-b]pyridin-3-yl)-1H-pyrazolo[4,3-c]quinoline-8-carboxamide,
4-amino-7-chloro-N,1-dimethyl-N-((5R)-2-(trifluoromethyl)-6,7-dihydro-5H-cyclopenta[b]pyridin-5-yl)-1H-pyrazolo[4,3-c]quinoline-8-carboxamide,
4-amino-N-((4S)-8-fluoro-7-(trifluoromethyl)-3,4-dihydro-1H-2-benzopyran-4-yl)-N,1-dimethyl-1H-pyrazolo[4,3-c]quinoline-8-carboxamide,
4-amino-N-((5S)-2-bromo-5,8-dihydro-6H-pyrano[3,4-b]pyridin-5-yl)-N,1-dimethyl-1H-pyrazolo[4,3-c]quinoline-8-carboxamide,
4-amino-N,1-dimethyl-N-((3S)-6-(trifluoromethyl)-2,3-dihydro-1-benzofuran-3-yl)-1H-pyrazolo[4,3-c]quinoline-8-carboxamide,
(3R)-4-amino-N,3-dimethyl-N-((5S)-2-(trifluoromethyl)-5,8-dihydro-6H-pyrano[3,4-b]pyridin-5-yl)-1,3-dihydrofuro[3,4-c]quinoline-8-carboxamide,
4-amino-N-methyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamide,
4-amino-7-fluoro-N,1-dimethyl-N-((5S)-2-(trifluoromethyl)-5,8-dihydro-6H-pyrano[3,4-b]pyridin-5-yl)-1H-pyrazolo[4,3-c]quinoline-8-carboxamide,
4-amino-N-methyl-N-((3S)-6-(trifluoromethyl)-2,3-dihydro-1-benzofuran-3-yl)-1,3-dihydrofuro[3,4-c][1,7]naphthyridine-8-carboxamide,
4-amino-7-fluoro-N,1-dimethyl-N-((3S)-6-(trifluoromethyl)-2,3-dihydro-1-benzofuran-3-yl)-1H-pyrazolo[4,3-c]quinoline-8-carboxamide, and
4-amino-7-fluoro-N,1-dimethyl-N-((3S)-6-(trifluoromethyl)-2,3-dihydrofuro[3,2-c]pyridin-3-yl)-1H-pyrazolo[4,3-c]quinoline-8-carboxamide.

13. A pharmaceutical composition comprising a compound of any of claims 1 or 12, the tautomer thereof, the stereoisomer thereof, or the pharmaceutically acceptable salt of any of the foregoing and a pharmaceutically acceptable carrier.

14. The compound of any of claims 1 or 12, the tautomer thereof, the stereoisomer thereof, or the pharmaceutically acceptable salt of any of the foregoing for use in treating cancer.

15. The compound, the tautomer thereof, the stereoisomer thereof, or the pharmaceutically acceptable salt of any of the foregoing for use according to claim 14,
wherein the cancer is MTAP-null cancer, preferably
wherein the cancer is selected from ovarian, lung, HNSCC, lymphoid, glioblastoma, colon, melanoma, gastric, bile duct, pancreatic or bladder cancer.

## Patentansprüche

1. Eine Verbindung der Formel I 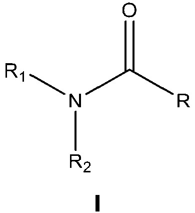
ein Tautomer davon, ein Stereoisomer davon oder ein pharmazeutisch verträgliches Salz einer der vorgenannten Verbindungen,
wobei R ein Tricyclus ist, der unabhängig aus der Formel IA ausgewählt ist: 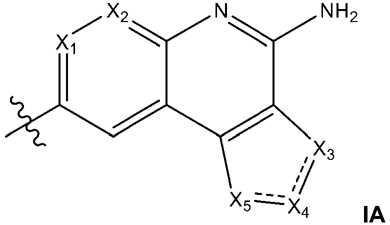
wobei 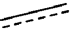 eine Einfach- oder Doppelbindung ist;
X' und X² in jedem Fall unabhängig voneinander ausgewählt sind aus gegebenenfalls substituiertem N und C, wobei die Substituenten unabhängig voneinander ausgewählt sind aus C₁₋₃-Alkyl;
wobei sowohl X¹ als auch X² nicht gleichzeitig N sein können;
wobei, wenn X¹ gleich C ist, es gegebenenfalls mit Halo, Halo-C₁₋₃-Alkyl oder -CN substituiert sein kann;
X³, X⁴ und X⁵ jeweils unabhängig ausgewählt sind aus gegebenenfalls substituiertem C, O und N, wobei die Substituenten unabhängig ausgewählt sind aus C₁₋₃-Alkyl und C₁₋₃-Alkyl(OH), wobei Alkyl gegebenenfalls mit Halogen substituiert sein kann;
wobei R¹ ein Radikal ist, unabhängig ausgewählt aus den Formeln IB, **IC** und ID, gegebenenfalls substituiert mit R⁴; 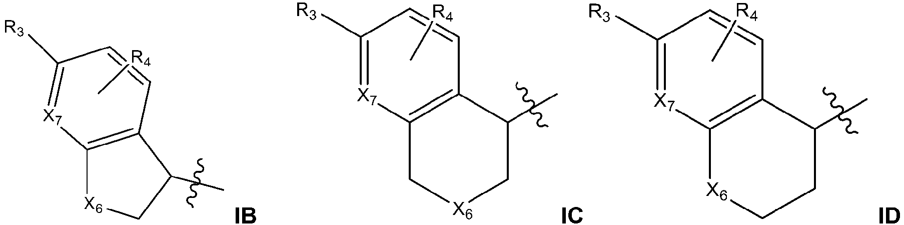
wobei X⁶ in jedem Fall unabhängig ausgewählt ist aus O und C;
wobei X⁷ in jedem Fall unabhängig ausgewählt ist aus N und C;
wobei R² in jedem Fall unabhängig ausgewählt ist aus einem gegebenenfalls substituierten C₁₋₆-Alkyl oder gegebenenfalls substituierten C₁₋₆-Cycloalkyl, wobei die Substituenten ausgewählt sind aus -CN oder C₁₋₆-Cycloalkyl;
wobei R³ in jedem Fall unabhängig ausgewählt ist aus C₁₋₆-Alkyl, C₁₋₆-Cycloalkyl, Halogen, C₁₋₆-Halogenalkyl, -S(=O)_{(2) C1-6}-Alkyl, -S(O)(NH) C₁₋₆-Alkyl, -S(O)(N-C₁₋₃-Alkyl)C₍₁₋₆)-Alkyl, - CN, -OC₁₋₆-Alkyl, -OC₁₋₆-Halogenalkyl, -N(=O)-OC₁₋₆-Alkyl, -C(O)C₍₁₋₆)-Alkyl, -C(O)C₁₋₆-Halogenalkyl, 3,6-Dihydro-2H-pyranyl und Pentafluorosulfanyl;
wobei R⁴ in jedem Fall unabhängig ausgewählt ist aus C₁₋₆-Alkyl, Halogen und C₁₋₆-Halogenalkyl.

2. Die Verbindung nach Anspruch 1, das Tautomer davon, das Stereoisomer davon oder das pharmazeutisch verträgliche Salz einer der vorgenannten Verbindungen, wobei R 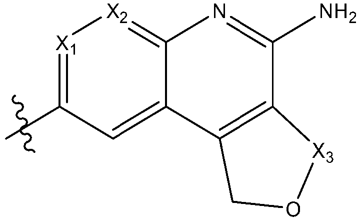 ist.

3. Die Verbindung nach Anspruch 2, das Tautomer davon, das Stereoisomer davon oder das pharmazeutisch verträgliche Salz einer der vorgenannten Verbindungen, wobei X¹ gleich C ist, das gegebenenfalls mit Halogen substituiert ist; oder wobei X¹ gleich N ist.

4. Die Verbindung nach Anspruch 2, das Tautomer davon, das Stereoisomer davon oder das pharmazeutisch verträgliche Salz einer der vorgenannten Verbindungen, wobei X³ ein gegebenenfalls substituiertes C ist.

5. Die Verbindung nach Anspruch 1, das Tautomer davon, das Stereoisomer davon oder das pharmazeutisch verträgliche Salz einer der vorgenannten Verbindungen, wo bei R 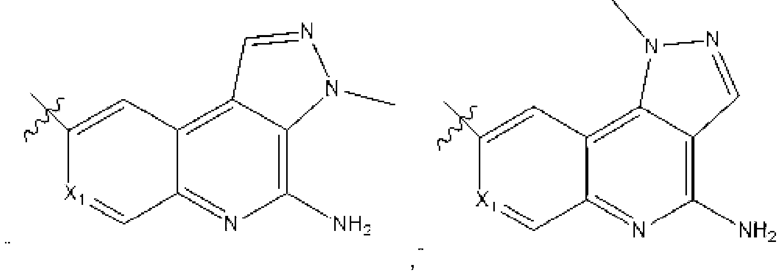 oder 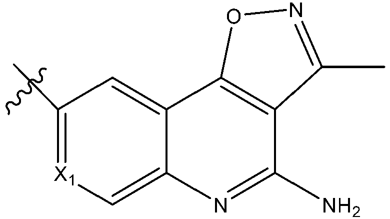 ist.

6. Die Verbindung nach Anspruch 5, das Tautomer davon, das Stereoisomer davon oder das pharmazeutisch verträgliche Salz einer der vorgenannten Verbindungen, worin X¹C ist, gegebenenfalls substituiert, vorzugsweise mit Halogen.

7. Die Verbindung nach einem der Ansprüche 1 oder 5, das Tautomer davon, das Stereoisomer davon oder das pharmazeutisch verträgliche Salz einer der vorgenannten Verbindungen, wobei R¹ gleich **IB, IC** oder **ID** ist.

8. Die Verbindung nach Anspruch 7, das Tautomer davon, das Stereoisomer davon oder das pharmazeutisch verträgliche Salz einer der vorgenannten Verbindungen, wobei R¹ mit R⁴ substituiert ist.

9. Die Verbindung nach Anspruch 8, das Tautomer davon, das Stereoisomer davon oder das pharmazeutisch verträgliche Salz einer der vorgenannten Verbindungen, wobei R⁴ Halogen ist.

10. Die Verbindung nach Anspruch 1, das Tautomer davon, das Stereoisomer davon oder das pharmazeutisch verträgliche Salz einer der vorgenannten Verbindungen, wobei
a) R³ in jedem Fall unabhängig ausgewählt ist aus C₁₋₆-Alkyl, Halogen und C₁₋₆-Halogenalkyl; oder
b) R³ in jedem Fall unabhängig ausgewählt ist aus -S(=O)₂C₁₋₆ Alkyl und -CN.

11. Die Verbindung nach einem der Ansprüche 1 oder 5, das Tautomer davon, das Stereoisomer davon oder das pharmazeutisch verträgliche Salz einer der vorgenannten Verbindungen, wobei R² unabhängig voneinander aus Methyl, Ethyl und Cyclopropyl ausgewählt ist.

12. Die Verbindung, das Tautomer davon, das Stereoisomer davon oder das pharmazeutisch verträgliche Salz nach Anspruch 1, wobei die Verbindung ausgewählt ist aus:
4-Amino-7-chlor-N,1-dimethyl-N-((5R)-2-(trifluormethyl)-6,7-dihydro-5H-cyclopenta[b]pyridin-5-yl)-1H-pyrazolo[4,3-c]chinolin-8-carboxamid,
4-Amino-N-((4S)-8-fluor-7-(trifluormethyl)-3,4-dihydro-1H-2-benzopyran-4-yl)-N,1-dimethyl-1H-pyrazolo[4,3-c]chinolin-8-carboxamid,
4-amino-N-((5S)-2-bromo-5,8-dihydro-6H-pyrano[3,4-b]pyridin-5-yl)-N,1-dimethyl-1H-pyrazolo[4,3-c]quinoline-8-carboxamide,
4-Amino-7-chlor-N,1-dimethyl-N-((5S)-2-(trifluormethyl)-5,8-dihydro-6H-pyrano[3,4-b]pyridin-5-yl)-1H-pyrazolo[4,3-c]chinolin-8-carboxamid,
4-Amino-7-fluor-N-methyl-N-((5R)-2-(trifluormethyl)-6,7-dihydro-5H-cyclopenta[b]pyridin-5-yl)-1,3-dihydrofuro[3,4-c]chinolin-8-carboxamid,
(3R)-4-Amino-7-fluor-N,3-dimethyl-N-((5R)-2-(trifluormethyl)-6,7-dihydro-5H-cyclopenta[b]pyridin-5-yl)-1,3-dihydrofuro[3,4-c]chinolin-8-carboxamid,
4-Amino-N,1-dimethyl-N-((5R)-2-(trifluormethyl)-6,7-dihydro-5H-cyclopenta[b]pyridin-5-yl)-1H-pyrazolo[4,3-c]chinolin-8-carboxamid,
4-Amino-N, 1-dimethyl-N-((3S)-6-(pentafluoro-lambda-6--sulfanyl)-2,3-dihydro-1-benzofuran-3-yl)-1H-pyrazolo[4,3-c]chinolin-8-carboxamid,
4-Amino-N,1-dimethyl-N-((5R)-2-(trifluormethyl)-6,7-dihydro-5H-cyclopenta[b]pyridin-5-yl)-1H-pyrazolo[4,3-c][1,7]naphthyridin-8-carboxamid,
4-Amino-7-fluor-N,1-dimethyl-N-((5R)-2-(trifluormethyl)-6,7-dihydro-5H-cyclopenta[b]pyridin-5-yl)-1H-pyrazolo[4,3-c]chinolin-8-carboxamid,
4-Amino-7-fluor-N,1-dimethyl-N-((3S)-6-(trifluormethyl)-2,3-dihydrofuro[2,3-b]pyridin-3-yl)-1H-pyrazolo[4,3-c]chinolin-8-carboxamid,
4-Amino-7-chlor-N,1-dimethyl-N-((5R)-2-(trifluormethyl)-6,7-dihydro-5H-cyclopenta[b]pyridin-5-yl)-1H-pyrazolo[4,3-c]chinolin-8-carboxamid,
4-Amino-N-((4S)-8-fluor-7-(trifluormethyl)-3,4-dihydro-1H-2-benzopyran-4-yl)-N,1-dimethyl-1H-pyrazolo[4,3-c]chinolin-8-carboxamid,
4-amino-N-((5S)-2-bromo-5,8-dihydro-6H-pyrano[3,4-b]pyridin-5-yl)-N,1-dimethyl-1H-pyrazolo[4,3-c]quinoline-8-carboxamid,
4-Amino-N,1-dimethyl-N-((3S)-6-(trifluormethyl)-2,3-dihydro-1-benzofuran-3-yl)-1H-pyrazolo[4,3-c]chinolin-8-carboxamid,
(3R)-4-Amino-N,3-dimethyl-N-((5S)-2-(trifluormethyl)-5,8-dihydro-6H-pyrano[3,4-b]pyridin-5-yl)-1,3-dihydrofuro[3,4-c]chinolin-8-carboxamid,
4-amino-N-methyl-N-((5S)-2-(trifluoromethyl)-5,8-dihydro-6H-pyrano[3,4-b]pyridin-5-yl)-1,3-dihydrofuro[3,4-c][1,7]naphthyridine-8-carboxamid,
4-Amino-7-fluor-N,1-dimethyl-N-((5S)-2-(trifluormethyl)-5,8-dihydro-6H-pyrano[3,4-b]pyridin-5-yl)-1H-pyrazolo[4,3-c]chinolin-8-carboxamid,
4-Amino-N-methyl-N-((3S)-6-(trifluormethyl)-2,3-dihydro-1-benzofuran-3-yl)-1,3-dihydrofuro[3,4-c][1,7]naphthyridin-8-carboxamid,
4-Amino-7-fluor-N,1-dimethyl-N-((3S)-6-(trifluormethyl)-2,3-dihydro-1-benzofuran-3-yl)-1H-pyrazolo[4,3-c]chinolin-8-carboxamid und
4-Amino-7-fluor-N,1-dimethyl-N-((3S)-6-(trifluormethyl)-2,3-dihydrofuro[3,2-c]pyridin-3-yl)-1H-pyrazolo[4,3-c]chinolin-8-carboxamid.

13. Pharmazeutische Zusammensetzung, umfassend eine Verbindung nach einem der Ansprüche 1 oder 12, das Tautomer davon, das Stereoisomer davon oder das pharmazeutisch verträgliche Salz einer der vorgenannten Verbindungen und einen pharmazeutisch annehmbaren Träger.

14. Die Verbindung nach einem der Ansprüche 1 oder 12, das Tautomer davon, das Stereoisomer davon oder das pharmazeutisch verträgliche Salz einer der vorgenannten Verbindungen zur Verwendung in der Behandlung von Krebs.

15. Die Verbindung, das Tautomer davon, das Stereoisomer davon oder das pharmazeutisch verträgliche Salz einer der vorgenannten Verbindungen zur Verwendung nach Anspruch 14, wobei der Krebs MTAP-null-Krebs ist, vorzugsweise wobei der Krebs ausgewählt ist aus Eierstock-, Lungen-, HNSCC-, Lymphoid-, Glioblastom-, Kolon-, Melanom-, Magen-, Gallengangs-, Pankreas- oder Blasenkrebs.

## Revendications

1. Composé de Formule I 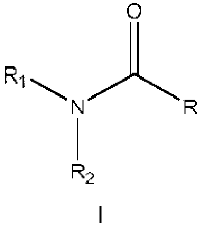
tautomère de celui-ci, stéréoisomère de celui-ci, ou sel pharmaceutiquement acceptable de l'un quelconque des précédents;
dans lequel R est cycle triple choisi indépendamment à partir de la formule IA : 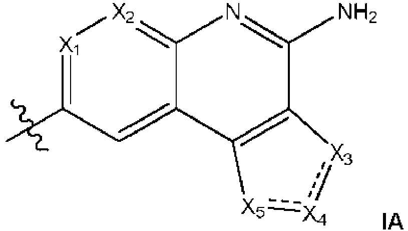
dans laquelle 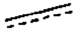 est une liaison simple ou double ;
X¹ et X² sont, à chaque instance, choisis indépendamment parmi N et C éventuellement substitués, dans lequel les substituants sont choisis indépendamment parmi des alkyles en C₁₋₃ ;
dans laquelle X¹ et X² ne peuvent pas être tous deux N en même temps ;
dans laquelle si X¹ est C, il peut être éventuellement substitué par un halogéno, un haloalkyle en C₁₋₃ ou -CN ;
X³, X⁴ et X⁵ sont, pour chaque instance, choisis indépendamment parmi C, O et N éventuellement substitués, dans laquelle les substituants sont choisis indépendamment parmi un alkyle en C₁₋₃ et un alkyle en C₁₋₃(OH), l'alkyle pouvant être éventuellement substitué par un halogéno ;
dans laquelle R¹ est un cycle double choisi indépendamment parmi les formules IB, IC et ID, éventuellement substitué par R⁴ ; 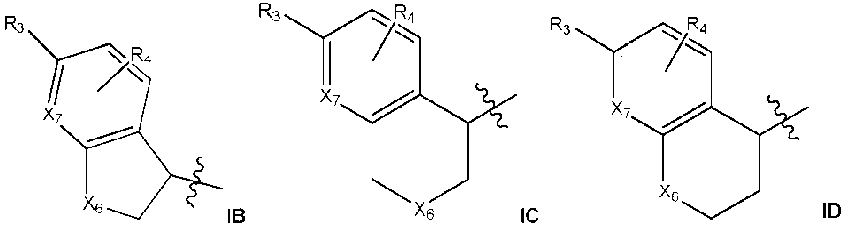
où X⁶ est, à chaque instance, choisi indépendamment parmi O et C ;
où X⁷ est, à chaque instance, choisi indépendamment parmi N et C ;
où R² est à chaque instance choisi indépendamment parmi un alkyle en C₁₋₆ éventuellement substitué ou un cycloalkyle en C₁₋₆ éventuellement substitué, les substituants étant choisis parmi -CN ou un cycloalkyle en C₁₋₆ ;
où R³ est, à chaque instance, choisi indépendamment parmi un alkyle en C₁₋₆, cycloalkyle en C₁₋₆, halogéno, haloalkyle en C₁₋₆, -S(=O)₂-alkyle en C₁₋₆, - S(O)(NH)alkyle en C₁₋₆, - S(O)(N-alkyle en C₁₋₃)alkyle en C₁₋₆, -CN, -O-alkyle en C₁₋₆, -O-haloalkyle en C₁₋₆, -N(=O)-O-alkyle en C₁₋₆, -C(O)alkyle en C₁₋₆, -C(O)haloalkyle en C₁₋₆, 3,6-dihydro-2H-pyranyle et pentafluorosulfanyle ;
où R⁴ est, à chaque instance, choisi indépendamment parmi un alkyle en C₁₋₆, un halogéno et un haloalkyle en C₁₋₆.

2. Composé selon la revendication 1, tautomère de celui-ci, stéréoisomère de celui-ci, ou sel pharmaceutiquement acceptable de l'un quelconque des précédents, dans lequel R est

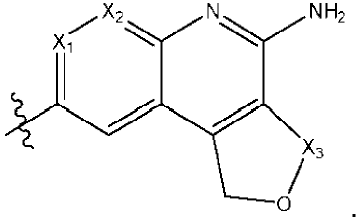

3. Composé selon la revendication 2, tautomère de celui-ci, stéréoisomère de celui-ci, ou sel pharmaceutiquement acceptable de l'un quelconque des précédents, dans lequel X¹ est C, éventuellement substitué par un halogéno ; ou dans lequel X¹ est N.

4. Composé selon la revendication 2, tautomère de celui-ci, stéréoisomère de celui-ci, ou sel pharmaceutiquement acceptable de l'un quelconque des précédents, dans lequel X³ est C éventuellement substitué.

5. Composé selon la revendication 1, tautomère de celui-ci, stéréoisomère de celui-ci, ou sel pharmaceutiquement acceptable de l'un quelconque des précédents, dans lequel R est 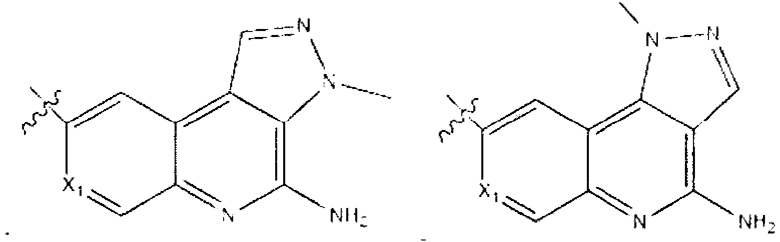 ou

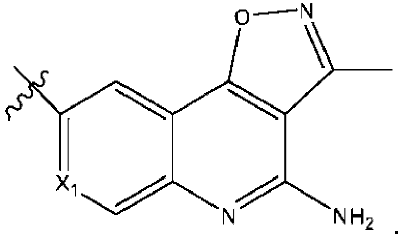

6. Composé selon la revendication 5, tautomère de celui-ci, stéréoisomère de celui-ci, ou sel pharmaceutiquement acceptable de l'un quelconque des précédents, dans lequel X¹ est C, éventuellement substitué, de préférence par un halogéno.

7. Composé selon l'une quelconque des revendications 1, ou 5, tautomère de celui-ci, stéréoisomère de celui-ci, ou sel pharmaceutiquement acceptable de l'un quelconque des précédents, dans lequel R¹ est IB, IC ou ID.

8. Composé selon la revendication 7, tautomère de celui-ci, stéréoisomère de celui-ci, ou sel pharmaceutiquement acceptable de l'un quelconque des précédents, dans lequel R¹ est substitué par R⁴.

9. Composé selon la revendication 8, tautomère de celui-ci, stéréoisomère de celui-ci, ou sel pharmaceutiquement acceptable de l'un quelconque des précédents, dans lequel R⁴ est un halogéno.

10. Composé selon la revendication 1, tautomère de celui-ci, stéréoisomère de celui-ci, ou sel pharmaceutiquement acceptable de l'un quelconque des précédents, dans lequel
a) R³ est, à chaque instance, choisi indépendamment parmi un alkyle en C₁₋₆, un halogéno et un haloalkyle en C₁₋₆ ; ou
b) R³ est, à chaque instance, choisi indépendamment parmi -S(=O)₂-alkyle en C₁₋₆ et -CN.

11. Composé selon l'une quelconque des revendications 1, ou 5, tautomère de celui-ci, stéréoisomère de celui-ci, ou sel pharmaceutiquement acceptable de l'un quelconque des précédents, dans lequel R² est choisi indépendamment parmi un méthyle, un éthyle et un cyclopropyle.

12. Composé, tautomère de celui-ci, stéréoisomère de celui-ci, ou sel pharmaceutiquement acceptable de l'un quelconque des précédents selon la revendication 1, dans lequel le composé est choisi parmi le:
4-amino-7-chloro-N,1-diméthyl-N-((5R)-2-(trifluorométhyl)-6,7-dihydro-5H-cyclopenta[b]pyridin-5-yl)-1H-pyrazolo[4,3-c]quinoline-8-carboxamide,
4-amino-N-((4S)-8-fluoro-7-(trifluorométhyl)-3,4-dihydro-1H-2-benzopyran-4-yl)-N,1-diméthyl-1H-pyrazolo[4,3-c]quinoline-8-carboxamide,
4-amino-N-((5S)-2-bromo-5,8-dihydro-6H-pyrano[3,4-b]pyridin-5-yl)-N,1-diméthyl-1H-pyrazolo[4,3-c]quinoline-8-carboxamide,
4-amino-7-chloro-N,1-diméthyl-N-((5S)-2-(trifluorométhyl)-5,8-dihydro-6H-pyrano[3,4-b]pyridin-5-yl)-1H-pyrazolo[4,3-c]quinoline-8-carboxamide,
4-amino-7-fluoro-N-méthyl-N-((5R)-2-(trifluorométhyl)-6,7-dihydro-5H-cyclopenta[b]pyridin-5-yl)-1,3-dihydrofuro[3,4-c]quinoline-8-carboxamide,
(3R)-4-amino-7-fluoro-N,3-diméthyl-N-((5R)-2-(trifluorométhyl)-6,7-dihydro-5H-cyclopenta[b]pyridin-5-yl)-1,3-dihydrofuro[3,4-c]quinoline-8-carboxamide,
4-amino-N,1-diméthyl-N-((5R)-2-(trifluorométhyl)-6,7-dihydro-5H-cyclopenta[b]pyridin-5-yl)-1H-pyrazolo[4,3-c]quinoline-8-carboxamide,
4-amino-N,1-diméthyl-N-((3S)-6-(pentafluoro-lambda∼6∼-sulfanyl)-2,3-dihydro-1-benzofuran-3-yl)-1H-pyrazolo[4,3-c]quinoline-8-carboxamide,
4-amino-N,1-diméthyl-N-((5R)-2-(trifluorométhyl)-6,7-dihydro-5H-cyclopenta[b]pyridin-5-yl)-1H-pyrazolo[4,3-c][1,7]naphtyridine-8-carboxamide,
4-amino-7-fluoro-N,1-diméthyl-N-((5R)-2-(trifluorométhyl)-6,7-dihydro-5H-cyclopenta[b]pyridin-5-yl)-1H-pyrazolo[4,3-c]quinoline-8-carboxamide,
4-amino-7-fluoro-N,1-diméthyl-N-((3S)-6-(trifluorométhyl)-2,3-dihydrofuro[2,3-b]pyridin-3-yl)-1H-pyrazolo[4,3-c]quinoline-8-carboxamide,
4-amino-7-chloro-N,1-diméthyl-N-((5R)-2-(trifluorométhyl)-6,7-dihydro-5H-cyclopenta[b]pyridin-5-yl)-1H-pyrazolo[4,3-c]quinoline-8-carboxamide,
4-amino-N-((4S)-8-fluoro-7-(trifluorométhyl)-3,4-dihydro-1H-2-benzopyran-4-yl)-N,1-diméthyl-1H-pyrazolo[4,3-c]quinoline-8-carboxamide,
4-amino-N-((5S)-2-bromo-5,8-dihydro-6H-pyrano[3,4-b]pyridin-5-yl)-N,1-diméthyl-1H-pyrazolo[4,3-c]quinoline-8-carboxamide,
4-amino-N,1-diméthyl-N-((3S)-6-(trifluorométhyl)-2,3-dihydro-1-benzofuran-3-yl)-1H-pyrazolo[4,3-c]quinoline-8-carboxamide,
(3R)-4-amino-N,3-diméthyl-N-((5S)-2-(trifluorométhyl)-5,8-dihydro-6H-pyrano[3,4-b]pyridin-5-yl)-1,3-dihydrofuro[3,4-c]quinoline-8-carboxamide,
4-amino-N-méthyl-N-((5S)-2-(trifluorométhyl)-5,8-dihydro-6H-pyrano[3,4-b]pyridin-5-yl)-1,3-dihydrofuro [3,4-c][1,7]naphtyridine-8-carboxamide,
4-amino-7-fluoro-N,1-diméthyl-N-((5S)-2-(trifluorométhyl)-5,8-dihydro-6H-pyrano[3,4-b]pyridin-5-yl)-1H-pyrazolo[4, 3-c]quinoline-8-carboxamide,
4-amino-N-méthyl-N-((3S)-6-(trifluorométhyl)-2,3-dihydro-1-benzofuran-3-yl)-1,3-dihydrofuro[3,4-c][1,7]naphtyridine-8-carboxamide,
4-amino-7-fluoro-N,1-diméthyl-N-((3S)-6-(trifluorométhyl)-2,3-dihydro-1-benzofuran-3-yl)-1H-pyrazolo[4,3-c]quinoline-8-carboxamide et le
4-amino-7-fluoro-N,1-diméthyl-N-((3S)-6-(trifluorométhyl)-2,3-dihydrofuro[3,2-c]pyridin-3-yl)-1H-pyrazolo[4,3-c]quinoline-8-carboxamide.

13. Composition pharmaceutique comprenant un composé selon l'une quelconque des revendications 1 ou 12, le tautomère de celui-ci, le stéréoisomère de celui-ci, ou le sel pharmaceutiquement acceptable de l'un quelconque des précédents et un véhicule pharmaceutiquement acceptable.

14. Composé selon l'une quelconque des revendications 1 ou 12, tautomère de celui-ci, stéréoisomère de celui-ci, ou sel pharmaceutiquement acceptable de l'un quelconque des précédents, destiné à être utilisé dans le traitement d'un cancer.

15. Composé, tautomère de celui-ci, stéréoisomère de celui-ci, ou sel pharmaceutiquement acceptable de l'un quelconque des précédents destiné à être utilisé selon la revendication 14,
le cancer étant un cancer avec expression nulle de MTAP,
le cancer étant de préférence choisi parmi un cancer de l'ovaire, du poumon, un carcinome épidermoïde de la tête et du cou (HNSCC), un cancer lymphoïde, un glioblastome, un cancer du côlon, un mélanome, un cancer de l'estomac, des voies biliaires, du pancréas ou de la vessie.
